# EUROPEAN PATENT APPLICATION

(11) **EP 4 403 558 A1**
(43) Date of publication of application: **24.07.2024**
(21) Application number: 22869364.4
(22) Date of filing: 16.09.2022
(51) Int. Cl.: C07D 491/18, C07D 491/22, C07D 519/00, C07F 9/6561, A61K 31/439, A61K 31/496, A61P 35/00, A61P 35/02

(54) **AZAINDAZOLE MACROCYCLIC COMPOUND AND USE THEREOF**

(30) Priority: 18.09.2021 CN 202111098709
(71) Applicant: Broadenbio Co., Ltd, Beijing 101111 (CN)
(72) Inventor: DUAN, Gongping, Beijing 101111 (CN); ZHANG, Xingmin, Beijing 101111 (CN); LI, Min, Beijing 101111 (CN)
(74) Representative: Winter, Brandl - Partnerschaft mbB
(86) International application number: PCT/CN2022/119179
(87) International publication number: WO 2023/040996

(57) **Abstract**

Provided in the present application are macrocyclic azaindazole compounds of Formula (I), and the use thereof. The compounds have the effect of inhibiting the activity of multiple protein kinases, comprising, for example, HPK1, FLT3 and KDR.

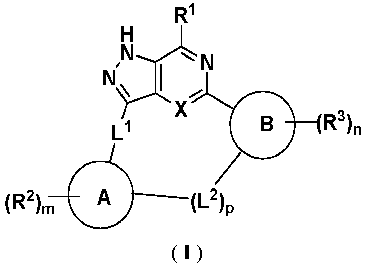

## Description

### TECHNICAL FIELD

The present invention relates to azaindazole macrocycle compounds, particularly azaindazole macrocycle derivatives as well as their pharmaceutical compositions with multiple protein kinase inhibitory activities. The invention also relates to methods of using said compounds and compositions for treating or ameliorating protein kinase mediated diseases, such as cancer.

### BACKGROUND OF THE INVENTION

Various cancers, as major threats to human health and life, their incidence and mortality rates are still showing a significant trend of increasing. The proliferation, metastasis and apoptosis of tumor cells are closely related to abnormalities in a series of cell signaling pathways. On the other hand, the immune escape of tumor cells, and the exhaustion or suppression of immune system are also important factors in the development and progression of cancers. Therefore, inhibiting the activity of key kinases in tumor cell signaling pathways, while activating immune cells and improving the innate immune function of patients, can effectively inhibit the proliferation and metastasis of tumor cells, showing broad prospects in cancer treatment.

Hematopoietic progenitor kinase 1 (HPK1), belonging to the mitogen-activated protein kinase kinase kinase kinase-4 (MAP4K) family, is a serine/threonine kinase originally cloned from hematopoietic progenitor cells (Hu, M. C. et al, Genes Dev. 1996; 10: 2251-2264; Keifer, F. et al., The EMBO Journal 1996; 15: 7013-7025). HPK1 is mainly distributed in lymphoid organs and lymphoid tissues, such as bone marrow, lymph nodes, thymus, etc., and is expressed predominantly in immune cells (T cells, B cells, dendritic cells, macrophages, etc.) (Hu, M.C. et al., Genes Dev. 1996; 10:2251-2264).

Studies have shown that HPK1 is a negative regulator protein of the T cell receptor (TCR) signaling pathway. TCR signaling causes the activation of HPK1. Activated HPK1 phosphorylates the Ser376 residue of SLP-76, promoting the binding of SLP-76 to 14-3-3 protein (Di Bartolo, V. et al., J. Exp. Med. 2007; 204: 681-691; Shui, J. et al., Nature Immuno. 2007; 8: 84-91). The SLP-76/14-3-3 interaction downregulates ERK signaling and calcium flux, and triggers the ubiquitination of SLP-76. The degradation of the SLP-76 complex blocks the TCR activation pathway consequently, thereby inhibiting T cell function (Lasserre, R. et al., J. Cell Biol. 2011; 195: 839-853).

In in vivo experiments, HPK1 knockout mice showed enhanced T cell function under antigen stimulation and produced more cytokines, such as IL-2 and IFN-γ (Shui, J. et al., Nature Immuno. 2007; 8: 84-91; Alzabin, S. et al., J. Immunol. 2009; 182: 6187-6194; Alzabin, S. et al., Cancer Immunol. Immunother. 2010; 59: 419-429). Further studies demonstrated that the kinase activity of HPK1 plays a key role in the negative regulation of immune cells. Compared with wild-type mice, mice with blockade of the kinase activity of HPK1 showed enhanced CD8⁺ T cell function, faster clearance of chronic lymphocytic meningitis virus, and better inhibition of tumor growth (Hernandez, S. et al., Cell Reports 2018; 25: 80-94). With Lewis lung cancer (LLC) model, mice transfected with HPK1^{-/-} T cells exhibited stronger antitumor immune responses than wild-type (Sawasdikosol, S. et al., Immunol. Res. 2012; 54: 262-265). Similar studies revealed that the immunosuppression of HPK1 on B cells (Sauer, K. et al., J. Biol. Chem. 2001; 276: 45207-45216; Tsuji, S. et al., J. Exp. Med. 2001; 194: 529-539; Wang, X. et al., J. Biol. Chem. 2012; 287: 34091-34100; Königsberger, S. et al., PLos One, 2010; 5: e12468), dendritic cells (Alzabin, S. et al., J. Immunol. 2009; 182: 6187-6194), NK cells and Treg cells are also derived from its kinase activity (Liu, J. et al., PLos One, 2019; 14: e0212670).

Clinical studies have found that, comparing with healthy controls, the HPK1 levels were significantly down-regulated in tissues from patients of systemic lupus erythematosus (Zhang, Q. et al., J. Autoimmun., 2011; 37: 180-189) and psoriatic arthritis (Stoeckman, A. K. et al., Genes Immun 2006; 7: 583-591; Baltiwalla, F. M. et al., Mol. Med. 2005; 11: 21-29), suggesting that HPK1 down-regulation contributes to the enhancement of autoimmune responses. On the other hand, upregulation of HPK1 levels has been observed in various cancers, such as acute myeloid leukemia (Chen-Deutsch, X. et al., Leuk. Res. 2012; 36: 884-888; Chen-Deutsch, X. et al., Cell Cycle 2012; 11: 1364-1373), bladder urothelial carcinoma (Wang. Y et al., Mol. Med. Rep. 2012; 5: 260-265), extramammary Paget's disease (Qian, Y et al, Am J. Dermatopathol. 2011; 33: 681-686) and colon cancer (Yang, H.S. et al., Mol. Cell Biol. 2006; 26: 1297-1306).

Therefore, HPK1 is a potential target for the treatment of tumors and viral diseases. The development of small molecule HPK1 inhibitors has important clinical prospect.

FMS-like tyrosine kinase 3 (FLT3) is a receptor tyrosine kinase expressed in hematopoietic progenitor cells and stem cells, and plays important roles in the survival, proliferation and differentiation of hematopoietic cells. Mutations and abnormal expression of FLT3 lead to blood diseases such as leukemia (Gilliland, D.G. et al., Blood, 2002; 100: 1532-1542; Stirewalt, D.L. et al., Nat. Rev. Cancer, 2003; 3: 650-665). About 30% of adult patients with Acute Myelocytic Leukemia (AML) have FLT3 gene mutations (Nakao, M.S. et al., Leukemia, 1996; 10: 1911-1918; Kottaridis P.D., Blood, 2001; 98: 1742-1759), which are the most common gene mutations among patients, and the most common factors associated with poor prognosis. This makes FLT3 an important target for the development of small molecule drugs for the treatment of such tumors.

Midostaurin, the first small molecule FLT3 inhibitor developed by Novartis, was approved by FDA in 2017 for the treatment of newly diagnosed FLT3-mutated AML patients in combination with chemotherapy. Since then, Gilteritinib, developed by Astellas, and Quizartinib, developed by Daiichi Sankyo, have been launched successively for the treatment of AML related to abnormal expression or mutation of FLT3 as single agent or in combination therapy. Although there are several drugs on the market, small molecule FLT3 inhibitors as a treatment option for AML still have many problems, such as short duration of clinical effects and secondary drug resistance. Clinical data show that FLT3 inhibitor can effectively eliminate peripheral blood blasts, but are less effective on bone marrow blasts (Bortheakur, G. et al., Haematologica, Jan. 2011; 96: 62-68). One possible reason for the above problem is the existence of alternative signaling pathways. Therefore, it is still necessary to develop a new generation of FLT3 inhibitors, especially small molecule compounds that inhibit multiple signaling pathways at the same time, for unmet clinical needs.

Vascular Endothelial Growth Factor Receptor (VEGFR) belongs to the class III receptor tyrosine kinase family. VEGFR2, also known as Kinase insert Domain Receptor (KDR), is widely distributed in vascular endothelial cells. It plays a very important role in inducing the development, proliferation and migration of new endothelial cells around tumors.

Currently, a number of VEGFR2 inhibitors have been approved for the treatment of cancers. Sunitinib, small molecule VEGFRs inhibitor developed by Pfizer, was approved in 2016 for the treatment of gastrointestinal stromal tumors and metastatic renal cell carcinoma. As a multi-target inhibitor, Sunitinib not only strongly inhibit the activity of VEGFRs, but also inhibit other kinases such as PDGFR and c-Kit. The multi-target inhibiton makes the drug not only potentially applicable to multiple indications, but also potentially can reduce drug resistance caused by activation of bypass signaling pathways. Since then, a number of multi-target small molecule inhibitors, such as Sorafenib, Cabozantininb, and Lenvatininb, have been approved and achieved great success.

Although some patent applications for small molecule HPK1 inhibitors have been published, such as WO2018049191, WO2018049200, WO2018102366, WO2018183964, WO2019090198, WO2019206049, WO2019238067 and WO2020092528 etc., no drug targeting HPK1 has been approved yet. In particular, small molecule drugs that simultaneously inhibit HPK1 and other kinases tartget are still rare. This type of small molecule compounds can eliminate tumor cells by directly inhibiting tumor cell proliferation and simultaneously activating innate immune function. Therefore, the development of novel and potent small molecule HPK1 inhibitors is urgently needed in clinical practice.

### SUMMARY OF THE INVENTION

This application is to provide an azaindazole compound of Formula (I), 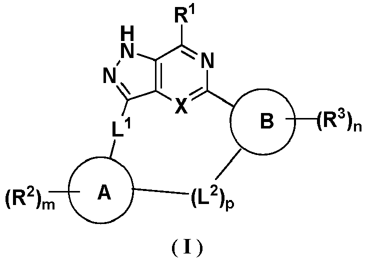
or an isomer, pharmaceutically acceptable salt, polymorph, isotope labeled compound, active metabolite or prodrug thereof,
wherein, the structure and atomic number of the azaindazole ring are as shown in Formula (Ia), 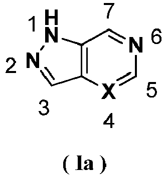
in the structure of Formula (I), X is selected from CR^{x} and N, wherein R^{x} is independently selected from H and halogen;
R¹ is selected from:
   1) hydrogen, halogen, cyano, -C(=O)OR^{a1}, -C(=O)NR^{a1}R^{b1}, -OR^{a1}, -NR^{a1}R^{b1}, -NR^{e1}C(=O)R^{a1}, -NR^{e1}C(=O)NR^{a1}R^{b1}, -SR^{a1}, -S(=O)R^{a1} and -S(=O)₂R^{a1}; or
   2) C₁₋₆ alkyl, C₂₋₄ alkenyl, C₂₋₄ alkynyl, C₃₋₈ cycloalkyl, and 3- to 8-membered aliphatic heterocyclyl, optionally substituted with 0, 1, 2, 3 or 4 of R¹¹;
wherein, R^{a1}, R^{b1} and R^{e1} are each independently selected from:
   1) hydrogen; or
   2) C₁₋₆ alkyl, C₃₋₆ monocyclic cycloalkyl, and 3- to 6-membered monocyclic aliphatic heterocyclyl, optionally substituted with 0, 1, 2, 3 or 4 of R¹¹;
   or, R^{a1} and R^{b1} attached to the same nitrogen atom, taken together with the nitrogen atom to which they are attached, form a 4- to 6-membered aliphatic heterocyclyl, optionally substituted with 0, 1, 2, 3 or 4 of R¹¹;
wherein, R¹¹ is selected from oxo, halogen, hydroxyl, amino, cyano, C₁₋₃ alkyl, C₁₋₃ alkoxy, C₁₋₃ alkylamino, C₃₋₆ cycloalkyl and C₃₋₆ cycloalkoxy;
L¹ is selected from:
   1) single bond; or
   2) 6- to 10-membered aryl, and 5- to 10-memebred heteroaryl; said aryl or heteroaryl group is connected to the azaindazole ring at the 3-position and to the ring A through a single bond, respectively; in addition to being connected to the azaindazole ring at the 3-position and to the ring A, said aryl or heteroaryl group is optionally substituted with 0, 1, 2, 3, 4, 5 or 6 of R4;
ring A is selected from monocyclic or bicyclic 3- to 12-membered carbocyclyl group and 3- to 12-membered heterocyclyl group, said carbocyclyl or heterocyclyl group is connected to L¹ and L² through two different atoms respectively; and in addition to being connected to L¹ and L², said carbocyclyl or heterocyclyl group is optionally substituted with m of R²;
ring B is selected from monocyclic or bicyclic 6- to 12-membered carbocyclyl group and 5- to 12-membered heterocyclyl group, said carbocyclyl or heterocyclyl group is connected to L² and to the azaindazole ring at the 5-position through two different atoms respectively; wherein,
   1) when ring B is selected from monocyclic carbocyclyl or monocyclic heterocyclyl group, the shortest distance between the two atoms on ring B, through which ring B is connected to L² and to the azaindazole ring at the 5-position, is 1, 2 or 3 chemical bonds;
   2) when ring B is selected from bicyclic carbocyclyl or bicyclic heterocyclyl group, the shortest distance between the two atoms on ring B, through which ring B is connected to L² and to the azaindazole ring at the 5-position, is 1, 2, 3, 4 or 5 chemical bonds;
and, in addition to being connected to L² and to the azaindazole ring at the 5-position, ring B is optionally substituted with n of R³;
R² is each independently selected from:
   1) oxo, halogen, cyano, -C(=O)R^{a2}, -C(=O)OR^{a2}, -C(=O)NR^{a2}R^{b2}, -C(=NR^{d2})NR^{a2}R^{b2}, -OR^{a2}, -OC(=O)R^{a2}, -OC(=O)OR^{c2}, -OC(=O)NR^{a2}R^{b2}, -SR^{a2}, -S(=O)R^{c2}, -S(=O)2R^{c2}, sulfonic acid group, -S(=O)NR^{a2}R^{b2}, -S(=O)2NR^{a2}R^{b2}, -S(=O)(=NR^{d2})R^{c2}, -NR^{a2}R^{b2}, -NR^{a2}C(=O)R^{b2}, -NR^{a2}C(=O)OR^{c2}, -NR^{e2}C(=O)NR^{a2}R^{b2}, -NR^{e2}C(=NR^{d2})NR^{a2}R^{b2}, -NR^{a2}S(=O)₂R^{c}₂, -NR^{e2}S(=O)₂NR^{a2}R^{b2}, nitro, -PR^{c2}R^{f2}, -P(=O)R^{c2}R^{f2} and phosphonic acid group; or
   2) C₁₋₆ alkyl, C₂₋₆ alkenyl, C₂₋₆ alkynyl, phenyl, 5- to 10-membered heteroaryl, C₃₋₁₂ cycloalkyl and 3- to 12-membered aliphatic heterocyclyl, optionally substituted with 0, 1, 2, 3, 4, 5 or 6 of R²²; or
   3) divalent bridged C₁₋₅ alkylene, 3- to 6-membered oxaalkylene, and 2- to 6-membered azaalkylene connected to ring A through two different ring-forming atoms thereof; said alkylene, oxaalkylene and azaalkylene are optionally substituted with 0, 1, 2, 3, 4, 5 or 6 of R²²;
or, two R² groups attached to one or two ring-forming atom(s) of the ring A, taken together with the one or two ring-forming atom(s) to which they are attached, form a C₅₋₁₂ aliphatic carbocyclyl, or 5- to 12-membered aliphatic heterocyclyl, optionally substituted with 0, 1, 2, 3, 4 or 5 of R²²;
wherein, R^{a2}, R^{b2} and R^{e2} are each independently selected from:
   1) hydrogen; or
   2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4, 5 or 6 of R²²;

   or, R^{a2} and R^{b2} attached to the same nitrogen atom, taken together with the nitrogen atom to which they are attached, form a 3- to 12-membered aliphatic heterocyclyl, optionally substituted with 0, 1, 2, 3, 4 or 5 of R²²;
   R^{c2} and R^{f2} are each independently selected from C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4 or 5 of R²²; or, R^{c2} and R^{f2} attached to the same phosphine atom, taken together with the phosphine atom to which they are attached, form a 3- to 12-membered aliphatic heterocyclyl, optionally substituted with 0, 1, 2, 3, 4 or 5 of R²²;
   R^{d2} is selected from:
      1) hydrogen, cyano, nitro and -S(=O)₂R^{G}; or
      2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4 or 5 of R²²,
   R²² is selected from:
      1) oxo, halogen, cyano, -C(=O)R^{a22}, -C(=O)OR^{a22}, -C(=O)NR^{a22}R^{b22}, -C(=NR^{d22})NR^{a22}R^{b22}, -OR^{a22}, -OC(=O)R^{a22}, -OC(=O)OR^{c22}, -OC(=O)NR^{a22}R^{b22}, -SR^{a22}, -S(=O)R^{c22}, -S(=O)₂R^{c22}, sulfonic acid group, -S(=O)NR^{a22}R^{b22}, -S(=O)₂NR^{a22}R^{b22}, -S(=O)(=NR^{d22})R^{c22}, -NR^{a22}R^{b22}, -NR^{a22}C(=O)R^{b22}, -NR^{a22}C(=O)OR^{c22}, -NR^{e22}C(=O)NR^{a22}R^{b22}, -NR^{e22}C(=NR^{d22})NR^{a22}R^{b22}, -NR^{a22}S(=O)2R^{c22}, -NR^{e22}S(=O)₂NR^{a22}R^{b22}, nitro, -PR^{c22}R^{f22}, -P(=O)R^{c22}R^{f22}, phosphonic acid group, and =N-R^{d22}; or
      2) C₁₋₆ alkyl, C₁₋₆ alkylene, C₂₋₆ alkenyl, C₂₋₆ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R²³;
   R^{a22}, R^{b22} and R^{e22} are each independently selected from:
      1) hydrogen; or
      2) C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R²³;
   or, R^{a22} and R^{b22} attached to the same nitrogen atom, taken together with the nitrogen atom to which they are attached, form a 3- to 10-membered aliphatic heterocyclyl, optionally substituted with 0, 1, 2, 3, 4 or 5 of R²³;
   R^{c22} and R^{f22} are each independently selected from C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R²³; or, R^{c22} and R^{f22} attached to the same phosphine atom, taken together with the phosphine atom to which they are attached, form a 3- to 10-membered aliphatic heterocyclyl, optionally substituted with 0, 1, 2, 3, 4 or 5 of R²³;
   R^{d22} is selected from:
      1) hydrogen, cyano, nitro and -S(=O)₂R^{G}; or
      2) C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R²³;
   R²³ is selected from:
      1) oxo, halogen, cyano, -C(=O)R^{a23}, -C(=O)OR^{a23}, -C(=O)NR^{a23}R^{b23}, -C(=NR^{d23})NR^{a23}R^{b23}, -OR^{a23}, -OC(=O)R^{a23}, -OC(=O)OR^{c23}, -OC(=O)NR^{a23}R^{b23}, -SR^{a23}, -S(=O)R^{c23}, -S(=O)₂R^{c23}, sulfonic acid group, -S(=O)NR^{a23}R^{b23}, -S(=O)₂NR^{a23}R^{b23}, -S(=O)(=NR^{d23})R^{c23}, -NR^{a23}R^{b23}, -NR^{a23}C(=O)R^{b23}, -NR^{a23}C(=O)OR^{c23}, -NR^{e23}C(=O)NR^{a23}R^{b23}, -NR^{e23}C(=NR^{d23})NR^{a23}R^{b23}, -NR^{a23}S(=O)₂R^{c23}, -NR^{e23}S(=O)₂NR^{a23}R^{b23}, nitro, -PR^{c23}R^{f23}, -P(=O)R^{c23}R^{f23}, phosphonic acid group, and =N-R^{d23}; or
      2) C₁₋₄ alkyl, C₁₋₄ alkylene, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
   R^{a23}, R^{b23} and R^{e23} are each independently selected from:
      1) hydrogen; or
      2) C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
   or, R^{a23} and R^{b23} attached to the same nitrogen atom, taken together with the nitrogen atom to which they are attached, form a 3- to 8-membered aliphatic heterocyclyl, optionally substituted with 0, 1, 2, 3, or 4 of R^{G};
   R^{c23} and R^{f23} are each independently selected from C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3, or 4 of R^{G};
   or, R^{c23} and R^{f23} attached to the same phosphine atom, taken together with the phosphine atom to which they are attached, form a 3- to 8-membered aliphatic heterocyclyl, optionally substituted with 0, 1, 2, 3 or 4 of R^{G};
   R^{d23} is selected from:
      1) hydrogen, cyano, nitro and -S(=O)₂R^{G}; or
      2) C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
   R³ is independently selected from:
      1) oxo, halogen, cyano, -C(=O)R^{a3}, -C(=O)OR^{a3}, -C(=O)NR^{a3}R^{b3}, -C(=NR^{d3})NR^{a3}R^{b3}, -OR^{a3}, -OC(=O)R^{a3}, -OC(=O)OR^{c3}, -OC(=O)NR^{a3}k^{b3}, -SR^{a3}, -S(=O)R^{c3}, -S(=O)₂R^{c3}, sulfonic acid group, -S(=O)NR^{a3}R^{b3}, -S(=O)₂NR^{a3}R^{b3}, -S(=O)(=NR^{d3})R^{c3}, -NR^{a3}R^{b3}, -NR^{a3}C(=O)R^{b3}, -NR^{a3}C(=O)OR^{c3}, -NR^{e3}C(=O)NR^{a3}R^{b3}, -NR^{e3}C(-NR^{d3})NR^{a3}R^{b3}, -NR^{a3}S(=O)₂R^{c3}, -NR^{e3}S(=O)₂NR^{a3}R^{b3}, nitro, -PR^{c3}R^{f3}, -P(=O)R^{c3}R^{f3} and phosphonic acid group; or
      2) C₁₋₆ alkyl, C₂₋₆ alkenyl, C₂₋₆ alkynyl, 6- to 10-membered aryl, 5- to 10-membered heteroaryl, C₃₋₁₂ cycloalkyl and 3- to 12-membered aliphatic heterocyclyl, optionally substituted with 0, 1, 2, 3, 4, 5 or 6 of R³²; or
      3) divalent bridged C₁₋₆ alkylene connected to ring B through two different ring-forming atoms thereof;
   or, two R³ groups attached to one or two ring-forming atom(s) of the ring B, taken together with the one or two ring-forming atom(s) to which they are attached, form a C₅₋₁₂ aliphatic carbocyclyl, or 5- to 12-membered aliphatic heterocyclyl, optionally substituted with 0, 1, 2, 3, 4 or 5 of R³²;
      wherein, R^{a3}, R^{b3} and R^{e3} are each independently selected from:
         1) hydrogen; or
         2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4, 5 or 6 of R³²;
      or, R^{a3} and R^{b3} attached to the same nitrogen atom, taken together with the nitrogen atom to which they are attached, form a 3- to 12-membered aliphatic heterocyclyl, optionally substituted with 0, 1, 2, 3, 4 or 5 of R³²;
      R^{c3} and R^{f3} are each independently selected from C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4 or 5 of R³²;
      or, R^{c3} and R^{f3} attached to the same phosphine atom, taken together with the phosphine atom to which they are attached, form a 3- to 12-membered aliphatic heterocyclyl, optionally substituted with 0, 1, 2, 3, 4 or 5 of R³²;
      R^{d3} is selected from:
         1) hydrogen, cyano, nitro and -S(=O)₂R^{G}; or
         2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4 or 5 of R³²;
      R³² is selected from:
         1) oxo, halogen, cyano, -C(=O)R^{a32}, -C(=O)OR^{a32}, -C(=O)NR^{a32}R^{b32}, -C(=NR^{d32})NR^{a32}R^{b32}, -OR^{a32}, -OC(=O)R^{a32}, -OC(=O)OR^{c32}, -OC(=O)NR^{a32}R^{b32}, -SR^{a32}, -S(=O)R^{c32}, -S(=O)₂R^{c32}, sulfonic acid group, -S(=O)NR^{a32}R^{b32}, -S(=O)₂NR^{a32}R^{b32}, -S(=O)(=NR^{d32})R^{c32}, -NR^{a32}R^{b32}, -NR^{a32}C(=O)R^{b32}, -NR^{a32}C(=O)OR^{c32}, -NR^{e32}C(=O)NR^{a32}R^{b32}, -NR^{e32}C(=NR^{d32})NR^{a32}R^{b32}, -NR^{a32}S(=O)₂R^{c32}, -NR^{e32}S(-O)₂NR^{a32}Rb³², nitro, -PR^{c32}R^{f32}, -P(=O)R^{c32}R^{f32}, phosphonic acid group, and =N-R^{d32}; or
         2) C₁₋₆ alkyl, C₁₋₆ alkylene, C₂₋₆ alkenyl, C₂₋₆ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R³³;
      R^{a32}, R^{b32} and R^{e32} are each independently selected from:
         1) hydrogen; or
         2) C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R³³;
      or, R^{a32} and R^{b32} attached to the same nitrogen atom, taken together with the nitrogen atom to which they are attached, form a 3- to 10-membered aliphatic heterocyclyl, optionally substituted with 0, 1, 2, 3, 4 or 5 of R³³;
      R^{c32} and R^{f32} are each independently selected from C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R³³;
      or, R^{c32} and R^{f32} attached to the same phosphine atom, taken together with the phosphine atom to which they are attached, form a 3- to 10-membered aliphatic heterocyclyl, optionally substituted with 0, 1, 2, 3, 4 or 5 of R³³;
      R^{d32} is selected from:
         1) hydrogen, cyano, nitro and -S(=O)₂R^{G}; or
         2) C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R³³;
      R³³ is selected from:
         1) oxo, halogen, cyano, -C(=O)R^{a33}, -C(=O)OR^{a33}, -C(=O)NR^{a33}R^{b33}, -C(=NR^{d33})NR^{a33}R^{b33}, -OR^{a33}, -OC(=O)R^{a33}, -OC(=O)OR^{c33}, -OC(=O)NR^{a33}R^{b33}, -SR^{a33}, -S(=O)R^{c33}, -S(=O)₂R^{c33}, sulfonic acid group, -S(=O)NR^{a33}R^{b33}, -S(=O)₂NR^{a33}R^{b33}, -S(=O)(=NR^{d33})R^{c33}, -NR^{a33}R^{b33}, -NR^{a33}C(=O)R^{b33}, -NR^{a33}C(=O)OR^{c33}, -NR^{e33}C(=O)NR^{a33}R^{b33}, -NR^{e33}C(=NR^{d33})NR^{a33}R^{b33}, -NR^{a33}S(=O)₂R^{c33}, -NR^{e33}S(=O)₂NR^{a33}R^{b33}, nitro, -PR^{c33}R^{f33}, -P(=O)R^{c33}R^{f33}, phosphonic acid group, and =N-R^{d33}; or
         2) C₁₋₄ alkyl, C₁₋₄ alkylene, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
      R^{a33}, R^{b33} and R^{e33} are each independently selected from:
         1) hydrogen; or
         2) C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
      or, R^{a33} and R^{b33} attached to the same nitrogen atom, taken together with the nitrogen atom to which they are attached, form a 3- to 8-membered aliphatic heterocyclyl, optionally substituted with 0, 1, 2, 3, or 4 of R^{G};
      R^{c33} and R^{f33} are each independently selected from C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3, or 4 of R^{G};
      or, R^{c33} and R^{f33} attached to the same phosphine atom, taken together with the phosphine atom to which they are attached, form a 3- to 8-membered aliphatic heterocyclyl, optionally substituted with 0, 1, 2, 3 or 4 of R^{G};
      R^{d33} is selected from:
         1) hydrogen, cyano, nitro and -S(=O)₂R^{G}; or
         2) C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
   R⁴ is independently selected from:
      1) oxo, halogen, cyano, -C(=O)R^{a4}, -C(=O)OR^{a4}, -C(=O)NR^{a4}R^{b4}, -C(=NR^{d4})NR^{a4}R^{b4}, -OR^{a4}, -OC(=O)R^{a4}, -OC(=O)OR^{c4}, -OC(=O)NR^{a4}R^{b4}, -SR^{a4}, -S(=O)R^{c4}, -S(=O)₂R^{c4}, -S(=O)NR^{a4}R^{b4}, -S(=O)₂NR^{a4}R^{b4}, -S(=O)(=NR^{d4})R^{c4}, -NR^{a4}R^{b4}, -NR^{a4}C(=O)R^{b4}, -NR^{a4}C(=O)OR^{c4}, -NR^{e4}C(=O)NR^{a4}R^{b4}, -NR^{e4}C(=NR^{d4})NR^{a4}R^{b4}, -NR^{a4}S(=O)₂R^{c4} and -NR^{e4} S(=O)₂NR^{a4}R^{b4}; or
      2) C₁₋₆ alkyl, C₂₋₆ alkenyl, C₂₋₆ alkynyl, phenyl, 5- to 10-membered heteroaryl, C₃₋₁₂ cycloalkyl and 3- to 12-membered aliphatic heterocyclyl, optionally substituted with 0, 1, 2, 3, 4, 5 or 6 of R⁴²;
   or, two R⁴ groups attached to two ring-forming atoms of the ring L¹, taken together with the two ring-forming atoms to which they are attached, form a C₅₋₁₂ aliphatic carbocyclyl, or 5-to 12-membered aliphatic heterocyclyl, optionally substituted with 0, 1, 2, 3, 4, 5 or 6 of R⁴²;
      R^{a4}, R^{b4} and R^{e4} are each independently selected from:
         1) hydrogen; or
         2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4, 5 or 6 of R⁴²;
      or, R^{a4} and R^{b4} attached to the same nitrogen atom, taken together with the nitrogen atom to which they are attached, form a 3- to 12-membered aliphatic heterocyclyl, optionally substituted with 0, 1, 2, 3, 4 or 5 of R⁴²;
      R^{c4} and R^{f4} are each independently selected from C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4 or 5 of R⁴²;
      R^{d4} is selected from:
         1) hydrogen, cyano, nitro and -S(=O)₂R^{G}; or
         2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4 or 5 of R⁴²;
      R⁴² is selected from:
         1) oxo, halogen, cyano, -C(=O)R^{a42}, -C(=O)OR^{a42}, -C(=O)NR^{a42}R^{b42}, -C(=NR^{d42})NR^{a42}R^{b42}, -OR^{a42}, -OC(=O)R^{a42}, -OC(=O)OR^{c42}, -OC(=O)NR^{a42}R^{b42}, -SR^{a42}, -S(=O)R^{c42}, S(=O)₂R^{c42}, -S(=O)NR^{a42}R^{b42}, -S(=O)₂NR^{a42}R^{b42}, -S(=O)(=NR^{d42})R^{c42}, -NR^{a42}R^{b42}, -NR^{a42}C(=O)R^{b42}, -NR^{a42}C(=O)OR^{c42}, -NR^{e42}C(=O)NR^{a42}R^{b42}, -NR^{e42}C(=NR^{d42})NR^{a42}R^{b42}, -NR^{a42}S(=O)₂R^{c42}, -NR^{e42}S(=O)₂NR^{a42}R^{b42} and =N-R^{d42}; or
         2) C₁₋₆ alkyl, C₁₋₆ alkylene, C₂₋₆ alkenyl, C₂₋₆ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R⁴³;
      R^{a42}, R^{b42} and R^{e42} are each independently selected from:
         1) hydrogen; or
         2) C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R⁴³,
      or, R^{a42} and R^{b42} attached to the same nitrogen atom, taken together with the nitrogen atom to which they are attached, form a 3- to 10-membered aliphatic heterocyclyl, optionally substituted with 0, 1, 2, 3, 4 or 5 of R⁴³,
      R^{c42} and R^{f42} are each independently selected from C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R⁴³;
      R^{d42} is selected from:
         1) hydrogen, cyano, nitro and -S(=O)₂R^{G}; or
         2) C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R⁴³,
      R⁴³ is selected from:
         1) oxo, halogen, cyano, -C(=O)R^{a43}, -C(=O)OR^{a43}, -C(=O)NR^{a43}R^{b43}, -C(=NR^{d43})NR^{a43}R^{b43}, -OR^{a43}, -OC(=O)R^{a43}, -OC(=O)OR^{c43}, -OC(=O)NR^{a43}R^{b43}, -SR^{a43}, -S(=O)R^{c43}, -S(=O)₂R^{c43}, -S(=O)NR^{a43}R^{b43}, -S(=O)₂NR^{a43}R^{b43}, -S(=O)(=NR^{d43})R^{c43}, -NR^{a43}R^{b43}, -NR^{a43}C(=O)R^{b43}, -NR^{a43}C(=O)OR^{c43}, -NR^{e43}C(=O)NR^{a43}R^{b43}, -NR^{e43}C(=NR^{d43})NR^{a43}R^{b43}, -NR^{a43}S(=O)₂R^{c43}, -NR^{e43}S(=O)₂NR^{a43}R^{b43} and =N-R^{d43}; or
         2) C₁₋₄ alkyl, C₁₋₄ alkylene, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
      R^{a43}, R^{b43} and R^{e43} are each independently selected from:
         1) hydrogen; or
         2) C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
      or, R^{a43} and R^{b43} attached to the same nitrogen atom, taken together with the nitrogen atom to which they are attached, form a 3- to 8-membered aliphatic heterocyclyl, optionally substituted with 0, 1, 2, 3, or 4 of R^{G};
      R^{c43} and R^{f43} are each independently selected from C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3, or 4 of R^{G};
      R^{d43} is selected from:
         1) hydrogen, cyano, nitro and -S(=O)₂R^{G}; or
         2) C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
   L² is each independently selected from -CH₂-, -NH-, -O-, and -S-; wherein, each L² is independently substituted with 0, 1, or 2 of R⁵; two adjacent L² may be connected through single bond, double bond, or triple bond, provided that the chemical valence allows; p of L² connected sequentially form a linker connecting ring A and ring B; wherein the two L² at both ends are attached to ring A and ring B through single bond, respectively;
   R⁵ is independently selected from:
      1) oxo, halogen, cyano, -C(=O)R^{a5}, -C(=O)OR^{a5}, -C(=O)NR^{a5}R^{b5}, -C(=NR^{d5})NR^{a5}R^{b5}, -OR^{a5}, -OC(=O)R^{a5}, -OC(=O)OR^{c5}, -OC(=O)NR^{a5}R^{b5}, -OP(=O)(R^{G1})₂, -SR^{a5}, -S(=O)R^{c5}, -S(=O)₂R^{c5}, sulfonic acid group, -S(=O)NR^{a5}R^{b5}, -S(=O)₂NR^{a5}R^{b5}, -S(=O)(=NR^{d5})R^{c5}, -NR^{a5}R^{b5}, -NR^{a5}C(=O)R^{b5}, -NR^{a5}C(=O)OR^{c5}, -NR^{e5}C(=O)NR^{a5}R^{b5}, -NR^{e5}C(=NR^{d5})NR^{a5}R^{b5}, -NR^{a5}S(=O)₂R^{c5}, -NR^{e5}S(=O)₂NR^{a5}R^{b5}, -NR^{G2}P(=O)(R^{G1})₂, -P(=O)R^{c5}R^{f5}, -P(=O)(R^{G1})₂ and =N-R^{d5}; or
      2) C₁₋₆ alkyl, C₂₋₆ alkenyl, C₂₋₆ alkynyl, phenyl, 5- to 6-membered heteroaryl, C₃₋₆ cycloalkyl and 3- to 6-membered aliphatic heterocyclyl, optionally substituted with 0, 1, 2, 3 or 4 of R⁵²; or
      3) two R⁵ groups attached to the same L² or attached to two adjacent L², taken together with the one or two L² atom(s) to which they are attached, form a C₃₋₆ aliphatic carbocyclyl, or 3- to 6-membered aliphatic heterocyclyl, optionally substituted with 0, 1, 2, 3, or 4 of R⁵², or
      4) two R⁵ groups attached to two nonadjacent L², taken together with the two L² atoms to which they are attached and all other L² atom(s) between said two L² atoms, form a C₃₋₆ aliphatic carbocyclyl, or 3- to 6-membered aliphatic heterocyclyl, optionally substituted with 0, 1, 2, 3, or 4 of R⁵²;

      R^{a5}, R^{b5} and R^{e5} are independently selected from:
         1) hydrogen; or
         2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3 or 4 of R⁵²;
      or, R^{a5} and R^{b5} attached to the same nitrogen atom, taken together with the nitrogen atom to which they are attached, form a 3- to 6-membered aliphatic heterocyclyl, optionally substituted with 0, 1, 2, 3 or 4 of R⁵²;
      R^{c5} and R^{f5} are each independently selected from C₁₋₄ alkyl, C₂₋₄ alkenyl, C₂₋₄ alkynyl, phenyl, 5- to 6-membered heteroaryl, C₃₋₆ cycloalkyl and 3- to 6-membered aliphatic heterocyclyl, optionally substituted with 0, 1, 2, 3 or 4 of R⁵²;
      or, R^{c5} and R^{f5} attached to the same phosphine atom, taken together with the phosphine atom to which they are attached, form a 3- to 6-membered aliphatic heterocyclyl, optionally substituted with 0, 1, 2, 3 or 4 of R⁵²;
      R^{d5} is selected from:
         1) hydrogen, hydroxyl, C₁₋₄ alkoxy, cyano, nitro and -S(=O)₂R^{G}; or
         2) C₁₋₄ alkyl, C₂₋₄ alkenyl, C₂₋₄ alkynyl, phenyl, 5- to 6-membered heteroaryl, C₃₋₆ cycloalkyl and 3- to 6-membered aliphatic heterocyclyl, optionally substituted with 0, 1, 2, 3 or 4 of R⁵²;
      R⁵² is selected from:
         1) oxo, halogen and cyano; or
         2) -C(=O)R^{G1}, -C(=O)R^{G2}, -C(=NR^{G3})N(R^{G2})₂, -OC(=O)R^{G2}, -OP(=O)(R^{G1})₂, -S(=O)R^{G2}, -S(=O)₂R^{G2}, sulfonic acid group, -S(=O)N(R^{G2})₂, -S(=O)₂N(R^{G2})₂, -S(=O)(=NR^{G3})R^{G2}, -N(R^{G2})C(=O)R^{G1}, -N(R^{G2})C(=O)R^{G2}, -N(R^{G2})C(=NR^{G3})N(R^{G2})₂, -N(R^{G2})S(=O)₂R^{G2}, -N(R^{G2})S(=O)₂N(R^{G2})₂, -N(R^{G2})P(=O)(R^{G1})_{2,} -P(=O)(R^{G2})₂, -P(=O)(R^{G1})₂, =N-R^{G3} and R^{G1}; or
         3) R^{G2};
      R^{G} is selected from:
         1) halogen, oxo, cyano, carboxyl, hydroxyl, C₁₋₄ alkoxy, amino, C₁₋₄ alkylamino, C₁₋₄ alkyl sulfinyl, C₁₋₄ alkyl sulfonyl, and C₁₋₄ alkylaminosulfonyl; or
         2) C₁₋₄ alkyl, C₁₋₄ alkylene, C₂₋₄ alkenyl, C₂₋₄ alkynyl, C₃₋₆ cycloalkyl and 3- to 8-membered aliphatic heterocyclyl, optionally substituted with 0, 1, 2, 3 or 4 substituents independently selected from: oxo, halogen, hydroxyl, hydroxymethyl, carboxyl, cyano, C₁₋₃ alkoxy, amino, and C₁₋₃ alkylamino;
      R^{G1} is selected from -OR^{G2} and -N(R^{G2})₂;
      R^{G2} is selected from:
         1) hydrogen; or
         2) C₁₋₄ alkyl, C₁₋₄ alkylene, C₃₋₆ cycloalkyl, 3- to 6-membered aliphatic heterocyclyl and phenyl, optionally substituted with 0, 1, 2 or 3 substituents independently selected from: oxo, halogen, hydroxyl, hydroxymethyl, carboxyl, cyano, C₁₋₃ alkoxy, amino, and C₁₋₃ alkylamino; or
         3) two R^{G2} attached to the same nitrogen atom, taken together with the nitrogen atom to which they are attached, form a 3- to 6-membered aliphatic heterocyclyl, optionally substituted with 0, 1, 2 or 3 substituents independently selected from: oxo, halogen, hydroxyl, hydroxymethyl, carboxyl, cyano, C₁₋₃ alkoxy, amino, and C₁₋₃ alkylamino;
      R^{G3} is selected from: hydrogen, cyano, nitro, -OR^{G2}, -S(=O)₂R^{G2} and R^{G2};
   p is selected from 1, 2, 3, 4, 5, 6, 7, 8, 9 and 10;
   m is selected from 0, 1, 2, 3, 4, 5 and 6;
   n is selected from 0, 1, 2, 3, 4, 5 and 6.

In one embodiment, the present invention also provides a pharmaceutical composition comprising the compound of Formula (I) herein, or a pharmaceutically acceptable salt, hydrate, solvate, active metabolite, polymorph, isotope labeled compound, isomer or prodrug thereof and a pharmaceutically acceptable carrier.

In one embodiment, the present invention further provides use of the compound of Formula (I) herein, or a pharmaceutically acceptable salt, hydrate, solvate, active metabolite, polymorph, isotope labeled compound, isomer or prodrug thereof, or a pharmaceutical composition thereof, for the prevention or treatment of diseases mediated by protein kinases.

In one embodiment, the present invention further provides use of the compound of Formula (I) herein, or a pharmaceutically acceptable salt, hydrate, solvate, active metabolite, polymorph, isotope labeled compound, isomer or prodrug thereof, or a pharmaceutical composition thereof, in the preparation of a medicament for the prevention or treatment of diseases mediated by protein kinases, said diseases comprise one or more kinds of cancer.

In one embodiment, the present invention further provides use of the compound of Formula (I) herein, or a pharmaceutically acceptable salt, hydrate, solvate, active metabolite, polymorph, isotope labeled compound, isomer or prodrug thereof, or a pharmaceutical composition thereof, for the treatment or amelioration of diseases, said diseases comprise one or more kinds of cancer.

In one embodiment, the present invention further provides a method for inhibiting the activity of HPK1, comprising administering the compound of Formula (I) herein, or a pharmaceutically acceptable salt, hydrate, solvate, active metabolite, polymorph, isotope labeled compound, isomer or prodrug thereof, or a pharmaceutical composition thereof to a patient.

In one embodiment, the present invention further provides a method for simultaneously inhibiting the activities of multiple protein kinases, comprising administering the compound of Formula (I) herein, or a pharmaceutically acceptable salt, hydrate, solvate, active metabolite, polymorph, isotope labeled compound, isomer or prodrug thereof, or a pharmaceutical composition thereof to a patient; said protein kinases comprise HPK1, FLT3 and KDR.

In one embodiment, the present invention further provides a method for treating a disease or disorder in a patient, comprising administering therapeutically effective amount of the compound of Formula (I) herein, or a pharmaceutically acceptable salt, hydrate, solvate, active metabolite, polymorph, isotope labeled compound, isomer or prodrug thereof, or a pharmaceutical composition thereof to the patient.

In one embodiment of the present invention, the compound of Formula (I) herein has the effect of inhibiting the activities of multiple protein kinases, comprising HPK1, FLT3 and KDR.

### DETAILED DESCRIPTION

The present application will be further described in details through the Examples. The features and advantages of the present application will become clearer through the descriptions thereof.

The word "such as" as used herein means "serving as an example or illustration." Any example described herein as "such as" is not necessarily to be considered as superior to other examples.

In addition, technical features related to different embodiments of the present application described herein can be combined with each other, provides they do not conflict with each other.

### Definition of Terms

In this disclosure, unless otherwise specified, a definition of a certain group applies to all groups containing this group. For example, the definition of alkyl is applicable to C₁-C₆ alkyl, C₁-C₃ alkyl, etc.; the definition of C₁-C₆ alkyl is applicable to "C₁-C₆ alkoxy", etc., and the following definitions are applicable to the claims and the description.

When a structure contains multiple substituents represented by the same symbol, the substituents may be the same or different; For example, the ring B contains two R³ as substituents, wherein the two R³ may be both methoxy, or one may be methoxy, and the other may be methyl.

The term "halogen" includes fluorine, chlorine, bromine, and iodine.

The term "Cₘ₋ₙ" (where m and n is an integer, and indicates the range that includes the end point) represents corresponding groups containing m-n carbon atoms, for example, C₁₋₆ alkyl represents an alkyl containing 1-6 carbon atoms, and C₂₋₆ alkenyl represents an alkenyl containing 2-6 carbon atoms.

The term "n membered" (where n is an integer) usually describes the number of ring-forming atoms, where the number of ring-forming atoms is n. "m-n membered" indicates the range that includes the end point, representing that the corresponding ring structure contains m-n ring-forming atoms. For example, piperidinyl is an example of a 6-membered heterocyclyl, and pyrazolyl is an example of a 5-membered heteroaryl.

The term "substituted" refers to a hydrogen of a structure is displaced by a "substituent". Unless otherwise indicated, the term "substituted" means any degree of substitution as long as said substitution is permitted. The choice of substituents is independent and the substitution may be in any chemically accessible position. It should be understood that substitution on a given atom is limited by chemical valence. It should be understood that substitution on a given atom produces chemically stable molecules. One divalent substituent (e.g., oxo) will displace two hydrogen atoms.

"The rest of the compound" refers to the portion of the whole molecular structure except for the "substituent" described. The rest of the compound is connected to the substituent by one or more unsaturated valences. The rest of the compound may contain one or more "connection point(s)", and two or more connection points may be on the same atom or different atoms.

The term "alkyl" refers to a straight-chain or branched-chain saturated hydrocarbon group. Alkyl is a group formed by the loss of a hydrogen of an alkane. Examples of alkyl include methyl, ethyl, n-propyl, isopropyl, n-butyl, isobutyl, sec-butyl, tert-butyl, n-pentyl, isopentyl, 2-methylbutyl, neopentyl, 1-ethylpropyl, n-hexyl, 1,2,2-trimethylpropyl and the like.

The term "alkenyl" refers to a straight or branched chain hydrocarbon group having one or more carbon - carbon double bonds. Alkenyl are a group formed by the loss of a hydrogen atom in an olefin. Examples of the alkenyl may be vinyl, 1-propenyl, 2-propenyl, allyl, 1-butenyl, 2-butenyl, (E)-but-2-ene-1-yl, (Z)-but-2-ene-1-yl, 2-methyl-propy-1-ene-1-yl, 1,3-butadiene-1-yl, 1,3-butadiene-2-yl and the like.

The term "alkynyl" refers to a straight or branched chain hydrocarbon group having one or more carbon-carbon triple bonds. Alkynyl is a group formed by the loss of one hydrogen atom from an alkyne. Examples of alkynyl may be ethynyl, 1-propynyl, propargyl, 1-butynyl, but-2-yn-1-yl, but-3-yn-1-yl, but-3-ene-1-alkynyl, 3-methylpent-2-ene-4-yn-1-yl and the like.

The term "alkylene" refers to a divalent group formed by losing two hydrogen atoms on the carbon atom of an alkane at the same time, wherein the two valences may be located on the same atom, or located on two atoms respectively. The two valences as described may be connected to the same atom of the rest of the compound, or connected to two separate atoms of the rest of the compound respectively. For example, methylene (-CH₂- or =CH₂), 1,1-ethylene (-CH(CH₃)- or =CH-CH₃), 1,2-ethylene (-CH₂CH₂-), but-1,4-diyl, but-1,3-diyl, 2,2-dimethyl-prop-1,3-diyl, and the like.

The term "n-membered oxaalkylene" refers to a bivalent group formed by replacing one or more carbon atoms in the main chain of an n-membered alkylene group with oxygen atom(s). The two valences as described may be connected to the same atom of the rest of the compound, or connected to two separate atoms of the rest of the compound respectively. For example, 2-oxa-1,3-propylene (-CH2OCH2-) is an example of 3-membered oxaalkylene group, 2-oxa-1,4-butylene (-CH2OCH2CH2-) is an example of a 4-membered oxaalkylene group, and the like. An alkylene group in which only branched-chain carbon atom(s) is replaced by oxygen atom(s) should not be considered "oxaalkylenes"; for example, in 2-methyl-1,3-propylene, when the branched-chain methyl is substituted by oxygen, and the resulted group (-CH2CH(OH)CH2-) shall be regarded as 2-hydroxy substituted 1,3-propylene.

The term "n-membered azaalkylene" refers to a divalent group formed by replacing one or more carbon atoms in the main chain of an n-membered alkylene with nitrogen atom(s), and the two valences described may be connected to the same atom of the rest of the compound, or connected to two separate atoms of the rest of the compound respectively. For example, 2-aza-1,3-propylene (-CH2NHCH2-) is an example of 3-membered azaalkylene, and aza-1,2-ethylene (-CH2NH-) is an example of 2-membered azaalkylene, and the like. An alkylene group in which only branched-chain carbon atom(s) is substituted with nitrogen atom(s) should not be considered "azaalkylene"; for example, in 2-methyl-1,3-propylene, when the branched-chain methyl is replaced by nitrogen, and the resulted group (-CH2CH(NH2)CH2-) should be regarded as 2-amino substituted 1,3-propylene.

The term "alkoxy" refers to a group with formula "-O-alkyl", wherein the alkyl group is as defined above. Alkoxy may be, for example, methoxy, ethoxy, n-propoxy, isopropoxy, n-butoxy, tert-butoxy and n-hexoxy and the like.

The term "alkylthio" refers to a group of formula "-S-alkyl", wherein the alkyl group is as defined above. "Alkylthio" may be, for example, methylthio, ethylthio, n-propylthio, isopropylthio, n-butylthio, tert-butylthio and n-hexylthio and the like.

The term "alkylamino" includes a group of formula "-NH-alkyl" and a group of formula "-N-(alkyl)₂", wherein the alkyl group is as defined above. The group of formula "-NH-alkyl" may be, for example, methylamino, ethylamino, isopropylamino and n-hexylamino and the like; The group of formula "-N-(alkyl)₂" may be, for example, dimethylamino, diethylamino, methylethylamino, methylisopropylamino and ethyl-n-hexylamino and the like.

The term "alkyl sulfinyl" refers to a group of formula "-S(=O)-alkyl", wherein the alkyl group is as defined above. For example, it may be methyl sulfinyl, ethyl sulfinyl, isopropyl sulfinyl and the like.

The term "alkyl sulfonyl" refers to a group of formula "-S(=O)₂-alkyl", wherein the alkyl group is as defined above. For example, it may be methyl sulfonyl, ethyl sulfoyl, isopropyl sulfonyl and the like.

The term "alkylaminosulfinyl" comprises a group of formula "-S(=O)-NH-alkyl" and a group of formula "-S(=O)-N(alkyl)₂", wherein the alkyl group is as defined above. The group of formula "-S(=O)-NH-alkyl" may be, for example, methylaminosulfinyl, ethylaminosulfinyl, isopropylaminosulfinyl, tert-butylaminosulfinyl and the like. The group of formula "-S(=O)-N(alkyl)₂" may be, for example, dimethylaminosulfinyl, diethylaminosulfinyl, methylethylaminosulfinyl, ethyl isobutylaminosulfinyl and the like.

The term "alkylaminosulfonyl" comprises a group of formula "-S(=O)₂-NH-alkyl" and a group of formula "-S(=O)₂-N(alkyl)₂", wherein the alkyl group is as defined above. The group of formula "-S(=O)₂-NH-alkyl" may be, for example, methylaminosulfonyl, ethylaminosulfonyl, isopropylaminosulfonyl, tert-butylaminosulfonyl, etc. The group of formula "-S(=O)₂-N(alkyl)₂" may be, for example, dimethylaminosulfonyl, diethylaminosulfonyl, methyl isopropylaminosulfonyl, ethyl tert-butylaminosulfonyl and the like.

The term "carbonyl" refers to a group of formula "-(C=O)-", which may also be represented by "-C(O)-".

The term "cyano" refers to a group of formula "-C=N", which may also be represented by "-CN".

The term "hydroxymethyl" refers to a group of formula "-CH₂OH".

The term "oxo" refers to an oxygen atom as a divalent substituent, when connected to a carbon atom to form a carbonyl group, or to the heteroatom to form a sulfinyl or sulfonyl, or *N-*oxide group and the like. In some embodiments, cycloalkyl and heterocyclyl may optionally be substituted by one or two oxo groups.

The term "imino group" or "=N-R", refers to a divalent substituent derived from ammonia or amine, wherein two valents of the same nitrogen atom are connected to one atom selected from the rest of the compound to form a double bond, and the third valence of the nitrogen atom is connected to the R group defined by the context. The "imino group" may form an imine, amidine or guanidine group when said nitrogen atom is connected to a carbon atom, or forms a sulfinyl imide or the like when said nitrogen atom is connected to a heteroatom.

The term "carbocyclyl" includes monocyclic and polycyclic aliphatic carbocyclyl and aromatic carbocyclyl. The monocyclic aliphatic carbocyclyl contains a single hydrocarbon ring, including cyclized alkyl and alkenyl. Polycyclic aliphatic carbocyclyl contains two or more hydrocarbon rings wherein at least one hydrocarbon ring is an aliphatic hydrocarbon ring (including cyclized alkyl and alkenyl), the other rings may be aliphatic and/or aromatic ring(s); wherein, any one of the rings is connected to at least one another ring to form a spiro ring (two rings share one ring-forming atom) or a bridged ring (two rings share two or more ring-forming atoms). The polycyclic carbocyclyl is connected to the rest of the compound by a ring-forming carbon atom on the hydrocarbon ring. It may be, for example, cyclopropyl, cyclobutyl, cyclopentyl, cyclohexyl, cyclopentenyl, cyclohexadienyl, bicyclo[3.1.0]hexyl, norbornyl, norpinanyl, bicyclo[1.1.1]pentyl, 1*H*-inden-1-yl, 2,3-dihydro-1*H*-inden-2-yl and the like. When a carbocyclyl only contains saturated rings, the carbocyclyl is a saturated carbocyclyl, also named as the "cycloalkyl".

The carbocyclyl includes a "carbocyclylene", i.e., the carbocyclyl is connected by two chemical valences to two connection points of the rest of the compound, and the two chemical valences described may be on the same carbon atom of the carbocyclylene, or may be on two different carbon atoms of the carbocyclylene. The two connection points may be located on the same atom of the rest of the compound, or located on two different atoms of the rest of the compound. For example, it may be 1,1-cyclobutylene, 1,3-cyclobutylene, and the like.

The term "aryl" refers to an aromatic monocyclic carbocyclyl or poly-carbocyclyl. For example, it may be phenyl, naphthyl, and the like.

The term "heterocyclyl" refers to a monocyclic or polycyclic group having at least one ring-forming heteroatom selected from oxygen, nitrogen, sulfur and phosphorus. The poly-heterocyclyl contains two or more rings, wherein at least one ring has at least one ring-forming heteroatom selected from oxygen, nitrogen, sulfur and phosphorus, and the other ring may have ring-forming heteroatoms or not. In poly-heterocyclyl, any one of the rings is connected to at least one other ring to form a spiro ring (two rings share one ring-forming atom) or a bridged ring (two rings share two or more ring-forming atoms). The heterocyclyl may be connected to the rest of the compound by an optional ring-forming carbon atom, or by an optional ring-forming heteroatom. In one embodiment, any of the ring carbon atoms in the heterocyclyl may be substituted by an oxo group to form a carbonyl group. In one embodiment, any ring nitrogen atom in the heterocyclyl may be *N*-oxide. In one embodiment, any ring nitrogen atom in the heterocyclyl may be quaternary ammonium cation.

Heterocyclyl includes aromatic heterocyclyl (i.e., "heteroaryl") and aliphatic heterocyclyl.

The term "heteroaryl" refers to an aromatic monocyclic heterocyclyl or poly-heterocyclyl having at least one ring-forming heteroatom selected from oxygen, nitrogen and sulfur. The heteroaryl group may be connected to the rest of the compound by an optional carbon atom, or by an optional heteroatom, provided that the chemical valence of the carbon atom or heteroatom allows. In one embodiment, any of the ring-forming carbon atoms in the heteroaryl may be substituted by an oxo group to form a carbonyl group. In one embodiment, any ring-forming nitrogen atom in the heteroaryl may be N oxide. In one embodiment, any ring nitrogen atom in the heteroaryl may be quaternary ammonium ion. For example, the heteroaryl may be pyrrolyl (including pyrrol-1-yl, pyrrol-2-yl and pyrrol-3-yl), pyrazolyl, imidazolyl, oxazolyl, isoxazolyl, thiazolyl, isothiazolyl, triazolyl, tetrazolyl, pyridyl, pyridin-2(1*H*)-one-1-yl, pyridin-4(1*H*)-one-1-yl, pyrazinyl, pyrimidinyl, pyridazinyl, pyrazin-3(2*H*)-one-2-yl, 1,2,4-triazinyl, 1,3,5-triazinyl, indolyl, benzofuranyl, benzothienyl, indazolyl, benzoimidazolyl, benzisothiazolyl, quinolinyl, isoquinolinyl, naphthyridinyl, imidazo[1,2-b]thiazolyl, purinyl, etc..

The term "aliphatic heterocyclyl" includes monocyclic or polycyclic aliphatic heterocyclyl. The monocyclic aliphatic heterocyclyl (aliphatic monocyclic heterocyclyl) may not contain double bond or contain one or more double bonds in the rings. The polycyclic aliphatic heterocyclyl (aliphatic poly-heterocyclyl) contains at least one alicycle, and other rings may be aliphatic or aromatic rings. The polycyclic aliphatic heterocyclyl may not contain a double bond or contain one or more double bonds in the rings. For example, it may be azetidinyl, oxetanyl, tetrahydropyrrolyl, tetrahydrofuranyl, 2-oxo-oxazolidinyl, piperidinyl, 3-oxo-piperidinyl, piperazinyl, morpholinyl, azepanyl, 2-oxa-6-azaspiro[3.3]heptyl, 1,2,3,4-tetrahydroquinolinyl, etc.

The term "heterocyclyl" includes a heterocyclylene, i.e., a heterocyclyl is connected to two points of attachment to the rest of the compound through two valences, and the two valences may be in the same ring-forming atom of the heterocyclylene. It may also be located on the two ring-forming atoms of the heterocyclylene group. The two connection points may be located on the same atom in the rest of the compound, or may be located on two atoms in the rest of the compound. For example, 1,1-(3-oxetanylene), 1,3-(2-azacyclopentylene), and the like.

The term "isomer" refers to isomers that result from different spatial arrangements of atoms in a molecule. "Stereoisomers" of the compounds described herein refers to all stereoisomers. For example, when the compound has asymmetric carbon atoms, enantiomers and diastereomers are produced; when the compound has carbon-carbon double bonds, carbon-nitrogen double bonds, or ring structures, *cis-* or trans-isomers are produced. Unless otherwise indicated, the compounds described herein include all isomers thereof, such as optical isomers, geometric isomers, rotational isomers, tautomers, stably existing conformational isomers, and the like; and the compounds may exist as a mixture of isomers or as isolated isomers.

Methods for preparing optically active products from optically inactive starting materials are known in the art, e.g., by resolution of racemic mixtures or by stereoselective synthesis.

Resolution of racemic mixtures of compounds may be carried out by any of a number of methods known in the art. One method involves fractional recrystallization using a chiral acid that is an optically active salt-forming organic acid. Suitable resolving agents for fractional recrystallization may be optically active acids such as D-tartaric acid, L-tartaric acid, diacetyltartaric acid, dibenzoyltartaric acid, mandelic acid, malic acid, L-camphorsulfonic acid, and the like. Other suitable resolving agents for fractional recrystallization include, for example, methylbenzylamine, 2-phenylglycinol, cyclohexylethylamine and the like in stereoisomerically pure form.

Methods for resolution of racemic mixtures also include, for example, the separation of diastereomers obtained by reaction with appropriate optically active species (such as chiral alcohols or Mosher's acid chloride), and then conversion (such as hydrolysis) back to the corresponding single optical isomer. For example, it may be performed by elution on a chromatographic column packed with an optically active resolving agent. Suitable chromatographic column and elution solvents may be determined by those skilled in the art.

The term "isotope labeled compounds" refers to a compound of the present application in which one or more atoms are replaced by a particular isotopic atom thereof. For example, the isotopic atom in the compound of the present application may include various isotopes of elements H, C, N, O, F, P, S, Cl and I, such as ²H, ³H, ¹³C, ¹⁴C, ¹⁵N, ¹⁷O, ¹⁸O, ¹⁸F, ³⁰P, ³²P, ³⁵S, ³⁶S, ¹²³I, ¹²⁴I and ¹²⁵I, etc. This application includes various isotope labeled compounds as defined. For example, they may be those compounds in which radioactive isotopes such as ³H and ¹⁴C are present, or those in which non-radioactive isotopes such as ²H and ¹³C are present. Such isotope labeled compounds are suitable for metabolic studies (using ¹⁴C), reaction kinetic studies (using e.g., ²H or ³H), detection or imaging techniques such as positron emission tomography (PET) or single photon emission computed tomography (SPECT), including drug or substrate tissue distribution analysis; or radiotherapy for patients, and the like.

In particular, ¹⁸F compounds may be particularly desirable for PET or SPECT studies. Isotope labeled compounds of formula (I) may generally be prepared by conventional techniques known to those skilled in the art or by methods analogous to those described in the accompanying examples and preparations using an appropriate isotope labeled reagent in place of the unlabeled reagents.

Furthermore, substitution with heavier isotopes, especially deuterium (i.e, ²H or D), may yield certain therapeutic advantages resulting from greater metabolic stability, such as increased in vivo half-life or reduced dose requirements or improved therapeutic index, and thus in some cases it may be preferred.

The term "pharmaceutically acceptable" refers to those compounds, materials, compositions and/or dosage forms, which are within the scope of sound medical judgment, suitable for use in contact with human and animal tissue without excessive of toxicity, irritation, allergic reactions or other problems or complications commensurate with a reasonable risk-benefit ratio.

The term "pharmaceutically acceptable salt" refers to a salt that retains the biological activity and properties of the compounds of the present invention and generally have no biologically or otherwise undesirable effects. In many cases, the compounds of the present invention are capable of forming acid and/or base addition salts via the presence of amino and/or carboxyls or the like.

"Pharmaceutically acceptable acid addition salts" may be formed with inorganic and organic acids. Examples of inorganic acids include, but are not limited to, hydrochloric acid, hydrobromic acid, sulfuric acid, nitric acid, phosphoric acid, and the like. Examples of organic acids include, but are not limited to, acetic acid, propionic acid, glycolic acid, oxalic acid, malonic acid, succinic acid, maleic acid, fumaric acid, tartaric acid, citric acid, benzoic acid, mandelic acid, methanesulfonic acid, ethanesulfonic acid, benzenesulfonic acid, p-toluenesulfonic acid, 1,2-ethanedisulfonic acid, sulfosalicylic acid, aspartic acid, glutamic acid, and the like.

"Pharmaceutically acceptable base addition salts" may be formed with inorganic and organic bases. Examples of inorganic bases include, but are not limited to, alkaline compounds of sodium, potassium, ammonium, calcium, magnesium, iron, silver, zinc and copper; particularly suitable salts include ammonium, potassium, sodium, calcium and magnesium salts. Organic bases include primary amines, secondary amines and tertiary amines, substituted amines including naturally occurring substituted amines, cyclic amines, alkaline ion-exchange resins, and the like. Examples of organic bases include, but are not limited to, isopropylamine, benzylamine, choline, diethanolamine, diethylamine, dicyclohexylamine, lysine, arginine, meglumine, piperazine, tromethamine, and the like.

All compounds and pharmaceutically acceptable salts thereof may be found or may be isolated (e.g., as hydrates and solvates) with other substances (e.g., solvents, including water and other solvents, etc.). When in the solid state, the compounds described herein and salts thereof may exist in various forms, including hydrates and solvates. Hydrates and solvates of compounds and salts thereof described herein include those in which water and solvents may be isotopically labeled, such as D₂O, methanol-*d*₃, methanol-*d*₄, acetone-*d*₆, DMSO-*d*₆. The presence of hydrates and solvates may be identified by those skilled in the art using means such as nuclear magnetic resonance (NMR).

The term "polymorph" refers to compounds of the present application that exist in different crystal lattice forms, as well as in amorphous form. Polymorphs of the compounds of the present application and salts thereof also include mixtures of various lattice forms, as well as mixtures of one or several lattice forms and amorphous form. The presence of polymorphs may be identified by those skilled in the art using means such as X-ray diffraction.

Therefore, unless expressly stated otherwise, references to compounds and salts thereof in this specification are to be understood to encompass any solid-state form of the compounds.

The term "active metabolite" refers to an active derivative of a compound that is formed when the compound is metabolized.

The term "a pharmaceutically acceptable prodrug" refers to any pharmaceutically acceptable ester, salt of the ester, amide or other derivative of the compound of the present application, which, upon administration to a subject, is capable of directly or indirectly providing the compound of the present application or its pharmacologically active metabolites or residues. Particularly preferred derivatives or prodrugs are those that increase the bioavailability of the compound of the present application when administered to a patient (e.g., make orally administered compounds more readily absorbed into the blood), or promote the delivery of the compound to biological organs or the site of action.

The term "a pharmaceutical composition" refers to a biologically active compound optionally in admixture with at least one pharmaceutically acceptable chemical component or agent, which is a "carrier" that facilitates for introducing the active compound into cells or tissues, include but are not limited to stabilizers, diluents, suspending agents, thickening agents and/or excipients. The pharmaceutical composition includes, but are not limited to, the following forms: tablets, pills, powders, lozenges, sachets, cachets, elixirs, suspensions, emulsions, solutions, syrups, aerosols (solid or dissolved in liquid vehicles), ointments, soft and hard gelatin capsules, suppositories, transdermal patches, sterile injectable solutions and sterile packaged powders, etc.

The term "pharmaceutically acceptable carriers" includes solvents, dispersion media, coatings, surfactants, antioxidants, preservatives (e.g., antibacterial, antifungal), isotonic agents, absorption delaying agents, salts, preservatives, pharmaceutical stabilizers, binders, excipients, disintegrants, lubricants, sweeteners, flavoring agents, dyes, and the like, well known to those skilled in the art, and combinations thereof. Unless being incompatible with the active compound, any conventional carrier is included in the therapeutic or pharmaceutical compositions.

The term "administration" or "administering" includes the means by which the compounds and pharmaceutical compositions thereof are introduced into a subject to achieve their intended function. The route of administration depends on whether the treatment is local or systemic and the area to be treated. Examples of routes of administration that may be used include injection (including subcutaneous, intravenous, intraarterial, parenteral, intraperitoneal, intrathecal and other local injections), topical administration (including transdermal, epidermal, ocular and mucosal, including intranasal, vaginal and rectal delivery), oral administration, inhalation, or insufflation (intratracheally or intranasally, e.g. by inhalation or insufflation of powders or aerosols, including by nebulizer). Injection administration may be by way of a single bolus dose, or may be by infusion, for example, by a continuous perfusion pump.

The term "therapeutically effective amount" refers to the amount of the compound of the present application that induces a biological or medical response in a subject, such as reducing or inhibiting activity of enzyme or protein or ameliorating symptoms, alleviating a condition, slowing or delaying disease progression, or preventing disease, etc..

The term "subject" or "patient" refers to an individual, including mammals and non-mammals, suffering from a disease, disorder, condition, or the like. Examples of mammal include, but are not limited to, any member of the class Mammalia: humans; non-human primates (e.g., chimpanzees and other apes and monkeys); livestock, such as cattle, horses, sheep, goats, pigs; other domesticated animals, such as rabbits, dogs, and cats; laboratory animals, including rodents, such as rats, mice, and guinea pigs, and the like. Examples of non-mammals include, but are not limited to, birds, fish, and the like.

### Compound

In one aspect, the present application provides azaindazole compound of Formula (I), 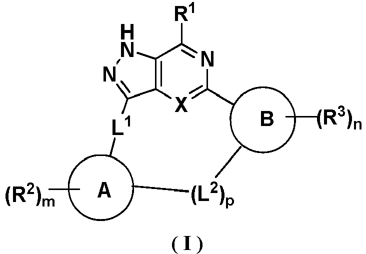
or an isomer, pharmaceutically acceptable salt, polymorph, isotope labeled compound, active metabolite or prodrug thereof,
wherein, the structure and atomic number of the azaindazole ring are as shown in Formula (Ia), 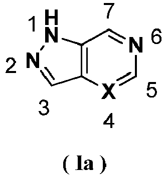
in the structure of Formula (I), X is selected from CR^{x} and N, wherein R^{x} is independently selected from H and halogen;
R¹ is selected from:
   1) hydrogen, halogen, cyano, -C(=O)OR^{a1}, -C(=O)NR^{a1}R^{b1}, -OR^{a1}, -NR^{a1}R^{b1}, -NR^{e1}C(=O)R^{a1}, -NR^{e1}C(=O)NR^{a1}R^{b1}, -SR^{a1}, -S(=O)R^{a1} and -S(=O)₂R^{a1}; or
   2) C₁₋₆ alkyl, C₂₋₄ alkenyl, C₂₋₄ alkynyl, C₃₋₈ cycloalkyl, and 3- to 8-membered aliphatic heterocyclyl, optionally substituted with 0, 1, 2, 3 or 4 of R¹¹;
wherein, R^{a1}, R^{b1} and R^{e1} are each independently selected from:
   1) hydrogen; or
   2) C₁₋₆ alkyl, C₃₋₆ monocyclic cycloalkyl, and 3- to 6-membered monocyclic aliphatic heterocyclyl, optionally substituted with 0, 1, 2, 3 or 4 of R¹¹;

   or, R^{a1} and R^{b1} attached to the same nitrogen atom, taken together with the nitrogen atom to which they are attached, form a 4- to 6-membered aliphatic heterocyclyl, optionally substituted with 0, 1, 2, 3 or 4 of R¹¹;
   wherein, R¹¹ is selected from oxo, halogen, hydroxyl, amino, cyano, C₁₋₃ alkyl, C₁₋₃ alkoxy, C₁₋₃ alkylamino, C₃₋₆ cycloalkyl and C₃₋₆ cycloalkoxy;
L¹ is selected from:
   1) single bond; or
   2) 6- to 10-membered aryl, and 5- to 10-memebred heteroaryl; said aryl and heteroaryl group is connected to the azaindazole ring at the 3-position and to the ring A through a single bond, respectively; in addition to being connected to the azaindazole ring at the 3-position and to the ring A, said aryl and heteroaryl group is optionally substituted with 0, 1, 2, 3, 4, 5 or 6 of R4;
ring A is selected from monocyclic or bicyclic 3- to 12-membered carbocyclyl group and 3- to 12-membered heterocyclyl group, said carbocyclyl or heterocyclyl group is connected to L¹ and L² through two different atoms respectively; and in addition to being connected to L¹ and L², said carbocyclyl or heterocyclyl group is optionally substituted with m of R²;
ring B is selected from monocyclic or bicyclic 6- to 12-membered carbocyclyl group and 5- to 12-membered heterocyclyl group, said carbocyclyl or heterocyclyl group is connected to L² and to the azaindazole ring at the 5-position through two different atoms respectively; wherein,
   1) when ring B is selected from monocyclic carbocyclyl or monocyclic heterocyclyl group, the shortest distance between the two atoms on ring B, through which ring B is connected to L² and to the azaindazole ring at the 5-position, is 1, 2 or 3 chemical bonds;
   2) when ring B is selected from bicyclic carbocyclyl or bicyclic heterocyclyl group, the shortest distance between the two atoms on ring B, through which ring B is connected to L² and to the azaindazole ring at the 5-position, is 1, 2, 3, 4 or 5 chemical bonds;
and, in addition to being connected to L² and to the azaindazole ring at the 5-position, ring B is optionally substituted with n of R³;
R² is each independently selected from:
   1) oxo, halogen, cyano, -C(=O)R^{a2}, -C(=O)OR^{a2}, -C(=O)NR^{a2}R^{b2}, -C(=NR^{d2})NR^{a2}R^{b2}, -OR^{a2}, -OC(=O)R^{a2}, -OC(=O)OR^{c2}, -OC(=O)NR^{a2}R^{b2}, -SR^{a2}, -S(=O)R^{c2}, -S(=O)2R^{c2}, sulfonic acid group, -S(=O)NR^{a2}R^{b2}, -S(=O)2NR^{a2}R^{b2}, -S(=O)(=NR^{d2})R^{c2}, -NR^{a2}R^{b2}, -NR^{a2}C(=O)R^{b2}, -NR^{a2}C(=O)OR^{c2}, -NR^{e2}C(=O)NR^{a2}R^{b2}, -NR^{e2}C(=NR^{d2})NR^{a2}R^{b2}, -NR^{a2}S(=O)₂R^{c2}, -NR^{e2}S(=O)₂NR^{a2}R^{b2}, nitro, -PR^{c2}R^{f2}, -P(=O)R^{c2}R^{f2} and phosphonic acid group; or
   2) C₁₋₆ alkyl, C₂₋₆ alkenyl, C₂₋₆ alkynyl, phenyl, 5- to 10-membered heteroaryl, C₃₋₁₂ cycloalkyl and 3- to 12-membered aliphatic heterocyclyl, optionally substituted with 0, 1, 2, 3, 4, 5 or 6 of R²²; or
   3) divalent bridged C₁₋₅ alkylene, 3- to 6-membered oxaalkylene, and 2- to 6-membered azaalkylene connected to ring A through two different ring-forming atoms thereof; said alkylene, oxaalkylene and azaalkylene are optionally substituted with 0, 1, 2, 3, 4, 5 or 6 of R²²;
or, two R² groups attached to one or two ring-forming atom(s) of the ring A, taken together with the one or two ring-forming atom(s) to which they are attached, form a C₅₋₁₂ aliphatic carbocyclyl, or 5- to 12-membered aliphatic heterocyclyl, optionally substituted with 0, 1, 2, 3, 4 or 5 of R²²;
wherein, R^{a2}, R^{b2} and R^{e2} are each independently selected from:
   1) hydrogen; or
   2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4, 5 or 6 of R²²;

   or, R^{a2} and R^{b2} attached to the same nitrogen atom, taken together with the nitrogen atom to which they are attached, form a 3- to 12-membered aliphatic heterocyclyl, optionally substituted with 0, 1, 2, 3, 4 or 5 of R²²;
   R^{c2} and R^{f2} are each independently selected from C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4 or 5 of R²²; or, R^{c2} and R^{f2} attached to the same phosphine atom, taken together with the phosphine atom to which they are attached, form a 3- to 12-membered aliphatic heterocyclyl, optionally substituted with 0, 1, 2, 3, 4 or 5 of R²²;
   R^{d2} is selected from:
      1) hydrogen, cyano, nitro and -S(=O)₂R^{G}; or
      2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4 or 5 of R²²;
   R²² is selected from:
      1) oxo, halogen, cyano, -C(=O)R^{a22}, -C(=O)OR^{a22}, -C(-O)NR^{a22}R^{b22}, -C(=NR^{d22})NR^{a22}R^{b22}, -OR^{a22}, -OC(=O)R^{a22}, -OC(=O)OR^{c22}, -OC(=O)NR^{a22}R^{b22}, -SR^{a22}, -S(=O)R^{c22}, -S(=O)₂R^{c22}, sulfonic acid group, -S(=O)NR^{a22}R^{b22}, -S(=O)₂NR^{a22}R^{b22}, -S(=O)(=NR^{d22})R^{c22}, -NR^{a22}R^{b22}, -NR^{a22}C(=O)R^{b22}, -NR^{a22}C(=O)OR^{c22}, -NR^{e22}C(=O)NR^{a22}R^{b22}, -NR^{e22}C(=NR^{d22})NR^{a22}R^{b22}, -NR^{a22}S(=O)₂R^{c22}, -NR^{e22}S(=O)₂NR^{a22}R^{b22}, nitro, -PR^{c22}R^{f22}, -P(=O)R^{c22}R^{f22}, phosphonic acid group, and =N-R^{d22}; or
      2) C₁₋₆ alkyl, C₁₋₆ alkylene, C₂₋₆ alkenyl, C₂₋₆ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R²³;
   R^{a22}, R^{b22} and R^{e22} are each independently selected from:
      1) hydrogen; or
      2) C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R²³;
   or, R^{a22} and R^{b22} attached to the same nitrogen atom, taken together with the nitrogen atom to which they are attached, form a 3- to 10-membered aliphatic heterocyclyl, optionally substituted with 0, 1, 2, 3, 4 or 5 of R²³;
   R^{c22} and R^{f22} are each independently selected from C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R²³; or, R^{c22} and R^{f22} attached to the same phosphine atom, taken together with the phosphine atom to which they are attached, form a 3- to 10-membered aliphatic heterocyclyl, optionally substituted with 0, 1, 2, 3, 4 or 5 of R²³;
   R^{d22} is selected from:
      1) hydrogen, cyano, nitro and -S(=O)₂R^{G}; or
      2) C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R²³;
   R²³ is selected from:
      1) oxo, halogen, cyano, -C(=O)R^{a23}, -C(=O)OR^{a23}, -C(=O)NR^{a23}R^{b23}, C(=NR^{d23})NR^{a23}R^{b23}, -OR^{a23}, -OC(=O)R^{a23}, -OC(=O)OR^{c23}, -OC(=O)NR^{a23}R^{b23}, -SR^{a23}, -S(=O)R^{c23}, -S(=O)₂R^{c23}, sulfonic acid group, -S(=O)NR^{a23}R^{b23}, -S(=O)₂NR^{a23}R^{b23}, -S(=O)(=NR^{d23})R^{c23}, -NR^{a23}R^{b23}, -NR^{a23}C(=O)R^{b23}, -NR^{a23}C(=O)OR^{c23}, -NR^{e23}C(=O)NR^{a23}R^{b23}, -NR^{e23}C(=NR^{d23})NR^{a23}R^{b23}, -NR^{a23}S(=O)₂R^{c23}, -NR^{e23}S(=O)₂NR^{a23}R^{b23}, nitro, -PR^{c23}R^{f23}, -P(=O)R^{c23}R^{f23}, phosphonic acid group, and =N-R^{d23}; or
      2) C₁₋₄ alkyl, C₁₋₄ alkylene, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
   R^{a23}, R^{b23} and R^{e23} are each independently selected from:
      1) hydrogen; or
      2) C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
   or, R^{a23} and R^{b23} attached to the same nitrogen atom, taken together with the nitrogen atom to which they are attached, form a 3- to 8-membered aliphatic heterocyclyl, optionally substituted with 0, 1, 2, 3, or 4 of R^{G};
   R^{c23} and R^{f23} are each independently selected from C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3, or 4 of R^{G};
   or, R^{c23} and R^{f23} attached to the same phosphine atom, taken together with the phosphine atom to which they are attached, form a 3- to 8-membered aliphatic heterocyclyl, optionally substituted with 0, 1, 2, 3 or 4 of R^{G};
   R^{d23} is selected from:
      1) hydrogen, cyano, nitro and -S(=O)₂R^{G}; or
      2) C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
R³ is independently selected from:
   1) oxo, halogen, cyano, -C(=O)R^{a3}, -C(=O)OR^{a3}, -C(=O)NR^{a3}R^{b3}, -C(=NR^{d3})NR^{a3}R^{b3}, -OR^{a3}, -OC(=O)R^{a3}, -OC(=O)OR^{c3}, -OC(=O)NR^{a3}R^{b3}, -SR^{a3}, -S(=O)R^{c3}, -S(=O)₂R^{c3}, sulfonic acid group, -S(=O)NR^{a3}R^{b3}, -S(=O)₂NR^{a3}R^{b3}, -S(=O)(=NR^{d3})R^{c3}, -NR^{a3}R^{b3}, -NR^{a3}C(=O)R^{b3}, -NR^{a3}C(=O)OR^{c3}, -NR^{e3}C(=O)NR^{a3}R^{b3}, -NR^{e3}C(=NR^{d3})NR^{a3}R^{b3}, -NR^{a3}S(=O)₂R^{c3}, -NR^{e3}S(=O)₂NR^{a3}R^{b3}, nitro, -PR^{c3}R^{f3}, -P(=O)R^{c3}R^{f3} and phosphonic acid group; or
   2) C₁₋₆ alkyl, C₂₋₆ alkenyl, C₂₋₆ alkynyl, 6- to 10-membered aryl, 5- to 10-membered heteroaryl, C₃₋₁₂ cycloalkyl and 3- to 12-membered aliphatic heterocyclyl, optionally substituted with 0, 1, 2, 3, 4, 5 or 6 of R³²; or
   3) divalent bridged C₁₋₆ alkylene connected to ring B through two different ring-forming atoms thereof;

   or, two R³ groups attached to one or two ring-forming atom(s) of the ring B, taken together with the one or two ring-forming atom(s) to which they are attached, form a C₅₋₁₂ aliphatic carbocyclyl, or 5- to 12-membered aliphatic heterocyclyl, optionally substituted with 0, 1, 2, 3, 4 or 5 of R³²;
   wherein, R^{a3}, R^{b3} and R^{e3} are each independently selected from:
      1) hydrogen; or
      2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4, 5 or 6 of R³²;
   or, R^{a3} and R^{b3} attached to the same nitrogen atom, taken together with the nitrogen atom to which they are attached, form a 3- to 12-membered aliphatic heterocyclyl, optionally substituted with 0, 1, 2, 3, 4 or 5 of R³²;
   R^{c3} and R^{f3} are each independently selected from C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4 or 5 of R³²;
   or, R^{c3} and R^{f3} attached to the same phosphine atom, taken together with the phosphine atom to which they are attached, form a 3- to 12-membered aliphatic heterocyclyl, optionally substituted with 0, 1, 2, 3, 4 or 5 of R³²;
   R^{d3} is selected from:
      1) hydrogen, cyano, nitro and -S(=O)₂R^{G}; or
      2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4 or 5 of R³²;
   R³² is selected from:
      1) oxo, halogen, cyano, -C(=O)R^{a32}, -C(=O)OR^{a32}, -C(=O)NR^{a32}R^{b32}, -C(=NR^{d32})NR^{a32}R^{b32}, -OR^{a32}, -OC(=O)R^{a32}, -OC(=O)OR^{c32}, -OC(=O)NR^{a32}R^{b32}, -SR^{a32}, -S(=O)R^{c32}, -S(=O)₂R^{c32}, sulfonic acid group, -S(=O)NR^{a32}R^{b32}, -S(=O)₂NR^{a32}R^{b32}, -S(=O)(=NR^{d32})R^{c32}, -NR^{a32}R^{b32}, -NR^{a32}C(=O)R^{b32}, -NR^{a32}C(=O)OR^{c32}, -NR^{e32}C(=O)NR^{a32}R^{b32}, -NR^{e32}C(=NR^{d32})NR^{a32}R^{b32}, -NR^{a32}S(=O)₂R^{c32}, -NR^{e32}S(=O)₂NR^{a32}R^{b32}, nitro, -PR^{c32}R^{f32}, -P(=O)R^{c32}R^{f32}, phosphonic acid group, and =N-R^{d32}; or
      2) C₁₋₆ alkyl, C₁₋₆ alkylene, C₂₋₆ alkenyl, C₂₋₆ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R³³;
   R^{a32}, R^{b32} and R^{e32} are each independently selected from:
      1) hydrogen; or
      2) C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R³³;
   or, R^{a32} and R^{b32} attached to the same nitrogen atom, taken together with the nitrogen atom to which they are attached, form a 3- to 10-membered aliphatic heterocyclyl, optionally substituted with 0, 1, 2, 3, 4 or 5 of R³³;
   R^{c32} and R^{f32} are each independently selected from C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R³³;
   or, R^{c32} and R^{f32} attached to the same phosphine atom, taken together with the phosphine atom to which they are attached, form a 3- to 10-membered aliphatic heterocyclyl, optionally substituted with 0, 1, 2, 3, 4 or 5 of R³³;
   R^{d32} is selected from:
      1) hydrogen, cyano, nitro and -S(=O)₂R^{G}; or
      2) C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R³³;
   R³³ is selected from:
      1) oxo, halogen, cyano, -C(=O)R^{a33}, -C(=O)OR^{a33}, -C(=O)NR^{a33}R^{b33}, -C(=NR^{d33})NR^{a33}R^{b33}, -OR^{a33}, -OC(=O)R^{a33}, -OC(=O)OR^{c33}, -OC(=O)NR^{a33}R^{b33}, -SR^{a33}, -S(=O)R^{c33}, -S(=O)₂R^{c33}, sulfonic acid group, -S(=O)NR^{a33}R^{b33}, -S(=O)₂NR^{a33}R^{b33}, -S(=O)(=NR^{d33})R^{c33}, -NR^{a33}R^{b33}, -NR^{a33}C(=O)R^{b33}, -NR^{a33}C(=O)OR^{c33}, -NR^{e33}C(=O)NR^{a33}R^{b33}, -NR^{e33}C(=NR^{d33})NR^{a33}R^{b33}, -NR^{a33}S(=O)₂R^{c33}, -NR^{e33}S(=O)₂NR^{a33}R^{b33}, nitro, -PR^{c33}R^{f33}, -P(=O)R^{c33}R^{f33}, phosphonic acid group, and =N-R^{d33}; or
      2) C₁₋₄ alkyl, C₁₋₄ alkylene, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
   R^{a33}, R^{b33} and R^{e33} are each independently selected from:
      1) hydrogen; or
      2) C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
   or, R^{a33} and R^{b33} attached to the same nitrogen atom, taken together with the nitrogen atom to which they are attached, form a 3- to 8-membered aliphatic heterocyclyl, optionally substituted with 0, 1, 2, 3, or 4 of R^{G};
   R^{c33} and R^{f33} are each independently selected from C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3, or 4 of R^{G};
   or, R^{c33} and R^{f33} attached to the same phosphine atom, taken together with the phosphine atom to which they are attached, form a 3- to 8-membered aliphatic heterocyclyl, optionally substituted with 0, 1, 2, 3 or 4 of R^{G};
   R^{d33} is selected from:
      1) hydrogen, cyano, nitro and -S(=O)₂R^{G}; or
      2) C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
R⁴ is independently selected from:
   1) oxo, halogen, cyano, -C(=O)R^{a4}, -C(=O)OR^{a4}, -C(=O)NR^{a4}R^{b4}, -C(=NR^{d4})NR^{a4}R^{b4}, -OR^{a4}, -OC(=O)R^{a4}, -OC(=O)OR^{c4}, -OC(=O)NR^{a4}R^{b4}, -SR^{a4}, -S(=O)R^{c4}, -S(=O)₂R^{c4}, -S(=O)NR^{a4}R^{b4}, -S(=O)₂NR^{a4}R^{b4}, -S(=O)(=NR^{d4})R^{c4}, -NR^{a4}R^{b4}, -NR^{a4}C(=O)R^{b4}, -NR^{a4}C(=O)OR^{c4}, -NR^{e4}C(=O)NR^{a4}R^{b4}, -NR^{e4}C(=NR^{d4})NR^{a4}R^{b4}, -NR^{a4}S(=O)₂R^{c4} and -NR^{e4}S(=O)₂NR^{a4}R^{b4}; or
   2) C₁₋₆ alkyl, C₂₋₆ alkenyl, C₂₋₆ alkynyl, phenyl, 5- to 10-membered heteroaryl, C₃₋₁₂ cycloalkyl and 3- to 12-membered aliphatic heterocyclyl, optionally substituted with 0, 1, 2, 3, 4, 5 or 6 of R⁴²;
or, two R⁴ groups attached to two ring-forming atoms of the ring L¹, taken together with the two ring-forming atoms to which they are attached, form a C₅₋₁₂ aliphatic carbocyclyl, or 5-to 12-membered aliphatic heterocyclyl, optionally substituted with 0, 1, 2, 3, 4, 5 or 6 of R⁴²;
   R^{a4}, R^{b4} and R^{e4} are each independently selected from:
      1) hydrogen; or
      2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4, 5 or 6 of R⁴²;
   or, R^{a4} and R^{b4} attached to the same nitrogen atom, taken together with the nitrogen atom to which they are attached, form a 3- to 12-membered aliphatic heterocyclyl, optionally substituted with 0, 1, 2, 3, 4 or 5 of R⁴²;
   R^{c4} and R^{f4} are each independently selected from C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4 or 5 of R⁴²;
   R^{d4} is selected from:
      1) hydrogen, cyano, nitro and -S(=O)₂R^{G}; or
      2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4 or 5 of R⁴²;
   R⁴² is selected from:
      1) oxo, halogen, cyano, -C(=O)R^{a42}, -C(=O)OR^{a42}, -C(=O)NR^{a42}R^{b42}, -C(=NR^{d42})NR^{a42}R^{b42}, -OR^{a42}, -OC(=O)R^{a42}, -OC(=O)OR^{c42}, -OC(=O)NR^{a42}R^{b42}, -SR^{a42}, -S(=O)R^{c42}, -S(=O)₂R^{c42}, -S(=O)NR^{a42}R^{b42}, -S(=O)₂NR^{a42}R^{b42}, -S(=O)(=NR^{d42})R^{c42}, -NR^{a42}R^{b42}, -NR^{a42}C(=O)R^{b42}, -NR^{a42}C(=O)OR^{c42}, -NR^{e42}C(=O)NR^{a42}R^{b42}, -NR^{e42}C(=NR^{d42})NR^{a42}R^{b42}, -NR^{a42}S(=O)₂R^{c42}, -NR^{e42}S(=O)₂NR^{a42}R^{b42} and =N-R^{d42}; or
      2) C₁₋₆ alkyl, C₁₋₆ alkylene, C₂₋₆ alkenyl, C₂₋₆ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R⁴³;
   R^{a42}, R^{b42} and R^{e42} are each independently selected from:
      1) hydrogen; or
      2) C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R⁴³;
   or, R^{a42} and R^{b42} attached to the same nitrogen atom, taken together with the nitrogen atom to which they are attached, form a 3- to 10-membered aliphatic heterocyclyl, optionally substituted with 0, 1, 2, 3, 4 or 5 of R⁴³;
   R^{c42} and R^{f42} are each independently selected from C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R⁴³;
   R^{d42} is selected from:
      1) hydrogen, cyano, nitro and -S(=O)₂R^{G}; or
      2) C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R⁴³;
   R⁴³ is selected from:
      1) oxo, halogen, cyano, -C(=O)R^{a43}, -C(=O)OR^{a43}, -C(=O)NR^{a43}R^{b43}, -C(=NR^{d43})NR^{a43}R^{b43}, -OR^{a43}, -OC(=O)R^{a43}, -OC(=O)OR^{c43}, -OC(=O)NR^{a43}R^{b43}, -SR^{a43}, -S(=O)R^{c43}, -S(=O)₂R^{c43}, -S(=O)NR^{a43}R^{b43}, -S(=O)₂NR^{a43}R^{b43}, -S(=O)(=NR^{d43})R^{c43}, -NR^{a43}R^{b43}, -NR^{a43}C(=O)R^{b43}, -NR^{a43}C(=O)OR^{c43}, -NR^{e43}C(=O)NR^{a43}R^{b43}, -NR^{e43}C(=NR^{d43})NR^{a43}R^{b43}, -NR^{a43}S(=O)₂R^{c43}, -NR^{e43}S(=O)₂NR^{a43}R^{b43} and =N-R^{d43}; or
      2) C₁₋₄ alkyl, C₁₋₄ alkylene, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
   R^{a43}, R^{b43} and R^{e43} are each independently selected from:
      1) hydrogen; or
      2) C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
   or, R^{a43} and R^{b43} attached to the same nitrogen atom, taken together with the nitrogen atom to which they are attached, form a 3- to 8-membered aliphatic heterocyclyl, optionally substituted with 0, 1, 2, 3, or 4 of R^{G};
   R^{c43} and R^{f43} are each independently selected from C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3, or 4 of R^{G};
   R^{d43} is selected from:
      1) hydrogen, cyano, nitro and -S(=O)₂R^{G}; or
      2) C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
L² is each independently selected from -CH₂-, -NH-, -O-, and -S-; wherein, each L² is independently substituted with 0, 1, or 2 of R⁵; two adjacent L² may be connected through single bond, double bond, or triple bond, provided that the chemical valence allows; p of L² connected sequentially form a linker connecting ring A and ring B; wherein the two L² at both ends are attached to ring A and ring B through single bond, respectively;
R⁵ is independently selected from:
   1) oxo, halogen, cyano, -C(=O)R^{a5}, -C(=O)OR^{a5}, -C(=O)NR^{a5}R^{b5}, -C(=NR^{d5})NR^{a5}R^{b5}, -OR^{a5}, -OC(=O)R^{a5}, -OC(=O)OR^{c5}, -OC(=O)NR^{a5}R^{b5}, -OP(=O)(R^{G1})₂, -SR^{a5}, -S(=O)R^{c5}, -S(=O)₂R^{c5}, sulfonic acid group, -S(=O)NR^{a5}R^{b5}, -S(=O)₂NR^{a5}R^{b5}, -S(=O)(=NR^{d5})R^{c5}, -NR^{a5}R^{b5}, -NR^{a5}C(=O)R^{b5}, -NR^{a5}C(=O)OR^{c5}, -NR^{e5}C(=O)NR^{a5}R^{b5}, -NR^{e5}C(=NR^{d5})NR^{a5}R^{b5}, -NR^{a5}S(=O)₂R^{c5}, -NR^{e5}S(=O)₂NR^{a5}R^{b5}, -NR^{G2}P(=O)(R^{G1})₂, -P(=O)R^{c5}R^{f5}, -P(=O)(R^{G1})₂ and =N-R^{d5}; or
   2) C₁₋₆ alkyl, C₂₋₆ alkenyl, C₂₋₆ alkynyl, phenyl, 5- to 6-membered heteroaryl, C₃₋₆ cycloalkyl and 3- to 6-membered aliphatic heterocyclyl, optionally substituted with 0, 1, 2, 3 or 4 of R⁵²; or
   3) two R⁵ groups attached to the same L² or attached to two adjacent L², taken together with the one or two L² atom(s) to which they are attached, form a C₃₋₆ aliphatic carbocyclyl, or 3- to 6-membered aliphatic heterocyclyl, optionally substituted with 0, 1, 2, 3, or 4 of R⁵²; or
   4) two R⁵ groups attached to two nonadjacent L², taken together with the two L² atoms to which they are attached and all other L² atom(s) between said two L² atoms, form a C₃₋₆ aliphatic carbocyclyl, or 3- to 6-membered aliphatic heterocyclyl, optionally substituted with 0, 1, 2, 3, or 4 of R⁵²;

   R^{a5}, R^{b5} and R^{e5} are independently selected from:
      1) hydrogen; or
      2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3 or 4 of R⁵²;
   or, R^{a5} and R^{b5} attached to the same nitrogen atom, taken together with the nitrogen atom to which they are attached, form a 3- to 6-membered aliphatic heterocyclyl, optionally substituted with 0, 1, 2, 3 or 4 of R⁵²;
   R^{c5} and R^{f5} are each independently selected from C₁₋₄ alkyl, C₂₋₄ alkenyl, C₂₋₄ alkynyl, phenyl, 5- to 6-membered heteroaryl, C₃₋₆ cycloalkyl and 3- to 6-membered aliphatic heterocyclyl, optionally substituted with 0, 1, 2, 3 or 4 of R⁵²;
   or, R^{c5} and R^{f5} attached to the same phosphine atom, taken together with the phosphine atom to which they are attached, form a 3- to 6-membered aliphatic heterocyclyl, optionally substituted with 0, 1, 2, 3 or 4 of R⁵²;
   R^{d5} is selected from:
      1) hydrogen, hydroxyl, C₁₋₄ alkoxy, cyano, nitro and -S(=O)₂R^{G}; or
      2) C₁₋₄ alkyl, C₂₋₄ alkenyl, C₂₋₄ alkynyl, phenyl, 5- to 6-membered heteroaryl, C₃₋₆ cycloalkyl and 3- to 6-membered aliphatic heterocyclyl, optionally substituted with 0, 1, 2, 3 or 4 of R⁵²;
   R⁵² is selected from:
      1) oxo, halogen and cyano; or
      2) -C(=O)R^{G1}, -C(=O)R^{G2}, -C(=NR^{G3})N(R^{G2})₂, -OC(=O)R^{G2}, -OP(=O)(R^{G1})₂, -S(=O)R^{G2}, -S(=O)₂R^{G2}, sulfonic acid group, -S(=O)N(R^{G2})₂, -S(=O)₂N(R^{G2})₂, -S(=O)(=NR^{G3})R^{G2}, -N(R^{G2})C(=O)R^{G1}, -N(R^{G2})C(=O)R^{G2}, -N(R^{G2})C(=NR^{G3})N(R^{G2})₂, -N(R^{G2})S(=O)₂R^{G2}, -N(R^{G2})S(=O)₂N(R^{G2})₂, -N(R^{G2})P(=O)(R^{G1})_{2,} -P(=O)(R^{G2})₂, -P(=O)(R^{G1})₂, =N-R^{G3} and R^{G1}; or
      3) R^{G2};
   R^{G} is selected from:
      1) halogen, oxo, cyano, carboxyl, hydroxyl, C₁₋₄ alkoxy, amino, C₁₋₄ alkylamino, C₁₋₄ alkyl sulfinyl, C₁₋₄ alkyl sulfonyl, and C₁₋₄ alkylaminosulfonyl; or
      2) C₁₋₄ alkyl, C₁₋₄ alkylene, C₂₋₄ alkenyl, C₂₋₄ alkynyl, C₃₋₆ cycloalkyl and 3- to 8-membered aliphatic heterocyclyl, optionally substituted with 0, 1, 2, 3 or 4 substituents independently selected from: oxo, halogen, hydroxyl, hydroxymethyl, carboxyl, cyano, C₁₋₃ alkoxy, amino, and C₁₋₃ alkylamino;
   R^{G1} is selected from -OR^{G2} and -N(R^{G2})₂;
   R^{G2} is selected from:
      1) hydrogen; or
      2) C₁₋₄ alkyl, C₁₋₄ alkylene, C₃₋₆ cycloalkyl, 3- to 6-membered aliphatic heterocyclyl and phenyl, optionally substituted with 0, 1, 2 or 3 substituents independently selected from: oxo, halogen, hydroxyl, hydroxymethyl, carboxyl, cyano, C₁₋₃ alkoxy, amino, and C₁₋₃ alkylamino; or
      3) two R^{G2} attached to the same nitrogen atom, taken together with the nitrogen atom to which they are attached, form a 3- to 6-membered aliphatic heterocyclyl, optionally substituted with 0, 1, 2 or 3 substituents independently selected from: oxo, halogen, hydroxyl, hydroxymethyl, carboxyl, cyano, C₁₋₃ alkoxy, amino, and C₁₋₃ alkylamino;
   R^{G3} is selected from: hydrogen, cyano, nitro, -OR^{G2}, -S(=O)₂R^{G2} and R^{G2};
p is selected from 1, 2, 3, 4, 5, 6, 7, 8, 9 and 10;
m is selected from 0, 1, 2, 3, 4, 5 and 6;
n is selected from 0, 1, 2, 3, 4, 5 and 6.

In one embodiment, X is selected from CH and N.

In one embodiment, X is CH.

In one embodiment, X is C-F.

In one embodiment, X is N.

In one embodiment, R¹ is selected from:
1) hydrogen, halogen, cyano, -C(=O)OR^{a1}, -C(=O)NR^{a1}R^{b1}, -OR^{a1}, and -NR^{a1}R^{b1}; or
2) C₁₋₆ alkyl, C₂₋₄ alkenyl, C₂₋₄ alkynyl, C₃₋₈ cycloalkyl, and 3- to 8-membered aliphatic heterocyclyl, optionally substituted with 0, 1, 2, 3 or 4 of R¹¹;

In one embodiment, R¹ is selected from:
1) hydrogen, halogen, cyano, ethynyl, -OR^{a1}, and -NR^{a1}R^{b1}; or
2) C₁₋₄ alkyl, C₃₋₅ monocyclic cycloalkyl, and 4- to 5-membered monocyclic aliphatic heterocyclyl, optionally substituted with 0, 1, 2, 3 or 4 of R¹¹;

In one embodiment, R¹ is selected from: hydrogen, fluorine, cyano, C₁₋₄ alkyl, C₃₋₄ cycloalkyl, 3- to 4-membered aliphatic heterocyclyl, and -OR^{a1}.

In one embodiment, R¹ is selected from: hydrogen, fluorine, cyano, methyl, ethyl, 1-propyl, 2-propyl, cyclopropyl, methoxy, ethoxy, cyclopropoxy and 2-propoxy.

In one embodiment, R¹ is hydrogen.

In one embodiment, R^{a1}, R^{b1} and R^{e1} are each independently selected from: hydrogen, C₁₋₃ alkyl, and C₃₋₄ cycloalkyl;
or, R^{a1} and R^{b1} attached to the same nitrogen atom, taken together with the nitrogen atom to which they are attached, form a 4- to 6-membered aliphatic heterocyclyl.

In one embodiment, R^{a1}, R^{b1} and R^{e1} are each independently selected from: hydrogen, methyl, ethyl, and cyclopropyl.

In one embodiment, R¹¹ is selected from oxo, halogen, hydroxyl, amino, cyano, C₁₋₃ alkyl, C₁₋₃ alkoxy, C₁₋₃ alkylamino, C₃₋₆ cycloalkyl and C₃₋₆ cycloalkoxy.

In one embodiment, R¹¹ is selected from halogen, hydroxyl, cyano, C₁₋₃ alkoxy, cyclopropoxy, C₁₋₃ alkyl, and cyclopropyl.

In one embodiment, R¹¹ is selected from fluorine, hydroxyl, cyano, methoxy, ethoxy, cyclopropoxy, methyl, ethyl, and cyclopropyl.

In one embodiment, L¹ is single bond.

In one embodiment, L¹ is selected from: 6- to 10-membered aryl, and 5- to 10-memebred heteroaryl; said aryl or heteroaryl group is connected to the azaindazole ring at the 3-position and to the ring A, respectively; in addition to being connected to the azaindazole ring and to the ring A, said aryl and heteroaryl group is optionally substituted with 0, 1, 2, 3, 4, 5 or 6 of R⁴.

In one embodiment, L¹ is selected from: phenyl, 5- to 6-memebred heteroaryl, and 9- to 10-memebred bicyclic heteroaryl; said aryl or heteroaryl group is connected to the azaindazole ring at the 3-position and to the ring A through two nonadjacent ring-forming atoms respectively; in addition to being connected to the azaindazole ring and to the ring A, said aryl and heteroaryl group is optionally substituted with 0, 1, 2, 3, 4, 5 or 6 of R⁴.

In one embodiment, L¹ is selected from: phenyl, and 5- to 6-memebred heteroaryl; said aryl or heteroaryl group is connected to the azaindazole ring at the 3-position and to the ring A, respectively; in addition to being connected to the azaindazole ring and to the ring A, said aryl and heteroaryl group is optionally substituted with 0, 1, 2, or 3 of R⁴.

In one embodiment, L¹ is other than single bond, ring A is selected from monocyclic or bicyclic 3- to 12-membered carbocyclyl group and 3- to 12-membered heterocyclyl group, and is connected to L¹ and L² through two different atoms thereof, respectively:
1) said monocyclic or bicyclic structure does not contain aromatic ring;
2) said monocyclic or bicyclic carbocyclyl group and heterocyclyl group contains at least one aromatic ring.

In one embodiment, L¹ is other than single bond, ring A is selected from monocyclic or bicyclic 5- to 11-membered carbocyclyl group and 5- to 11-membered heterocyclyl group, and is connected to L¹ and L² through two different atoms thereof, respectively:
1) said monocyclic or bicyclic structure does not contain aromatic ring;
2) said monocyclic or bicyclic carbocyclyl group and heterocyclyl group contains at least one aromatic ring.

In one embodiment, L¹ is other than single bond, ring A is selected from monocyclic or bicyclic C₅, C₆, C₇, C₈, and C₉ carbocyclyl group and 5-, 6-, 7-, 8-, and 9-membered heterocyclyl group, and is connected to L¹ and L² through two different atoms thereof, respectively:
1) said monocyclic or bicyclic structure does not contain aromatic ring;
2) said monocyclic or bicyclic carbocyclyl group and heterocyclyl group contains at least one aromatic ring.

In one embodiment, L¹ is other than single bond, ring A is selected from monocyclic or bicyclic C₅, C₆, C₇, C₈, and C₉ carbocyclyl group and 5-, 6-, 7-, 8-, and 9-membered heterocyclyl group, and is connected to L¹ and L² through two different atoms thereof respectively, wherein said monocyclic or bicyclic structure does not contain aromatic ring.

In one embodiment, L¹ is other than single bond, ring A is selected from phenyl and 5- to 6-membered monocyclic heterocyclyl group, and is connected to L¹ and L² through two different atoms thereof, respectively.

In one embodiment, L¹ is other than single bond, ring A is selected from 6- to 11-membered bicyclic carbocyclyl group and 6- to 11-membered bicyclic heterocyclyl group, and is connected to L¹ and L² through two different atoms respectively, wherein said bicyclic group contains at least one aromatic ring.

In one embodiment, ring B is selected from phenyl, naphthyl, 5- to 6-membered monocyclic heteroaryl, and 9- to 10-membered bicyclic heteroaryl, and is connected to L² and to the azaindazole ring at the 5-position through two different ring-forming atoms thereof, respectively:
1) When ring B is selected from phenyl and 5- to 6-membered monocyclic heteroaryl, the shortest distance between said two ring-forming atoms is 1, 2 or 3 chemical bonds; preferably, the shortest distance between said two ring-forming atoms is 1 or 2 chemical bonds;
2) When ring B is selected from naphthyl and 9- to 10-membered bicyclic heteroaryl, the shortest distance between said two ring-forming atoms is 1, 2, 3, 4 or 5 chemical bonds; preferably, the shortest distance between said two ring-forming atoms is 1, 2, 3 or 4 chemical bonds.

In one embodiment, ring B is selected from phenyl and 5- to 6-membered monocyclic heteroaryl, and is connected to L² and to the azaindazole ring at the 5-position through two different ring-forming atoms thereof, respectively; wherein the shortest distance between said two ring-forming atoms is 1, 2 or 3 chemical bonds; preferably, the shortest distance between said two ring-forming atoms is 1 or 2 chemical bonds.

In one embodiment, ring B is selected from naphthyl and 9- to 10-membered bicyclic heteroaryl, and is connected to L² and to the azaindazole ring at the 5-position through two different ring-forming atoms thereof, respectively; wherein the shortest distance between said two ring-forming atoms is 1, 2, 3, 4 or 5 chemical bonds; preferably, the shortest distance between said two ring-forming atoms is 1, 2, 3 or 4 chemical bonds.

In one embodiment, ring B is selected from naphthyl and 9- to 10-membered bicyclic heteroaryl, and is connected to L² and to the azaindazole ring at the 5-position through two different ring-forming atoms thereof, respectively; wherein the shortest distance between said two ring-forming atoms is 1, 2 or 3 chemical bonds; preferably, the shortest distance between said two ring-forming atoms is 1 or 2 chemical bonds.

In one embodiment, ring B is selected from naphthyl and 9- to 10-membered bicyclic heteroaryl, and is connected to L² and to the azaindazole ring at the 5-position through two different ring-forming atoms thereof, respectively; wherein the shortest distance between said two ring-forming atoms is 3, 4 or 5 chemical bonds; preferably, the shortest distance between said two ring-forming atoms is 3 or 4 chemical bonds.

In one embodiment, ring B is selected from bridged C₉₋₁₂ bicyclic carbocyclyl and bridged 8- to 12-membered bicyclic heterocyclyl, said bicyclic carbocyclyl or bicyclic heterocyclyl is formed by one monocyclic aryl group and one monocyclic alicyclic group; and is connected to L² and to the azaindazole ring at the 5-position through two different ring-forming atoms thereof, respectively; wherein said monocyclic aryl group is selected from phenyl and 5- to 6-membered heteroaryl; wherein said monocyclic alicyclic group is selected from C₅₋₇ monocyclic carbocyclyl and 5- to 7-membered monocyclic aliphatic heterocyclyl; wherein the ring-forming atom connected to the azaindazole ring at the 5-position belongs to said monocyclic aryl group; wherein the ring-forming atom connected to L² belongs to said monocyclic alicyclic group; wherein the shortest distance between said two ring-forming atoms is 3, 4 or 5 chemical bonds; preferably, the shortest distance between said two ring-forming atoms is 3 or 4 chemical bonds.

In one embodiment, ring B is selected from phenyl and pyridyl, and is connected to L² and to the azaindazole ring at the 5-position through two different ring-forming atoms thereof, respectively; wherein said two ring-forming atoms are in *ortho-, meta-* or *para*-position relationship; preferably, said two ring-forming atoms are in *ortho-* or *meta*-position relationship.

In one embodiment, ring B is selected from phenyl and pyridyl, and is connected to L² and to the azaindazole ring at the 5-position through two different ring-forming atoms thereof, respectively; wherein said two ring-forming atoms are in *ortho*-position relationship.

In one embodiment, ring B is selected from phenyl, and is connected to L² and to the azaindazole ring at the 5-position through two different ring-forming atoms thereof, respectively; wherein said two ring-forming atoms are in *ortho-, meta-* or *para*-position relationship; preferably, said two ring-forming atoms are in *ortho-* or *meta*-position relationship.

In one embodiment, ring B is selected from phenyl, and is connected to L² and to the azaindazole ring at the 5-position through two different ring-forming atoms thereof, respectively; wherein said two ring-forming atoms are in *ortho*-position relationship.

In one embodiment, L¹ is selected as single bond, therefore, the compound in the present application has the structure of Formula (II), 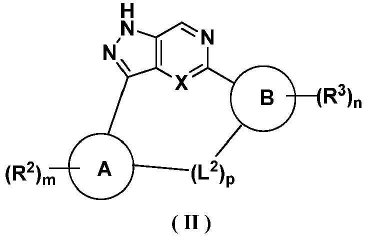
wherein, ring A is selected from monocyclic or bicyclic 3- to 12-membered carbocyclyl group and 3- to 12-membered heterocyclyl group, and is connected to the azaindazole ring at the 3-position and to L² through two different atoms respectively; in addition to being connected to the azaindazole ring at the 3-position and to L², ring A is optionally substituted with m of R²;
X, ring B, R², R³, L², p, m, and n are as defined herein.

In one embodiment, ring A is selected from monocyclic or bicyclic C₆₋₁₂ carbocyclyl group and 5- to 12-membered heterocyclyl group, and is connected to the azaindazole ring at the 3-position and to L² through two different ring-forming atoms respectively, and the single ring directly connected to the azaindazole ring at the 3-position is aromatic ring; wherein,
1) when ring A is selected from phenyl and monocyclic heteroaryl, the shortest distance between the two ring-forming atoms on ring A attached to the azaindazole ring at the 3-position and to L² respectively, is 1, 2 or 3 chemical bonds; preferably, the shortest distance between said two ring-forming atoms is 1 or 2 chemical bonds;
2) when ring A is selected from bicyclic carbocyclyl and bicyclic heterocyclyl, the shortest distance between the two ring-forming atoms on ring A attached to the azaindazole ring at the 3-position and to L² respectively, is 1, 2, 3, 4 or 5 chemical bonds; preferably, the shortest distance between said two ring-forming atoms is 1, 2, 3 or 4 chemical bonds.

In one embodiment, ring B is selected from phenyl, naphthyl, 5- to 6-membered monocyclic heteroaryl, and 9- to 10-membered bicyclic heteroaryl, and is connected to L² and to the azaindazole ring at the 5-position through two different ring-forming atoms thereof, respectively:
1) when ring B is selected from phenyl and 5- to 6-membered monocyclic heteroaryl, the shortest distance between said two ring-forming atoms is 1, 2 or 3 chemical bonds;
2) when ring B is selected from naphthyl and 9- to 10-membered bicyclic heteroaryl, the shortest distance between said two ring-forming atoms is 1, 2, 3, 4 or 5 chemical bonds.

In one embodiment, ring B is selected from phenyl and 5- to 6-membered monocyclic heteroaryl, and is connected to L² and to the azaindazole ring at the 5-position through two different ring-forming atoms thereof, respectively; wherein the shortest distance between said two ring-forming atoms is 1, 2 or 3 chemical bonds.

In one embodiment, R² is each independently selected from:
1) halogen, cyano, hydroxyl, C₁₋₄ alkoxy, amino, C₁₋₄ alkylamino, and C₁₋₃ alkyl sulfonyl; or
2) C₁₋₄ alkyl, C₂₋₄ alkenyl, C₂₋₄ alkynyl, phenyl, 5- to 6-membered heteroaryl, C₃₋₆ cycloalkyl and 3- to 8-membered aliphatic heterocyclyl, optionally substituted with 0, 1, 2, 3 or 4 of R²²; or
3) C₁₋₅ alkylene, 3- to 6-membered oxaalkylene, and 2- to 6-membered azaalkylene, optionally substituted with 0, 1, 2, 3 or 4 of R²².
R²² is each independently selected from:
1) oxo, halogen, cyano, hydroxyl, C₁₋₄ alkoxy, amino, C₁₋₄ alkylamino, C₁₋₃ alkyl sulfonyl, and carboxyl; or
2) C₁₋₄ alkyl, C₂₋₄ alkenyl, C₂₋₄ alkynyl, C₃₋₆ cycloalkyl and 3- to 8-membered aliphatic heterocyclyl, optionally substituted with 0, 1, 2, 3 or 4 substituents independently selected from: oxo, halogen, hydroxyl, hydroxy-C₁₋₃ alkyl, C₁₋₃ alkoxy-C₁₋₃ alkyl, cyano, C₁₋₃ alkoxy, amino, C₁₋₃ alkylamino, amino-C₁₋₃ alkyl, and C₁₋₃ alkylamino-C₁₋₃ alkyl; or
3) C₁₋₅ alkylene, 3- to 6-membered oxaalkylene, and 2- to 6-membered azaalkylene, optionally substituted with 0, 1, 2, 3 or 4 substituents independently selected from: oxo, halogen, hydroxyl, hydroxy-C₁₋₃ alkyl, C₁₋₃ alkoxy-C₁₋₃ alkyl, cyano, C₁₋₃ alkoxy, amino, C₁₋₃ alkylamino, amino-C₁₋₃ alkyl, and C₁₋₃ alkylamino-C₁₋₃ alkyl.

In one embodiment, R³ is independently selected from:
1) halogen, cyano, -C(=O)NR^{a3}R^{b3} and -OR^{a3}; or
2) C₁₋₃ alkyl, C₂₋₆ alkynyl, phenyl, 5- to 6-membered heteroaryl, C₃₋₆ cycloalkyl and 3- to 6-membered aliphatic heterocyclyl, optionally substituted with 0, 1, 2, 3 or 4 of R³²;

wherein, R^{a3} and R^{b3} are each independently selected from:
   1) hydrogen; or
   2) C₁₋₄ alkyl, C₃₋₆ cycloalkyl and 4- to 6-membered aliphatic heterocyclyl, optionally substituted with 0, 1, 2, 3 or 4 of R³²;
or, R^{a3} and R^{b3} attached to the same nitrogen atom, taken together with the nitrogen atom to which they are attached, form a 4- to 6-membered aliphatic heterocyclyl, optionally substituted with 0, 1, 2, 3 or 4 of R³²;
R³² is each independently selected from:
   1) oxo, halogen, cyano, hydroxyl, C₁₋₄ alkoxy, amino, and C₁₋₄ alkylamino; or
   2) C₁₋₄ alkyl, C₃₋₆ cycloalkyl and 3- to 6-membered aliphatic heterocyclyl, optionally substituted with 0, 1 or 2 substituents independently selected from: oxo, halogen, hydroxyl, hydroxymethyl, cyano, C₁₋₃ alkoxy, amino, and C₁₋₃ alkylamino.

In one embodiment, L² is each independently selected from -CH₂-, -NH-, -O-, and -S-; wherein, each L² is independently substituted with 0, 1, or 2 of R⁵; two adjacent L² may be connected through single bond; p of L² connected sequentially form a linker connecting ring A and ring B;
when L² is selected from -CH₂-, the R⁵ thereof is each independently selected from:
   1) oxo, halogen, cyano, -C(=O)R^{a5}, carboxyl, -C(=O)NR^{a5}R^{b5}, -OR^{a5}, -NR^{a5}R^{b5}, -NR^{a5}C(=O)R^{b5}, -NR^{e5}C(=O)NR^{a5}R^{b5}, -NR^{a5}S(=O)₂R^{c5}, -NR^{e5}S(=O)₂NR^{a5}R^{b5}, and -NR^{G2}P(=O)(R^{G1})₂; or
   2) C₁₋₄ alkyl, C₃₋₆ cycloalkyl and 3- to 6-membered aliphatic heterocyclyl, optionally substituted with 0, 1, 2, 3 or 4 of R⁵²; or
   3) two R⁵ groups attached to the same L², taken together with the L² atom to which they are attached, form a cyclic group selected from: 1,1-cyclopropyl, 1,1-cyclobutyl, 1,1-cyclopentyl, 1,1-cyclohexyl, 3,3-oxetanyl, 3,3-azetidinyl, 3,3-tetrahydrofuryl, 3,3-tetrahydropyrrolyl, 3,3-tetrahydropyranyl, 4,4-tetrahydropyranyl, 3,3-piperidinyl and 4,4-piperidyl, optionally substituted with 0, 1, 2, 3, or 4 of R⁵²; or
   4) two R⁵ groups attached to two adjacent L², taken together with the two L² atoms to which they are attached, form a cyclic group selected from: 1,2-cyclopropyl, 1,2-cyclobutyl, 1,2-cyclopentyl, 1,2-cyclohexyl, 3,4-tetrahydrofuryl, 3,4-tetrahydropyrrolyl, 3,4-tetrahydropyranyl and 3,4-piperidinyl, optionally substituted with 0, 1, 2, 3, or 4 of R⁵²; or
   5) two R⁵ groups attached to two nonadjacent L², taken together with the two L² atoms to which they are attached and all other L² atom(s) between said two L² atoms, form a cyclic group selected from: 1,3-cyclobutyl, 1,3-cyclopentyl, 1,3-cyclohexyl, 1,4-cyclohexyl, 2,5-tetrahydrofuryl, 2,5-tetrahydropyrrolyl, 2,6-tetrahydropyranyl, 3,5-tetrahydropyranyl, 2,6-piperidinyl, 3,5-piperidinyl, 2,6-morpholinyl, 2,5-morpholinyl, 3,5-morpholinyl, 2,6-piperazinyl and 2,5-piperazinyl, optionally substituted with 0, 1, 2, 3, or 4 of R⁵²;
when L² is selected from -NH-, the R⁵ thereof is each independently selected from:
   1) -C(=O)R^{a5}, -C(=O)NR^{a5}R^{b5}, -C(=NR^{d5})NR^{a5}R^{b5}, -S(=O)₂R^{c5}, -S(=O)₂NR^{a5}R^{b5}, -S(=O)(=NR^{d5})R^{c5} and -P(=O)(R^{G1})₂; or
   2) C₁₋₄ alkyl, C₃₋₆ cycloalkyl and 3- to 6-membered aliphatic heterocyclyl, optionally substituted with 0, 1, 2, 3 or 4 of R⁵²; or
   3) two R⁵ groups attached to two adjacent L², taken together with the two L² atoms to which they are attached, form a cyclic group selected from: 1,2-aziridinyl, 1,2-azetidinyl, 1,2-tetrahydropyrrolyl, 1,2-piperidinyl, 3,4-morpholinyl and 1,2-piperanyl, optionally substituted with 0, 1, 2, 3, or 4 of R⁵²; or
   4) two R⁵ groups attached to two nonadjacent L², taken together with the two L² atoms to which they are attached and all other L² atom(s) between said two L² atoms, form a cyclic group selected from: 1,3-azetidinyl, 1,3-tetrahydropyrrolyl, 1,3-piperidinyl, 1,4-piperidinyl and 1,4-piperazinyl, optionally substituted with 0, 1, 2, 3, or 4 of R⁵²; or
when L² is selected from -S-, the R⁵ thereof is each independently selected from oxo;
wherein, R^{a5}, R^{b5} and R^{e5} are independently selected from:
   1) hydrogen; or
   2) C₁₋₃ alkyl, C₃₋₆ cycloalkyl, 3-oxetanyl, 3-azetidinyl, tetrahydrofuryl, tetrahydropyrrolyl, tetrahydropyranyl and piperidinyl, optionally substituted with 0, 1, 2, 3 or 4 of R⁵²;
or, R^{a5} and R^{b5} attached to the same nitrogen atom, taken together with the nitrogen atom to which they are attached, form a cyclic group selected from: 1-azetidinyl, 1-tetrahydropyrrolyl, 1-piperidinyl, 1-piperazinyl and morpholinyl, optionally substituted with 0, 1, 2, 3 or 4 of R⁵²;
R^{c5} is each independently selected from C₁₋₃ alkyl, C₃₋₆ cycloalkyl, 3-oxetanyl, 3-azetidinyl, tetrahydrofuryl, tetrahydropyrrolyl, tetrahydropyranyl and piperidinyl, optionally substituted with 0, 1, 2, 3 or 4 of R⁵²;
R^{d5} is selected from:
   1) hydrogen, cyano, nitro and -S(=O)₂-(C₁₋₃ alkyl); or
   2) C₁₋₃ alkyl and cyclopropyl;
R⁵² is selected from: oxo, fluorine, cyano, hydroxyl, C₁₋₃ alkoxy, cyclopropoxy, C₁₋₃ alkyl, cyclopropyl, difluoromethyl, trifluoromethyl, hydroxymethyl, 1-hydroxyethyl, 2-hydroxyethyl, 2-hydroxy-2-propyl, amino and C₁₋₃ alkylamino;
R^{G1} is selected from -OR^{G2} and -N(R^{G2})₂;
R^{G2} is selected from:
   1) hydrogen; or
   2) C₁₋₄ alkyl, C₁₋₄ alkylene, C₃₋₆ cycloalkyl, 3- to 6-membered aliphatic heterocyclyl and phenyl, optionally substituted with 0, 1, 2 or 3 substituents independently selected from: oxo, halogen, hydroxyl, hydroxymethyl, carboxyl, cyano, C₁₋₃ alkoxy, amino, and C₁₋₃ alkylamino; or
   3) two R^{G2} attached to the same nitrogen atom, taken together with the nitrogen atom to which they are attached, form a 3- to 6-membered aliphatic heterocyclyl, optionally substituted with 0, 1, 2 or 3 substituents independently selected from: oxo, halogen, hydroxyl, hydroxymethyl, carboxyl, cyano, C₁₋₃ alkoxy, amino, and C₁₋₃ alkylamino;
R^{G3} is selected from: hydrogen, cyano, nitro, -OR^{G2}, -S(=O)₂R^{G2} and R^{G2}.

In one embodiment, L¹ is selected as single bond, the compound described herein has the structure of Formula (IIa), 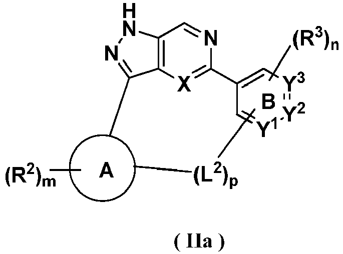
X is selected from CH and N;
ring A is selected from phenyl and 5- to 6-membered heteroaryl, and is connected to the azaindazole ring at the 3-position and to L² through two different ring-forming atoms respectively; in addition to being connected to the azaindazole ring at the 3-position and to L², ring A is optionally substituted with m of R²;
ring B is connected to L² and to the azaindazole ring at the 5-position through two different ring-forming atoms respectively; wherein, the shortest distance between said two ring-forming atoms is 1, 2 or 3 chemical bonds; wherein, Y¹, Y² and Y³ are each independently selected from CR³ and N, provided that no more than two of Y¹, Y² and Y³ is selected from N;
m is selected from 0, 1, 2, 3 and 4;
n is selected from 0, 1, 2 and 3;
R², R³, L² and p are as defined herein.

In one embodiment, the compound described herein has the structure of Formula (IIb), 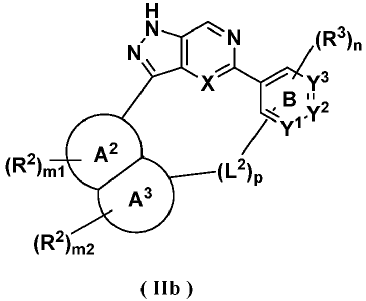 wherein,
X is selected from CH and N;
ring A² and ring A³ form a fused ring system, wherein: ring A² is selected from phenyl and 5- to 6-membered heteroaryl, and is connected to the azaindazole ring at the 3-position through a ring-forming atom thereof; ring A³ is selected from C₅, C₆, and C₇ monocyclic carbocyclyl and 5-, 6- and 7-membered monocyclic heterocyclyl, and is connected to L² through a ring-forming atom thereof; and in addition to being connected to the azaindazole ring at the 3-position, ring A² is optionally substituted with m1 of R²; and in addition to being connected to L², ring A³ is unsubstituted or optionally substituted with m2 of R²;
ring B is connected to L² and to the azaindazole ring at the 5-position through two different ring-forming atoms respectively; wherein, the shortest distance between said two ring-forming atoms is 1, 2 or 3 chemical bonds; wherein, Y¹, Y² and Y³ are each independently selected from CR³ and N, provided that no more than two of Y¹, Y² and Y³ is selected from N;
m1 and m2 is selected from integers between 0 and 4; the sum of m1 and m2 is m, and m is selected from 0, 1, 2, 3 and 4;
n is selected from 0, 1, 2 and 3;
R², R³, L² and p are as defined herein.

In one embodiment, the compound described herein has the structure of Formula (IIc), 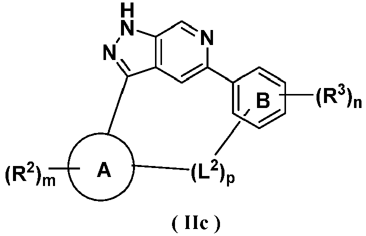 wherein, ring A, R², R³, L², p, m, and n are as defined herein.

In one embodiment, in Formula (IIa) and Formula (IIc), ring A is selected from phenyl and 5- to 6-membered mono-heteroaryl, and is connected to the azaindazole ring at the 3-position and to L² through two different ring-forming atoms respectively; wherein the shortest distance between said two ring-forming atoms is 1 or 2 chemical bonds; preferably, the shortest distance between said two ring-forming atoms is 2 chemical bonds.

In one embodiment, in Formula (IIa) and Formula (IIc), ring A is selected from naphthyl and 9- to 10-membered bicyclic heteroaryl, and is connected to L¹ and L² through two different ring-forming atoms respectively; wherein the shortest distance between said two ring-forming atoms is 1, 2, 3, 4 or 5 chemical bonds; preferably, the shortest distance between said two ring-forming atoms is 1, 2, 3 or 4 chemical bonds.

In one embodiment, in Formula (IIa) and Formula (IIc), ring A is selected from naphthyl and 9- to 10-membered bicyclic heteroaryl, and is connected to L¹ and L² through two different ring-forming atoms respectively; wherein all said ring-forming atoms connected to L¹ and L² belong to the same monocyclic aryl group; wherein the shortest distance between said two ring-forming atoms is 1, 2 or 3 chemical bonds; preferably, the shortest distance between said two ring-forming atoms is 1 or 2 chemical bonds.

In one embodiment, in Formula (IIa) and Formula (IIc), ring A is selected from bridged C₉₋₁₂ bicyclic carbocyclyl and bridged 8- to 12-membered bicyclic heterocyclyl, said bicyclic carbocyclyl orbicyclic heterocyclyl is formed by one monocyclic aryl group and one monocyclic alicyclic group; and is connected to L¹ and L² through two different ring-forming atoms thereof, respectively; wherein said monocyclic aryl group is selected from phenyl and 5-to 6-membered heteroaryl; wherein said monocyclic alicyclic group is selected from C₅₋₇ monocyclic carbocyclyl and 5- to 7-membered monocyclic aliphatic heterocyclyl; wherein all said ring-forming atoms connected to L¹ and L² belong to the same monocyclic aryl group; wherein the shortest distance between said two ring-forming atoms is 1, 2 or 3 chemical bonds; preferably, the shortest distance between said two ring-forming atoms is 1 or 2 chemical bonds.

In some embodiments, in Formula (IIa) and Formula (IIc), ring A is selected from phenyl and 5- to 6-membered mono-heteroaryl, and is connected to the azaindazole ring at the 3-position and to L² through two different ring-forming atoms respectively; wherein the shortest distance between said two ring-forming atoms is 1 or 2 chemical bonds; preferably, the shortest distance between said two ring-forming atoms is 2 chemical bonds.

ring B is selected from phenyl, and, Y¹, Y² and Y³ are each independently selected from CR³; and ring B is connected to L² and to the azaindazole ring at the 5-position through two different ring-forming atoms respectively; wherein said two ring-forming atoms are in *ortho-, meta-* or *para*-position relationship.

In some embodiments, in Formula (IIb), the fused ring system formed of ring A² and ring A³ is selected from naphthyl and 9- to 10-membered bicyclic heteroaryl, wherein, ring A² is connected to the azaindazole ring at the 3-position through one ring-forming atom, ring A³ is connected to L² through one ring-forming atom; and in addition to being connected to the azaindazole ring at the 3-position, ring A² is optionally and independently substituted with m1 of R²; and in addition to being connected to L², ring A³ is unsubstituted or optionally and independently substituted with m2 of R²; wherein, the shortest distance between said two ring-forming atoms is 3, 4 or 5 chemical bonds; preferably, the shortest distance between said two ring-forming atoms is 3 or 4 chemical bonds.

In some embodiments, in Formula (IIb), ring A² is selected from phenyl and 5- to 6-membered heteroaryl, and is connected to the azaindazole ring at the 3-position through one ring-forming atom; ring A³ is selected from C₅, C₆, and C₇ monocyclic carbocyclyl and 5-, 6-and 7-membered monocyclic heterocyclyl, and is connected to L² through one ring-forming atom; wherein, the shortest distance between said two ring-forming atoms is 3, 4 or 5 chemical bonds; preferably, the shortest distance between said two ring-forming atoms is 3 or 4 chemical bonds.

In some embodiments, in Formula (IIb), ring A² is selected from phenyl and 5- to 6-membered heteroaryl, and is connected to the azaindazole ring at the 3-position through one ring-forming atom; ring A³ is selected from C₅, C₆, and C₇ monocyclic carbocyclyl and 5-, 6-and 7-membered monocyclic heterocyclyl, and is connected to L² through one ring-forming atom; and in addition to being connected to the azaindazole ring at the 3-position, ring A² is optionally and independently substituted with m1 of R²; and in addition to being connected to L², ring A³ is unsubstituted or optionally and independently substituted with m2 of R².

In some embodiments, in Formula (IIa) and Formula (IIb), ring B is phenyl, and, Y¹, Y² and Y³ are each independently selected from CR³; and ring B is connected to L² and to the azaindazole ring at the 5-position through two different ring-forming atoms respectively; wherein said two ring-forming atoms are in *ortho-, meta-* or para-position relationship.

In some embodiments, ring A is selected from one of the following structures: 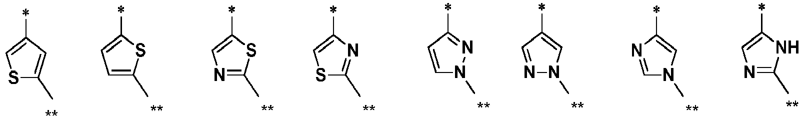 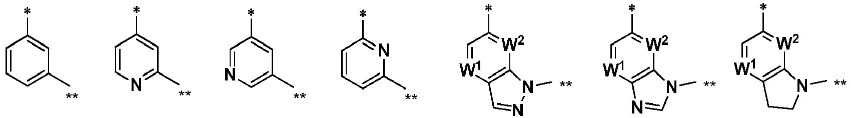 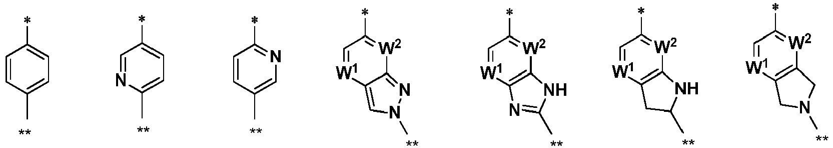 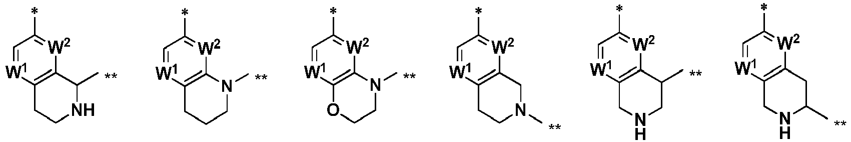 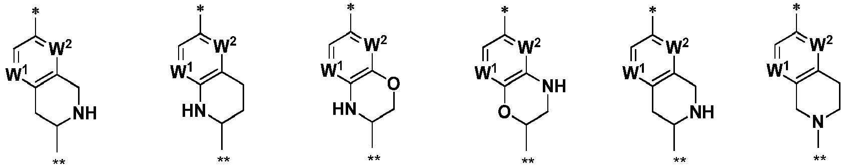 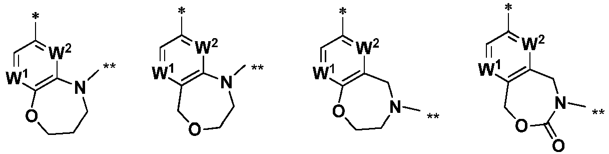 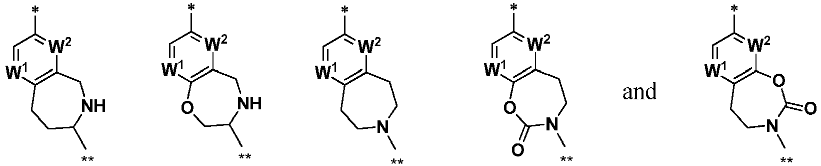
wherein, the "*" at the end of the chemical bond in each structure means that the structure is connected to the azaindazole ring at the 3-position through the bond, the "**" at the end of the chemical bond in each structure means that the structure is connected to L² through the bond;
W¹ and W² are each independently selected from CH and N;
the ring-forming carbon atom(s) and/or ring-forming nitrogen atom(s) in these formulas is optionally substituted with 0, 1, 2, 3 or 4 of R².

In some embodiments, ring A is a single ring selected from one of the following structures: 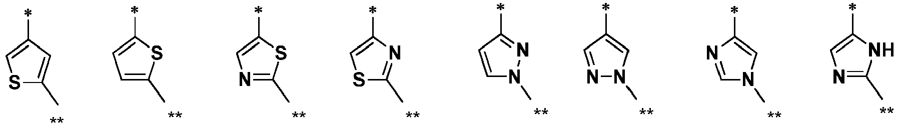 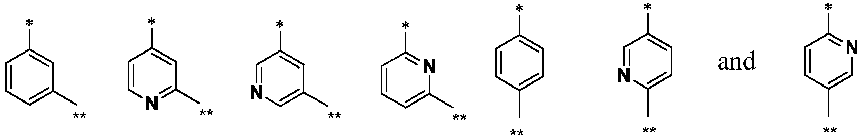
wherein, the "*" at the end of the chemical bond in each structure means that the structure is connected to the azaindazole ring at the 3-position through the bond, the "**" at the end of the chemical bond in each structure means that the structure is connected to L² through the bond;
the ring-forming carbon atom(s) in these formulas is optionally substituted with 0, 1 or 2 of R².

In some embodiments, the fused ring system formed of ring A² and ring A³ is selected from one of the following structures: 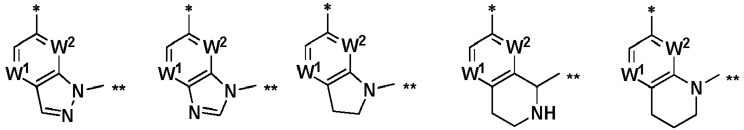 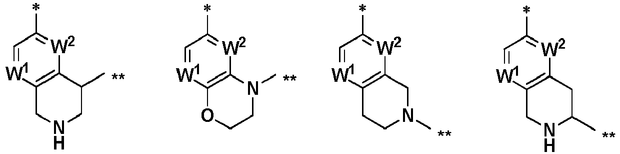 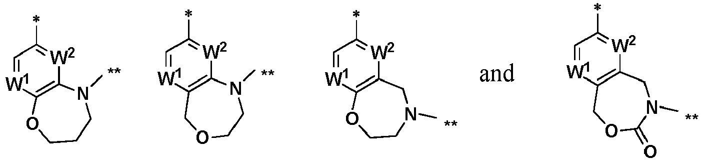
wherein, the "*" at the end of the chemical bond in each structure means that the structure is connected to the azaindazole ring at the 3-position through the bond, the "**" at the end of the chemical bond in each structure means that the structure is connected to L² through the bond;
W¹ and W² are each independently selected from CH and N;
the ring-forming carbon atom(s) and/or ring-forming nitrogen atom(s) in these formulas is optionally substituted with 0, 1, 2, 3 or 4 of R².

In some embodiments, ring B is selected from phenyl and 6-membered heteroaryl.

In one embodiment, the compound described herein has the structure of Formula (IIIb), 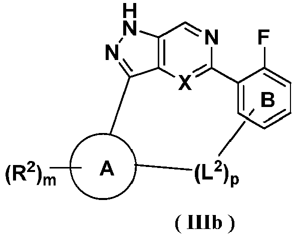 wherein,
X is selected from N and CH;
ring A is selected from phenyl and pyridyl, and is connected to the azaindazole ring at the 3-position and to L² through two different ring-forming atoms respectively; wherein the shortest distance between said two ring-forming atoms is 1 or 2 chemical bonds; preferably, the shortest distance between said two ring-forming atoms is 2 chemical bonds;
ring B is selected from phenyl, and is connected to L² and to the azaindazole ring at the 5-position through two different ring-forming atoms respectively; wherein said two ring-forming atoms are in *ortho-* or meta-position relationship; preferably, said two ring-forming atoms are in *ortho*-position relationship;
R², L², p, and m are as defined herein.

In some embodiments, R² is each independently selected from:
1) oxo, fluorine, chlorine, cyano, hydroxyl, methoxy, ethoxy, isopropoxy, cyclopropoxy, methyl, ethyl, 1-propyl, isopropyl, cyclopropyl, difluoromethyl, trifluoromethyl, hydroxymethyl, 1-hydroxyethyl, 2-hydroxyethyl, 2-hydroxy-2-propyl, 2-amino-2-propyl, *N*-methyl-2-amino-2-propyl, *N*,N-dimethyl-2-amino-2-propyl, and carboxyl; or
2) one of the following aliphatic heterocyclyl, wherein the "*" at the end of the chemical bond in each structure means that the structure is connected to the ring A through the bond: 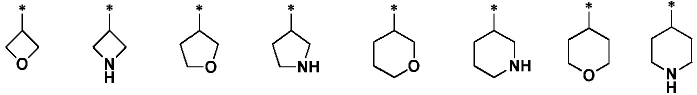 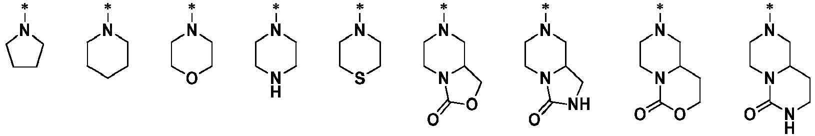 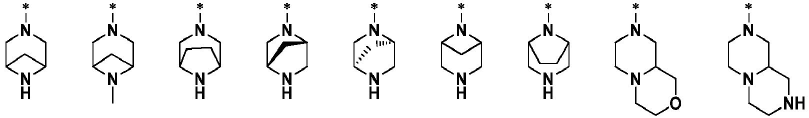 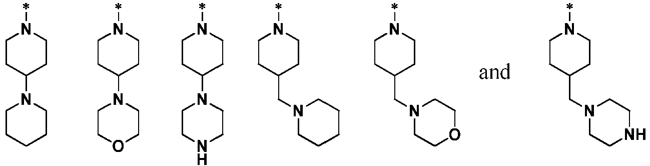 wherein, said aliphatic heterocyclyl is optionally substituted with 0, 1, 2 or 3 of substituent R²²¹ independently selected from: oxo, fluorine, chlorine, cyano, hydroxyl, C₁-C₆ alkoxy, C₁-C₆ alkylamino, C₁-C₆ alkyl, C₁-C₆ fluoroalkyl, C₁-C₆ hydroxyalkyl, C₁-C₆ aminoalkyl, C₃-C₆ cycloalkyl, C₃-C₆ oxacycloalkyl, -C(=O)-(C₁-C₆ alkyl), and -S(=O)₂-(C₁-C₆ alkyl); or
3) ethylene, propylene, butylene, pentylene, oxapropylene, oxabutylene, oxapentylene, azapropylene, azabutylene and azapentylene, optionally substituted with 0, 1 or 2 substituents selected from fluorine, hydroxyl and methyl; or
4) phenyl, pyridyl, pyrimidinyl, imidazolyl, pyrazolyl and thiazolyl, optionally substituted with 0, 1, 2 or 3 substituents independently selected from: oxo, fluorine, chlorine, cyano, hydroxyl, C₁-C₆ alkoxy, C₁-C₆ alkyl, C₁-C₆ fluoroalkyl, C₁-C₆ hydroxyalkyl, C₁-C₆ aminoalkyl, C₃-C₆ cycloalkyl, and C₃-C₆ oxacycloalkyl;
m is selected from 0, 1 and 2.

In one embodiment, in Formula (IIIb), R² is each independently selected from:
1) fluorine, chlorine, cyano, hydroxyl, methoxy, ethoxy, isopropoxy, cyclopropoxy, methyl, ethyl, 1-propyl, isopropyl, cyclopropyl, difluoromethyl, trifluoromethyl, hydroxymethyl, 1-hydroxyethyl, 2-hydroxyethyl, 2-hydroxy-2-propyl, 2-amino-2-propyl, *N*-methyl-2-amino-2-propyl, and *N,N-*dimethyl-2-amino-2-propyl; or
2) one of the following aliphatic heterocyclyl, wherein the "*" at the end of the chemical bond in each structure means that the structure is connected to the ring A through the bond: 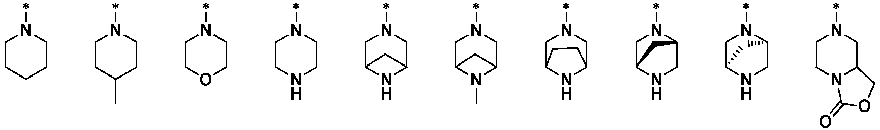 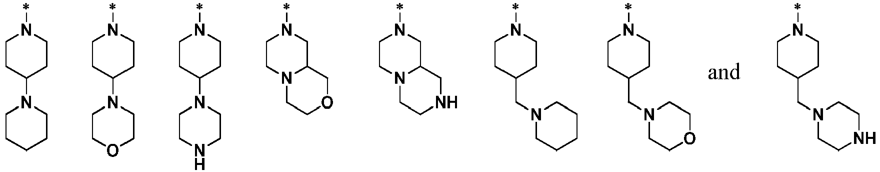 wherein, said aliphatic heterocyclyl is optionally substituted with 0, 1, 2 or 3 of substituent R²²¹ independently selected from: oxo, fluorine, chlorine, cyano, hydroxyl, C₁-C₆ alkoxy, C₁-C₆ alkylamino, C₁-C₆ alkyl, C₁-C₆ fluoroalkyl, C₁-C₆ hydroxyalkyl, C₁-C₆ aminoalkyl, C₃-C₆ cycloalkyl, C₃-C₆ oxacycloalkyl, -C(=O)-(C₁-C₆ alkyl), and -S(=O)₂-(C₁-C₆ alkyl).

In one embodiment, in Formula (IIIb), R²²¹ is each independently selected from: oxo, fluorine, chlorine, cyano, hydroxyl, methoxy, ethoxy, isopropoxy, cyclopropoxy, dimethylamino, methyl, ethyl, 1-propyl, isopropyl, cyclopropyl, difluoromethyl, trifluoromethyl, hydroxymethyl, 1-hydroxyethyl, 2-hydroxyethyl, 2-hydroxy-2-propyl, 2-amino-2-propyl, *N*-methyl-2-amino-2-propyl, *N,N*-dimethyl-2-amino-2-propyl, oxetanyl and tetrahydropyranyl.

In one embodiment, the compound described herein has the structure of Formula (IIIa), 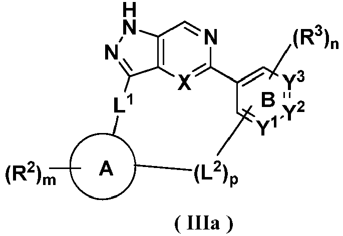 wherein,
X is selected from CH and N;
L¹ is selected from: phenyl, 5- to 6-memebred heteroaryl, and 9- to 10-memebred bicyclic heteroaryl; said aryl or heteroaryl group is connected to the azaindazole ring at the 3-position and to the ring A through two nonadjacent ring-forming atoms respectively; in addition to being connected to the azaindazole ring and to the ring A, said aryl and heteroaryl group is optionally substituted with 0, 1, 2, 3, 4, 5 or 6 of R⁴;
ring A is selected from monocyclic or bicyclic C₅, C₆, C₇, C₈, and C₉ aliphatic carbocyclyl group and 5-, 6-, 7-, 8-, and 9-membered aliphatic heterocyclyl group, and is connected to L¹ and L² through two different atoms thereof, respectively; said monocyclic or bicyclic structure does not contain aromatic ring;
ring B is connected to L² and to the azaindazole ring at the 5-position through two different ring-forming atoms respectively; wherein, the shortest distance between the two ring-forming atoms thereof, is 1, 2 or 3 chemical bonds; wherein, Y¹, Y² and Y³ are each independently selected from CR³ and N, provided that no more than two of Y¹, Y² and Y³ is selected from N;
R² is each independently selected from:
   1) halogen, cyano, hydroxyl, C₁₋₄ alkoxy, amino, C₁₋₄ alkylamino, and C₁₋₃ alkyl sulfonyl; or
   2) C₁₋₄ alkyl, C₁₋₅ alkylene, 3- to 6-membered oxaalkylene, 2- to 6-membered azaalkylene, C₂₋₄ alkenyl, C₂₋₄ alkynyl, C₃₋₆ cycloalkyl and 3- to 8-membered aliphatic heterocyclyl, optionally substituted with 0, 1, 2, 3 or 4 substituents independently selected from: oxo, halogen, hydroxyl, hydroxy-C₁₋₃ alkyl, C₁₋₃ alkoxy-C₁₋₃ alkyl, cyano, C₁₋₃ alkoxy, amino, C₁₋₃ alkylamino, amino-C₁₋₃ alkyl, and C₁₋₃ alkylamino-C₁₋₃ alkyl;
R⁴ is each independently selected from:
   1) halogen, cyano, hydroxyl, C₁₋₄ alkoxy, amino, C₁₋₄ alkylamino, and C₁₋₃ alkyl sulfonyl; or
   2) C₁₋₄ alkyl, C₂₋₄ alkenyl, C₂₋₄ alkynyl, C₃₋₆ cycloalkyl and 3- to 8-membered aliphatic heterocyclyl, optionally substituted with 0, 1, 2, 3 or 4 substituents independently selected from: oxo, halogen, hydroxyl, hydroxy-C₁₋₃ alkyl, C₁₋₃ alkoxy-C₁₋₃ alkyl, cyano, C₁₋₃ alkoxy, amino, C₁₋₃ alkylamino, amino-C₁₋₃ alkyl, and C₁₋₃ alkylamino-C₁₋₃ alkyl; or
   3) phenyl and 5- to 6-membered heteroaryl, optionally substituted with 0, 1, 2 or 3 of R⁴²,
wherein, R⁴² is each independently selected from:
   1) halogen, cyano, hydroxyl, C₁₋₃ alkoxy, amino, C₁₋₃ alkylamino, and C₁₋₃ alkyl sulfonyl; or
   2) C₁₋₃ alkyl, C₂₋₄ alkynyl, C₃₋₆ cycloalkyl and 3- to 6-membered aliphatic heterocyclyl, optionally substituted with 0, 1, 2, 3 or 4 substituents independently selected from: halogen, hydroxyl, hydroxymethyl, cyano, C₁₋₃ alkoxy, amino, and C₁₋₃ alkylamino;
L² is each independently selected from -CH₂-, -NH-, -O-, and -S-; wherein, each L² is independently substituted with 0, 1, or 2 of R⁵; two adjacent L² may be connected through single bond; p of L² connected sequentially form a linker connecting ring A and ring B;
   when L² is selected from -CH₂-, the R⁵ thereof is each independently selected from:
      1) oxo, halogen, cyano, -C(=O)R^{a5}, carboxyl, -C(=O)NR^{a5}R^{b5}, -OR^{a5}, -NR^{a5}R^{b5}, -NR^{a5}C(=O)R^{b5}, -NR^{e5}C(=O)NR^{a5}R^{b5}, -NR^{a5}S(=O)₂R^{c5}, -NR^{e5}S(=O)₂NR^{a5}R^{b5}, and -NR^{G2}P(=O)(R^{G1})₂; or
      2) C₁₋₄ alkyl, C₃₋₆ cycloalkyl and 3- to 6-membered aliphatic heterocyclyl, optionally substituted with 0, 1, 2, 3 or 4 of R⁵²; or
      3) two R⁵ groups attached to the same L², taken together with the L² atom to which they are attached, form a cyclic group selected from: 1,1-cyclopropyl, 1,1-cyclobutyl, 1,1-cyclopentyl, 1,1-cyclohexyl, 3,3-oxetanyl, 3,3-azetidinyl, 3,3-tetrahydrofuryl, 3,3-tetrahydropyrrolyl, 3,3-tetrahydropyranyl, 4,4-tetrahydropyranyl, 3,3-piperidinyl and 4,4-piperidyl, optionally substituted with 0, 1, 2, 3, or 4 of R⁵²; or
      4) two R⁵ groups attached to two adjacent L², taken together with the two L² atoms to which they are attached, form a cyclic group selected from: 1,2-cyclopropyl, 1,2-cyclobutyl, 1,2-cyclopentyl, 1,2-cyclohexyl, 3,4-tetrahydrofuryl, 3,4-tetrahydropyrrolyl, 3,4-tetrahydropyranyl and 3,4-piperidinyl, optionally substituted with 0, 1, 2, 3, or 4 of R⁵², or
      5) two R⁵ groups attached to two nonadjacent L², taken together with the two L² atoms to which they are attached and all other L² atom(s) between said two L² atoms, form a cyclic group selected from: 1,3-cyclobutyl, 1,3-cyclopentyl, 1,3-cyclohexyl, 1,4-cyclohexyl, 2,5-tetrahydrofuryl, 2,5-tetrahydropyrrolyl, 2,6-tetrahydropyranyl, 3,5-tetrahydropyranyl, 2,6-piperidinyl, 3,5-piperidinyl, 2,6-morpholinyl, 2,5-morpholinyl, 3,5-morpholinyl, 2,6-piperazinyl and 2,5-piperazinyl, optionally substituted with 0, 1, 2, 3, or 4 of R⁵²,
   when L² is selected from -NH-, the R⁵ thereof is each independently selected from:
      1) -C(=O)R^{a5}, -C(=O)NR^{a5}R^{b5}, -C(=NR^{d5})R^{a5}R^{b5}, -S(=O)₂R^{c5}, -S(=O)₂NR^{a5}R^{b5}, -S(=O)(=NR^{d5})R^{c5} and -P(=O)(R^{G1})₂; or
      2) C₁₋₄ alkyl, C₃₋₆ cycloalkyl and 3- to 6-membered aliphatic heterocyclyl, optionally substituted with 0, 1, 2, 3 or 4 of R⁵²; or
      3) two R⁵ groups attached to two adjacent L², taken together with the two L² atoms to which they are attached, form a cyclic group selected from: 1,2-aziridinyl, 1,2-azetidinyl, 1,2-tetrahydropyrrolyl, 1,2-piperidinyl, 3,4-morpholinyl and 1,2-piperanyl, optionally substituted with 0, 1, 2, 3, or 4 of R⁵²; or
      4) two R⁵ groups attached to two nonadjacent L², taken together with the two L² atoms to which they are attached and all other L² atom(s) between said two L² atoms, form a cyclic group selected from: 1,3-azetidinyl, 1,3-tetrahydropyrrolyl, 1,3-piperidinyl, 1,4-piperidinyl and 1,4-piperazinyl, optionally substituted with 0, 1, 2, 3, or 4 of R⁵²,
   when L² is selected from -S-, the R⁵ thereof is each independently selected from oxo;
   wherein, R^{a5}, R^{b5} and R^{e5} are independently selected from:
      1) hydrogen; or
      2) C₁₋₃ alkyl, C₃₋₆ cycloalkyl, 3-oxetanyl, 3-azetidinyl, tetrahydrofuryl, tetrahydropyrrolyl, tetrahydropyranyl and piperidinyl, optionally substituted with 0, 1, 2, 3 or 4 of R⁵²;
   or, R^{a5} and R^{b5} attached to the same nitrogen atom, taken together with the nitrogen atom to which they are attached, form a cyclic group selected from: 1-azetidinyl, 1-tetrahydropyrrolyl, 1-piperidinyl, 1-piperazinyl and morpholinyl, optionally substituted with 0, 1, 2, 3 or 4 of R⁵²;
   R^{c5} is each independently selected from C₁₋₃ alkyl, C₃₋₆ cycloalkyl, 3-oxetanyl, 3-azetidinyl, tetrahydrofuryl, tetrahydropyrrolyl, tetrahydropyranyl and piperidinyl, optionally substituted with 0, 1, 2, 3 or 4 of R⁵²,
   R^{d5} is selected from:
      1) hydrogen, cyano, nitro and -S(=O)₂-(C₁₋₃ alkyl); or
      2) C₁₋₃ alkyl and cyclopropyl;
R⁵² is selected from: oxo, fluorine, cyano, hydroxyl, C₁₋₃ alkoxy, cyclopropoxy, C₁₋₃ alkyl, cyclopropyl, difluoromethyl, trifluoromethyl, hydroxymethyl, 1-hydroxyethyl, 2-hydroxyethyl, 2-hydroxy-2-propyl, amino and C₁₋₃ alkylamino;
   R^{G1} is selected from -OR^{G2} and -N(R^{G2})₂;
   R^{G2} is selected from:
      1) hydrogen; or
      2) C₁₋₄ alkyl, C₁₋₄ alkylene, C₃₋₆ cycloalkyl, 3- to 6-membered aliphatic heterocyclyl and phenyl, optionally substituted with 0, 1, 2 or 3 substituents independently selected from: oxo, halogen, hydroxyl, hydroxymethyl, carboxyl, cyano, C₁₋₃ alkoxy, amino, and C₁₋₃ alkylamino; or
      3) two R^{G2} attached to the same nitrogen atom, taken together with the nitrogen atom to which they are attached, form a 3- to 6-membered aliphatic heterocyclyl, optionally substituted with 0, 1, 2 or 3 substituents independently selected from: oxo, halogen, hydroxyl, hydroxymethyl, carboxyl, cyano, C₁₋₃ alkoxy, amino, and C₁₋₃ alkylamino;
   R^{G3} is selected from: hydrogen, cyano, nitro, -OR^{G2}, -S(=O)₂R^{G2} and R^{G2};
m is selected from 0, 1, 2, 3, 4, 5 and 6;
p is selected from 2, 3, 4, 5, 6, 7, 8 and 9;
n is selected from 0, 1, 2 and 3;
R³ is as defined herein.

In some embodiments, the compound described herein has the structure of Formula (IIIa), or an isomer, pharmaceutically acceptable salt, polymorph, isotope labeled compound, active metabolite or prodrug thereof; wherein,
L¹ is selected from:
1) phenyl, thienyl and pyridyl; or
2) 5- and 6-membered monocyclic heteroaryl containing two ring-forming heteroatoms, wherein one of the ring-forming heteroatom is selected from nitrogen, and the other ring-forming heteroatom is selected from nitrogen, oxygen and sulfur; wherein, L¹ is connected to the azaindazole ring at the 3-position and to the ring A through two nonadjacent ring-forming atoms respectively; in addition to being connected to the azaindazole ring and to the ring A, L¹ is optionally substituted with 0, 1, 2 or 3 of R⁴.

In some embodiments, in the compound of Formula (IIIa),
L¹ is selected from:
1) phenyl, thienyl and pyridyl; or
2) 5- and 6-membered monocyclic heteroaryl containing two ring-forming heteroatoms, wherein one of the ring-forming heteroatom is selected from nitrogen, and the other ring-forming heteroatom is selected from nitrogen, oxygen and sulfur; wherein, L¹ is connected to the azaindazole ring at the 3-position and to the ring A through two nonadjacent ring-forming atoms respectively; in addition to being connected to the azaindazole ring and to the ring A, L¹ is optionally substituted with 0, 1 or 2 of R⁴; or
3) quinolyl, isoquinolyl, indolyl, indazolyl, benzimidazolyl, benzothiazolyl and benzisothiazolyl; wherein, L¹ is connected to the azaindazole ring at the 3-position and to the ring A through two nonadjacent ring-forming atoms respectively, said two ring-forming atoms belong to the benzene ring; in addition to being connected to the azaindazole ring and to the ring A, L¹ is optionally substituted with 0, 1, 2 or 3 of R⁴.

In some embodiments, in the compound of Formula (IIIa), L¹ is selected from:
1) phenyl, thienyl and pyridyl; or
2) 5- and 6-membered monocyclic heteroaryl containing two ring-forming heteroatoms, wherein one of the ring-forming heteroatom is selected from nitrogen, and the other ring-forming heteroatom is selected from nitrogen, oxygen and sulfur; wherein, L¹ is connected to the azaindazole ring at the 3-position and to the ring A through two adjacent ring-forming atoms respectively; in addition to being connected to the azaindazole ring and to the ring A, L¹ is optionally substituted with 0, 1 or 2 of R⁴.

In some embodiments, the compound described herein has the structure of Formula (IIIa), or an isomer, pharmaceutically acceptable salt, polymorph, isotope labeled compound, active metabolite or prodrug thereof; wherein,
L¹ is selected from: naphthyl, and 9- to 10-memebred heteroaryl; said naphthyl or heteroaryl group is connected to the azaindazole ring at the 3-position and to the ring A through two nonadjacent ring-forming atoms respectively; wherein said heteroaryl group contains at least one ring-forming nitrogen atom, and does not contain other ring-forming heteroatoms or contains one or two ring-forming heteroatom(s) selected from nitrogen, oxygen and sulfur; in addition to being connected to the azaindazole ring and to the ring A, said naphthyl and heteroaryl group is optionally substituted with 0, 1, 2, 3 or 4 of R⁴.

In some embodiments, in the compound of Formula (IIIa),
L¹ is selected from: naphthyl, and 9- to 10-memebred heteroaryl; said 9- to 10-memebred heteroaryl contains at least one benzene ring; said naphthyl or heteroaryl group is connected to the azaindazole ring at the 3-position and to the ring A through two nonadjacent ring-forming atoms respectively, and all said two ring-forming atoms belong to the benzene ring; said heteroaryl group contains at least one ring-forming nitrogen atom, and does not contain other ring-forming heteroatoms or contains one or two ring-forming heteroatom(s) selected from nitrogen, oxygen and sulfur; in addition to being connected to the azaindazole ring and to the ring A, said naphthyl and heteroaryl group is optionally substituted with 0, 1, 2, 3 or 4 of R⁴.

In some embodiments, in the compound of Formula (IIIa),
L¹ is selected from quinolyl, isoquinolyl, indolyl, indazolyl, benzimidazolyl, benzothiazolyl and benzisothiazolyl; said L¹ is connected to the azaindazole ring at the 3-position and to the ring A through two nonadjacent ring-forming atoms respectively, and all said two ring-forming atoms belong to the benzene ring; in addition to being connected to the azaindazole ring and to the ring A, said L¹ is optionally substituted with 0, 1, 2 or 3 of R⁴.

In some embodiments, in the compound of Formula (IIIa), L¹ is selected from quinolyl, isoquinolyl, indolyl, indazolyl, benzimidazolyl, benzothiazolyl and benzisothiazolyl; said L¹ is connected to the azaindazole ring at the 3-position and to the ring A through two nonadjacent ring-forming atoms respectively, and all said two ring-forming atoms belong to the benzene ring; in addition to being connected to the azaindazole ring and to the ring A, said L¹ is optionally substituted with 0, 1 or 2 of R⁴.

In some embodiments, in the compound of Formula (IIIa), L¹ is selected from one of the following structures; wherein, the "*" at the end of the chemical bond in each structure means that the structure is connected to the azaindazole ring at the 3-position through the bond, the "**" at the end of the chemical bond in each structure means that the structure is connected to ring A through the bond: 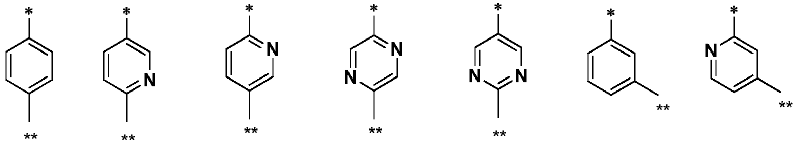 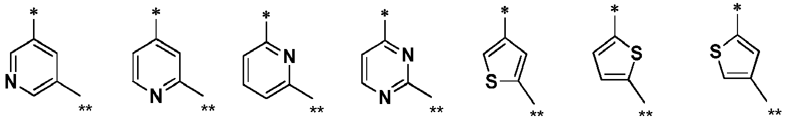 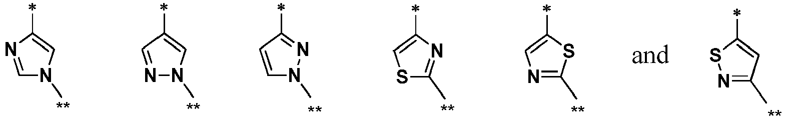 and, in addition to being connected to the azaindazole ring and to the ring A, L¹ is optionally substituted with 0, 1 or 2 of R⁴.

In one embodiment, in the compound of Formula (IIIa), ring B is selected from phenyl and 6-membered heteroaryl, wherein Y¹, Y² and Y³ are each independently selected from CR³ and N, provided that no more than two of Y¹, Y² and Y³ is selected from N;

In one embodiment, in the compound of Formula (IIIa), Y¹, Y² and Y³ are each independently selected from CR³.

In one embodiment, in the compound of Formula (IIIa), Y¹ is selected from N, Y² and Y³ are each independently selected from CR³.

In one embodiment, in the compound of Formula (IIIa), Y² is selected from N, Y¹ and Y³ are each independently selected from CR³.

In one embodiment, in the compound of Formula (IIIa), Y³ is selected from N, Y¹ and Y² are each independently selected from CR³.

In one embodiment, in the compound of Formula (IIIa), Y¹ and Y² are selected from N, Y³ is selected from CR³.

In one embodiment, in the compound of Formula (IIIa), Y² and Y³ are selected from N, Y¹ is selected from CR³.

In one embodiment, ring A is selected from monocyclic or bicyclic C₅, C₆, C₇, C₈, and C₉ carbocyclyl group and 5-, 6-, 7-, 8-, and 9-membered heterocyclyl group, and is connected to L¹ and L² through two different ring-forming atoms respectively; said monocyclic or bicyclic structure does not contain aromatic ring.

In one embodiment, ring A is selected from one of the following structures; the "*" at the end of the chemical bond in each structure means that the structure is connected to L¹ through the bond, the "**" at the end of the chemical bond in each structure means that the structure is connected to L² through the bond: 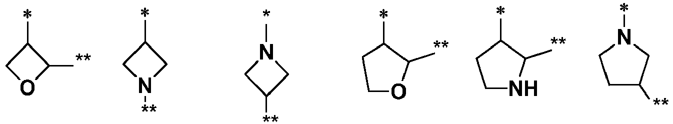 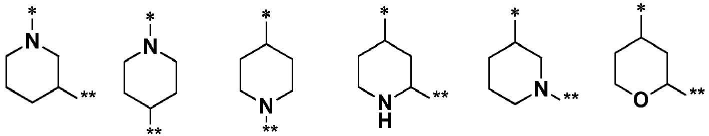 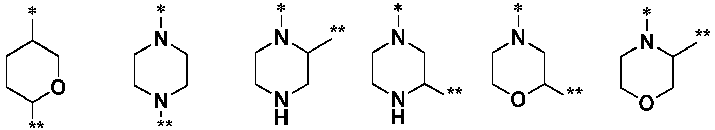 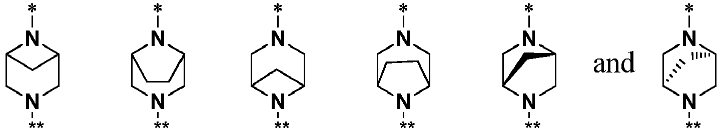 and, in addition to being connected to L¹ and L², ring A is optionally substituted with 0, 1 or 2 of R².

In one embodiment, R² is independently selected from:
1) oxo, fluorine, chlorine, cyano, hydroxyl, methoxy, ethoxy, isopropoxy, cyclopropoxy, methyl, ethyl, 1-propyl, isopropyl, cyclopropyl, difluoromethyl, trifluoromethyl, hydroxymethyl, 1-hydroxyethyl, 2-hydroxyethyl, 2-hydroxy-2-propyl, 2-amino-2-propyl, N-methyl-2-amino-2-propyl, *N*,N-dimethyl-2-amino-2-propyl, and carboxyl; or
2) cyclopropyl, cyclobutyl, cyclopentyl, cyclohexyl, oxetanyl, tetrahydrofuryl, tetrahydropyranyl, azetidinyl, tetrahydropyrrolyl, piperidinyl, piperazinyl, and morpholinyl, optionally substituted with 0, 1, 2 or 3 of substituent R²²¹ independently selected from: oxo, fluorine, chlorine, cyano, hydroxyl, C₁-C₆ alkoxy, C₁-C₆ alkylamino, C₁-C₆ alkyl, C₁-C₆ fluoroalkyl, C₁-C₆ hydroxyalkyl, C₁-C₆ aminoalkyl, C₃-C₆ cycloalkyl, and C₃-C₆ oxacycloalkyl; or
3) ethylene, propylene, butylene, pentylene, oxapropylene, oxabutylene, oxapentylene, azapropylene, azabutylene or azapentylene, optionally substituted with 0, 1 or 2 of substituents selected from fluorine, hydroxy and methyl;
m is selected from 0, 1 and 2.

In one embodiment, R²²¹ is each independently selected from: oxo, fluorine, chlorine, cyano, hydroxyl, methoxy, ethoxy, isopropoxy, cyclopropoxy, dimethylamino, methyl, ethyl, 1-propyl, isopropyl, cyclopropyl, difluoromethyl, trifluoromethyl, hydroxymethyl, 1-hydroxyethyl, 2-hydroxyethyl, 2-hydroxy-2-propyl, 2-amino-2-propyl, *N*-methyl-2-amino-2-propyl, *N,N-*dimethyl-2-amino-2-propyl, oxetanyl and tetrahydropyranyl.

In one embodiment, R³ is each independently selected from: fluorine, chlorine, cyano, C₁₋₃ alkoxy, difluoromethoxy, trifluoromethoxy, cyclopropoxy, C₁₋₃ alkyl, difluoromethyl, trifluoromethyl and cyclopropyl, and at least one of R³ is located in the *ortho*-position of the ring-forming atom of ring B connected to the azaindazole ring at the 5-position; said R³ located in the *ortho*-position of the ring-forming atom of ring B connected to the azaindazole ring at the 5-position is selected from fluorine, chlorine, methoxy, methyl and ethyl;
n is selected from 1, 2 and 3.

In one embodiment, R³ is independently selected from: fluorine, chlorine, cyano, methoxy, methyl and ethyl, and at least one of R³ is located in the *ortho*-position of the ring-forming atom of ring B connected to the azaindazole ring at the 5-position; said R³ located in the *ortho*-position of the ring-forming atom of ring B connected to the azaindazole ring at the 5-position is selected from fluorine, chlorine, methoxy, methyl and ethyl;
n is selected from 1 and 2.

In one embodiment, R³ is independently selected from: fluorine, chlorine, cyano and methoxy, and at least one of R³ is located in the *ortho*-position of the ring-forming atom of ring B connected to the azaindazole ring at the 5-position; said R³ located in the *ortho*-position of the ring-forming atom of ring B connected to the azaindazole ring at the 5-position is selected from fluorine, chlorine and methoxy; preferably, said R³ is selected from fluorine;
n is selected from 1 and 2; preferably, n is selected from 1.

In one embodiment, R³ is independently selected from fluorine and methoxy, and at least one of R³ is located in the *ortho-*position of the ring-forming atom of ring B connected to the azaindazole ring at the 5-position; preferably, said R³ is selected from fluorine;
n is selected from 1 and 2; preferably, n is selected from 1.

In one embodiment, ring B is selected from phenyl and 6-membered heteroaryl, and is connected to L² and to the azaindazole ring at the 5-position through two different ring-forming atoms respectively; wherein the shortest distance between said two ring-forming atoms is 1, 2 or 3 chemical bonds; preferably, wherein Y¹, Y² and Y³ are each independently selected from CR³ and N, provided that no more than two of Y¹, Y² and Y³ is selected from N.

In one embodiment, ring B is selected from phenyl; Y¹, Y² and Y³ are each independently selected from CR³; and ring B is connected to L² and to the azaindazole ring at the 5-position through two different ring-forming atoms respectively; wherein said two ring-forming atoms are in *ortho-, meta-* or *para*-position relationship.

In one embodiment, R² is independently selected from:
1) halogen, cyano, -C(=O)NR^{a2}R^{b2}, -OR^{a2}, -S(=O)R^{c2}, -S(=O)2R^{c2}, -S(=O)2NR^{a2}R^{b2}, -NR^{a2}R^{b2}, -NR^{a2}C(=O)R^{b2}, -NR^{e2}C(=O)NR^{a2}R^{b2}, -NR^{a2}S(=O)₂R^{c2}, and -NR^{e2}S(=O)₂NR^{a2}R^{b2}; or
2) C₁₋₄ alkyl, C₁₋₅ alkylene, 3- to 6-membered oxaalkylene, and 2- to 6-membered azaalkylene, C₂₋₄ alkynyl, phenyl, 5- to 6-membered heteroaryl, C₃₋₆ cycloalkyl and 3- to 8-membered aliphatic heterocyclyl, optionally substituted with 0, 1, 2, 3 or 4 of R²²,

wherein, R^{a2}, R^{b2} and R^{e2} are each independently selected from:
   1) hydrogen; or
   2) C₁₋₄ alkyl, phenyl, 5- to 6-membered heteroaryl, C₃₋₆ cycloalkyl, and 4- to 6-membered aliphatic heterocyclyl, optionally substituted with 0, 1, 2, 3 or 4 of R²²;
   or, R^{a2} and R^{b2} attached to the same nitrogen atom, taken together with the nitrogen atom to which they are attached, form a 4- to 6-membered aliphatic heterocyclyl, optionally substituted with 0, 1, 2, 3 or 4 of R²²;
R^{c2} is selected from C₁₋₄ alkyl, phenyl, 5- to 6-membered heteroaryl, C₃₋₆ cycloalkyl and 4- to 6-membered aliphatic heterocyclyl, optionally substituted with 0, 1, 2, 3 or 4 of R²²;
R²² is independently selected from:
   1) oxo, halogen, cyano, hydroxyl, C₁₋₄ alkoxy, amino and C₁₋₄ alkylamino; or
   2) C₁₋₄ alkyl, C₂₋₄ alkenyl, C₂₋₄ alkynyl, C₃₋₆ cycloalkyl and 3- to 6-membered aliphatic heterocyclyl, optionally substituted with 0, 1, 2, 3 or 4 substituents independently selected from: oxo, halogen, hydroxyl, hydroxymethyl, cyano, C₁₋₃ alkoxy, amino, and C₁₋₃ alkylamino.

In one embodiment, R² is independently selected from:
1) halogen, cyano, -C(=O)NR^{a2}R^{b2}, -OR^{a2}, -S(=O)R^{c2}, -S(=O)2R^{c2}, -S(=O)2NR^{a2}R^{b2}, -NR^{a2}R^{b2}, -NR^{a2}C(=O)R^{b2}, -NR^{e2}C(=O)NR^{a2}R^{b2}, -NR^{a2}S(=O)₂R^{c2}, and -NR^{e2}S(=O)₂NR^{a2}R^{b2}; or
2) C₁₋₄ alkyl, C₂₋₄ alkynyl, phenyl, 5- to 6-membered heteroaryl, C₃₋₆ cycloalkyl and 3- to 8-membered aliphatic heterocyclyl, optionally substituted with 0, 1, 2, 3 or 4 of R^{G};

wherein, R^{a2}, R^{b2} and R^{e2} are each independently selected from:
   1) hydrogen; or
   2) C₁₋₄ alkyl, phenyl, 5- to 6-membered heteroaryl, C₃₋₆ cycloalkyl, and 4- to 6-membered aliphatic heterocyclyl, optionally substituted with 0, 1, 2, 3 or 4 of R^{G};
   or, R^{a2} and R^{b2} attached to the same nitrogen atom, taken together with the nitrogen atom to which they are attached, form a 4- to 6-membered aliphatic heterocyclyl, optionally substituted with 0, 1, 2, 3 or 4 of R^{G};
R^{c2} is selected from C₁₋₄ alkyl, phenyl, 5- to 6-membered heteroaryl, C₃₋₆ cycloalkyl and 4- to 6-membered aliphatic heterocyclyl, optionally substituted with 0, 1, 2, 3 or 4 of R^{G}.

In one embodiment, R² is independently selected from:
two R² groups attached to one or two ring-forming atom(s) of the ring A, taken together with the one or two ring-forming atom(s) to which they are attached, form a C₅₋₆ aliphatic carbocyclyl, or 5- to 6-membered aliphatic heterocyclyl, optionally substituted with 0, 1, 2, 3 or 4 of R²².

In one embodiment, R² is independently selected from:
1) halogen, cyano, hydroxyl, C₁₋₄ alkoxy, amino, C₁₋₄ alkylamino, and C₁₋₃ alkyl sulfonyl; or
2) C₁₋₄ alkyl, C₁₋₅ alkylene, 3- to 6-membered oxaalkylene, 2- to 6-membered azaalkylene, C₂₋₄ alkenyl, C₂₋₄ alkynyl, C₃₋₆ cycloalkyl and 3- to 8-membered aliphatic heterocyclyl, optionally substituted with 0, 1, 2, 3 or 4 substituents independently selected from: oxo, halogen, hydroxyl, hydroxy-C₁₋₃ alkyl, C₁₋₃ alkoxy-C₁₋₃ alkyl, cyano, C₁₋₃ alkoxy, amino, C₁₋₃ alkylamino, amino-C₁₋₃ alkyl, and C₁₋₃ alkylamino-C₁₋₃ alkyl.

In one embodiment, R² is independently selected from:
fluorine, chlorine, cyano, hydroxyl, methoxy, ethoxy, isopropoxy, cyclopropoxy, methyl, ethyl, 1-propyl, isopropyl, cyclopropyl, difluoromethyl, trifluoromethyl, hydroxymethyl, 1-hydroxyethyl, 2-hydroxyethyl, 2-hydroxy-2-propyl, 2-amino-2-propyl, *N*-methyl-2-amino-2-propyl, *N,N-*dimethyl-2-amino-2-propyl, and carboxyl.

In one embodiment, R² is independently selected from 3- to 8-membered aliphatic heterocyclyl, wherein said aliphatic heterocyclyl contains 1 or 2 ring-forming heteroatom(s) optionally selected from N, O and S; said aliphatic heterocyclyl is optionally substituted with 0, 1, 2 or 3 substituents independently selected from: oxo, halogen, hydroxyl, hydroxy-C₁₋₃ alkyl, C₁₋₃ alkoxy-C₁₋₃ alkyl, cyano, C₁₋₃ alkoxy, amino, C₁₋₃ alkylamino, amino-C₁₋₃ alkyl, and C₁₋₃ alkylamino-C₁₋₃ alkyl.

In one embodiment, R² is each independently selected from: ethylene, propylene, butylene, pentylene, oxapropylene, oxabutylene, oxapentylene, azapropylene, azabutylene and azapentylene, optionally substituted with 0, 1 or 2 substituents selected from fluorine, hydroxy and methyl.

In one embodiment, R²²¹ is each independently selected from: oxo, fluorine, chlorine, cyano, hydroxyl, methoxy, ethoxy, isopropoxy, cyclopropoxy, dimethylamino, methyl, ethyl, 1-propyl, isopropyl, cyclopropyl, difluoromethyl, trifluoromethyl, hydroxymethyl, 1-hydroxyethyl, 2-hydroxyethyl, 2-hydroxy-2-propyl, 2-amino-2-propyl, *N*-methyl-2-amino-2-propyl, *N,N-*dimethyl-2-amino-2-propyl, oxetanyl and tetrahydropyranyl.

In one embodiment, R³ is independently selected from:
1) halogen, cyano, -C(=O)NR^{a3}R^{b3}, -OR^{a3}, -S(=O)R^{c3}, -S(=O)₂R^{c3}, -S(=O)₂NR^{a3}R^{b3}, -NR^{a3}R^{b3}, -NR^{a3}C(=O)R^{b3}, -NR^{e3}C(=O)NR^{a3}R^{b3}, -NR^{a3}S(=O)₂R^{c3}, and -NR^{e3}S(=O)₂NR^{a3}R^{b3}; or
2) C₁₋₄ alkyl, C₂₋₄ alkynyl, phenyl, 5- to 6-membered heteroaryl, C₃₋₆ cycloalkyl and 3- to 8-membered aliphatic heterocyclyl, optionally substituted with 0, 1, 2, 3 or 4 of R³²; or
3) two R³ groups attached to two ring-forming atoms of the ring A, taken together with the two ring-forming atoms to which they are attached, form a C₅₋₆ aliphatic carbocyclyl, or 5-to 6-membered aliphatic heterocyclyl, optionally substituted with 0, 1, 2, 3 or 4 of R³²;

wherein, R^{a3}, R^{b3} and R^{e3} are each independently selected from:
   1) hydrogen; or
   2) C₁₋₄ alkyl, phenyl, 5- to 6-membered heteroaryl, C₃₋₆ cycloalkyl and 3- to 6-membered aliphatic heterocyclyl, optionally substituted with 0, 1, 2, 3 or 4 of R³²,
or, R^{a3} and R^{b3} attached to the same nitrogen atom, taken together with the nitrogen atom to which they are attached, form a 4- to 6-membered aliphatic heterocyclyl, optionally substituted with 0, 1, 2, 3 or 4 of R³²;
R^{c3} is selected from C₁₋₄ alkyl, phenyl, 5- to 6-membered heteroaryl, C₃₋₆ cycloalkyl and 4- to 6-membered aliphatic heterocyclyl, optionally substituted with 0, 1, 2, 3 or 4 of R³²;
R³² is each independently selected from:
   1) oxo, halogen, cyano, hydroxyl, C₁₋₄ alkoxy, amino, and C₁₋₄ alkylamino; or
   2) C₁₋₄ alkyl, C₂₋₄ alkenyl, C₂₋₄ alkynyl, C₃₋₆ cycloalkyl and 3- to 6-membered aliphatic heterocyclyl, optionally substituted with 0, 1, 2, 3 or 4 substituents independently selected from: oxo, halogen, hydroxyl, hydroxymethyl, cyano, C₁₋₃ alkoxy, amino, and C₁₋₃ alkylamino.

In one embodiment, R³ is independently selected from:
1) halogen, cyano, -C(=O)NR^{a3}R^{b3} and -OR^{a3}; or
2) C₁₋₃ alkyl, C₂₋₄ alkynyl, phenyl, 5- to 6-membered heteroaryl, C₃₋₆ cycloalkyl and 3- to 6-membered aliphatic heterocyclyl, optionally substituted with 0, 1, 2, 3 or 4 of R³²,

Wherein, R^{a3} and R^{b3} are each independently selected from:
   1) hydrogen; or
   2) C₁₋₄ alkyl, C₃₋₆ cycloalkyl and 4- to 6-membered aliphatic heterocyclyl, optionally substituted with 0, 1, 2, 3 or 4 of R³²;
or, R^{a3} and R^{b3} attached to the same nitrogen atom, taken together with the nitrogen atom to which they are attached, form a 4- to 6-membered aliphatic heterocyclyl, optionally substituted with 0, 1, 2, 3 or 4 of R³²;
R³² is each independently selected from:
   1) oxo, halogen, cyano, hydroxyl, C₁₋₄ alkoxy, amino, and C₁₋₄ alkylamino; or
   2) C₁₋₄ alkyl, C₃₋₆ cycloalkyl and 3- to 6-membered aliphatic heterocyclyl, optionally substituted with 0, 1, 2, 3 or 4 substituents independently selected from: oxo, halogen, hydroxyl, hydroxymethyl, cyano, C₁₋₃ alkoxy, amino, and C₁₋₃ alkylamino.

In one embodiment, R³ is independently selected from:
two R³ groups attached to two ring-forming atoms of the ring B, taken together with the two ring-forming atoms to which they are attached, form a C₅₋₆ aliphatic carbocyclyl, or 5- to 6-membered aliphatic heterocyclyl, optionally substituted with 0, 1, 2, 3 or 4 of R³².

In one embodiment, R³² is independently selected from:
1) oxo, halogen, cyano, hydroxyl, C₁₋₄ alkoxy, amino, and C₁₋₄ alkylamino; or
2) C₁₋₄ alkyl, C₃₋₆ cycloalkyl and 3- to 6-membered aliphatic heterocyclyl, optionally substituted with 0, 1, 2, 3 or 4 substituents independently selected from: oxo, halogen, hydroxyl, hydroxymethyl, cyano, C₁₋₃ alkoxy, amino, and C₁₋₃ alkylamino.

In one embodiment, R³ is independently selected from: fluorine, chlorine, cyano, C₁₋₃ alkoxy, difluoromethoxy, trifluoromethoxy, cyclopropoxy, C₁₋₃ alkyl, difluoromethyl, trifluoromethyl and cyclopropyl, and at least one of R³ is located in the *ortho*-position of the ring-forming atom of ring B connected to the azaindazole ring at the 5-position; said R³ is optionally selected from fluorine, chlorine, methoxy, methyl and ethyl.

In one embodiment, R³ is independently selected from: fluorine, chlorine, cyano, methoxy, methyl and ethyl; and at least one of R³ is located in the *ortho-*position of the ring-forming atom of ring B connected to the azaindazole ring at the 5-position; said R³ is selected from fluorine, chlorine, methoxy, methyl and ethyl.

In one embodiment, R³ is independently selected from: fluorine, chlorine, cyano and methoxy; and at least one of R³ is located in the *ortho*-position of the ring-forming atom of ring B connected to the azaindazole ring at the 5-position; said R³ is selected from fluorine, chlorine, and methoxy.

In one embodiment, R³ is independently selected from: fluorine and methoxy; and at least one of R³ is located in the *ortho*-position of the ring-forming atom of ring B connected to the azaindazole ring at the 5-position.

In one embodiment, R³ is selected from fluorine; and said fluorine atom is located in the *ortho*-position of the ring-forming atom of ring B connected to the azaindazole ring at the 5-position.

In one embodiment, R⁴ is independently selected from:
1) halogen, cyano, -C(=O)NR^{a4}R^{b4}, -OR^{a4}, -S(=O)R^{c4}, -S(=O)₂R^{c4}, -S(=O)₂NR^{a4}R^{b4}, -NR^{a4}R^{b4}, -NR^{a4}C(=O)R^{b4}, -NR^{e4}C(=O)NR^{a4}R^{b4}, -NR^{a4}S(=O)₂R^{c4} and -NR^{e4}S(=O)₂NR^{a4}R^{b4}; or
2) C₁₋₄ alkyl, C₂₋₄ alkynyl, phenyl, 5- to 6-membered heteroaryl, C₃₋₆ cycloalkyl and 3- to 8-membered aliphatic heterocyclyl, optionally substituted with 0, 1, 2, 3 or 4 of R⁴²; or
3) two R⁴ groups attached to two ring-forming atoms of the ring L¹, taken together with the two ring-forming atoms to which they are attached, form a C₅₋₆ aliphatic carbocyclyl, or 5-to 6-membered aliphatic heterocyclyl, optionally substituted with 0, 1, 2, 3 or 4 of R⁴²,

R^{a4}, R^{b4} and R^{e4} are each independently selected from:
   1) hydrogen; or
   2) C₁₋₄ alkyl, phenyl, 5- to 6-membered heteroaryl, C₃₋₆ cycloalkyl and 4- to 6-membered aliphatic heterocyclyl, optionally substituted with 0, 1, 2, 3 or 4 of R⁴²,
or, R^{a4} and R^{b4} attached to the same nitrogen atom, taken together with the nitrogen atom to which they are attached, form a 4- to 6-membered aliphatic heterocyclyl, optionally substituted with 0, 1, 2, 3 or 4 of R⁴²,
R^{c4} is selected from C₁₋₄ alkyl, phenyl, 5- to 6-membered heteroaryl, C₃₋₆ cycloalkyl and 4- to 6-membered aliphatic heterocyclyl, optionally substituted with 0, 1, 2, 3 or 4 of R⁴²;
wherein,
R⁴² is independently selected from:
   1) oxo, halogen, cyano, hydroxyl, C₁₋₄ alkoxy, amino, and C₁₋₄ alkylamino; or
   2) C₁₋₄ alkyl, C₂₋₄ alkenyl, C₂₋₄ alkynyl, C₃₋₆ cycloalkyl and 3- to 6-membered aliphatic heterocyclyl, optionally substituted with 0, 1, 2, 3 or 4 substituents independently selected from: oxo, halogen, hydroxyl, hydroxymethyl, cyano, C₁₋₃ alkoxy, amino, and C₁₋₃ alkylamino.

In one embodiment, R⁴ is independently selected from:
1) halogen, cyano, -C(=O)NR^{a4}R^{b4}, -OR^{a4}, -S(=O)R^{c4}, -S(=O)₂R^{c4}, -S(=O)₂NR^{a4}R^{b4}, -NR^{a4}R^{b4}, -NR^{a4}C(=O)R^{b4}, -NR^{e4}C(=O)NR^{a4}R^{b4}, -NR^{a4}S(=O)₂R^{c4} and -NR^{e4}S(=O)₂NR^{a4}R^{b4}; or
2) C₁₋₄ alkyl, C₂₋₄ alkynyl, phenyl, 5- to 6-membered heteroaryl, C₃₋₆ cycloalkyl and 3- to 8-membered aliphatic heterocyclyl, optionally substituted with 0, 1, 2, 3 or 4 of R^{G};

wherein,
R^{a4}, R^{b4} and R^{e4} are each independently selected from:
   1) hydrogen; or
   2) C₁₋₄ alkyl, phenyl, 5- to 6-membered heteroaryl, C₃₋₆ cycloalkyl and 4- to 6-membered aliphatic heterocyclyl, optionally substituted with 0, 1, 2, 3 or 4 of R^{G};
   or,
R^{a4} and R^{b4} attached to the same nitrogen atom, taken together with the nitrogen atom to which they are attached, form a 4- to 6-membered aliphatic heterocyclyl, optionally substituted with 0, 1, 2, 3 or 4 of R^{G};
R^{c4} is selected from C₁₋₄ alkyl, phenyl, 5- to 6-membered heteroaryl, C₃₋₆ cycloalkyl and 4- to 6-membered aliphatic heterocyclyl, optionally substituted with 0, 1, 2, 3 or 4 of R^{G}.

In one embodiment, R⁴ is independently selected from:
two R⁴ groups attached to two ring-forming atoms of the ring L¹, taken together with the two ring-forming atoms to which they are attached, form a C₅₋₆ aliphatic carbocyclyl, or 5- to 6-membered aliphatic heterocyclyl, optionally substituted with 0, 1, 2, 3 or 4 of R⁴²,

In one embodiment, R⁴ is independently selected from:
1) halogen, cyano, hydroxyl, C₁₋₄ alkoxy, amino, C₁₋₄ alkylamino and C₁₋₃ alkyl sulfonyl; or
2) C₁₋₄ alkyl, C₂₋₄ alkenyl, C₂₋₄ alkynyl, C₃₋₆ cycloalkyl and 3- to 8-membered aliphatic heterocyclyl, optionally substituted with 0, 1, 2, 3 or 4 substituents independently selected from: oxo, halogen, hydroxyl, hydroxy-C₁₋₃ alkyl, C₁₋₃ alkoxy-C₁₋₃ alkyl, cyano, C₁₋₃ alkoxy, amino, C₁₋₃ alkylamino, amino-C₁₋₃ alkyl, and C₁₋₃ alkylamino-C₁₋₃ alkyl.

In one embodiment, R⁴ is independently selected from phenyl and 5- to 6-membered heteroaryl optionally substituted with 0, 1, 2, 3 or 4 of R⁴²:
wherein,
R⁴² is each independently selected from:
1) halogen, cyano, hydroxyl, C₁₋₄ alkoxy, amino, C₁₋₄ alkylamino and C₁₋₃ alkyl sulfonyl; or
2) C₁₋₄ alkyl, C₂₋₄ alkynyl, C₃₋₆ cycloalkyl and 3- to 6-membered aliphatic heterocyclyl, optionally substituted with 0, 1, 2 or 3 substituents independently selected from: halogen, hydroxyl, hydroxymethyl, cyano, C₁₋₃ alkoxy, amino, and C₁₋₃ alkylamino.

In one embodiment, R⁴ is independently selected from: fluorine, chlorine, cyano, hydroxyl, methoxy, ethoxy, isopropoxy, cyclopropoxy, methyl, ethyl, 1-propyl, isopropyl, cyclopropyl, difluoromethyl, trifluoromethyl, hydroxymethyl, 1-hydroxyethyl, 2-hydroxyethyl, 2-hydroxy-2-propyl, 2-amino-2-propyl, *N*-methyl-2-amino-2-propyl and *N,N*-dimethyl-2-amino-2-propyl.

In one embodiment, R⁴ is selected from 3- to 8-membered aliphatic heterocyclyl, wherein said aliphatic heterocyclyl contains 1 or 2 ring-forming heteroatom(s) optionally selected from N, O and S; said aliphatic heterocyclyl is unsubstituted or optionally substituted with 1, 2 or 3 substituents independently selected from: oxo, halogen, hydroxyl, hydroxy-C₁₋₃ alkyl, C₁₋₃ alkoxy-C₁₋₃ alkyl, cyano, C₁₋₃ alkoxy, amino, C₁₋₃ alkylamino, amino-C₁₋₃ alkyl, and C₁₋₃ alkylamino-C₁₋₃ alkyl.

In one embodiment, R⁴ is selected from one of the following aliphatic heterocyclyl, wherein the "*" at the end of the chemical bond in each structure means that the structure is connected to the ring of L¹ through the bond: 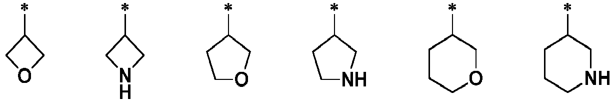 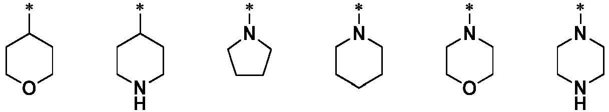 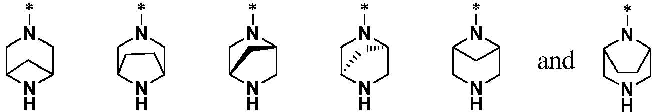 wherein, said aliphatic heterocyclyl is optionally substituted with 0, 1, 2 or 3 of substituent independently selected from: oxo, fluorine, chlorine, cyano, hydroxyl, methoxy, ethoxy, isopropoxy, cyclopropoxy, methyl, ethyl, 1-propyl, isopropyl, cyclopropyl, difluoromethyl, trifluoromethyl, hydroxymethyl, 1-hydroxyethyl, 2-hydroxyethyl, 2-hydroxy-2-propyl, 2-amino-2-propyl, *N*-methyl-2-amino-2-propyl and *N*,*N*-dimethyl-2-amino-2-propyl.

In one embodiment, R⁴ is independently selected from phenyl, pyridyl, pyrimidinyl, pyrazinyl, imidazolyl, pyrazolyl and thiazolyl, optionally substituted with 0, 1 or 2 of R⁴²,
wherein,
R⁴² is independently selected from: fluorine, chlorine, cyano, hydroxyl, methoxy, ethoxy, isopropoxy, cyclopropoxy, methyl, ethyl, 1-propyl, isopropyl, cyclopropyl, difluoromethyl, trifluoromethyl, hydroxymethyl, 1-hydroxyethyl, amino, methylamino, dimethylamino and diethylamino.

In one embodiment, L² is each independently selected from -CH₂-, -NH-, -O- and -S-; wherein, each L² is independently substituted with 0, 1, or 2 of R⁵.

In one embodiment, L² is each independently selected from -CH₂-, -NH- and -O-.

In one embodiment, L² is each independently selected from -CH₂- and -O-.

In one embodiment, L² is each independently selected from -CH₂-, -NH-, -O- and -S-; wherein, each L² is independently substituted with 0, 1, or 2 of R⁵; two adjacent L² may be connected through triple bond, provided said two L² are both selected from carbon.

In one embodiment, L² is each independently selected from -CH₂-, -NH-, -O- and -S-; wherein, each L² is independently substituted with 0, 1, or 2 of R⁵; two adjacent L² may be connected through double bond, in *Z*-form or *E*-form, provided that one of said two L² is selected from carbon, and the other one of said two L² is selected from nitrogen.

In one embodiment, L² is each independently selected from -CH₂-, -NH-, -O- and -S-; wherein, each L² is independently substituted with 0, 1, or 2 of R⁵; two adjacent L² may be connected through double bond, in *Z*-form or *E*-form, provided that said two L² are both selected from nitrogen.

In one embodiment, L² is each independently selected from -CH₂-, -NH-, -O- and -S-; wherein, each L² is independently substituted with 0, 1, or 2 of R⁵; any two adjacent L² may be connected through single bond.

In one embodiment, L² is each independently selected from -CH₂-, -NH- and -O-; wherein, each L² is independently substituted with 0, 1, or 2 of R⁵; any two adjacent L² may be connected through single bond.

In one embodiment, when L² is selected from -CH₂-, each said L² is optionally substituted with 0, 1, or 2 of R⁵.

In one embodiment, when L² is selected from -NH-, each said L² is optionally substituted with 0 or 1 of R⁵.

In one embodiment, when L² is selected from -S-, each said L² is optionally substituted with 0, 1 or 2 oxo group(s).

In one embodiment, when L² is selected from -CH₂-, the substituent R⁵ of said L² is each independently selected from:
1) oxo, halogen, cyano, -C(=O)R^{a5}, carboxyl, -C(=O)NR^{a5}R^{b5}, -C(=NR^{d5})NR^{a5}R^{b5}, -OR^{a5}, -OP(=O)(R^{G1})₂, -S(=O)₂R^{c5}, -S(=O)₂NR^{a5}R^{b5}, -S(=O)(=NR^{d5})R^{c5}, -NR^{a5}R^{b5}, -NR^{a5}C(=O)R^{b5}, -NR^{e5}C(=O)NR^{a5}R^{b5}, -NR^{e5}C(=NR^{d5})NR^{a5}R^{b5}, -NR^{a5}S(=O)₂R^{c5}, -NR^{e5}S(=O)₂NR^{a5}R^{b5}, -NR^{G2}P(=O)(R^{G1})₂, -P(=O)R^{c5}R^{f5}, -P(=O)(R^{G1})₂ and =N-R^{d5}; or
2) C₁₋₆ alkyl, phenyl, 5- to 6-membered heteroaryl, C₃₋₆ cycloalkyl and 3- to 6-membered aliphatic heterocyclyl, optionally substituted with 0, 1, 2, 3 or 4 of R⁵²; or
3) two R⁵ groups attached to the same L² or attached to two adjacent L², taken together with the one or two L² atom(s) to which they are attached, form a C₃₋₆ aliphatic carbocyclyl, or 3- to 6-membered aliphatic heterocyclyl, optionally substituted with 0, 1, 2, 3, or 4 of R⁵²; or
4) two R⁵ groups attached to two nonadjacent L², taken together with the two L² atoms to which they are attached and all other L² atom(s) between said two L² atoms, form a C₃₋₆ aliphatic carbocyclyl, or 3- to 6-membered aliphatic heterocyclyl, optionally substituted with 0, 1, 2, 3, or 4 of R⁵².

In one embodiment, when L² is selected from -NH-, the substituent R⁵ of said L² is each independently selected from:
-C(=O)R^{a5}, -C(=O)NR^{a5}R^{b5}, -C(=NR^{d5})NR^{a5}R^{b5}, -S(=O)₂R^{c5}, -S(=O)₂NR^{a5}R^{b5}, -S(=O)(=NR^{d5})R^{c5}, -P(=O)R^{c5}R^{f5} and -P(=O)(R^{G1})₂; or
2) C₁₋₆ alkyl, phenyl, 5- to 6-membered heteroaryl, C₃₋₆ cycloalkyl and 3- to 6-membered aliphatic heterocyclyl, optionally substituted with 0, 1, 2, 3 or 4 of R⁵²; or
3) two R⁵ groups attached to two adjacent L², taken together with the two L² atoms to which they are attached, form 3- to 6-membered aliphatic heterocyclyl, optionally substituted with 0, 1, 2, 3, or 4 of R⁵²; or
4) two R⁵ groups attached to two nonadjacent L², taken together with the two L² atoms to which they are attached and all other L² atom(s) between said two L² atoms, form 3- to 6-membered aliphatic heterocyclyl, optionally substituted with 0, 1, 2, 3, or 4 of R⁵²,

R^{a5}, R^{b5} and R^{e5} are each independently selected from:
   1) hydrogen; or
   2) C₁₋₄ alkyl, phenyl, 5- to 6-membered heteroaryl, C₃₋₆ cycloalkyl and 3- to 6-membered aliphatic heterocyclyl, optionally substituted with 0, 1, 2, 3 or 4 of R⁵²,
or, R^{a5} and R^{b5} attached to the same nitrogen atom, taken together with the nitrogen atom to which they are attached, form a 3- to 6-membered aliphatic heterocyclyl, optionally substituted with 0, 1, 2, 3 or 4 of R⁵²;
R^{c5} and R^{f5} are each independently selected from: C₁₋₄ alkyl, phenyl, 5- to 6-membered heteroaryl, C₃₋₆ cycloalkyl and 3- to 6-membered aliphatic heterocyclyl, optionally substituted with 0, 1, 2, 3 or 4 of R⁵²;
or, R^{c5} and R^{f5} attached to the same phosphine atom, taken together with the phosphine atom to which they are attached, form a 3- to 6-membered aliphatic heterocyclyl, optionally substituted with 0, 1, 2, 3 or 4 of R⁵²;
R^{d5} is selected from:
   1) hydrogen, hydroxyl, C₁₋₄ alkoxy, cyano, nitro and -S(=O)₂R^{G}; or
   2) C₁₋₄ alkyl, phenyl, 5- to 6-membered heteroaryl, C₃₋₆ cycloalkyl and 3- to 6-membered aliphatic heterocyclyl, optionally substituted with 0, 1, 2, 3 or 4 of R⁵²,
R⁵² is selected from:
   1) oxo, halogen and cyano; or
   2) R^{G2}; or
   3) R^{G1}, -C(=O)R^{G1}, -C(=O)R^{G2}, -OP(=O)(R^{G1})₂, -S(=O)₂R^{G2}, -S(=O)₂N(R^{G2})₂, -S(=O)(=NR^{G3})R^{G2}, -N(R^{G2})C(=O)R^{G1}, -N(R^{G2})C(=O)R^{G2}, -N(R^{G2})S(=O)₂R^{G2}, -N(R^{G2})S(=O)₂N(R^{G2})₂, -N(R^{G2})P(=O)(R^{G1})₂, -P(=O)(R^{G2})₂, -P(=O)(R^{G1})₂ and =N-R^{G3}.

In one embodiment, L² is each independently selected from -CH₂-, -NH-, -O- and -S-; wherein, each L² is independently substituted with 0, 1, or 2 of R⁵;

When L² is selected from -S-, the substituent R⁵ of said L² is independently selected from: oxo and =N-R^{d5};
R^{d5} is selected from: hydrogen, cyano, nitro and -S(=O)₂R^{G}.

In one embodiment, L² is each independently selected from -CH₂-, -NH-, -O- and -S-; each L² is independently substituted with 0, 1, or 2 of R⁵; R⁵ is independently selected from:
1) oxo, halogen, cyano, -C(=O)R^{a5}, carboxyl, -C(=O)NR^{a5}R^{b5}, -C(=NR^{d5})NR^{a5}R^{b5}, -OR^{a5}, -NR^{a5}R^{b5}, -NR^{a5}C(=O)R^{b5}, -NR^{e5}C(=O)NR^{a5}R^{b5}, -NR^{a5}S(=O)₂R^{c5}, -NR^{e5}S(=O)₂NR^{a5}R^{b5}, -NR^{G2}P(=O)(R^{G1})₂, -S(=O)₂R^{c5}, -S(=O)₂NR^{a5}R^{b5}, -S(=O)(=NR^{d5})R^{c5} and -P(=O)(R^{G1})₂; or
2) C₁₋₄ alkyl, C₂₋₅ alkylene, C₃₋₆ cycloalkyl and 3- to 6-membered aliphatic heterocyclyl, optionally substituted with 0, 1, 2, 3 or 4 of R⁵².

In one embodiment, L² is each independently selected from -CH₂-, -NH- and -O-; each L² is independently substituted with 0, 1, or 2 of R⁵; R⁵ is independently selected from:
1) oxo, halogen, cyano, -C(=O)R^{a5}, carboxyl, -C(=O)NR^{a5}R^{b5}, -C(=NR^{d5})NR^{a5}R^{b5}, -OR^{a5}, -NR^{a5}R^{b5}, -NR^{a5}C(=O)R^{b5}, -NR^{e5}C(=O)NR^{a5}R^{b5}, -NR^{a5}S(=O)₂R^{c5}, -NR^{e5}S(=O)₂NR^{a5}R^{b5}, -NR^{G2}P(=O)(R^{G1})₂, -S(=O)₂R^{c5}, -S(=O)₂NR^{a5}R^{b5}, -S(=O)(=NR^{d5})R^{c5} and -P(=O)(R^{G1})₂; or
2) C₁₋₄ alkyl, C₂₋₅ alkylene, C₃₋₆ cycloalkyl and 3- to 6-membered aliphatic heterocyclyl, optionally substituted with 0, 1, 2, 3 or 4 of R⁵².

In one embodiment, L² is each independently selected from -CH₂-, -NH-, -O- and -S-;
1) when L² is selected from -CH₂-, the substituent R⁵ of said L² is each independently selected from: oxo, halogen, cyano, -C(=O)R^{a5}, carboxyl, -C(=O)NR^{a5}R^{b5}, -OR^{a5}, -NR^{a5}R^{b5}, -NR^{a5}C(=O)R^{b5}, -NR^{e5}C(=O)NR^{a5}R^{b5}, -NR^{a5}S(=O)₂R^{c5}, -NR^{e5}S(=O)₂NR^{a5}R^{b5}, and -NR^{G2}P(=O)(R^{G1})₂; or
2) when L² is selected from -NH-, the substituent R⁵ of said L² is each independently selected from: -C(=O)R^{a5}, -C(=O)NR^{a5}R^{b5}, -C(=NR^{d5})NR^{a5}R^{b5}, -S(=O)₂R^{c5}, -S(=O)₂NR^{a5}R^{b5}, -S(=O)(=NR^{d5})R^{c5}, -P(=O)R^{c5}R^{f5} and -P(=O)(R^{G1})₂; or
3) when L² is selected from -CH₂- or -NH-, the substituent R⁵ of said L² is each independently selected from: C₁₋₄ alkyl, C₃₋₆ cycloalkyl and 3- to 6-membered aliphatic heterocyclyl, optionally substituted with 0, 1, 2, 3 or 4 of R⁵²;
4) when L² is selected from -S-, R⁵ is independently selected from oxo;
wherein,
R^{a5}, R^{b5} and R^{e5} are independently selected from:
   1) hydrogen; or
   2) C₁₋₃ alkyl, C₃₋₆ cycloalkyl, 3-oxetanyl, 3-azetidinyl, tetrahydrofuryl, tetrahydropyrrolyl, tetrahydropyranyl and piperidinyl, optionally substituted with 0, 1, 2, 3 or 4 of R⁵²,
or, R^{a5} and R^{b5} attached to the same nitrogen atom, taken together with the nitrogen atom to which they are attached, form a cyclic group selected from: 1-azetidinyl, 1-tetrahydropyrrolyl, 1-piperidinyl, 1-piperazinyl and morpholinyl, optionally substituted with 0, 1, 2, 3 or 4 of R⁵²;
R^{c5} is each independently selected from C₁₋₃ alkyl, C₃₋₆ cycloalkyl, 3-oxetanyl, 3-azetidinyl, tetrahydrofuryl, tetrahydropyrrolyl, tetrahydropyranyl and piperidinyl, optionally substituted with 0, 1, 2, 3 or 4 of R⁵²,
R^{d5} is selected from:
   1) hydrogen, cyano, nitro and -S(=O)₂-(C₁₋₃ alkyl); or
   2) C₁₋₃ alkyl and cyclopropyl;
R⁵² is selected from: oxo, fluorine, cyano, hydroxyl, C₁₋₃ alkoxy, cyclopropoxy, C₁₋₃ alkyl, cyclopropyl, difluoromethyl, trifluoromethyl, hydroxymethyl, 1-hydroxyethyl, 2-hydroxyethyl, 2-hydroxy-2-propyl, amino and C₁₋₃ alkylamino.

In one embodiment, L² is each independently selected from -CH₂-, -NH-, -O- and -S-; L² is each independently substituted with 0, 1 or 2 of R⁵; R⁵ is independently selected from:
1) two R⁵ groups attached to the same L² or attached to two adjacent L², taken together with the one or two L² atom(s) to which they are attached, form a C₃₋₆ aliphatic carbocyclylene, or 3- to 6-membered aliphatic heterocyclylene, optionally substituted with 0, 1, 2, 3, or 4 of R⁵², or
2) two R⁵ groups attached to two nonadjacent L², taken together with the two L² atoms to which they are attached and all other L² atom(s) between said two L² atoms, form a C₃₋₆ aliphatic carbocyclylene, or 3- to 6-membered aliphatic heterocyclylene, optionally substituted with 0, 1, 2, 3, or 4 of R⁵².

In one embodiment, L² is each independently selected from -CH₂-, -NH-, -O- and -S-; L² is each independently substituted with 0, 1 or 2 of R⁵;
when L² is selected from -CH₂-, R⁵ is independently selected from:
1) two R⁵ groups attached to the same L², taken together with the L² atom to which they are attached, form a cyclic group selected from: 1,1-cyclopropyl, 1,1-cyclobutyl, 1,1-cyclopentyl, 1,1-cyclohexyl, 3,3-oxetanyl, 3,3-azetidinyl, 3,3-tetrahydrofuryl, 3,3-tetrahydropyrrolyl, 3,3-tetrahydropyranyl, 4,4-tetrahydropyranyl, 3,3-piperidinyl and 4,4-piperidyl, optionally substituted with 0, 1, 2, 3, or 4 of R⁵²; or
2) two R⁵ groups attached to two adjacent L², taken together with the two L² atoms to which they are attached, form a cyclic group selected from: 1,2-cyclopropyl, 1,2-cyclobutyl, 1,2-cyclopentyl, 1,2-cyclohexyl, 3,4-tetrahydrofuryl, 3,4-tetrahydropyrrolyl, 3,4-tetrahydropyranyl and 3,4-piperidinyl, optionally substituted with 0, 1, 2, 3, or 4 of R⁵²; or
3) two R⁵ groups attached to two nonadjacent L², taken together with the two L² atoms to which they are attached and all other L² atom(s) between said two L² atoms, form a cyclic group selected from: 1,3-cyclobutyl, 1,3-cyclopentyl, 1,3-cyclohexyl, 1,4-cyclohexyl, 2,5-tetrahydrofuryl, 2,5-tetrahydropyrrolyl, 2,6-tetrahydropyranyl, 3,5-tetrahydropyranyl, 2,6-piperidinyl, 3,5-piperidinyl, 2,6-morpholinyl, 2,5-morpholinyl, 3,5-morpholinyl, 2,6-piperazinyl and 2,5-piperazinyl, optionally substituted with 0, 1, 2, 3, or 4 of R⁵².

In one embodiment, L² is each independently selected from -CH₂-, -NH-, -O- and -S-; L² is each independently substituted with 0, 1 or 2 of R⁵;
when L² is selected from -NH-, R⁵ is independently selected from:
1) two R⁵ groups attached to two adjacent L², taken together with the two L² atoms to which they are attached, form a cyclic group selected from: 1,2-aziridinyl, 1,2-azetidinyl, 1,2-tetrahydropyrrolyl, 1,2-piperidinyl, 3,4-morpholinyl and 1,2-piperanyl, optionally substituted with 0, 1, 2, 3, or 4 of R⁵²; or
2) two R⁵ groups attached to two nonadjacent L², taken together with the two L² atoms to which they are attached and all other L² atom(s) between said two L² atoms, form a cyclic group selected from: 1,3-azetidinyl, 1,3-tetrahydropyrrolyl, 1,3-piperidinyl, 1,4-piperidinyl and 1,4-piperazinyl, optionally substituted with 0, 1, 2, 3, or 4 of R⁵².

In one embodiment, R⁵² is selected from: oxo, fluorine, cyano, hydroxyl, C₁₋₃ alkoxy, cyclopropoxy, C₁₋₃ alkyl, cyclopropyl, difluoromethyl, trifluoromethyl, hydroxymethyl, 1-hydroxyethyl, 2-hydroxyethyl, 2-hydroxy-2-propyl, amino and C₁₋₃ alkylamino.

In one embodiment, L² is each independently selected from -CH₂-, -NH-, -O- and -S-; L² is each independently substituted with 0, 1 or 2 of R⁵, R⁵ is independently selected from:
1) oxo, halogen, cyano, hydroxyl, C₁₋₃ alkoxy, amino, C₁₋₃ alkylamino, and C₁₋₃ alkyl; or
2) when L² is selected from carbon, two R⁵ groups attached to the same L², taken together with said L² atom to which they are attached, form C₃, C₄ and C₅ cycloalkylene; wherein said cycloalkylene is optionally substituted with 0, 1, 2, 3, or 4 of R⁵²; or
3) when two adjacent L² are selected from carbon and nitrogen, two R⁵ groups attached to said two L², taken together with said two L² atoms to which they are attached, form C₄, C₅ and C₆ cycloalkylene or C₄, C₅ and C₆ heterocyclylene; wherein said cycloalkylene and heterocyclylene is optionally substituted with 0, 1, 2, 3, or 4 of R⁵²; or
4) when two nonadjacent L² are selected from carbon and nitrogen, two R⁵ groups attached to said two L², taken together with said two L² atoms to which they are attached, form C₄, C₅ and C₆ cycloalkylene or C₄, C₅ and C₆ heterocyclylene; wherein said cycloalkylene and heterocyclylene is optionally substituted with 0, 1, 2, 3, or 4 of R⁵²,
wherein,
R⁵² is selected from: halogen, cyano, hydroxyl, C₁₋₃ alkoxy and C₁₋₃ alkyl.

In one embodiment, L² is each independently selected from:
1) -CH₂-, -CF₂-, -CH(C₁₋₃ alkyl)-, -C(1,1-cyclopropylene)-, -C(C₁₋₃ alkyl)₂-, -CH(OH)-, -CH(C₁₋₃ alkoxy)-, -CH(CN)-, -C(=O)-, -CH(NH₂)-, -CH(NHC₁₋₃ alkyl)-, -CH(N(C₁₋₃ alkyl)₂)-, -NH-, -N(C₁₋₃ alkyl)-, -N(cyclopropyl)-, -N(CH₂CH₂OH)-, -N(C(=O)C₁₋₃ alkyl)-, -N(C(=O)-cyclopropyl)-, -N(C(=O)CH₂OH)-, -N(C(=O)CH₂N(C₁₋₃ alkyl)₂)-, -N(S(=O)₂C₁₋₃ alkyl)-, -N(S(=O)₂-cyclopropyl)-, -O-, -S(=O)-, and -S(=O)₂-; or
2) -CH(C₁₋₃ alkyl)-, -C(C₁₋₃ alkyl)₂-, -C(1,1-cyclopropylene)-, and -C(1,1-cyclobutylene)-, optionally substituted with 0, 1, 2 or 3 substituent selected from fluorine, hydroxyl, cyano and methoxy; or
3) two adjacent L² selected from carbon, taken together with a methylene group connected to said two L², form 1,2-cyclopropylene, optionally substituted with 0, 1, 2, or 3 of substituent selected from fluorine, hydroxyl, cyano and methoxy; or
4) three sequentially connected L² selected from carbon, taken together with a methylene group connected to the carbon atoms at both ends of said three L², form 1,3-cyclobutylene, optionally substituted with 0, 1, 2, or 3 of substituent selected from fluorine, hydroxyl, cyano and methoxy.

In one embodiment, R^{G} is selected from:
1) halogen, cyano, hydroxyl, C₁₋₃ alkoxy, amino and C₁₋₃ alkylamino; or
2) C₁₋₄ alkyl, C₁₋₄ alkylene, C₂₋₄ alkenyl, C₂₋₄ alkynyl, C₃₋₆ cycloalkyl and 3- to 8-membered aliphatic heterocyclyl, optionally substituted with 0, 1, 2, 3 or 4 substituents independently selected from: oxo, halogen, hydroxyl, hydroxymethyl, carboxyl, cyano, C₁₋₃ alkoxy, amino, and C₁₋₃ alkylamino.

In one embodiment, ring B is selected from phenyl and 5- to 6-membered monocyclic heteroaryl, and is connected to L² and to the azaindazole ring at the 5-position through two different ring-forming atoms thereof, respectively; wherein the shortest distance between said two ring-forming atoms is 1, 2 or 3 chemical bonds.

In one embodiment, ring B is selected from phenyl, and is connected to L² and to the azaindazole ring at the 5-position through two different ring-forming atoms thereof, respectively; wherein said two ring-forming atoms are in *ortho-, meta-* or para-position relationship.

In one embodiment, L² is each independently selected from -CH₂-, -NH-, -O-, and -S-; wherein, each L² is independently substituted with 0, 1, or 2 of R⁵; two adjacent L² may be connected through single bond, double bond, or triple bond, provided that the chemical valence allows; p of L² attached sequentially form a linker connecting ring A and ring B; wherein the two L² at both ends are attached to ring A and ring B through single bond, respectively;
R⁵ is independently selected from:
   1) oxo, halogen, cyano, hydroxyl, C₁₋₃ alkoxy, amino, C₁₋₃ alkylamino, and C₁₋₃ alkyl; or
   2) when L² is selected from carbon, two R⁵ groups attached to the same L², taken together with said L² atom to which they are attached, form C₃, C₄ and C₅ cycloalkylene; wherein said cycloalkylene is optionally substituted with 0, 1, 2, 3, or 4 of R⁵²; or
   3) when two adjacent L² are selected from carbon and nitrogen, two R⁵ groups attached to said two L², taken together with said two L² atoms to which they are attached, form C₄, C₅ and C₆ cycloalkylene or C₄, C₅ and C₆ heterocyclylene; wherein said cycloalkylene and heterocyclylene is optionally substituted with 0, 1, 2, 3, or 4 of R⁵²; or
   4) when two nonadjacent L² are selected from carbon and nitrogen, two R⁵ groups attached to said two L², taken together with said two L² atoms to which they are attached, form C₄, C₅ and C₆ cycloalkylene or C₄, C₅ and C₆ heterocyclylene; wherein said cycloalkylene and heterocyclylene is optionally substituted with 0, 1, 2, 3, or 4 of R⁵²;
wherein, R⁵² is selected from: halogen, cyano, hydroxyl, C₁₋₃ alkoxy and C₁₋₃ alkyl.

In one embodiment, p is selected from 2, 3, 4, 5, 6, 7, 8 and 9.

In one embodiment, p is selected from 1, 2, 3, 4, 5, 6, 7 and 8.

In one embodiment, p is selected from 4, 5, 6, 7 and 8.

In one embodiment, p is selected from 5, 6 and 7.

In one embodiment, p is selected from 1, 2, 3 and 4.

In one embodiment, p is selected from 2, 3 and 4.

In one embodiment, the sum of m1 and m2 is m.

In one embodiment, m is selected from 0, 1, 2, 3, 4, 5 and 6.

In one embodiment, m is selected from 0, 1, 2, 3 and 4.

In one embodiment, m is selected from 0, 1 and 2.

In one embodiment, m is selected from 0 and 1.

In one embodiment, n is selected from 0, 1, 2, 3, 4, 5 and 6.

In one embodiment, n is selected from 0, 1, 2 and 3.

In one embodiment, n is selected from 1 and 2.

In one embodiment, n is selected from 0 and 1.

In one embodiment, n is selected from 1.

In one embodiment, the compound described herein is selected from one of the following structures:

| Compound No. | Structure | Name |
|---|---|---|
| Compound 1 | 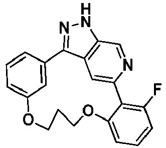 | 16-fluoro-7,11-dioxa-19,22,23-triazapentacyclo[16.5.2.1^{2,6}.0^{12,17}.0^{21,24}]hexacosa -1(23),2(26),3,5,12,14,16,18,20,24-decaene |
| Compound 3 | 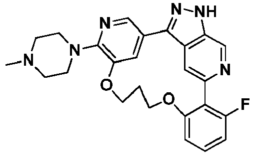 | 16-fluoro-5-(4-methylpiperazin-1 -yl)-7,11-dioxa-4,19,22,23-tetraazapentacyclo[16.5.2.1^{2,6}.0^{12,17}.0^{21,24}]hexac osa-1(23),2(26),3,5,12,14,16,18,20,24-decaene |
| Compound 4 | 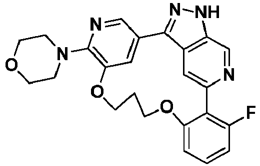 | 16-fluoro-5-(morpholin-4-yl)-7,11-dioxa-4,19,22,23-tetraazapentacyclo[16.5.2.1^{2,6}.0^{12,17}.0^{21,24}]hexac osa-1(23),2(26),3,5,12,14,16,18,20,24-decaene |
| Compound 5 | 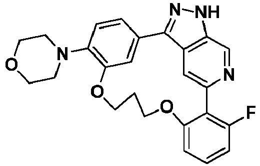 | 16-fluoro-5-(morpholin-4-yl)-7,11-dioxa-19,22,23-triazapentacyclo[16.5.2.1^{2,6}.0^{12,17}.0^{21,24}]hexacosa -1(23),2(26),3,5,12, 14,16, 18,20,24-decaene |
| Compound 6 | 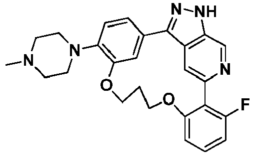 | 16-fluoro-5-(4-methylpiperazin-1-yl)-7,11-dioxa-19,22,23-triazapentacyclo[16.5.2.1^{2,6}.0^{12,17}.0^{21,24}]hexacosa -1(23),2(26),3,5,12, 14,16, 18,20,24-decaene |
| Compound 7 | 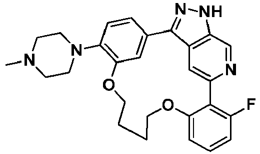 | 17-fluoro-5-(4-methylpiperazin-1-yl)-7,12-dioxa-20,23,24-triazapentacyclo[17.5.2.1^{2,6}.0^{13,18}.0^{22,25}]heptacos a-1(24),2(27),3,5,13,15,17,19,21,25-decaene |
| Compound 9 | 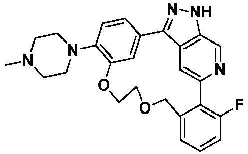 | 16-fluoro-5-(4-methylpiperazin-1-yl)-7,10-dioxa-19,22,23-triazapentacyclo[16.5.2.1^{2,6}.0^{12,17}.0^{21,24}]hexacosa -1(23),2(26),3,5,12, 14,16, 18,20,24-decaene |
| Compound 10 | 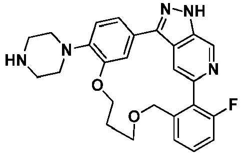 | 17-fluoro-5-(piperazin-1-yl)-7,11-dioxa-20,23,24-triazapentacyclo[17.5.2.1^{2,6}.0^{13,18}.0^{22,25}]heptacos a-1(24),2(27),3,5,13,15,17,19,21,25-decaene |
| Compound 11 | 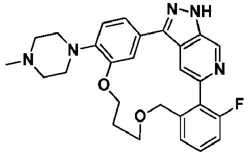 | 17-fluoro-5-(4-methylpiperazin-1-yl)-7,11-dioxa-20,23,24-triazapentacyclo[17.5.2.1^{2,6}.0^{13,18}.0^{22,25}]heptacos a-1(24),2(27),3,5,13,15,17,19,21,25-decaene |
| Compound 12 | 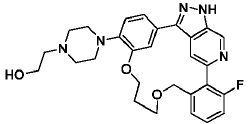 | 17-fluoro-5-(4-(2-hydroxyethyl)piperazin-1-yl)-7,11-dioxa-20,23,24-triazapentacyclo[17.5.2.1^{2,6}.0^{13,18}.0^{22,25}]heptacos a-1(24),2(27),3,5,13,15,17,19,21,25-decaene |
| Compound 13 | 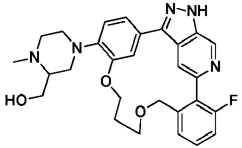 | 17-fluoro-5-(3-hydroxymethyl-4-methylpiperazin-1-yl)-7,11-dioxa-20,23,24-triazapentacyclo[17.5.2.1^{2,6}.0^{13,18}.0^{22,25}]heptacos a-1(24),2(27),3,5,13,15,17,19,21,25-decaene |
| Compound 14 | 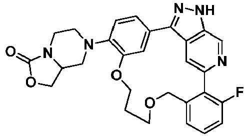 | 7-{17-fluoro-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-5-yl}-hexahydro-1*H*-[1,3]oxazolo[3,4-a]pyrazin-3-one |
| Compound 17 | 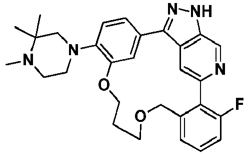 | 17-fluoro-5-(3,3,4-trimethylpiperazin-1-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 18 | 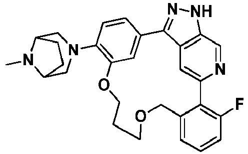 | 17-fluoro-5-{8-methyl-3,8-diazabicyclo[3.2. 1]octan-3-yl 1-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 19 | 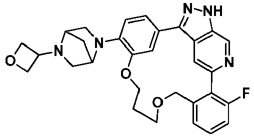 | 17-fluoro-5-[5-(oxetan-3-yl)-2,5-diazabicyclo[2.2.1]heptan-2-yl]-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 89 | 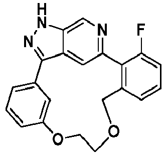 | 16-fluoro-7,10-dioxa-19,22,23-triazapentacyclo[16.5.2.1^{2,6}.0^{12,17}.0^{21,24}]hexacosa -1(23),2(26),3,5,12(17),13,15,18,20,24-decaene |
| Compound 90 | 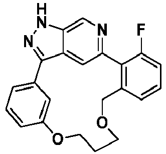 | 17-fluoro-7,11-dioxa-20,23,24-triazapentacyclo[17.5.2.1^{2,6}.0^{13,18}.0^{22,25}]heptacos a-1(24),2(27),3,5,13(18),14,16,19,21,25-decaene |
| Compound 91 | 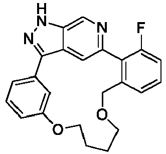 | 18-fluoro-7,12-dioxa-21,24,25-triazapentacyclo[18.5.2.1^{2,6}.0^{14,19}.0^{21,26}]octacosa -1(25),2(28),3,5,14(19),15,17,20,22,26-decaene |
| Compound 92 | 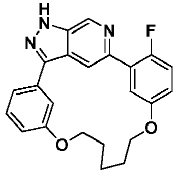 | 17-fluoro-7,13-dioxa-20,23,24-triazapentacyclo[17.5.2.1^{2,6}.1^{14,18}.0^{22,25}]octacosa -1(24),2(28),3,5,14,16,18(27),19,21,25-decaene |
| Compound 93 | 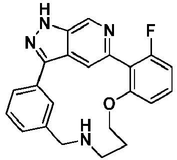 | 17-fluoro-12-oxa-8,20,23,24-tetraazapentacyclo[17.5.2.1^{2,6}.0^{13,18}.0^{22,25}]heptac osa- 1 (24),2(27),3,5,13 (18),14,16,19,21,25-decaene |
| Compound 94 | 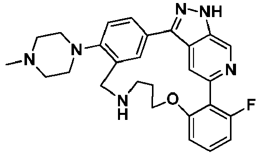 | 17-fluoro-5 -(4-methylpiperazin-1 -yl)-12-oxa-8,20,23,24-tetraazapentacyclo[17.5.2.1^{2,6}.0^{13,18}.0^{22,25}]heptac osa-1(24),2(27),3,5,13(18),14,16,19,21,25- decaene |
| Compound 95 | 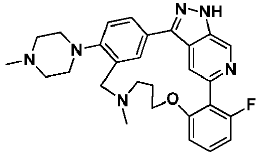 | 17-fluoro-8-methyl-5-(4-methylpiperazin-1-yl)-12-oxa-8,20,23,24-tetraazapentacyclo[17.5.2.1^{2,6}.0^{13,18}.0^{22,25}]heptac osa-1(24),2(27),3,5,13(18),14,16,19,21,25-decaene |
| Compound 96 | 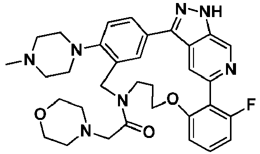 | 1-[17-fluoro-5-(4-methylpiperazin-1-yl)-12-oxa-8,20,23,24-tetraazapentacyclo[17.5.2.1^{2,6}.0^{13,18}.0^{22,25}]heptac osa-1(24),2(27),3,5,13(18),14,16,19,21,25-decaen-8-yl]-2-(morpholin-4-yl)ethan-1-one |
| Compound 97 | 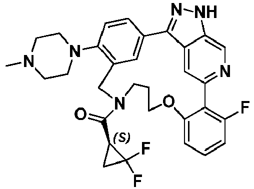 | 8-[(1*S*)-2,2-difluorocyclopropanecarbonyl]-17-fluoro-5-(4-methylpiperazin-1-yl)-12-oxa-8,20,23,24-tetraazapentacyclo[17.5.2.1^{2,6}.0^{13,18}.0^{22,25}]heptac osa-1(24),2(27),3,5,13(18),14,16,19,21,25- decaene |
| Compound 98 | 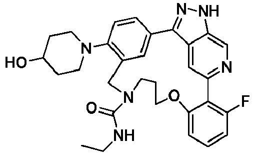 | *N-*ethyl-17-fluoro-5-(4-hydroxypiperidin-1-yl)-12-oxa-8,20,23,24-tetraazapentacyclo[17.5.2.1^{2,6}.0^{13,18}.0^{22,25}]heptac osa-1(24),2(27),3,5,13(18),14,16,19,21,25-decaene-8-carboxamide |
| Compound 99 | 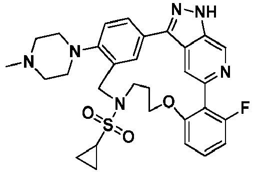 | 8-(cyclopropanesulfonyl)-17-fluoro-5-(4-methylpiperazin-1-yl)-12-oxa-8,20,23,24-tetraazapentacyclo[17.5.2.1^{2,6}.0^{13,18}.0^{22,25}]heptac osa-1(24),2(27),3,5,13(18),14,16,19,21,25-decaene |
| Compound 100 | 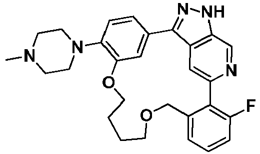 | 18-fluoro-5-(4-methylpiperazin-1-yl)-7,12-dioxa-21,24,25-triazapentacyclo[18.5.2.1^{2,6}.0^{14,19}.0^{23,26}]octacosa -1(25),2(28),3,5,14(19),15,17,20,22,26-decaene |
| Compound 101 | 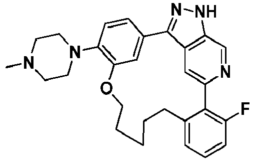 | 17-fluoro-5-(4-methylpiperazin-1-yl)-7-oxa-20,23,24-triazapentacyclo[17.5.2.1^{2,6}.0^{13,18}.0^{22,25}]heptacos a-1(24),2(27),3,5,13(18),14,16,19,21,25-decaene |
| Compound 102 | 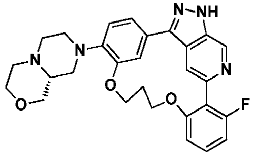 | 5-[(9aR)-octahydropyrazino[2,1-c][1,4]oxazin-8-yl]-16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 103 | 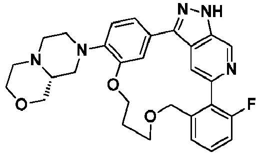 | 5-[(9aR)-octahydropyrazino[2,1-c][1,4]oxazin-8-yl]-17-fluoro-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 104 | 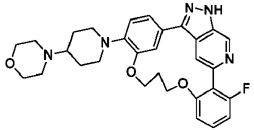 | 16-fluoro-5-[4-(morpholin-4-yl)piperidin-1-yl]-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 105 | 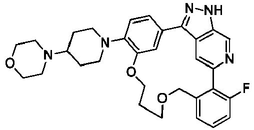 | 17-fluoro-5-[4-(morpholin-4-yl)piperidin-1-yl]-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 106 | 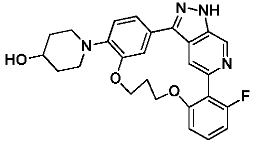 | 16-fluoro-5-(4-hydroxypiperidin-1-yl)-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 107 | 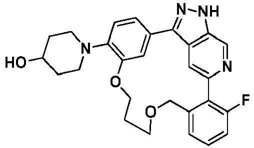 | 17-fluoro-5-(4-hydroxypiperidin-1-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 108 | 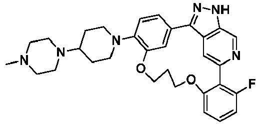 | 16-fluoro-5-[4-(4-methylpiperazin-1-yl)piperidin-1-yl]-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 109 | 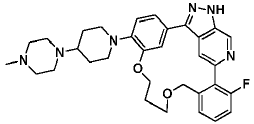 | 17-fluoro-5-[4-(4-methylpiperazin-1-yl)piperidin-1-yl]-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 110 | 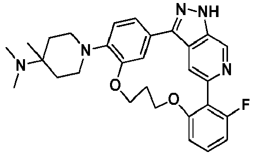 | 1-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-*N*,*N*,4-trimethylpiperidin-4-amine |
| Compound 111 | 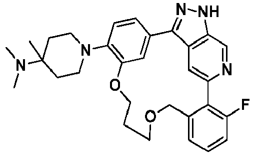 | 1-{17-fluoro-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-5-yl}-*N*,*N*,4-trimethylpiperidin-4-amine |
| Compound 112 | 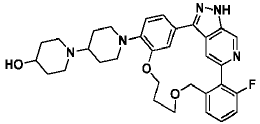 | 1'-{17-fluoro-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-5-yl}-[1,4'-bipiperidin]-4-ol |
| Compound 113 | 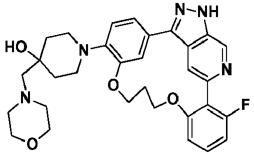 | 1-{16-fluoro-7,11-dioxa-19,22,23- triazapentacyclo[16.5.2.1^{2,6}.0^{12,17}.0^{21,24}]hexacosa -1(23),2,4,6(26),12(17),13,15,18,20,24-decaen-5-yl}-1-4-[(morpholin-4-yl)methyl]piperidin-4-ol |
| Compound 114 | 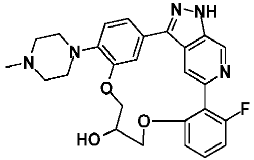 | 16-fluoro-5-(4-methylpiperazin-1 -yl)-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-9-ol |
| Compound 115 | 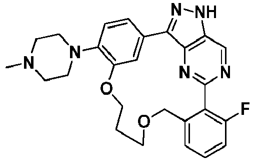 | 17-fluoro-5-(4-methylpiperazin-1-yl)-7,11-dioxa-20,23,24,26-tetraazapentacyclo[17.5.2.1^{2,6}.0^{13,18}.0^{22,25}]heptac osa-1(24),2,4,6(27),13(18),14,16,19,21,25-decaene |
| Compound 116 | 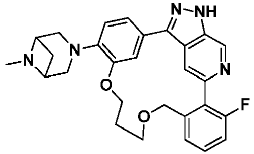 | 17-fluoro-5-{ 6-methyl-3,6-diazabicyclo[3.1.1]heptan-3-yl}-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 117 | 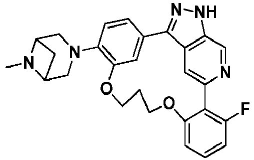 | 16-fluoro-5-{ 6-methyl-3,6-diazabicyclo[3.1.1]heptan-3-yl}-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 118 | 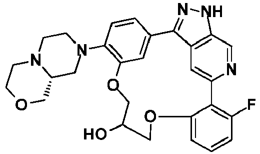 | 5-[(9a*R*)-octahydropyrazino[2,1-c][1,4]oxazin-8-yl]-16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-9-ol |
| Compound 119 | 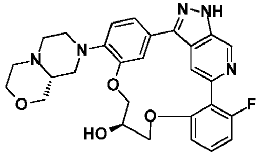 | (9*R*)-5-[(9a*R*)-octahydropyrazino[2,1-c][1,4]oxazin-8-yl]-16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-9-ol |
| Compound 120 | 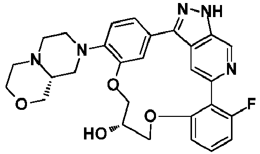 | (9*S*)-5-[(9a*R*)-octahydropyrazino[2,1-c][1,4]oxazin-8-yl]-16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-9-ol |
| Compound 121 | 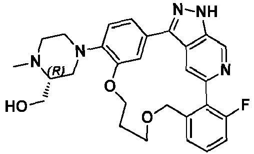 | [(2*R*)-4-{17-fluoro-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-5-yl}-1-methylpiperazin-2-yl]methanol |
| Compound 122 | 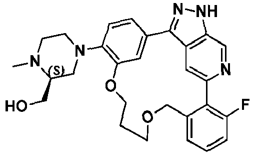 | [(2*S*)-4-{17-fluoro-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-5-yl}-1-methylpiperazin-2-yl]methanol |
| Compound 123 | 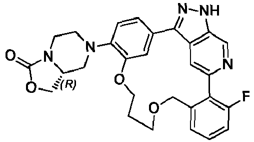 | (8a*R*)-7-{17-fluoro-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-5-yl}-hexahydro-1*H*-[1,3]oxazolo[3,4-a]pyrazin-3-one |
| Compound 124 | 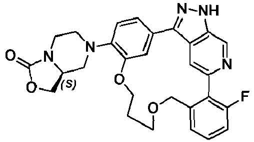 | (8a*S*)-7-{17-fluoro-7,1 1-dioxa-20,23,24-triazapentacyclo[17.5.2.1^{2,6}.0^{13,18}.0^{22,25}]heptacos a-1(24),2,4,6(27),13(18),14,16,19,21,25-decaen-5-yl}-hexahydro-1*H*-[1,3]oxazolo[3,4-a]pyrazin-3-one |
| Compound 125 | 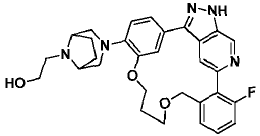 | 2-(3-{17-fluoro-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-5-yl}-3,8-diazabicyclo[3.2.1]octan-8-yl)ethan-1-ol |
| Compound 126 | 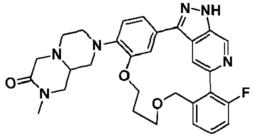 | 8-{17-fluoro-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-5-yl}-2-methyl-octahydro-1*H*-[1,4]diazino[1,2-a]pyrazin-3-one |
| Compound 127 | 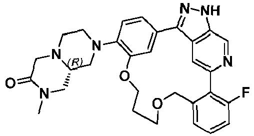 | (9a*R*)-8- {17-fluoro-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-5-yl}-2-methyl-octahydro-1*H*-[1,4]diazino[1,2-a]pyrazin-3-one |
| Compound 128 | 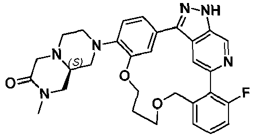 | (9a*S*)-8-{17-fluoro-7,11-dioxa-20,23,24-triazapentacyclo[17.5.2.1^{2,6}.0^{13,18}.₀^{22,25}]heptacos a-1(24),2,4,6(27),13(18),14,16,19,21,25-decaen-5-yl}-2-methyl-octahydro-1*H*-[1,4]diazino[1,2-a]pyrazin-3-one |
| Compound 129 | 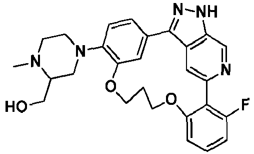 | [4-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-1-methylpiperazin-2-yl]methanol |
| Compound 130 | 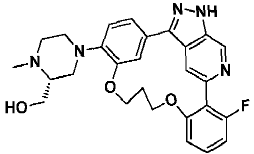 | [(2*R*)-4-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-1-methylpiperazin-2-yl]methanol |
| Compound 131 | 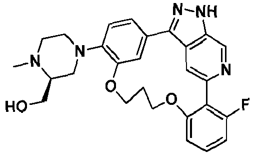 | [(2*S*)-4-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-1-methylpiperazin-2-yl]methanol |
| Compound 132 | 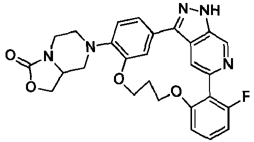 | 7-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-hexahydro-1*H*-[1,3]oxazolo[3,4-a]pyrazin-3-one |
| Compound 134 | 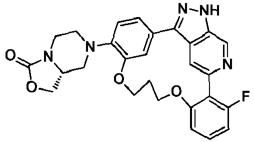 | (8a*R*)-7-{16-fluoro-7,11-dioxa-19,22,23- triazapentacyclo[16.5.2.1^{2,6}.0^{12,17}.0^{21,24}]hexacosa -1(23),2,4,6(26),12(17),13,15,18,20,24-decaen-5-yl}-hexahydro-1*H*-[1,3]oxazolo[3,4-a]pyrazin-3-one |
| Compound 135 | 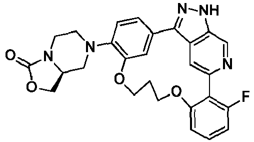 | (8a*S*)-7-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-hexahydro-1*H*-[1,3]oxazolo[3,4-a]pyrazin-3-one |
| Compound 136 | 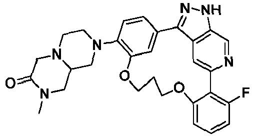 | 8-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-2-methyl-octahydro-1*H*-[1,4]diazino[1,2-a]pyrazin-3-one |
| Compound 137 | 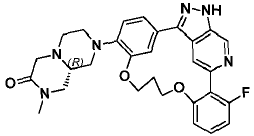 | (9a*R*)-8-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-2-methyl-octahydro-1*H*-[1,4]diazino[1,2-a]pyrazin-3-one |
| Compound 138 | 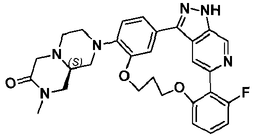 | (9a*S*)-8-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-2-methyl-octahydro-1*H*-[1,4]diazino[1,2-a]pyrazin-3-one |
| Compound 139 | 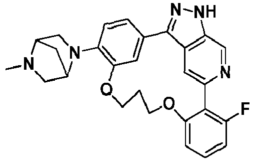 | 16-fluoro-5-[5-methyl-2,5-diazabicyclo[2.2.1]heptan-2-yl]-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 140 | 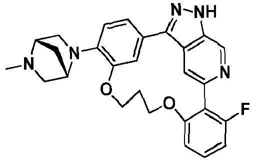 | 16-fluoro-5-[(1*S*,4*S*)-5-methyl-2,5-diazabicyclo[2.2.1]heptan-2-yl]-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 141 | 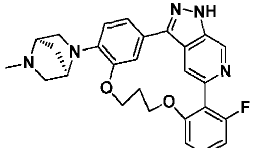 | 16-fluoro-5-[(1*R*,4*R*)-5-methyl-2,5-diazabicyclo[2.2.1]heptan-2-yl]-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 142 | 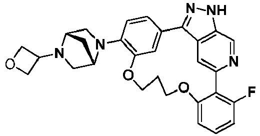 | 16-fluoro-5-[(1*S*,4*S*)-5-(oxetan-3-yl)-2,5-diazabicyclo[2.2.1]heptan-2-yl]-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 143 | 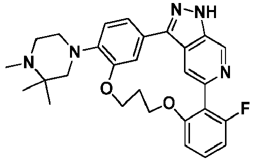 | 16-fluoro-5-(3,3,4-trimethylpiperazin-1-yl)-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 144 | 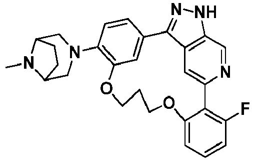 | 16-fluoro-5-{8-methyl-3,8-diazabicyclo[3.2.1]octan-3-yl}-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 145 | 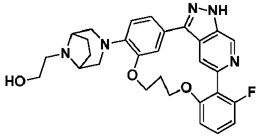 | 2-(3-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-3,8-diazabicyclo[3.2.1]octan-8-yl)ethan-1-ol |
| Compound 146 | 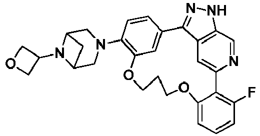 | 16-fluoro-5-[6-(oxetan-3-yl)-3,6-diazabicyclo[3 .1.1]heptan-3-yl]-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 147 | 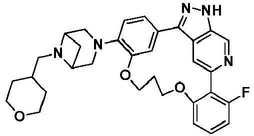 | 16-fluoro-5-{6-[(oxan-4-yl)methyl]-3,6-diazabicyclo[3.1.1]heptan-3-yl}-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 148 | 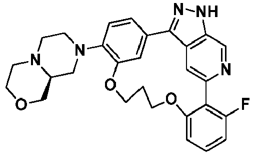 | 5-[(9a*S*)-octahydropyrazino[2,1-c][1,4]oxazin-8-yl]-16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 149 | 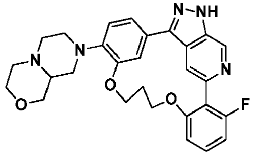 | 16-fluoro-5-{octahydropyrazino[2,1-c][1,4]oxazin-8-yl}-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 150 | 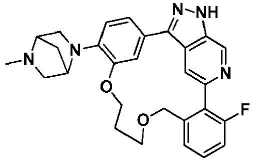 | 17-fluoro-5-[5-methyl-2,5-diazabicyclo[2.2.1]heptan-2-yl]-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 151 | 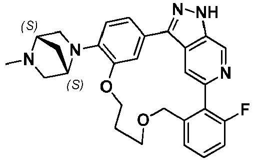 | 17-fluoro-5-[(1*S*,4*S*)-5-methyl-2,5-diazabicyclo[2.2.1]heptan-2-yl]-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 152 | 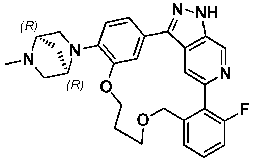 | 17-fluoro-5-[(IR,4R)-5-methyl-2,5-diazabicyclo[2.2.1]heptan-2-yl]-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 153 | 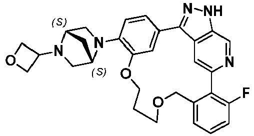 | 17-fluoro-5-[(1*S*,4*S*)-5-(oxetan-3-yl)-2,5-diazabicyclo[2.2.1]heptan-2-yl]-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 154 | 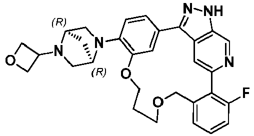 | 17-fluoro-5-[(1*R*,4*R*)-5-(oxetan-3-yl)-2,5-diazabicyclo[2.2.1]heptan-2-yl]-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |

In one embodiment, the compound described herein is selected from one of the following structures:

| Compound No. | Structure | Name |
|---|---|---|
| Compound 2 | 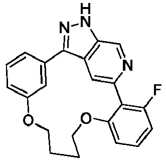 | 17-fluoro-7,12-dioxa-20,23,24-triazapentacyclo[17.5.2.1^{2,6}.0^{13,18}.0^{22,25}]heptacos a-1(24),2(27),3,5,13,15,17,19,21,25-decaene |
| Compound 8 | 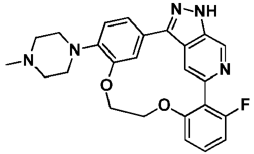 | 15-fluoro-5-(4-methylpiperazin-1-yl)-7,10-dioxa-18,21,22-triazapentacyclo[15.5.2.1^{2,6}.0^{11,16}.0^{20,23}]pentacos a-1(22),2(25),3,5,11,13,15,17,19,23-decaene |
| Compound 20 | 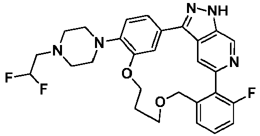 | 17-fluoro-5-(4-(2,2-difluoroethyl)-piperazin-1-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 21 | 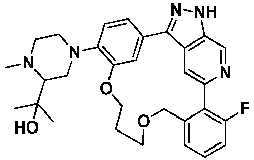 | 17-fluoro-5-(3-(2-hydroxy-2-methylethyl)-4-methylpiperazin-1-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 22 | 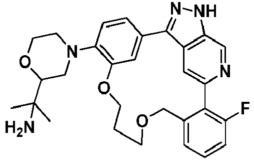 | 17-fluoro-5-(2-(2-amino-2-methylethyl)-morpholin-4-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 23 | 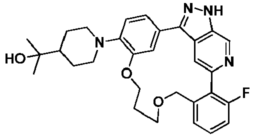 | 17-fluoro-5-(4-(2-hydroxy-2-methylethyl)-piperidinyl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 24 | 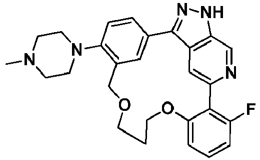 | 17-fluoro-5-(4-methylpiperazin-1-yl)-8,12-dioxa-20,23,24-triazapentacyclo[17.5.2.1^{2,6}.0^{13,18}.0^{22,25}]heptacos a-(24),2,4,6(27),13(18),14,16,19,21,25-decaene |
| Compound 25 | 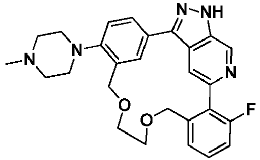 | 17-fluoro-5-(4-methylpiperazin-1-yl)-8,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 26 | 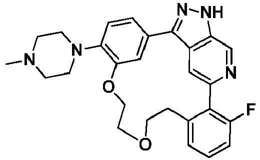 | 17-fluoro-5-(4-methylpiperazin-1-yl)-7,10-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 27 | 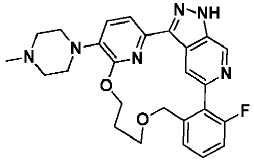 | 17-fluoro-5-(4-methylpiperazin-1-yl)-7,11-dioxa-20,23,24,27-tetraazapentacyclo[17.5.2.1^{2,6}.0^{13,18}.0^{22,25}]heptac osa-1(24),2,4,6(27),13(18),14,16,19,21,25-decaene |
| Compound 28 | 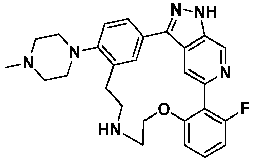 | 17-fluoro-5 -(4-methylpiperazin-1 -yl)-12-oxa-9,20,23,24-tetraazapentacyclo[17.5.2.1^{2,6}.0^{13,18}.0^{22,25}]heptac osa-1(24),2,4,6(27),13(18),14,16,19,21,25-decaene |
| Compound 29 | 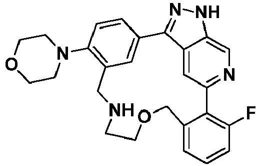 | 17-fluoro-5 -(morpholin-4-yl)-11 -oxa-8,20,23,24-tetraazapentacyclo[17.5.2.1^{2,6}.0^{13,18}.0^{22,25}]heptac osa-1(24),2,4,6(27),13(18),14,16,19,21,25-decaene |
| Compound 30 | 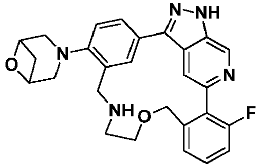 | 17-fluoro-5-{6-oxa-3-azabicyclo[3.1.1]heptan-3-yl}-11-oxa-8,20,23,24-tetraazapentacyclo[17.5.2.1^{2,6}.0^{13,18}.0^{22,25}]heptac osa-1(24),2,4,6(27),13(18),14,16,19,21,25-decaene |
| Compound 31 | 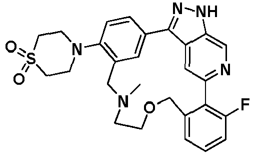 | 17-fluoro-8-methyl-5-(1,1-dioxothiomorpholin-4-yl)-11-oxa-8,20,23,24-tetraazapentacyclo[17.5.2.1^{2,6}.0^{13,18}.0^{22,25}]heptac osa-1(24),2,4,6(27),13(18),14,16,19,21,25-decaene |
| Compound 32 | 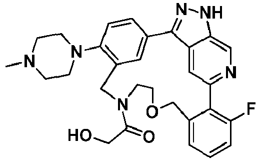 | 1-[17-fluoro-5-(4-methylpiperazin-1-yl)-11-oxa-8,20,23,24-tetraazapentacyclo[17.5.2.1^{2,6}.0^{13,18}.0^{22,25}]heptac osa-1(24),2,4,6(27),13(18),14,16,19,21,25-decaen-8-yl]-2-hydroxyethan-1-one |
| Compound 33 | 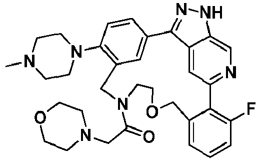 | 1-[17-fluoro-5-(4-methylpiperazin-1-yl)-11-oxa-8,20,23,24-tetraazapentacyclo[17.5.2.1^{2,6}.0^{13,18}.0^{22,25}]heptac osa-1(24),2,4,6(27),13(18),14,16,19,21,25-decaen-8-yl]-2-(morpholin-4-yl)ethan-1-one |
| Compound 34 | 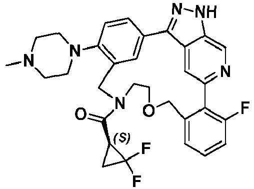 | 8-[(1S)-2,2-difluorocyclopropanecarbonyl]-17-fluoro-5-(4-methylpiperazin-1-yl)-11-oxa-8,20,23,24-tetraazapentacyclo[17.5.2.1^{2,6}.0^{13,18}.0^{22,25}]heptac osa-1(24),2,4,6(27),13(18),14,16,19,21,25-decaene |
| Compound 35 | 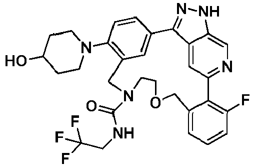 | 17-fluoro-5-(4-hydroxypiperidin-1-yl)-*N*-(2,2,2-trifluoroethyl)-11-oxa-8,20,23,24-tetraazapentacyclo[17.5.2.1^{2,6}.0^{13,18}.0^{22,25}]heptac osa-1(24),2,4,6(27),13(18),14,16,19,21,25-decaene-8-carboxamide |
| Compound 36 | 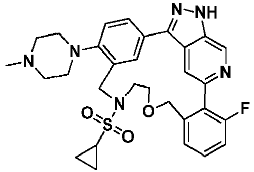 | 8-(cyclopropanesulfonyl)-17-fluoro-5-(4-methylpiperazin-1-yl)-11-oxa-8,20,23,24-tetraazapentacyclo[17.5.2.1^{2,6}.0^{13,18}.0^{22,25}]heptac osa-1(24),2,4,6(27),13(18),14,16,19,21,25-decaene |
| Compound 37 | 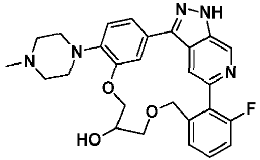 | 17-fluoro-9-hydroxy-5-(4-methylpiperazin-1-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 38 | 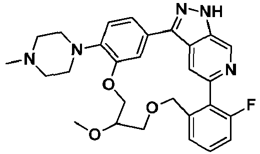 | 17-fluoro-9-methoxy-5-(4-methylpiperazin-1-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 39 | 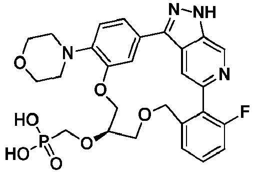 | ({[(9*R*)-17-fluoro-5-(morpholin-4-yl)-7,11- dioxa-20,23,24-triazapentacyclo[17.5.2.1^{2,6}.0^{13,18}.0^{22,25}]heptacos a-1(24),2,4,6(27),13(18),14,16,19,21,25-decaen-9-yl]oxy}methyl)phosphonic acid |
| Compound 40 | 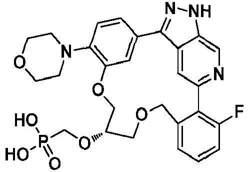 | ({[(9*S*)-17-fluoro-5-(morpholin-4-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-9-yl]oxy}methyl)phosphonic acid |
| Compound 41 | 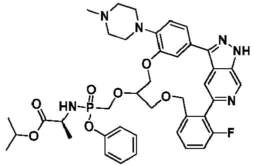 | Isopropyl (2*S*)-2-{[({[17-fluoro-5-(4-methylpiperazin-1-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-9-yl]oxy}methyl)(phenoxy)phosphoryl]amino}pro panoate |
| Compound 42 | 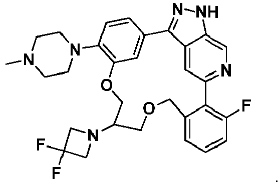 | 9-(3,3-difluoroazetidin-1-yl)-17-fluoro-5-(4-methylpiperazin-1-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 43 | 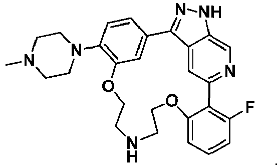 | 18-fluoro-5-(4-methylpiperazin-1-yl)-7,13-dioxa-10,21,24,25-tetraazapentacyclo[18.5.2.1^{2,6}.0^{14,19}.0^{23,26}]octaco sa-1(25),2,4,6(28),14(19),15,17,20,22,26-decaene |
| Compound 44 | 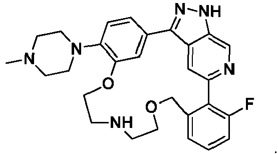 | 19-fluoro-5-(4-methylpiperazin-1-yl)-7,13-dioxa-10,22,25,26-tetraazapentacyclo[19.5.2.1^{2,6}.0^{15,20}.0^{24,27}]nonac osa-1(26),2,4,6(29),15(20),16,18,21,23,27-decaene |
| Compound 45 | 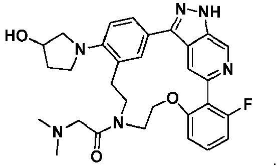 | 2-(dimethylamino)-1-[17-fluoro-5-(3-hydroxypyrrolidin-1-yl)-12-oxa-9,20,23,24-tetraazapentacyclo[17.5.2.1^{2,6}.0^{13,18}.0^{22,25}]heptac osa-1(24),2,4,6(27),13(18),14,16,19,21,25-decaen-9-yl]ethan-1-one |
| Compound 46 | 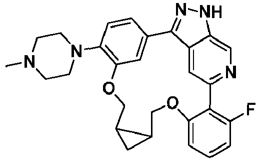 | 18-fluoro-5-(4-methylpiperazin-1-yl)-7,13-dioxa-21,24,25-triazahexacyclo[18.5.2.1^{2,6}.0^{9,11}.0^{14,19}.0^{23,26}]octa cosa-1(25),2,4,6(28),14(19),15,17,20,22,26-decaene |
| Compound 47 | 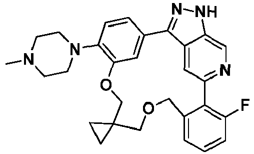 | 17'-fluoro-5'-(4-methylpiperazin-1-yl)-7',11'-dioxa-20',23',24'-triazaspiro[cyclopropane-1,9'-pentacyclo[17.5.2.1^{2,6}.0^{13,18}.0^{22,25}]heptacosane]-1'(24'),2',4',6'(27'),13'(18'),14',16',19',21',25'-decaene |
| Compound 48 | 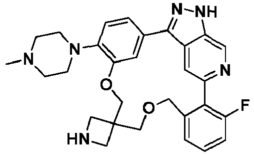 | 17'-fluoro-5'-(4-methylpiperazin-1-yl)-7',11'-dioxa-20',23',24'-triazaspiro[azetidine-3,9'-pentacyclo[17.5.2.1^{2,6}.0^{13,18}.0^{22,25}]heptacosane]-1'(24'),2',4',6'(27'),13'(18'),14',16',19',21',25'-decaene |
| Compound 52 | 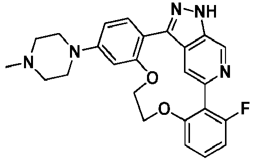 | 16-fluoro-5-(4-methylpiperazin-1-yl)-8,11-dioxa-19,22,23-triazapentacyclo[16.5.2.0^{2,7}.0^{12,17}.0^{21,24}]pentacos a-1(23),2,4,6,12(17),13,15,18,20,24-decaene |
| Compound 53 | 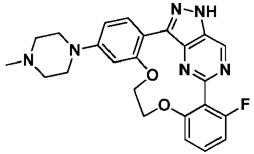 | 16-fluoro-5-(4-methylpiperazin-1-yl)-8,11-dioxa-19,22,23,25-tetraazapentacyclo[16.5.2.0^{2,7}.0^{12,17}.0^{21,24}]pentac osa-1(23),2,4,6,12(17),13,15,18,20,24-decaene |
| Compound 83 | 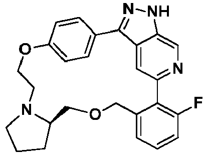 | (13*R*)-21-fluoro-6,15-dioxa-9,24,27,28-tetraazahexacyclo[21.5.2.2^{2,5}.0^{9,13}.0^{17,22}.0^{26,29}]do triaconta-1(28),2,4,17(22),18,20,23,25,29,31-decaene |

In one embodiment, the compound described herein is selected from one of the following structures:

| Compound No. | Structure | Name |
|---|---|---|
| Compound 49 | 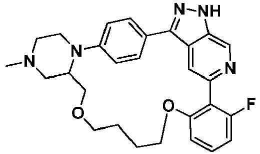 | 23-fluoro-9-methyl-13,18-dioxa-6,9,26,29,30-pentaazahexacyclo[23.5.2.2^{2,5}.0^{6,11}.0^{19,24}.0^{28,31}]te tratriaconta-1 (3 0),2,4,19(24),20,22,25,27,31,33-decaene |
| Compound 50 | 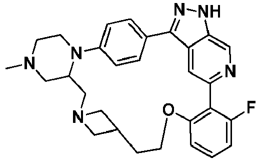 | 23-fluoro-9-methyl-18-oxa-6,9,13,26,29,30-hexaazaheptacyclo[23.5.2.2^{2,5}.1^{13,15}.0^{6,11}.0^{19,24}.0^{2 8,31}]pentatriaconta-1 (30),2,4,19(24),20,22,25,27,31,34-decaene |
| Compound 51 | 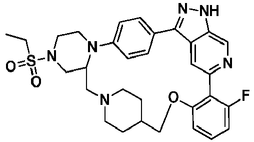 | 9-(ethanesulfonyl)-23-fluoro-18-oxa-6,9,13,26,29,30-hexaazaheptacyclo[23.5.2.2^{2,5}.2^{13,16}.0^{6,11}.01^{9,24}.0^{2 8,31}]hexatriaconta-1(30),2,4,19(24),20,22,25,27,31,35-decaene |
| Compound 55 | 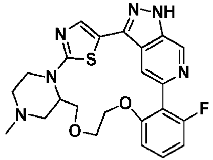 | 21-fluoro-9-methyl-13,16-dioxa-31-thia-4,6,9,24,27,28-hexaazahexacyclo[21.5.2.1^{2,5}.0^{6,11}.0^{17,22}.0^{26,29}]he ntriaconta-1(28),2,4,17(22),18,20,23,25,29-nonaene |
| Compound 56 | 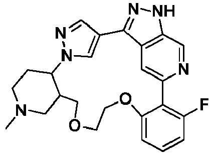 | 21-fluoro-9-methyl-13,16-dioxa-4,5,9,24,27,28-hexaazahexacyclo[21.5.2.1^{2,5}.0^{6,11}.0^{17,22}.0^{26,29}]he ntriaconta-1(28),2(31),3,17(22),18,20,23,25,29-nonaene |
| Compound 57 | 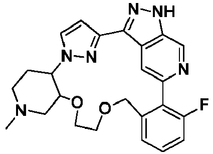 | 21-fluoro-9-methyl-12,15-dioxa-5,9,24,27,28,31-hexaazahexacyclo[21.5.2.1^{2,5}.0^{6,11}.0^{17,22}.0^{26,29}]he ntriaconta-1(28),2(31),3,17(22),18,20,23,25,29-nonaene |
| Compound 58 | 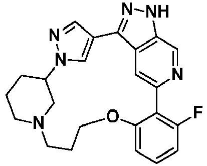 | 19-fluoro-14-oxa-4,5,10,22,25,26-hexaazahexacyclo[19.5.2.1^{2,5}.1^{6,10}.0^{15,20}.0^{24,27}]tri aconta-1 (26),2(3 0),3,15(20),16,18,21,23,27-nonaene |
| Compound 59 | 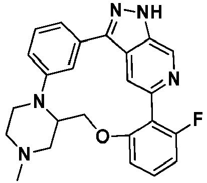 | 19-fluoro-10-methyl-14-oxa-7,10,22,25,26-pentaazahexacyclo[19.5.2.1^{2,6}.0^{7,12}.0^{15,20}.0^{24,27}]n onacosa-1(26),2,4,6(29),15(20),16,18,21,23,27-decaene |
| Compound 60 | 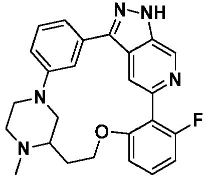 | 19-fluoro-10-methyl-14-oxa-7,10,22,25,26-pentaazahexacyclo[19.5.2.1^{2,6}.1^{7,11}.0^{15,20}.0^{24,27}]tr iaconta-1(26),2,4,6(30),15(20),16,18,21,23,27-decaene |
| Compound 61 | 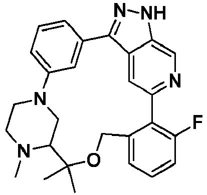 | 19-fluoro-10,12,12-trimethyl-13-oxa-7,10,22,25,26-pentaazahexacyclo[19.5.2.1^{2,6}.1^{7,11}.0^{15,20}.0^{24,27}]tr iaconta-1(26),2,4,6(30),15(20),16,18,21,23,27-decaene |
| Compound 62 | 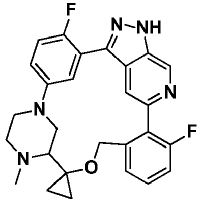 | 3',19'-difluoro-10'-methyl-13'-oxa-7',10',22',25',26'-pentaazaspiro[cyclopropane-1,12'-hexacyclo[19.5.2.1^{2,6}.1^{7,11}.0^{15,20}.0^{24,27}]triacontan e]-1'(26'),2',4',6'(30'),15'(20'),16',18',21',23',27'-decaene |
| Compound 63 | 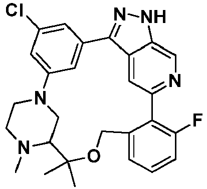 | 4-chloro-19-fluoro-10,12,12-trimethyl-13-oxa-7,10,22,25,26-pentaazahexacyclo[19.5.2.1^{2,6}.1^{7,11}.0^{15,20}.0^{24,27}]tr iaconta-1(26),2,4,6(30),15(20),16,18,21,23,27-decaene |
| Compound 64 | 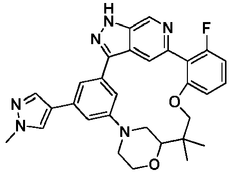 | 19-fluoro-12,12-dimethyl-4-(1-methyl-1*H-*pyrazol-4-yl)-10,14-dioxa-7,22,25,26-tetraazahexacyclo[19.5.2.1^{2,6}.1^{7,11}.0^{15,20}.0^{24,27}]tri aconta-1(26),2,4,6(30),15(20),16,18,21,23,27-decaene |
| Compound 65 | 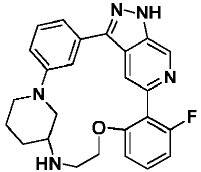 | 20-fluoro-15-oxa-7,12,23,26,27-pentaazahexacyclo[20.5.2.1^{2,6}.1^{7,11}.0^{16,21}.0^{25,28}]h entriaconta-1(27),2,4,6(31),16(21),17,19,22,24,28-decaene |
| Compound 66 | 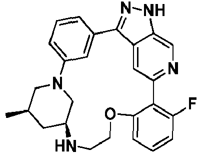 | (9*R*,11*S*)-20-fluoro-9-methyl-15-oxa-7,12,23,26,27-pentaazahexacyclo[20.5.2.1^{2,6}.1^{7,11}.0^{16,21}.0^{25,28}]h entriaconta-1(27),2,4,6(31),16(21),17,19,22,24,28-decaene |
| Compound 67 | 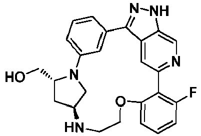 | [(8*R*,10*S*)-19-fluoro-14-oxa-7,11,22,25,26-pentaazahexacyclo[19.5.2.1^{2,6}.1^{7,10}.0^{15,20}.0^{24,27}]tr iaconta-1(26),2,4,6(30),15(20),16,18,21,23,27-decaen-8-yl]methanol |
| Compound 68 | 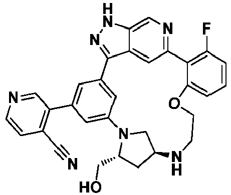 | 3-[(8*R*,10*S*)-19-fluoro-8-(hydroxymethyl)-14-oxa-7,11,22,25,26-pentaazahexacyclo[19.5.2.1^{2,6}.1^{7,10}.0^{15,20}.0^{24,27}]tr iaconta-1(26),2,4,6(30),15(20),16,18,21,23,27-decaen-4-yl]pyridine-4-carbonitrile |
| Compound 69 | 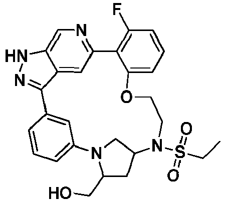 | [11-(ethanesulfonyl)-19-fluoro-14-oxa-7,11,22,25,26-pentaazahexacyclo[19.5.2.1^{2,6}.1^{7,10}.0^{15,20}.0^{24,27}]tr iaconta-1(26),2,4,6(30),15(20),16,18,21,23,27-decaen-8-yl]methanol |
| Compound 70 | 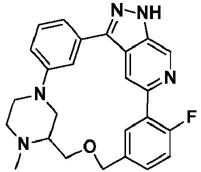 | 18-fluoro-10-methyl-13-oxa-7,10,21,24,25-pentaazahexacyclo[18.5.2.1^{2,6}.1^{7,11}.1^{15,19}.0^{23,26}]tr iaconta-1(25),2,4,6(30),15,17,19(28),20,22,26-decaene |
| Compound 84 | 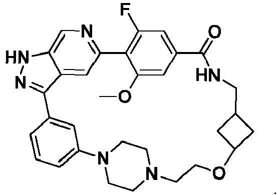 | 37-fluoro-3-methoxy-12-oxa-7,15,18,25,26,29-hexaazaheptacyclo[22.5.2.2^{2,5} .2^{15,11}.1^{9,11}.1^{19,23}.0^{2 7,31}]heptatriaconta-1(29),2,4,19(32),20,22,24,27,30,36-decaen-6-one |
| Compound 85 | 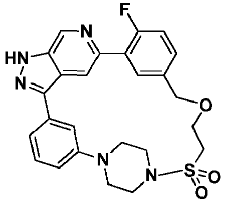 | 19-fluoro-14-oxa-11lambda6-thia-7,10,22,25,26-pentaazahexacyclo[19.5.2.2^{7,10}.1^{2,6}.1^{16,20}.0²⁴,²⁷]d otriaconta-1(26),2,4,6(32),16,18,20(29),21,23,27-decaene-11,11-dione |
| Compound 86 | 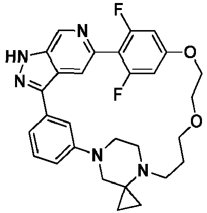 | 3',34'-difluoro-6',9'-dioxa-13',16',23',24',27'-pentaazaspiro[cyclopropane-1,14'-hexacyclo[20.5.2.2^{2,5}.2^{13,16}.1^{17,21}.0^{25,29}]tetratriac ontane]-1'(27'),2',4',17'(30'),18',20',22',25',28',33'-decaene |
| Compound 87 | 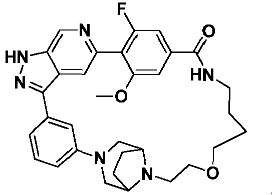 | 38-fluoro-3-methoxy-12-oxa-7,15,21,28,29,32-hexaazaheptacyclo[25.5.2.2^{2,5}.1^{16,21}.1^{22,26}.0^{15,19}.0 ^{30,34}]octatriaconta-1(32),2,4,22(35),23,25,27,30,33,37-decaen-6-one |
| Compound 88 | 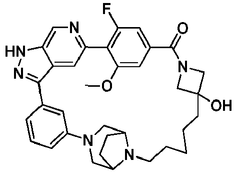 | 39-fluoro-9-hydroxy-3-methoxy-7,15,21,28,29,32-hexaazaoctacyclo[25.5.2.2^{2,5}.1^{7,9}.1^{16,21}.1^{22,26}.0^{15,1 9}.0^{30,34}]nonatriaconta-1(32),2,4,22(35),23,25,27,30,33,38-decaen-6-one |

In one embodiment, the compound described herein is selected from one of the following structures:

| Compound No. | Structure | Name |
|---|---|---|
| Compound 71 | 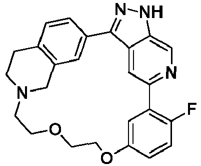 | 10-fluoro-14,17-dioxa-3,4,20,30-tetraazahexacyclo[18.5.3.2^{5,8}.1^{9,13}.0^{2,6}.0^{23,27}]hent riaconta-1(25),2,5(31),6,8(30),9(29),10,12,23,26-decaene |
| Compound 72 | 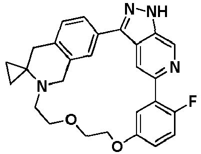 | 10'-fluoro-14',17'-dioxa-3',4',20',30'-tetraazaspiro[cyclopropane-1,21'-hexacyclo[18.5.3.2^{5,8}.1^{9,13}.0^{2,6}.0^{23,27}]hentriaconta ne]-1'(25'),2',5'(31'),6',8'(30'),9'(29'),10',12',23',26'-decaene |
| Compound 73 | 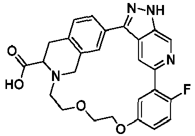 | 10-fluoro-14,17-dioxa-3,4,20,30-tetraazahexacyclo[18.5.3.2^{5,8}.1^{9,13}.0^{2,6}.0^{23,27}]hent riaconta-1(25),2,5(31),6,8(30),9(29),10,12,23,26-decaene-21-carboxylic acid |
| Compound 74 | 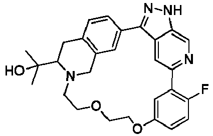 | 10-fluoro-14,17-dioxa-21-(2-hydroxyprop-2-yl)-3,4,20,30-tetraazahexacyclo[18.5.3.2^{5,8}.1^{9,13}.0^{2,6}.0^{23,27}]hent riaconta-1(25),2,5(31),6,8(30),9(29),10, 12,23,26-decaene |
| Compound 75 | 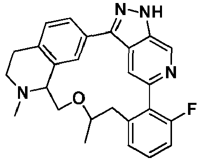 | 10-fluoro-16,20-dimethyl-17-oxa-3,4,20,28-tetraazahexacyclo[17.6.2.2^{5,8}.0^{2,6}.0^{9,14}.0^{23,27}]non acosa-1(25),2,5(29),6,8(28),9(14),10,12,23,26-decaene |
| Compound 76 | 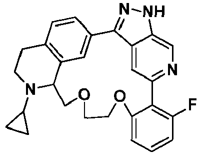 | 21-cyclopropyl-10-fluoro-15,18-dioxa-3,4,21,29-tetraazahexacyclo[18.6.2.2^{5,8}.0^{2,6}.0^{9,14}.0^{24,28}]triac onta-1(26),2,5(30),6,8(29),9(14),10,12,24,27-decaene |

| Compound No. | Structure | Name |
|---|---|---|
| Compound 77 | 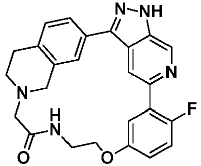 | 10-fluoro-14-oxa-3,4,17,20,30-pentaazahexacyclo[18.5.3.2^{5,8}.1^{9,13}.0^{2,6}.0^{23,27}]hen triaconta-1(25),2,5(3 1),6,8(30),9(29),10,12,23,26-decaen-18-one |
| Compound 78 | 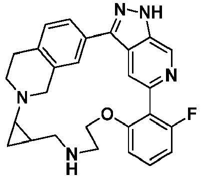 | 14-fluoro-9-oxa-1,6,20,21,33-pentaazaheptacyclo[21.5.3.2^{16,19}.0^{2,4}.0^{10,15}.0^{18,22}. 0^{26,30}]tritriaconta-10(15),11,13,16(33),17,19(32),21,23,25,30-decaene |
| Compound 79 | 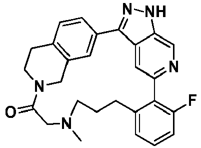 | 10-fluoro-18-methyl-3,4,18,21,30-pentaazahexacyclo[19.5.3.2^{5,8}.0^{2,6}.0^{9,14}.0^{24,28}]hen triaconta-1(26),2,5(31),6,8(30),9(14),10,12,24,27-decaen-20-one |
| Compound 80 | 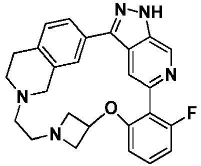 | 10-fluoro-15-oxa-3,4,18,21,31-pentaazaheptacyclo[19.5.3.2^{5,1}.1^{16,18}.0^{2,6}.0^{9,14}.0^{24 ,28}]dotriaconta-1(26),2,5(32),6,8(31),9(14),10,12,24,27-decaene |
| Compound 81 | 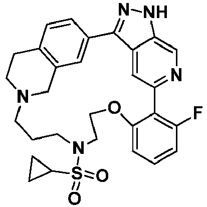 | 18-(cyclopropanesulfonyl)-10-fluoro-15-oxa-3,4,18,22,31-pentaazahexacyclo[20.5.3.2^{5,8}.0^{2,6}.0^{9,14}.0^{25,29}]dot riaconta-1(27),2,5(32),6,8(31),9(14),10,12,25,28-decaene |
| Compound 82 | 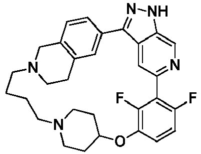 | 22,32-difluoro-18-oxa-9,14,25,28,29-pentaazaheptacyclo[22.5.2.2^{14,17}.1^{2,6}.1^{5,9}.1^{19,23}.0^{2 7,30}]hexatriaconta-1(29),2,4,6(36),19,21,23(32),24,26,30-decaene |

### Compositions and Methods of Use

In one embodiment, the invention provides a pharmaceutical composition comprising the compound of the present application or a pharmaceutically acceptable salt, hydrate, solvate, active metabolite, polymorph, isotope labeled compound, isomer or prodrug thereof, and a pharmaceutically acceptable carrier.

The pharmaceutical combination may be prepared in a manner well known in the pharmaceutical field, and may be administered by various routes. The mode of administration may be topical (including transdermal, epidermal, ocular, local injection and mucosal, including intranasal, vaginal, and rectal delivery), pulmonary (e.g., by inhalation or insufflation of powders or aerosols, including by nebulizer; intratracheal or intranasal), oral or parenteral.

In one embodiment, the composition is suitable for parenteral administration, including intravenous, intraarterial, subcutaneous, intraperitoneal, intramuscular and other local injections or infusions; or intracranial, such as intrathecal or intraventricular administration. Parenteral administration may be in the form of a single bolus dose, or (e.g.) continuous perfusion pump.

In one embodiment, the composition is suitable for topical administration, which may comprise transdermal patches, ointments, lotions, creams, gels, drops, suppositories, sprays, liquids and powders. Conventional pharmaceutical carriers, aqueous, powdered or oily matrices, thickeners and the like may be necessary or desired.

In one embodiment, the invention provides use of the compound of the present application or a pharmaceutically acceptable salt, hydrate, solvate, active metabolite, polymorph, isotope labeled compound, isomer or prodrug thereof, or a pharmaceutical composition thereof, for the prevention or treatment of a disease mediated by protein kinases.

In one embodiment, the invention provides use of the compound of the present application or a pharmaceutically acceptable salt, hydrate, solvate, active metabolite, polymorph, isotope labeled compound, isomer or prodrug thereof, or a pharmaceutical composition thereof, for the prevention or treatment of a disease mediated by HPK1.

In one embodiment, the invention provides a method for regulating, for example, inhibiting, the activity of HPK1, comprising administering the compound of Formula (I) or a pharmaceutically acceptable salt, hydrate, solvate, active metabolite, polymorph, isotope labeled compound, isomer or prodrug thereof, or a pharmaceutical composition thereof to a patient, in order to stimulate and/or boost immunity to cancers.

In one embodiment, the invention provides a method for simultaneously regulating, for example, inhibiting, the activity of multiple protein kinases (for example, FLT3, KDR, and the like), comprising administering the compound of Formula (I) or a pharmaceutically acceptable salt, hydrate, solvate, active metabolite, polymorph, isotope labeled compound, isomer or prodrug thereof, or a pharmaceutical composition thereof to a patient, in order to inhibit the proliferation of tumor cells.

In one embodiment, the invention provides a method for simultaneously regulating, for example, inhibiting, the activity of HPK1 and other multiple protein kinases (for example, FLT3, KDR, and the like), comprising administering the compound of Formula (I) or a pharmaceutically acceptable salt, hydrate, solvate, active metabolite, polymorph, isotope labeled compound, isomer or prodrug thereof, or a pharmaceutical composition thereof to a patient, in order to inhibit the proliferation of tumor cells.

In one embodiment, the invention provides a method for preventing, ameliorating or treating a disease mediated by HPK1 in a patient, comprising administering therapeutically effective amount of the compound of Formula (I) or a pharmaceutically acceptable salt, hydrate, solvate, active metabolite, polymorph, isotope labeled compound, isomer or prodrug thereof, or a pharmaceutical composition thereof to the patient.

In one embodiment, the invention provides a method for preventing, ameliorating or treating tumors (comprising benign tumors and malignant tumors) in a patient, comprising administering therapeutically effective amount of the compound of Formula (I) or a pharmaceutically acceptable salt, hydrate, solvate, active metabolite, polymorph, isotope labeled compound, isomer or prodrug thereof, or a pharmaceutical composition thereof to the patient.

In one embodiment, the invention provides a method for preventing, ameliorating or treating a disease caused by viruses in a patient, comprising administering therapeutically effective amount of the compound of Formula (I) or a pharmaceutically acceptable salt, hydrate, solvate, active metabolite, polymorph, isotope labeled compound, isomer or prodrug thereof, or a pharmaceutical composition thereof to the patient.

In one embodiment, the invention provides a method for preventing, ameliorating or treating myelodysplastic syndromes in a patient, comprising administering therapeutically effective amount of the compound of Formula (I) or a pharmaceutically acceptable salt, hydrate, solvate, active metabolite, polymorph, isotope labeled compound, isomer or prodrug thereof, or a pharmaceutical composition thereof to the patient.

In one embodiment, the invention provides use of the compound of Formula (I) or a pharmaceutically acceptable salt, hydrate, solvate, active metabolite, polymorph, isotope labeled compound, isomer or prodrug thereof, or a pharmaceutical composition thereof, for the treatment or amelioration of benign or malignant tumors, myelodysplastic syndromes and diseases caused by viruses.

In one embodiment, the invention provides use of the compound of Formula (I) or a pharmaceutically acceptable salt, hydrate, solvate, active metabolite, polymorph, isotope labeled compound, isomer or prodrug thereof, or a pharmaceutical composition thereof, for the treatment or amelioration of tumors.

In one embodiment, the invention provides use of the compound of Formula (I) or a pharmaceutically acceptable salt, hydrate, solvate, active metabolite, polymorph, isotope labeled compound, isomer or prodrug thereof, or a pharmaceutical composition thereof, for the treatment or amelioration of diseases caused by viruses.

In one embodiment, the invention provides use of the compound of Formula (I) or a pharmaceutically acceptable salt, hydrate, solvate, active metabolite, polymorph, isotope labeled compound, isomer or prodrug thereof, or a pharmaceutical composition thereof, in the preparation of a medicament for the treatment or amelioration of one or more particular diseases selected from benign or malignant tumors, myelodysplastic syndromes and diseases caused by viruses.

In one embodiment, the invention provides use of the compound of Formula (I) or a pharmaceutically acceptable salt, hydrate, solvate, active metabolite, polymorph, isotope labeled compound, isomer or prodrug thereof, or a pharmaceutical composition thereof, in the preparation of a medicament for the treatment or amelioration of tumors.

In one embodiment, the invention provides use of the compound of Formula (I) or a pharmaceutically acceptable salt, hydrate, solvate, active metabolite, polymorph, isotope labeled compound, isomer or prodrug thereof, or a pharmaceutical composition thereof, in the preparation of a medicament for the treatment or amelioration of diseased caused by viruses.

In one embodiment, the tumors comprise one or more of the following cancers: chronic or acute leukemia, primary CNS (central nervous system) lymphoma, lymphoma, multiple myeloma, lung cancer, hepatocellular carcinoma, cholangiocarcinoma, gallbladder cancer, gastric cancer, colorectal cancer, intestinal leiomyosarcoma, breast cancer, triple-negative breast cancer, ovarian cancer, cervical cancer, endometrial cancer, fallopian tube cancer, vaginal cancer, vulvar cancer, malignant teratoma, pancreatic cancer, pancreatic ductal adenocarcinoma, nasopharyngeal cancer, oral cancer, laryngeal cancer, esophageal squamous cell carcinoma, esophageal adenocarcinoma, thyroid cancer, kidney cancer, bladder cancer, malignant brain tumor, rhabdomyosarcoma, osteosarcoma, chondrosarcoma, osteofibrosarcoma, Ewing sarcoma, myxoma, malignant thymoma, malignant peripheral nerve sheath tumor, prostate cancer, testicular cancer, penile cancer, urethral cancer, as well as skin malignant tumors (comprising squamous cell carcinoma, basal cell carcinoma, malignant melanoma, and the like).

In one embodiment, the virus comprises one or more of the following viruses: hepatitis virus, human immunodeficiency virus, human papillomavirus, herpes simplex virus, measles virus, norovirus, Boca virus, Coxsackie virus, Ebola virus, enterovirus, lymphocytic meningitis virus, influenza virus, SARS virus and COVID-19 virus.

### Synthesis

The compounds of the present invention and their salts may be prepared using known organic synthesis techniques and may be prepared according to any of a number of possible synthetic routes (such as those in the schemes below).

The reactions used to prepare the compounds of the present application may be carried out in suitable solvents. Suitable solvents may be substantially non-reactive with the starting materials (reactants), intermediates or products at temperatures at which the reaction is carried out (e.g., temperatures that may range from the melting point of the solvent to the boiling point of the solvent). A given reaction may be carried out in one solvent or a mixture of solvents. Depending on a specific reaction step, one skilled in the art may select an appropriate solvent for a specific reaction step.

The preparation of the compounds of the present invention may involve the protection and deprotection of various chemical groups. One skilled in art may readily determine the need for protection and deprotection and the selection of appropriate protecting groups.

The following schemes provide general guidance related to the preparation of the compounds of the present invention. Those skilled in the art will appreciate that general knowledge of organic chemistry may be used to modify or optimize the methods shown in the schemes to prepare the various compounds of the present application.

Compounds described in this invention may be prepared according to methods as illustrated in the schemes below.

All methods described in this specification may be performed in any suitable order unless otherwise indicated or otherwise clearly contradicted by context. All examples or exemplary language (e.g., "such as") provided in this specification are used only to better clarify the invention, and are not intended to limit the scope of the application as otherwise claimed.

Hereinafter, the preparation and properties of the compounds of formula (I) in some embodiments will be further described with reference to specific examples. Among them, the starting materials used are known and commercially available, or may be synthesized using or according to methods known in the art.

Unless otherwise specified, all reactions in the Examples were carried out under continuous magnetic stirring, and the reaction temperature was in degrees Celsius.

The reactions may be monitored according to any suitable method known in the art, such as nuclear magnetic resonance spectroscopy (NMR), infrared spectroscopy (IR), spectrophotometry (e.g., UV-Vis spectroscopy), liquid mass spectrometry (LC-MS), mass spectrometry, high performance liquid chromatography, thin layer chromatography and so on. The products may be purified by any suitable method known in the art, such as column chromatography (normal or reverse phase), preparative thin layer chromatography, trituration, recrystallization, and the like. Generally, 100-200 mesh silica gel from Qingdao Haiyang Chemical Co., Ltd. is used as carrier (stationary phase) in normal phase column chromatography. Silica gel 60 F254 silica gel plate from Merck Ltd. is used in thin layer chromatography (TLC), and GF254 preparative silica gel plate from Anhui Liangchen Silicon Material Co., Ltd. is used in preparative thin layer chromatography (pre-TLC).

The structures of the compounds in the Examples were determined by nuclear magnetic resonance spectroscopy (NMR) and/or liquid chromatography-mass spectrometry (LC-MS). The nuclear magnetic resonance spectrum was measured by Bruker AVANCE-400 nuclear magnetic resonance apparatus, and the solvent was usually deuterated dimethyl sulfoxide (DMSO-*d*₆) or deuterated chloroform (CDCl₃). NMR chemical shifts (δ) were given in parts per million (ppm) using tetramethylsilane (TMS) as the internal standard. LC-MS was performed on an Agilent 1100 liquid chromatograph and a Bruker HCT-Ultra ion trap mass spectrometer.

### EXAMPLES

### Abbreviation Table

| | |
|---|---|
| DMF | *N*,*N*-Dimethylformamide |
| Pd(dppf)Cl₂ | (1,1'-Bis(diphenylphosphino)ferrocene)palladium(II) dichloride |
| Xphos-Pd-G2 | Chloro(2-dicyclohexylphosphino-2',4',6'-triisopropyl-1,1'-biphenyl)[2-(2'-amino-1,1'-biphenyl)]palladium(II) |
| BINAP | (2,2'-Bis(diphenylphosphino)-1,1'-binaphthyl) |
| TMAD | *N*,*N*,*N'*,*N'*-Tetramethylazodicarboxamide |

### Example 1.

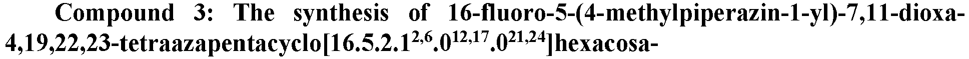

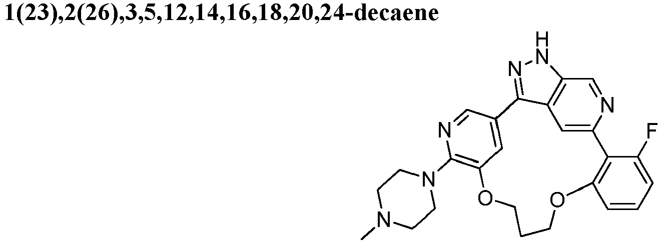

### Step 1: the synthesis of 3-(3-(benzyloxy)propoxy)-2-bromopyridine

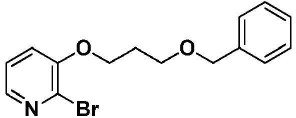

To a mixture of 2-bromopyridin-3-ol (7.50 g, 43.1 mmol) and ((3-bromopropoxy)methyl)benzene (11.85 g, 51.72 mmol) was added DMF (45 mL), followed by potassium iodide (716 mg, 4.31 mmol) and potassium carbonate (11.92 g, 86.21 mmol). The mixture was heated in oil bath at 80°C for 2 hours. After completion of the reaction, the reaction mixture was cooled to room temperature, and filtered. The filter cake was washed with ethyl acetate (20 mL × 4). The combined filtrate was concentrated to dryness, and the residue was purified by column chromatography (petroleum ether/ethyl acetate = 5/1) to afford the title product (13.08 g, yellow solid). Yield: 94.2%.

ESI-MS: 322.1, [M+H]⁺.

### Step 2: the synthesis of 1-(3-(3-(benzyloxy)propoxy)pyridin-2-yl)-4-methylpiperazine

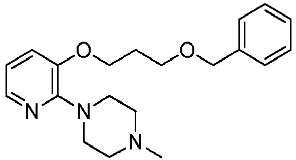

To a mixture of 3-(3-(benzyloxy)propoxy)-2-bromopyridine (4.50 g, 13.97 mmol), 1-methylpiperazine (7.35 mL, 69.8 mmol), BINAP (1.30 g, 2.09 mmol) and potassium tert-butoxide (3.41 g, 27.9 mmol) was added toluene (45 mL), and the mixture was stirred under nitrogen. To the mixture was then added tris(dibenzylideneacetone)dipalladium (1.28 g, 1.40 mmol), and stirred in oil bath at 110°C for 12 hours. After completion of the reaction, the reaction mixture was cooled to room temperature, and concentrated to dryness. The residue was purified by column chromatography (dichloromethane/methanol = 20/1) to afford the title product (4.660 g, orange-red oil). Yield: 97.7%.

ESI-MS: 342.2, [M+H]⁺.

### Step 3: the synthesis of 1- f 3-[3-(benzyloxy)propoxy]-5-bromopyridin-2-yl}-4-methylpiperazine

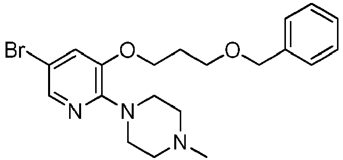

To 1-(3-(3-(benzyloxy)propoxy)pyridin-2-yl)-4-methylpiperazine (4.460 g, 13.06 mmol) was added dichloromethane (27 mL), and the mixture was stirred at 0°C under nitrogen. To the mixture was added a solution of N-bromosuccinimide (2.560 g, 14.37 mmol) in dry acetonitrile (27 mL), and further stirred at the same temperature for 2 hours. After completion of the conversion, the reaction was quenched by the addition of saturated sodium bisulfite aqueous solution (2 mL). The reaction mixture was then diluted with water (100 mL), and extracted with dichloromethane (100 mL × 4). The combined organic phase was concentrated to dryness, and the residue was purified by column chromatography (dichloromethane/methanol = 300/1 to 200/1) to afford the title product (5.090 g, orange-red solid). Yield: 92.7%.

ESI-MS: 420.2, [M+H]⁺.

### Step 4: the synthesis of 1-{3-[3-(benzyloxy)propoxy]-5-(4,4,5,5-tetramethyl-1,3,2-dioxaborolan-2-yl)pyridin-2-yl}-4-methylpiperazine

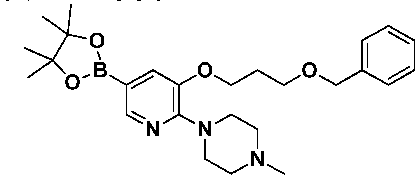

To a mixture of 1-{3-[3-(benzyloxy)propoxy]-5-bromopyridin-2-yl}-4-methylpiperazine (1.000 g, 2.38 mmol), bis(pinacolato)diboron (1.21 g, 4.76 mmol) and potassium acetate (0.700 g, 7.14 mmol) was added 1,4-dioxane (12 mL), and stirred at room temperature under nitrogen. To the mixture was then added Pd(dppf)Cl₂ (174 mg, 0.24 mmol), and stirred in oil bath at 90°C for 22 hours. After completion of the reaction, the reaction mixture was cooled to room temperature, and filtered. The filter cake was washed with 1,4-dioxane (5 mL × 4). The combined filtrate was concentrated to dryness to afford the title product (2.000 g, brown oil), which was directly used in the next step.

ESI-MS: 468.3, [M+H]⁺.

### Step 5: the synthesis of 3-(5-(3-(benzyloxy)propoxy)-6-(4-methylpiperazin-1-yl)pyridin-3-yl)-5-bromo-1-((2-(trimethylsilyl)ethoxy)methyl)-1H-pyrazolo[3,4-c]pyridine

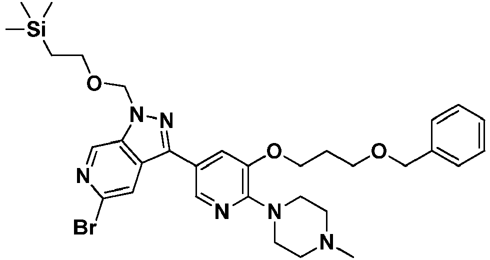

To a mixture of 5- bromo- 3- iodo-1-((2-(trimethylsilyl)ethoxy)methyl)-1*H*-pyrazolo[3,4-c]pyridine (800 mg, 1.76 mmol), 1-{3-[3-(benzyloxy)propoxy]-5-(4,4,5,5-tetramethyl-1,3,2-dioxaborolan-2-yl)pyridin-2-yl}-4-methylpiperazine (1.070 g, 2.29 mmol) and potassium carbonate (730 mg, 5.28 mmol) was added 1,4-dioxane (8 mL) and water (2 mL), and stirred at room temperature under nitrogen. To the mixture was then added Pd(dppf)Cl₂ (129 mg, 0.18 mmol), and stirred in oil bath at 80°C for 4 hours. After completion of the reaction, the reaction mixture was cooled to room temperature, and concentrated to dryness. The residue was purified by column chromatography (dichloromethane/methanol/triethylamine = 150/1/0.3) to afford the title product (503 mg, brown oil). Yield: 42.8%.

ESI-MS: 667.2, [M+H]⁺.

### Step 6: the synthesis of 3-(5-(3-(benzyloxy)propoxy)-6-(4-methylpiperazin-1-yl)pyridin-3-yl)-5-bromo-1H-pyrazolo[3,4-c]pyridine

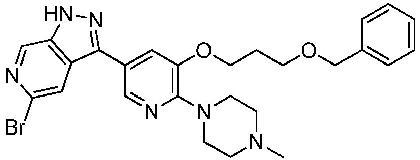

To 3-(5-(3-(benzyloxy)propoxy)-6-(4-methylpiperazin-1-yl)pyridin-3-yl)-5-bromo-1-((2-(trimethylsilyl)ethoxy)methyl)-1*H*-pyrazolo[3,4-c]pyridine (500 mg, 0.75 mmol) was added tetrahydrofuran (5 mL) and tetrabutylammonium fluoride solution in tetrahydrofuran (1M, 3.74 mL, 3.74 mmol), and stirred at 60°C under nitrogen for 16 hours. After completion of reaction, the reaction mixture was concentrated to dryness. The residue was diluted with water (10 mL) and saturated sodium carbonate aqueous solution (5 mL), and extracted with dichloromethane (20 mL × 4). The combined organic phase was concentrated to dryness, and the residue was purified by column chromatography (dichloromethane/methanol/triethylamine = 100/1/0.2 to 30/1/0.3) to afford the title product (180 mg, yellow oil). Yield: 44.7%.

ESI-MS: 537.2, [M+H]⁺.

### Step 7: the synthesis of 3-(5-(3-(benzyloxy)propoxy)-6-(4-methylpiperazin-1-yl)pyridin-3-yl)-5-bromo-1-tosyl-1H-pyrazolo[3,4-c]pyridine

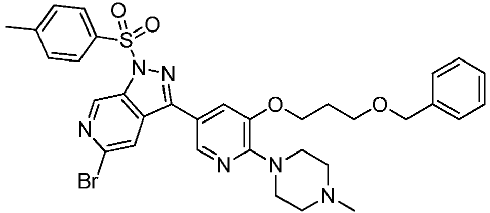

To a solution of 3-(5-(3-(benzyloxy)propoxy)-6-(4-methylpiperazin-1-yl)pyridin-3-yl)-5-bromo-1*H*-pyrazolo[3,4-c]pyridine (180 mg, 0.34 mmol) in 1,2-dichloroethane (9 mL) was added *N*,*N-*diisopropylethylamine (166 µL, 1.01 mmol) and p-toluenesulfonyl chloride (128 mg, 0.67 mmol). The mixture was heated in oil bath at 45°C under nitrogen for 15 hours. After completion of the reaction, the reaction mixture was concentrated to dryness, and the residue was purified by column chromatography (dichloromethane/methanol/triethylamine = 1000/5/3 to 800/10/3) to afford the title product (180 mg, yellow solid). Yield: 86.3%.

ESI-MS: 691.1, [M+H]⁺.

### Step 8: the synthesis of 3-((5-(5-bromo-1-tosyl-1H-pyrazolo[3,4-c]pyridin-3-yl)-2-(4-methylpiperazin-1 -yl)pyridin-3 -yl)oxy)propan-1 -ol

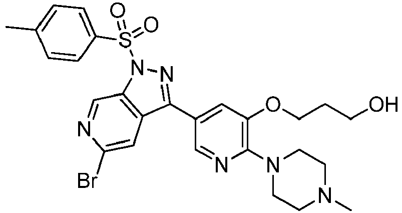

To a flask equipped with calcium chloride-drying tube was added 3-(5-(3-(benzyloxy)propoxy)-6-(4-methylpiperazin-1-yl)pyridin-3-yl)-5-bromo-1-tosyl-1*H-*pyrazolo[3,4-c]pyridine (200 mg, 0.29 mmol) and dichloromethane (6 mL). To the solution was added boron trichloride solution in dichloromethane (1M, 3.74 mL, 3.74 mmol) at 0°C, and then warmed to room temperature, and stirred for 5 hours. After completion of the reaction, the mixture was diluted with methyl *tert*-butyl ether (10 mL), and filtered. The filter cake was dried under vacuum to afford the title product (219 mg, brown solid) as the hydrochloride.

ESI-MS: 601.1, [M+H]⁺.

### Step 9: the synthesis of 3-fluoro-2-(3-(5-(3-hydroxypropoxy)-6-(4-methylpiperazin-1-yl)pyridin-3-yl)-1-tosyl-1H-pyrazolo[3,4-c]pyridin-5-yl)phenol

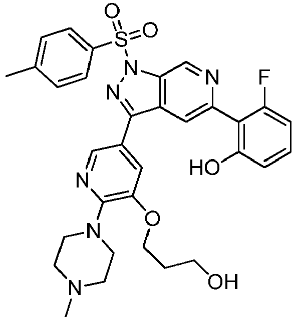

To a mixture of 3-((5-(5-bromo-1-tosyl-1*H*-pyrazolo[3,4-c]pyridin-3-yl)-2-(4-methylpiperazin-1-yl)pyridin-3-yl)oxy)propan-1-ol (200 mg, 0.33 mmol), (2-fluoro-6-hydroxyphenyl)boronic acid (78 mg, 0.50 mmol) and potassium phosphate (212 mg, 1.00 mmol) was added 1,4-dioxane (8 mL) and water (2 mL), and stirred at room temperature under nitrogen. To the mixture was then added XPhos-Pd-G2 (13 mg, 0.02 mmol), and stirred in oil bath at 90°C for 40 min. After completion of the reaction, the reaction mixture was cooled to room temperature, diluted with 5% brine (30 mL), and then extracted with dichloromethane (50 mL × 4). The combined organic phase was concentrated to dryness. The residue was purified by column chromatography (dichloromethane/methanol/triethylamine = 40/1/0.1 to 30/1/0.1) to afford the crude solid product. The solid was dissolved in dichloromethane (200 mL), washed with saturated sodium carbonate aqueous solution (50 mL) and water (50 mL × 4) successively, dried over anhydrous sodium sulfate, and filtered. The filtrate was concentrated to dryness to afford the title product (133 mg, yellow solid). Yield: 63.2%.

ESI-MS: 633.2, [M+H]⁺.

### Step 10: the synthesis of 22-(benzenesulfonyl)-16-fluoro-5-(4-methylpiperazin-1-yl)-7,11-dioxa-4,19,22,23-tetraazapentacyclo[16.5.2.1^{2,6}.0^{12,17}.0^{21,24}]hexacosa-1(23),2(26),3,5,12,14,16,18,20,24-decaene

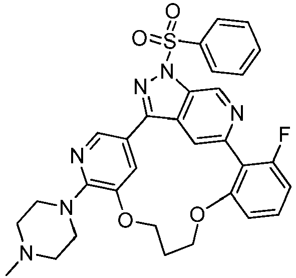

To the solution of 3-fluoro-2-(3-(5-(3-hydroxypropoxy)-6-(4-methylpiperazin-1-yl)pyridin-3-yl)-1-tosyl-1*H*-pyrazolo[3,4-c]pyridin-5-yl)phenol (80 mg, 0.13 mmol) in dry tetrahydrofuran (10 mL) was added triphenylphosphine (166 mg, 0.63 mmol), and stirred in oil bath at 40°C under nitrogen. To the solution was then added a solution of diisopropyl azodicarboxylate (125 µL, 0.63 mmol) in dry tetrahydrofuran (2 mL), and further stirred at 40°C for 3 hours. After completion of the reaction, the mixture was concentrated to dryness, and the residue was purified by column chromatography (dichloromethane/methanol/triethylamine = 50/1/0.1) to afford the crude product. The crude product was purified by pre-TLC (dichloromethane/methanol = 10/1) to afford the tile product (49 mg, yellow solid). Yield: 63.0%.

ESI-MS: 615.2, [M+H]⁺.

### Step 11: the synthesis of 16-fluoro-5-(4-methylpiperazin-1-yl)-7,11-dioxa-4,19,22,23-tetraazapentacyclo[16.5.2.1^{2,6}.0^{12,17}.0^{21,24}]hexacosa-1(23),2(26),3,5,12,14,16,18,20,24-decaene

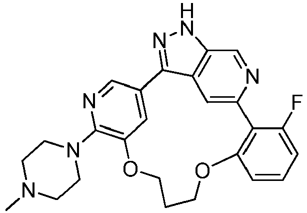

To a solution of 22-(benzenesulfonyl)-16-fluoro-5-(4-methylpiperazin-1-yl)-7,11-dioxa-4,19,22,23-tetraazapentacyclo[16.5.2.1^{2,6}.0^{12,17}.0^{21,24}]hexacosa-1(23),2(26),3,5,12,14,16,18,20,24-decaene (49 mg, 0.08 mmol) in methanol (3 mL) was added methanolic solution of potassium hydroxide (1M, 1.5 mL), and stirred at room temperature for 20 min. After completion of the reaction, the reaction mixture was purified by pre-TLC (dichloromethane/methanol = 10/1) to afford the tile product (31 mg, pale yellow solid). Yield: 84.5%.

ESI-MS: 461.2, [M+H]⁺.

### ¹H NMR (DMSO-d₆, 400 MHz) δ 13.70 (s, 1H), 9.11 (s, 1H), 8.71 (s, 1H), 8.36 (d, J= 1.6 Hz, 1H), 8.07 (s, 1H), 7.44 - 7.36 (m, 1H), 7.02 - 6.93 (m, 2H), 4.62 - 4.51 (m, 2H), 4.17 - 4.04 (m, 2H), 3.56 - 3.37 (m, 4H), 2.49-2.41 (m, 4H), 2.39 - 2.31 (m, 2H), 2.24 (s, 3H).

### Example 4.

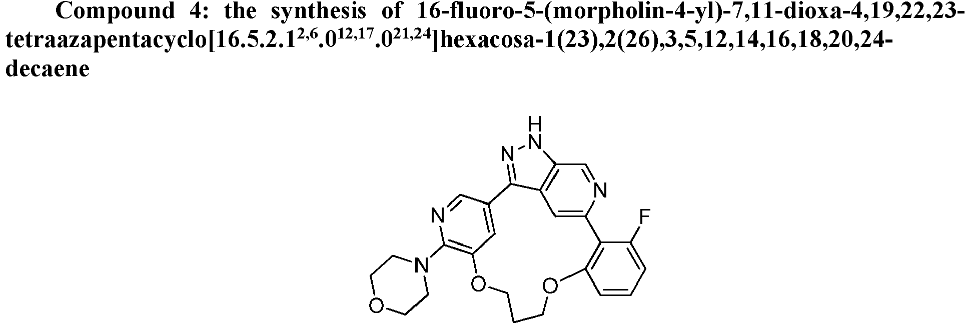

### Step 1: the synthesis of 4-(3-(3-(benzyloxy)propoxy)pyridin-2-yl)morpholine

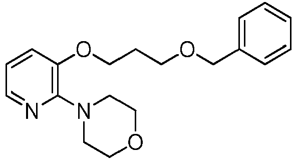

To a mixture of 3-(3-(benzyloxy)propoxy)-2-bromopyridine (4.50 g, 13.97 mmol), morpholine (6.08 mL, 69.8 mmol), BINAP (1.30 g, 2.09 mmol) and potassium tert-butoxide (3.41 g, 27.9 mmol) was added toluene (45 mL), and the mixture was stirred under nitrogen. To the mixture was then added tris(dibenzylideneacetone)dipalladium (1.28 g, 1.40 mmol), and stirred in oil bath at 110°C for 12 hours. After completion of the reaction, the reaction mixture was cooled to 0°C, diluted with saturated ammonium chloride aqueous solution (200 mL), and extracted with ethyl acetate (200 mL × 2). The organic phase was washed with brine (200 mL), and concentrated to dryness. The residue was purified by column chromatography (petroleum ether/ethyl acetate = 5/1 to 3/1) to afford the title product (3.730 g, brown oil). Yield: 81.3%.

ESI-MS: 329.2, [M+H]⁺.

### Step 2: the synthesis of 4-(3-(3-(benzyloxy)propoxy)-5-bromopyridin-2-yl)morpholine

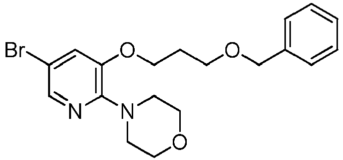

To 4-(3-(3-(benzyloxy)propoxy)pyridin-2-yl)morpholine (3.53 g, 10.8 mmol) was added dichloromethane (21 mL), and the mixture was stirred at 0°C under nitrogen. To the mixture was added a solution of N-bromosuccinimide (1.91 g, 10.75 mmol) in dry acetonitrile (21 mL), and further stirred at the same temperature for 0.5 hours. After completion of the conversion, the reaction was quenched by the addition of saturated sodium bisulfite aqueous solution (1 mL). The reaction mixture was then diluted with water (100 mL), and extracted with dichloromethane (100 mL × 3). The combined organic phase was concentrated to dryness, and the residue was purified by column chromatography (petroleum ether/ethyl acetate = 5/1) to afford the title product (3.60 g, yellow oil). Yield: 82.3%.

ESI-MS: 407.3/409.3, [M+H]⁺.

### Step 3: the synthesis of 4-(3-(3-(benzyloxy)propoxy)-5-(4,4,5,5-tetramethyl-1,3,2-dioxaborolan-2-yl)pyridin-2-yl)morpholine

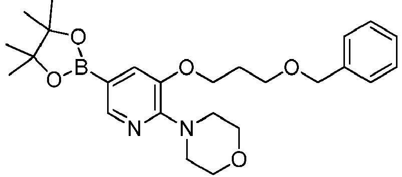

This compound was synthesized according to the procedure described in Example 1 (step 4) using 4-(3-(3-(benzyloxy)propoxy)-5-bromopyridin-2-yl)morpholine instead of 1-(3-(3-(benzyloxy)propoxy)-5-bromopyridin-2-yl)-4-methylpiperazine as the starting material. The crude product was directly used in the next step.

ESI-MS: 455.3, [M+H]⁺.

### Step 4: the synthesis of 4-(3-(3-(benzyloxy)propoxy)-5-(5-bromo-1-((2-(trimethylsilyl)ethoxy)methyl)-1H-pyrazolo[3,4-c]pyridin-3-yl)pyridin-2-yl)morpholine

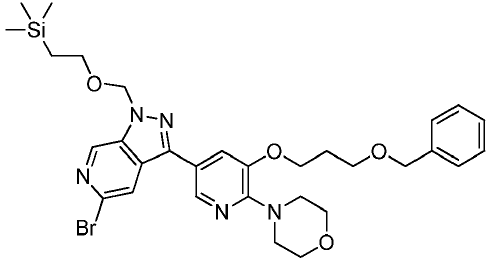

This compound was synthesized according to the procedure described in Example 1 (step 5) using 4-(3-(3-(benzyloxy)propoxy)-5-(4,4,5,5-tetramethyl-1,3,2-dioxaborolan-2-yl)pyridin-2-yl)morpholine instead of 1-(3-(3-(benzyloxy)propoxy)-5-(4,4,5,5-tetramethyl-1,3,2-dioxaborolan-2-yl)pyridin-2-yl)-4-methylpiperazine as the starting material. Yield: 56.0%.

ESI-MS: 654.2, [M+H]⁺.

### Step 5: the synthesis of 4-(3-(3-(benzyloxy)propoxy)-5-(5-bromo-1H-pyrazolo[3,4-c]pyridin-3-yl)pyridin-2-yl)morpholine

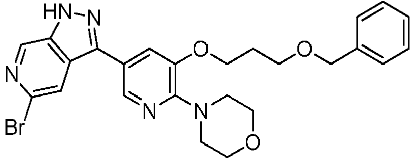

This compound was synthesized according to the procedure described in Example 1 (step 6) using 4-(3-(3-(benzyloxy)propoxy)-5-(5-bromo-1-((2-(trimethylsilyl)ethoxy)methyl)-1H-pyrazolo[3,4-c]pyridin-3-yl)pyridin-2-yl)morpholine instead of 3-(5-(3-(benzyloxy)propoxy)-6-(4-methylpiperazin-1-yl)pyridin-3-yl)-5-bromo-1-((2-(trimethylsilyl)ethoxy)methyl)-1*H-*pyrazolo[3,4-c]pyridine as the starting material. Yield: 62.6%.

### Step 6: the synthesis of 4-(3-(3-(benzyloxy)propoxy)-5-(5-bromo-1-tosyl-1H-pyrazolo[3,4-c]pyridin-3-yl)pyridin-2-yl)morpholine

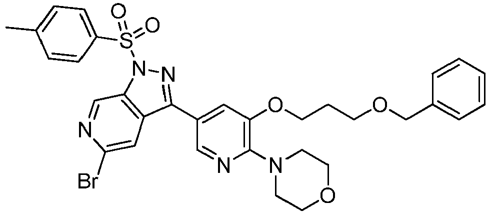

This compound was synthesized according to the procedure described in Example 1 (step 7) using 4-(3-(3-(benzyloxy)propoxy)-5-(5-bromo-1H-pyrazolo[3,4-c]pyridin-3-yl)pyridin-2-yl)morpholine instead of 3-(5-(3-(benzyloxy)propoxy)-6-(4-methylpiperazin-1-yl)pyridin-3-yl)-5-bromo-1*H*-pyrazolo[3,4-c]pyridine as the starting material. Yield: 91.3%.

ESI-MS: 678.1, [M+H]⁺.

### Step 7: the synthesis of 3-((5-(5-bromo-1-tosyl-1H-pyrazolo[3,4-c]pyridin-3-yl)-2-morpholinopyridin-3-yl)oxy)propan-1-ol

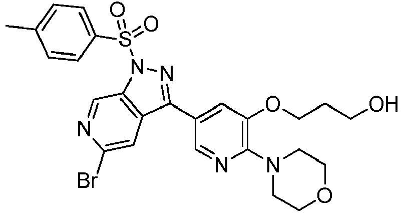

This compound was synthesized according to the procedure described in Example 1 (step 8) using 4-(3-(3-(benzyloxy)propoxy)-5-(5-bromo-1-tosyl-1H-pyrazolo[3,4-c]pyridin-3-yl)pyridin-2-yl)morpholine instead of 3-(5-(3-(benzyloxy)propoxy)-6-(4-methylpiperazin-1-yl)pyridin-3-yl)-5-bromo-1-tosyl-1*H*-pyrazolo[3,4-c]pyridine as the starting material. After completion of the reaction, the reaction mixture was concentrated to dryness at 30°C. To the residue was added water (20 mL) and saturated sodium carbonate aqueous solution (10 mL), and extracted with dichloromethane (50 mL) twice. The combined organic phase was concentrated to dryness, and the residue was purified by column chromatography (eluent: ethyl acetate) to afford the title product (yellow solid). Yield: 83.0%.

ESI-MS: 588.1, [M+H]⁺.

### Step 8: the synthesis of 3-fluoro-2-(3-(5-(3-hydroxypropoxy)-6-morpholinopyridin-3-yl)-1-tosyl-1H-pyrazolo[3,4-c]pyridin-5-yl)phenol

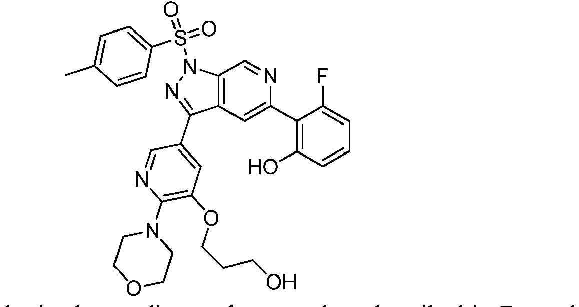

This compound was synthesized according to the procedure described in Example 1 (step 9) using 3-((5-(5-bromo-1-tosyl-1*H*-pyrazolo[3,4-c]pyridin-3-yl)-2-morpholinopyridin-3-yl)oxy)propan-1-ol instead of 3-((5-(5-bromo-1-tosyl-1*H*-pyrazolo[3,4-c]pyridin-3-yl)-2-(4-methylpiperazin-1-yl)pyridin-3-yl)oxy)propan-1-ol as the starting material. Yield: 74.5%.

ESI-MS: 620.2, [M+H]⁺.

### Step 9: the synthesis of 22-(benzenesulfonyl)-16-fluoro-5-(morpholin-4-yl)-7,11-dioxa-4,19,22,23-tetraazapentacyclo[16.5.2.1^{2,6}.0^{12,17}.0^{21,24}]hexacosa-1(23),2(26),3,5,12,14,16,18,20,24-decaene

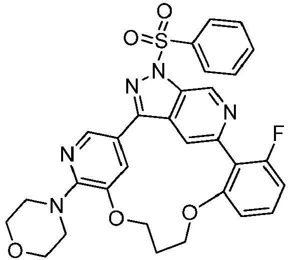

This compound was synthesized according to the procedure described in Example 1 (step 10) using 3-fluoro-2-(3-(5-(3-hydroxypropoxy)-6-morpholinopyridin-3-yl)-1-tosyl-1H-pyrazolo[3,4-c]pyridin-5-yl)phenol instead of 3-fluoro-2-(3-(5-(3-hydroxypropoxy)-6-(4-methylpiperazin-1-yl)pyridin-3-yl)-1-tosyl-1*H*-pyrazolo[3,4-c]pyridin-5-yl)phenol as the starting material. Yield: 28.2%.

ESI-MS: 602.1, [M+H]⁺.

### Step 10: the synthesis of 16-fluoro-5-(morpholin-4-yl)-7,1 1-dioxa-4,19,22,23-tetraazapentacyclo[16.5.2.1^{2,6}.0^{12,17}.0^{21,24}]hexacosa-1(23),2(26),3,5,12,14,16,18,20,24-decaene

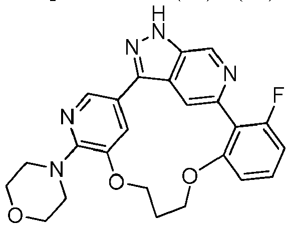

This compound was synthesized according to the procedure described in Example 1 (step 10) using 22-(benzenesulfonyl)-16-fluoro-5-(morpholin-4-yl)-7,11-dioxa-4,19,22,23-tetraazapentacyclo[16.5.2.1^{2,6}.0^{12,17}.0^{21,24}]hexacosa-1(23),2(26),3,5,12,14,16,18,20,24-decaene instead of 22-(benzenesulfonyl)-16-fluoro-5-(4-methylpiperazin-1-yl)-7,11-dioxa-4,19,22,23-tetraazapentacyclo[16.5.2.1^{2,6}.0^{12,17}.0^{21,24}]hexacosa-1(23),2(26),3,5,12,14,16,18,20,24-decaene as the starting material. Yield: 80.6%.

ESI-MS: 448.2, [M+H]⁺.

¹H NMR (DMSO-*d*₆, 400 MHz) δ 13.74 (s, 1H), 9.11 (s, 1H), 8.71 (s, 1H), 8.38 *(d, J=* 1.4 Hz, 1H), 8.09 (s, 1H), 7.45-7.31 (m, 1H), 7.06-6.84 (m, 2H), 4.67-4.50 (m,2H), 4.19-4.03 (m, 2H), 3.82-3.67 (m, 4H), 3.52-3.40 (m, 4H), 2.39-2.28 (m, 2H).

### Example 5.

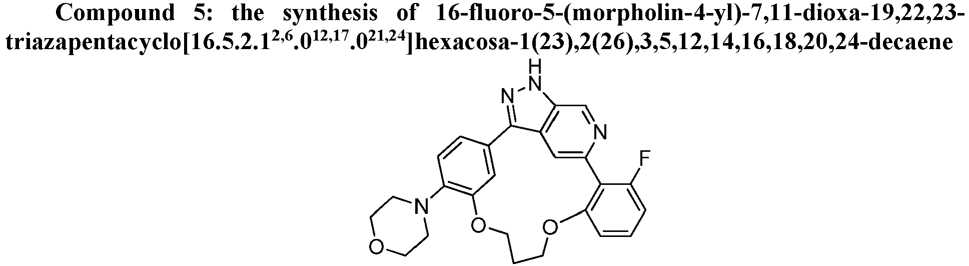

### Step 1: the synthesis of 1-(3-(benzyloxy)propoxy)-2-bromobenzene

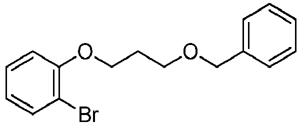

This compound was synthesized according to the procedure described in Example 1 (step 1) using 2-bromophenol instead of 2-bromopyridin-3-ol as the starting material. The crude product was directly used in the next step.

ESI-MS: 321.0, [M+H]⁺.

### Step 2: the synthesis of 4-(2-(3-(benzyloxy)propoxy)phenyl)morpholine

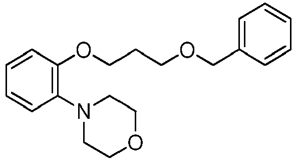

This compound was synthesized according to the procedure described in Example 1 (step 2) using 1-(3-(benzyloxy)propoxy)-2-bromobenzene instead of 3-(3-(benzyloxy)propoxy)-2-bromopyridine as the starting material. Overall yield: 72.8% (two steps).

ESI-MS: 328.2, [M+H]⁺.

### Step 3: the synthesis of 4-(2-(3-(benzyloxy)propoxy)-4-bromophenyl)morpholine

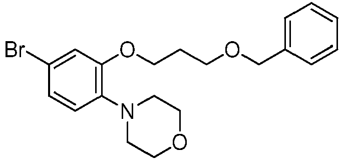

This compound was synthesized according to the procedure described in Example 1 (step 3) using 4-(2-(3-(benzyloxy)propoxy)phenyl)morpholine instead of 1-(3-(3-(benzyloxy)propoxy)pyridin-2-yl)-4-methylpiperazine as the starting material. The crude product was directly used in the next step.

ESI-MS: 406.1, [M+H]⁺.

### Step 4: the synthesis of 4-(2-(3-(benzyloxy)propoxy)-4-(4,4,5,5-tetramethyl-1,3,2-dioxaborolan-2-yl)phenyl)morpholine

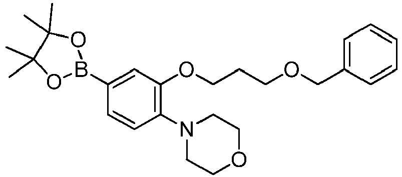

This compound was synthesized according to the procedure described in Example 1 (step 4) using 4-(2-(3-(benzyloxy)propoxy)-4-bromophenyl)morpholine instead of 1-(3-(3-(benzyloxy)propoxy)-5-bromopyridin-2-yl)-4-methylpiperazine as the starting material. The crude product was directly used in the next step.

ESI-MS: 454.3, [M+H]⁺.

### Step 5: the synthesis of 4-(2-(3-(benzyloxy)propoxy)-4-(5-bromo-1-((2-(trimethylsilyl)ethoxy)methyl)-1H-pyrazolo[3,4-c]pyridin-3-yl)phenyl)morpholine

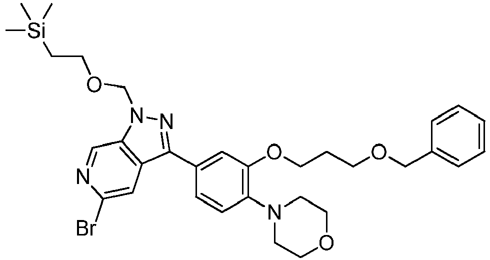

This compound was synthesized according to the procedure described in Example 1 (step 5) using 4-(2-(3-(benzyloxy)propoxy)-4-(4,4,5,5-tetramethyl-1,3,2-dioxaborolan-2-yl)phenyl)morpholine instead of 1-(3-(3-(benzyloxy)propoxy)-5-(4,4,5,5-tetramethyl-1,3,2-dioxaborolan-2-yl)pyridin-2-yl)-4-methylpiperazine as the starting material. Overall yield: 41.4% (three steps).

ESI-MS: 653.2, [M+H]⁺.

### Step 6: the synthesis of 4-(2-(3-(benzyloxy)propoxy)-4-(5-bromo-1H-indazol-3-yl)phenyl)morpholine

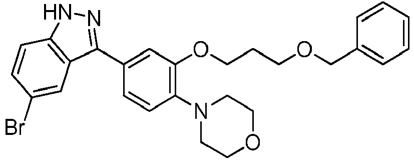

This compound was synthesized according to the procedure described in Example 1 (step 6) using 4-(2-(3-(benzyloxy)propoxy)-4-(5-bromo-1-((2-(trimethylsilyl)ethoxy)methyl)-1*H-*pyrazolo[3,4-c]pyridin-3-yl)phenyl)morpholine instead of 3-(5-(3-(benzyloxy)propoxy)-6-(4-methylpiperazin-1-yl)pyridin-3-yl)-5-bromo-1-((2-(trimethylsilyl)ethoxy)methyl)-1*H-*pyrazolo[3,4-c]pyridine as the starting material. Yield: 91.1%.

ESI-MS: 523.1, [M+H]⁺.

### Step 7: the synthesis of 4-(2-(3-(benzyloxy)propoxy)-4-(5-bromo-1-tosyl-1H-pyrazolo[3,4-c]pyridin-3-yl)phenyl)morpholine

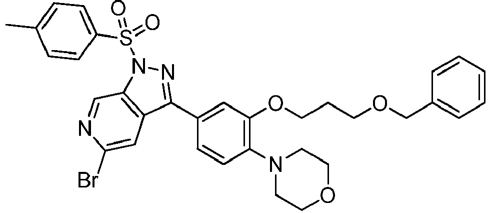

This compound was synthesized according to the procedure described in Example 1 (step 7) using 4-(2-(3-(benzyloxy)propoxy)-4-(5-bromo-1*H*-indazol-3-yl)phenyl)morpholine instead of 3-(5-(3-(benzyloxy)propoxy)-6-(4-methylpiperazin-1-yl)pyridin-3-yl)-5-bromo-1*H-*pyrazolo[3,4-c]pyridine as the starting material. Yield: 90.6%.

ESI-MS: 677.1, [M+H]⁺.

### Step 8: the synthesis of 3-(5-(5-bromo-1-tosyl-1H-pyrazolo[3,4-c]pyridin-3-yl)-2-morpholinophenoxy)propan-1-ol

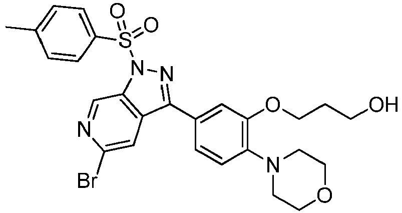

This compound was synthesized according to the procedure described in Example 2 (step 7) using 4-(2-(3-(benzyloxy)propoxy)-4-(5-bromo-1-tosyl-1H-pyrazolo[3,4-c]pyridin-3-yl)phenyl)morpholine instead of 4-(2-(3-(benzyloxy)propoxy)-4-(5-bromo-1-tosyl-1*H-*pyrazolo[3,4-c]pyridin-3-yl)phenyl)morpholine as the starting material. Yield: 53.6%.

ESI-MS: 587.1, [M+H]⁺.

### Step 9: the synthesis of 3-fluoro-2-(3-(3-(3-hydroxypropoxy)-4-morpholinophenyl)-1-tosyl-1H-pyrazolo[3,4-c]pyridin-5-yl)phenol

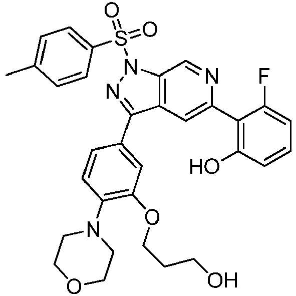

This compound was synthesized according to the procedure described in Example 1 (step 9) using 3-(5-(5-bromo-1-tosyl-1*H*-pyrazolo[3,4-c]pyridin-3-yl)-2-morpholinophenoxy)propan-1-ol instead of 3-((5-(5-bromo-1-tosyl-1*H*-pyrazolo[3,4-c]pyridin-3-yl)-2-(4-methylpiperazin-1-yl)pyridin-3-yl)oxy)propan-1-ol as the starting material. Yield: 93.9%.

ESI-MS: 619.2, [M+H]⁺.

### Step 10: the synthesis of 22-(benzenesulfonyl)-16-fluoro-5-(morpholin-4-yl)-7,11-dioxa-19,22,23-triazapentacyclo[16.5.2.1^{2,6}.0^{12,17}.0^{21,24}]hexacosa-1(23),2(26),3,5,12,14,16,18,20,24-decaene

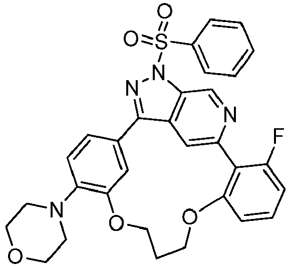

This compound was synthesized according to the procedure described in Example 1 (step 10) using 3-fluoro-2-(3-(3-(3-hydroxypropoxy)-4-morpholinophenyl)-1-tosyl-1*H*-pyrazolo[3,4-c]pyridin-5-yl)phenol instead of 3-((5-(5-bromo-1-tosyl-1H-pyrazolo[3,4-c]pyridin-3-yl)-2-(4-methylpiperazin-1-yl)pyridin-3-yl)oxy)propan-1-ol as the starting material. The crude product was directly used in the next step.

ESI-MS: 601.2, [M+H]⁺.

### Step 11: the synthesis of 16-fluoro-5-(morpholin-4-yl)-7,11-dioxa-19,22,23-triazapentacyclo[16.5.2.1^{2,6}.0^{12,17}.0^{21,24}]hexacosa-1(23),2(26),3,5,12,14,16,18,20,24-decaene

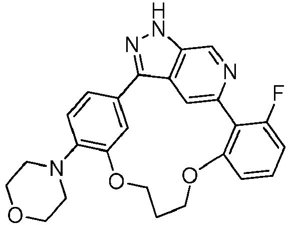

This compound was synthesized according to the procedure described in Example 1 (step 10) using 22-(benzenesulfonyl)-16-fluoro-5-(morpholin-4-yl)-7,11-dioxa-19,22,23-triazapentacyclo[16.5.2.1^{2,6}.0^{12,17}.0^{21,24}]hexacosa-1(23),2(26),3,5,12,14,16,18,20,24-decaene instead of 22-(benzenesulfonyl)-16-fluoro-5-(4-methylpiperazin-1-yl)-7,11-dioxa-4,19,22,23-tetraazapentacyclo[16.5.2.1^{2,6}.0^{12,17}.0^{21,24}]hexacosa-1(23),2(26),3,5,12,14,16,18,20,24-decaene as the starting material. Overall yield: 28.6% (two steps).

ESI-MS: 447.2, [M+H]⁺.

¹H NMR (DMSO-*d*₆, 400 MHz) δ 13.62 (s, 1H), 9.09 (s, 1H), 8.76 (s, 1H), 7.87 (d, *J=* 0.8 Hz, 1H), 7.50 (dd, *J* = 8.0, 1.2 Hz, 1H), 7.44-7.36 (m, 1H), 7.04 (d, *J* = 8.4 Hz, 1H), 7.01-6.95 (m, 2H), 4.60-4.52 (m, 2H), 4.15-4.07 (m, 2H), 3.79-3.72 (m, 4H), 3.10-3.03 (m, 4H), 3.38-3.28 (m, 2H).

### Example 6.

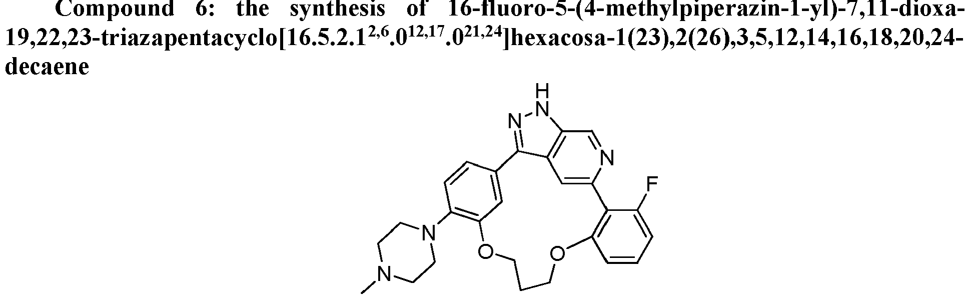

### Step 1: the synthesis of 1-{2-[3-(benzyloxy)propoxy]-4-[5-bromo-1-(4-methylbenzenesulfonyl)-1H-pyrazolo[3,4-c]pyridin-3-yl]phenyl}-4-methylpiperazine

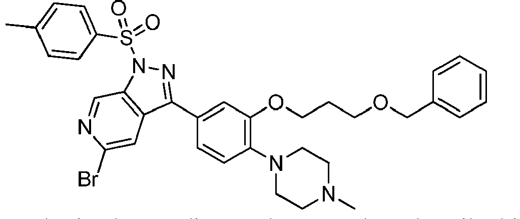

This compound was synthesized according to the procedure described in Example 1 (step 7) using 3-(3-(3-(benzyloxy)propoxy)-4-(4-methylpiperazin-1-yl)phenyl)-5-bromo-1*H-*indazole instead of 3-(5-(3-(benzyloxy)propoxy)-6-(4-methylpiperazin-1-yl)pyridin-3-yl)-5-bromo-1*H*-pyrazolo[3,4-c]pyridine as the starting material. Yield: 97%.

ESI-MS: 690.1, [M+H]⁺.

### Step 2: the synthesis of 3-(5-(5-bromo-1-tosyl-1H-pyrazolo[3,4-c]pyridin-3-yl)-2-(4-methylpiperazin-1 -yl)phenoxy)propan-1 -ol

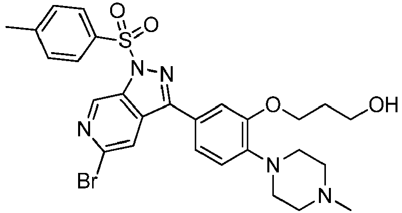

This compound was synthesized according to the procedure described in Example 1 (step 8) using 3-(3-(3-(benzyloxy)propoxy)-4-(4-methylpiperazin-1-yl)phenyl)-5-bromo-1-tosyl-1*H-*pyrazolo[3,4-c]pyridine instead of 3-(5-(3-(benzyloxy)propoxy)-6-(4-methylpiperazin-1-yl)pyridin-3-yl)-5-bromo-1-tosyl-1*H*-pyrazolo[3,4-c]pyridine as the starting material. Yield: 99% (as hydrochloride).

ESI-MS: 600.1, [M+H]⁺.

### Step 3: the synthesis of 2-[1-(benzenesulfonyl)-3-[3-(3-hydroxypropoxy)-4-(4-methylpiperazin-1-yl)phenyl]-1H-pyrazolo[3,4-c]pyridin-5-yl]-3-fluorophenol

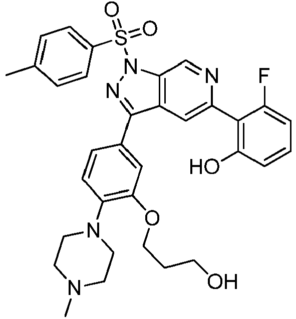

This compound was synthesized according to the procedure described in Example 1 (step 9) using 3-(5-(5-bromo-1-tosyl-1*H*-pyrazolo[3,4-c]pyridin-3-yl)-2-(4-methylpiperazin-1-yl)phenoxy)propan-1-ol instead of 3-((5-(5-bromo-1-tosyl-1*H*-pyrazolo[3,4-c]pyridin-3-yl)-2-(4-methylpiperazin-1-yl)pyridin-3-yl)oxy)propan-1-ol as the starting material. Yield: 36.8%.

ESI-MS: 632.2, [M+H]⁺.

### Step 4: the synthesis of 22-(benzenesulfonyl)-16-fluoro-5-(4-methylpiperazin-1-yl)-7,11-dioxa-19,22,23-triazapentacyclo[16.5.2.1^{2,6}.0^{12,17}.0^{21,24}]hexacosa-1(23),2(26),3,5,12,14,16,18,20,24-decaene

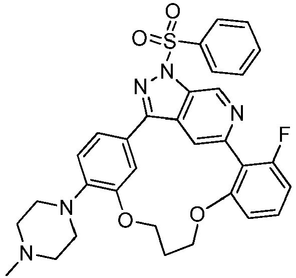

This compound was synthesized according to the procedure described in Example 1 (step 10) using 2-[1-(benzenesulfonyl)-3-[3-(3-hydroxypropoxy)-4-(4-methylpiperazin-1-yl)phenyl]-1*H*-pyrazolo[3,4-c]pyridin-5-yl]-3-fluorophenol instead of 3-((5-(5-bromo-1-tosyl-1*H-*pyrazolo[3,4-c]pyridin-3-yl)-2-(4-methylpiperazin-1-yl)pyridin-3-yl)oxy)propan-1-ol as the starting material. Yield: 39%.

ESI-MS: 614.2, [M+H]⁺.

### Step 5: the synthesis of 16-fluoro-5-(4-methylpiperazin-1-yl)-7,11-dioxa-19,22,23-triazapentacyclo[16.5.2.1^{2,6}.0^{12,17}.0^{21,24}]hexacosa-1(23),2(26),3,5,12,14,16,18,20,24-decaene

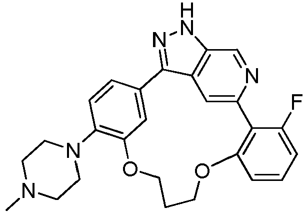

This compound was synthesized according to the procedure described in Example 1 (step 10) using 22-(benzenesulfonyl)-16-fluoro-5-(4-methylpiperazin-1-yl)-7,11-dioxa-19,22,23-triazapentacyclo[16.5.2.1^{2,6}.0^{12,17}.0^{21,24}]hexacosa-1(23),2(26),3,5,12,14,16,18,20,24-decaene instead of 22-(benzenesulfonyl)-16-fluoro-5-(4-methylpiperazin-1-yl)-7,11-dioxa-4,19,22,23-tetraazapentacyclo[16.5.2.1^{2,6}.0^{12,17}.0^{21,24}]hexacosa-1(23),2(26),3,5,12,14,16,18,20,24-decaene as the starting material. Yield: 62%.

ESI-MS: 460.2, [M+H]⁺.

¹H NMR (DMSO-*d*₆, 400 MHz) δ 13.62 (s, 1H), 9.09 (s, 1H), 8.75 (s, 1H), 7.85 (d, *J=* 0.8 Hz, 1H), 7.47 (dd, *J =* 8.4, 1.6 Hz, 1H), 7.44-7.36 (m, 1H), 7.02 (d, *J =* 8.0 Hz, 1H), 7.00 - 6.93 (m, 2H), 4.62 - 4.51 (m, 2H), 4.17 - 4.04 (m, 2H), 3.14-3.00 (m, 4H), 2.49-2.41 (m, 4H), 2.39 - 2.31 (m, 2H), 2.24 (s, 3H).

### Example 7.

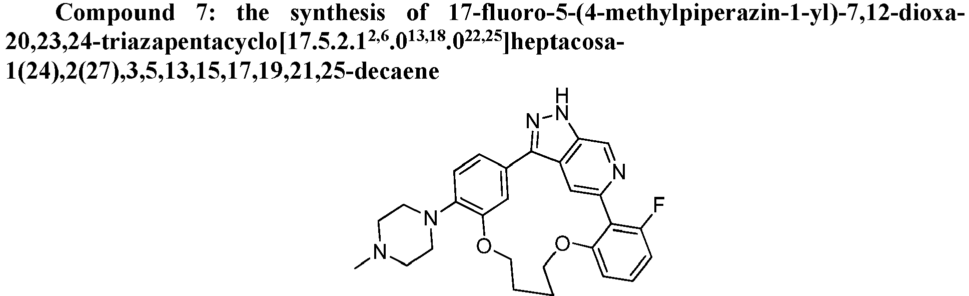

### Step 1: the synthesis of 1-(4-(benzyloxy)butoxy)-2-bromobenzene

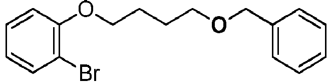

To a solution of 4-(benzyloxy)butan-1-ol (5.218 g, 21.46 mmol) and 2-bromophenol (3.898 g, 23.61 mmol) in DMF (50 mL) was added potassium carbonate (8.156 g, 59.0 mmol) at room temperature, and further stirred at room temperature overnight. The reaction was quenched by pouring into half-saturated sodium bicarbonate aqueous solution (500 mL), and then extracted with ethyl acetate (200 mL × 2). The organic phase was washed with water (400 mL) and brine (400 mL), dried over anhydrous sodium sulfate, and then filtered. The filtrate was concentrated to dryness to afford the crude product of title compound which was directly used in the next step.

ESI-MS: 335.0, [M+H]⁺.

### Step 2: the synthesis of 1-(2-(4-(benzyloxy)butoxy)phenyl)-4-methylpiperazine

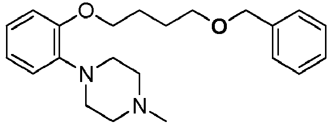

This compound was synthesized according to the procedure described in Example 1 (step 2) using 1-(4-(benzyloxy)butoxy)-2-bromobenzene instead of 3-(3-(benzyloxy)propoxy)-2-bromopyridine as the starting material. Overall yield: 79.7% (two steps).

ESI-MS: 355.2, [M+H]⁺.

### Step 3: the synthesis of 1-(2-(4-(benzyloxy)butoxy)-4-bromophenyl)-4-methylpiperazine

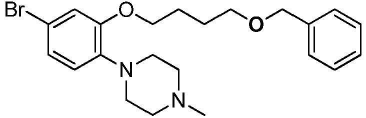

To a solution of 1-(2-(4-(benzyloxy)butoxy)phenyl)-4-methylpiperazine (6.064 g, 17.11 mmol) in acetic acid (78 mL) was added a solution of bromine (967 µL, 17.62 mmol) in acetic acid (20 mL) dropwise, and further stirred at room temperature for 2 hours. After completion of the reaction, the mixture was concentrated under vacuum. The residue was diluted with water (200 mL), and adjusted to pH = 12 with 2N NaOH aqueous solution, and then extracted with ethyl acetate (100 mL × 2). The combined organic phase was washed with brine (200 mL), dried over anhydrous sodium sulfate, and filtered. The filtrate was concentrated to dryness to afford the crude product of title compound which was directly used in the next step without further purification.

ESI-MS: 433.1, [M+H]⁺.

### Step 4: the synthesis of 1-{2-[4-(benzyloxy)butoxy]-4-(4,4,5,5-tetramethyl-1,3,2-dioxaborolan-2-yl)phenyl}-4-methylpiperazine

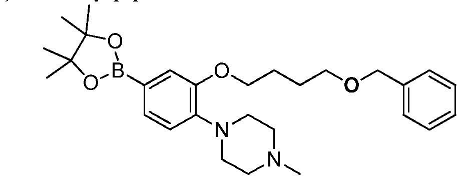

This compound was synthesized according to the procedure described in Example 1 (step 4) using 1-(2-(4-(benzyloxy)butoxy)-4-bromophenyl)-4-methylpiperazine instead of 1-(3-(3-(benzyloxy)propoxy)-5-bromopyridin-2-yl)-4-methylpiperazine as the starting material. The crude product of title compound was directly used in the next step without further purification.

ESI-MS: 481.3, [M+H]⁺.

### Step 5: the synthesis of 1-{2-[4-(benzyloxy)butoxy]-4-(5-bromo-1-{[2-(trimethylsilyl)ethoxy]methyl}-1H-pyrazolo[3,4-c]pyridin-3-yl)phenyl}-4-methylpiperazine

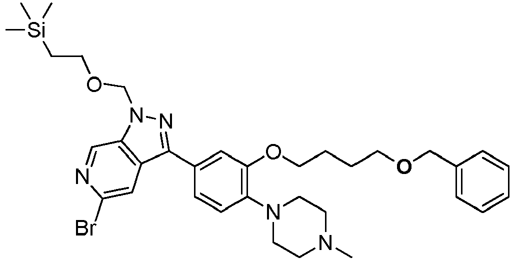

This compound was synthesized according to the procedure described in Example 1 (step 5) using 1-{2-[4-(benzyloxy)butoxy]-4-(4,4,5,5-tetramethyl-1,3,2-dioxaborolan-2-yl)phenyl}-4-methylpiperazine instead of 1-{3-[3-(benzyloxy)propoxy]-5-(4,4,5,5-tetramethyl-1,3,2-dioxaborolan-2-yl)pyridin-2-yl}-4-methylpiperazine as the starting material. Overall yield: 28% (three steps).

ESI-MS: 680.2, [M+H]⁺.

### Step 6: the synthesis of 3-{3-[4-(benzyloxy)butoxy]-4-(4-methylpiperazin-1-yl)phenyl}-5-bromo-1H-indazole

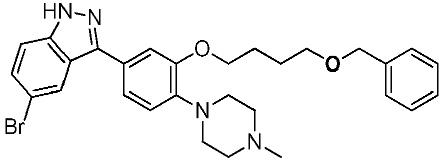

This compound was synthesized according to the procedure described in Example 1 (step 6) using 4-(2-(3-(benzyloxy)propoxy)-4-(5-bromo-1-((2-(trimethylsilyl)ethoxy)methyl)-1*H-*pyrazolo[3,4-c]pyridin-3-yl)phenyl)morpholine instead of 1-{3-[3-(benzyloxy)propoxy]-5-(5-bromo-1-{[2-(trimethylsilyl)ethoxy]methyl}-1*H*-pyrazolo[3,4-c]pyridin-3-yl)pyridin-2-yl}-4-methylpiperazine as the starting material. Yield: 50.7%.

ESI-MS: 550.1, [M+H]⁺.

### Step 7: the synthesis of 1-{2-[4-(benzyloxy)butoxy]-4-[5-bromo-1-(4-methylbenzenesulfonyl)-17/-pyrazolo[3,4-c]pyri din-3-yl]phenyl}-4-methylpiperazine

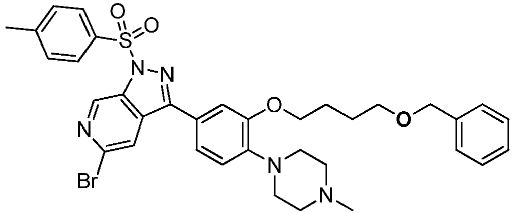

This compound was synthesized according to the procedure described in Example 1 (step 7) using 3 - {3 - [4-(benzyloxy)butoxy] -4-(4-methylpiperazin-1-yl)phenyl} -5 -bromo- 1*H*-indazole instead of 1-{3-[3-(benzyloxy)propoxy]-5-{5-bromo-1*H*-pyrazolo[3,4-c]pyridin-3-yl}pyridin-2-yl}-4-methylpiperazine as the starting material.

ESI-MS: 704.2, [M+H]⁺.

### Step 8: the synthesis of 4-{5-[5-bromo-1-(4-methylbenzenesulfonyl)-1H-pyrazolo[3,4-c]pyridin-3-yl]-2-(4-methylpiperazin-1-yl)phenoxy}butan-1-ol

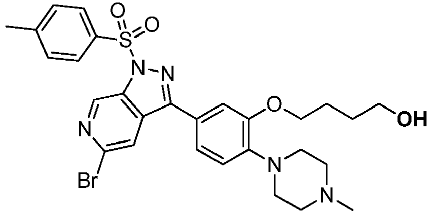

This compound was synthesized according to the procedure described in Example 1 (step 8) using 1-{2-[4-(benzyloxy)butoxy]-4-[5-bromo-1-(4-methylbenzenesulfonyl)-1*H-*pyrazolo[3,4-c]pyridin-3-yl]phenyl}-4-methylpiperazine instead of 1-{3-[3-(benzyloxy)propoxy]-5-[5-bromo-1-(4-methylbenzenesulfonyl)-1H-pyrazolo[3,4-c]pyridin-3-yl]pyridin-2-yl}-4-methylpiperazine as the starting material. Overall yield: 79.7% (two steps).

ESI-MS: 614.1, [M+H]⁺.

### Step 9: the synthesis of 3-fluoro-2- f 3-[3-(4-hydroxybutoxy)-4-(4-methylpiperazin-1-yl)phenyl]-1-(4-methylbenzenesulfonyl)-1H-pyrazolo[3,4-c]pyridin-5-yl}phenol

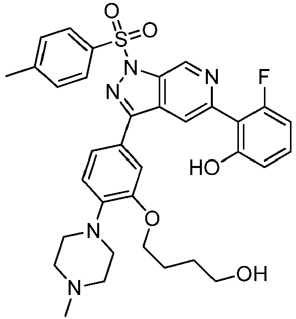

This compound was synthesized according to the procedure described in Example 1 (step 9) using 4-{5-[5-bromo-1-(4-methylbenzenesulfonyl)-1*H*-pyrazolo[3,4-c]pyridin-3-yl]-2-(4-methylpiperazin-1-yl)phenoxy}butan-1-ol instead of 3-({5-[5-bromo-1-(4-methylbenzenesulfonyl)-1*H*-pyrazolo[3,4-c]pyridin-3-yl]-2-(4-methylpiperazin-1-yl)pyridin-3-yl}oxy)propan-1-ol as the starting material.

ESI-MS: 646.2, [M+H]⁺.

### Step 10: the synthesis of 17-fluoro-23-(4-methylbenzenesulfonyl)-5-(4-methylpiperazin-1-yl)-7,12-dioxa-4,20,23,24-tetraazapentacyclo[17.5.2.1^{2,6}.0^{13,18}.0^{22,25}]heptacosa-1(24),2,4,6(27),13(18),14,16,19,21,25-decaene

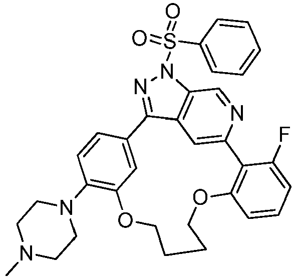

This compound was synthesized according to the procedure described in Example 1 (step 10) using 3-fluoro-2-{3-[3-(4-hydroxybutoxy)-4-(4-methylpiperazin-1-yl)phenyl]-1-(4-methylbenzenesulfonyl)-1*H*-pyrazolo[3,4-c]pyridin-5-yl}phenol instead of 3-({5-[5-bromo-1-(4-methylbenzenesulfonyl)-1*H*-pyrazolo[3,4-c]pyridin-3-yl]-2-(4-methylpiperazin-1-yl)pyridin-3-yl}oxy)propan-1-ol as the starting material. The crude product of title compound was directly used in the next step.

ESI-MS: 628.2, [M+H]⁺.

### Step 11: the synthesis of 17-fluoro-5-(4-methylpiperazin-1-yl)-7,12-dioxa-20,23,24-triazapentacyclo[17.5.2.1^{2,6}.0^{13,18}.0^{22,25}]heptacosa-1(24),2(27),3,5,13,15,17,19,21,25-decaene

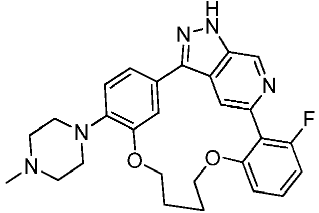

This compound was synthesized according to the procedure described in Example 1 (step 10) using 17-fluoro-23-(4-methylbenzenesulfonyl)-5-(4-methylpiperazin-1-yl)-7,12-dioxa-4,20,23,24-tetraazapentacyclo[17.5.2.1^{2,6}.0^{13,18}.0^{22,25}]heptacosa-1(24),2,4,6(27),13(18),14,16,19,21,25-decaene instead of 22-(benzenesulfonyl)-16-fluoro-5-(4-methylpiperazin-1-yl)-7,11-dioxa-4,19,22,23-tetraazapentacyclo[16.5.2.1^{2,6}.0^{12,17}.0^{21,24}]hexacosa-1(23),2(26),3,5,12,14,16,18,20,24-decaene as the starting material. Overall yield: 61.5% (two steps).

ESI-MS: 474.2, [M+H]⁺.

¹H NMR (DMSO-*d*₆, 400 MHz) δ 13.76 (s, 1H), 9.14 (s, 1H), 8.13 (s, 1H), 7.40-7.35 (m, 1H), 7.28 (dd, *J* = 8.4, 1.6 Hz, 1H), 7.22 (d, *J* = 0.8 Hz, 1H), 7.09 (d, *J* = 8.1 Hz, 1H), 6.98 (d, *J* = 8.5 Hz, 1H), 6.88 (dd, *J* = 10.0, 8.7 Hz, 1H), 4.30-4.22 (m, 2H), 4.15 - 4.07 (m, 2H), 3.50-2.80 (m, 8H), 2.65 (br, 3H), 2.28-2.14 (m, 4H), 1.84 - 1.72 (m, 2H).

The following compounds in the Examples were synthesized according to procedures similar to those described in Example 1 and using appropriate intermediates.

| Example | Compound | Structure | Characterization data |
|---|---|---|---|
| Example 102 | Compound 102 | 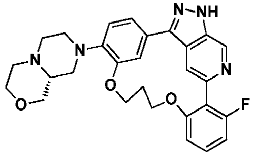 | ESI-MS: 502.3, [M+H]⁺. |
| | | | ¹H NMR (DMSO-*d*₆, 400 MHz) δ 13.61 (s, 1H), 9.09 (s, 1H), 8.75 (s, 1H), 7.84 (s, 1H), 7.50-7.46 (m, 1H), 7.43-7.36 (m, 1H), 7.02-6.93 (m, 3H), 4.58-4.53 (m, 2H), 4.12-4.10 (m, 2H), 3.79-3.75 (m, 1H), 3.71-3.67 (m, 1H), 3.58-3.47 (m, 2H), 3.28-3.20 (m, 1H), 3.16 (t, *J* = 10.2 Hz, 1H), 2.79-2.73 (m, 2H), 2.67-2.64 (m, 1H), 2.43-2.23 (m, 6H). |
| Example 104 | Compound 104 | 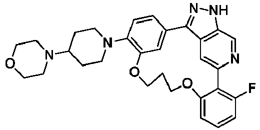 | ESI-MS: 530.3, [M+H]⁺. |
| | | | ¹H NMR (DMSO-*d*₆, 400 MHz) δ 13.58 (s, 1H), 9.09 (s, 1H), 8.75 (s, 1H), 7.84 (s, 1H), 7.50-7.43 (m, 1H), 7.43-7.36 (m, 1H), 7.03 (d, *J* = 8.2 Hz, 1H), 7.00-6.92 (m, 2H), 4.59-4.50 (m, 2H), 4.12-4.10 (m, 2H), 3.63-3.49 (m, 6H), 2.60 (t, *J* = 11.4 Hz, 2H), 2.51 (m, 4H) 2.40-2.30 (m, 3H), 1.86 (d,J = 11.7 Hz, 2H), 1.57 (m, 2H). |
| Example 106 | Compound 106 | 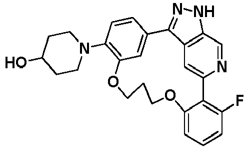 | ESI-MS: 461.2, [M+H]⁺. |
| Example 108 | Compound 108 | 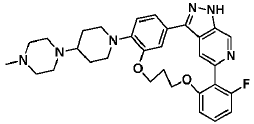 | ESI-MS: 543.3, [M+H]⁺. |
| | | | ¹H NMR (DMSO-*d*₆, 400 MHz) δ 13.60 (s, 1H), 9.09 (s, 1H), 8.75 (s, 1H), 7.84 (s, 1H), 7.47 (dd, *J* = 8.1, 1.6 Hz,1H), 7.43-7.37 (m, 1H), 7.03 (d, *J* = 8.2 Hz, 1H), 7.01-6.93 (m, 2H), 4.59-4.51 (m, 2H), 4.15-4.08 (m, 2H), 3.54 (d, *J* = 5.8 Hz, 2H), 2.63-2.50 (m, 6 H), 2.43-2.25 (m, 7H), 2.17 (s, 3H), 1.89-1.79 (m, 2H), 1.64-1.51 (m, 2H). |
| Example 110 | Compound 110 | 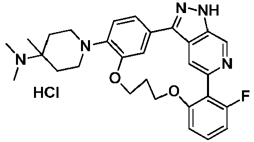 | ESI-MS: 502.2, [M+H-HCl]⁺. |
| | | | ¹H NMR (DMSO-*d*₆, 400 MHz) δ 13.65 (s, 1H), 10.45 (s, 1H), 10.19 (br, 1H), 9.09 (s, 1H), 8.75 (s, 1H), 7.87 (s,1 H), 7.49 (dd, *J* = 8.2, 1.5 Hz, 1H), 7.43-7.37 (m, 1H), 7.08 (d, *J* = 8.2 Hz, 1H), 7.00-6.93 (m, 2H), 4.60-4.52 (m, 2H), 4.14-4.08 (m, 2H), 3.56-3.46 (m, 2H), 2.78 (t, *J* = 11.4 Hz, 2H), 2.69 (s, 6H), 2.38-2.28 (m, 2H), 2.10-2.00 (m, 2H), 1.69-1.90 (m, 2H), 1.34 (s, 3H). |
| Example 113 | Compound 113 | 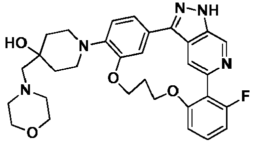 | ESI-MS: 560.3, [M+H]⁺. |
| Example 117 | Compound 117 | 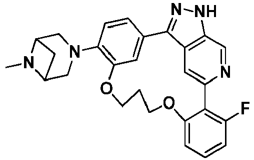 | ESI-MS: 472.3, [M+H]⁺. |
| | | | ¹H NMR (DMSO-*d*₆, 400 MHz) δ 13.56 (s, 1H), 9.08 (s, 1H), 8.73 (s, 1H), 7.80 (s, 1 H), 7.48-7.45 (m, 1H), 7.43-7.36 (m, 1H), 7.06 (d, *J* = 8.2 Hz, 1H), 7.01-6.93 (m, 2H), 4.56-4.52 (m, 2H), 4.13-4.10 (m, 2H), 3.80-3.60 (m, 6H), 2.40-2.30 (m, 3H), 2.15 (br, 3H), 1.83 (br, 1H). |
| Example 130 | Compound 130 | 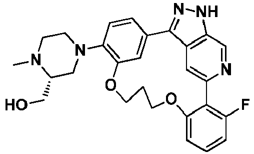 | ESI-MS: 490.3, [M+H]⁺. |
| | | | ¹H NMR (DMSO-*d*₆, 400 MHz) δ 13.61 (s, 1H), 9.09 (s, 1H), 8.75 (s, 1H), 7.84 (s, 1H), 7.50-7.46 (m, 1H), 7.43-7.36 (m, 1H), 7.02-6.93 (m, 3H), 4.58-4.53 (m, 3H), 4.12-4.10 (m, 2H), 3.70-3.62 (m, 1H), 3.53-3.46 (m, 1H), 3.40-3.30 (m, 4H), 2.84-2.70 (m, 2H), 2.56-2.50 (m, 1H), 2.42-2.19 (m, 7H). |
| Example 131 | Compound 131 | 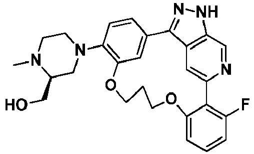 | ESI-MS: 490.3, [M+H]⁺. |
| | | | ¹H NMR (DMSO-*d*₆, 400 MHz) δ 13.60 (s, 1H), 9.09 (s, 1H), 8.75 (s, 1H), 7.84 (s, 1H), 7.50-7.46 (m, 1H), 7.43-7.36 (m, 1H), 7.02-6.93 (m, 3H), 4.58-4.53 (m, 3H), 4.12-4.10 (m, 2H), 3.70-3.62 (m, 1H), 3.53-3.46 (m, 1H), 3.40-3.30 (m, 4H), 2.84-2.70 (m, 2H), 2.56-2.50 (m, 1H), 2.42-2.19 (m, 7H). |
| Example 134 | Compound 134 | 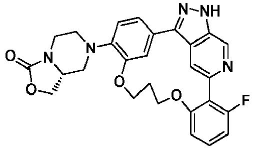 | ESI-MS: 502.3, [M+H]⁺. ¹H NMR (DMSO-*d*₆, 400 MHz) δ 13.58 (s, 1H), 9.09 (s, 1H), 8.75 (s, 1H), 7.84 (s, 1H), 7.50-7.43 (m, 1H), 7.43-7.36 (m, 1H), 7.03 (d, *J* = 8.2 Hz, 1H), 7.00-6.92 (m, 2H), 4.59-4.50 (m, 2H), 4.45-4.40 (m, 1H), 4.12-3.95 (m, 4H), 3.70-3.64 (m, 1H), 3.57-3.50 (m, 1H), 3.44-3.38 (m, 1H), 3.24-3.15 (m, 1H), 2.64-2.52 (m, 2H), 2.40-2.30 (m, 2H). |
| Example 135 | Compound 135 | 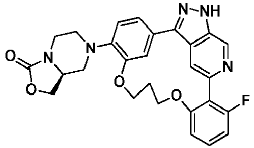 | ESI-MS: 502.3, [M+H]⁺. |
| | | | ¹H NMR (DMSO-*d*₆, 400 MHz) δ 13.58 (s, 1H), 9.09 (s, 1H), 8.75 (s, 1H), 7.84 (s, 1H), 7.50-7.43 (m, 1H), 7.43-7.36 (m, 1H), 7.03 (d, *J* = 8.2 Hz, 1H), 7.00-6.92 (m, 2H), 4.59-4.50 (m, 2H), 4.45-4.40 (m, 1H), 4.12-3.95 (m, 4H), 3.70-3.64 (m, 1H), 3.57-3.50 (m, 1H), 3.44-3.38 (m, 1H), 3.24-3.15 (m, 1H), 2.64-2.52 (m, 2H), 2.40-2.30 (m, 2H). |
| Example 137 | Compound 137 | 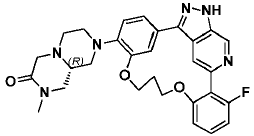 | ESI-MS: 529.3, [M+H]⁺. |
| | | | ¹H NMR (DMSO-*d*₆, 400 MHz) δ 13.59 (s, 1H), 9.09 (s, 1H), 8.75 (s, 1H), 7.84 (s, 1 H), 7.48-7.36 (m, 2H), 6.99-6.92 (m, 3H), 4.59-4.54 (m, 2H), 4.13-4.10 (m, 2H), 3.52-3.40 (m, 2H), 3.36-3.28 (m, 2H), 3.24 (dd, *J* = 11.6, 4.1 Hz, 1H), 3.13 (t, *J* = 11.1 Hz, 1H), 2.92-2.86 (m, 1H), 2.85-2.72 (m, 5H), 2.70-2.60 (m, 1H), 2.50-2.44 (m, 1H), 2.38-2.25 (m, 3H). |
| Example 138 | Compound 138 | 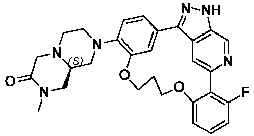 | ESI-MS: 529.3, [M+H]⁺. |
| | | | ¹H NMR (DMSO-*d*₆, 400 MHz) δ 13.59 (s, 1H), 9.09 (s, 1H), 8.75 (s, 1H), 7.84 (s, 1 H), 7.48-7.36 (m, 2H), 6.99-6.92 (m, 3H), 4.59-4.54 (m, 2H), 4.13-4.10 (m, 2H), 3.52-3.40 (m, 2H), 3.36-3.28 (m, 2H), 3.24 (dd, *J* = 11.6, 4.1 Hz, 1H), 3.13 (t, *J* = 11.1 Hz, 1H), 2.92-2.86 (m, 1H), 2.85-2.72 (m, 5H), 2.70-2.60 (m, 1H), 2.50-2.44 (m, 1H), 2.38-2.25 (m, 3H). |
| Example 140 | Compound 140 | 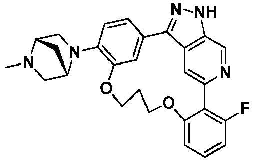 | ESI-MS: 472.2, [M+H]⁺. |
| | | | ¹H NMR (DMSO-*d*₆, 400 MHz) δ 13.62 (s, 1H), 9.09 (s, 1H), 8.75 (s, 1H), 7.84 (s, 1 H), 7.48-7.36 (m, 2H), 6.99-6.92 (m, 3H), 4.58-4.53 (m, 2H), 4.42-4.34 (m, 1H), 4.15-4.10 (m, 2H), 3.56-3.50 (m, 1H), 3.40-3.30 (m, 1H), 3.26-3.20 (m, 1H), 2.80-2.74 (m, 1H), 2.68 (d, *J* = 9.3 Hz, 1H), 2.40-2.25 (m, 5H), 1.82 (d, *J* = 9.1 Hz, 1H), 1.71 (d, J= 9.1 Hz, 1H). |
| Example 141 | Compound 141 | 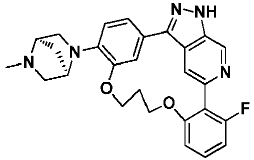 | ESI-MS: 472.2, [M+H]⁺. |
| | | | ¹H NMR (DMSO-*d*₆, 400 MHz) δ 13.62 (s, 1H), 9.09 (s, 1H), 8.75 (s, 1H), 7.84 (s, 1 H), 7.48-7.36 (m, 2H), 6.99-6.92 (m, 3H), 4.58-4.53 (m, 2H), 4.42-4.34 (m, 1H), 4.15-4.10 (m, 2H), 3.56-3.50 (m, 1H), 3.40-3.30 (m, 1H), 3.26-3.20 (m, 1H), 2.80-2.74 (m, 1H), 2.68 (d, *J* = 9.3 Hz, 1H), 2.40-2.25 (m, 5H), 1.82 (d, *J* = 9.1 Hz, 1H), 1.71 (d, J= 9.1 Hz, 1H). |
| Example 142 | Compound 142 | 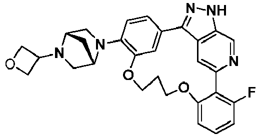 | ESI-MS: 514.2, [M+H]⁺. |
| | | | ¹H NMR (DMSO-*d*₆, 400 MHz) δ 13.62 (s, 1H), 9.08 (s, 1H), 8.73 (s, 1H), 7.78 (s, 1 H), 7.50-7.42 (m, 1H), 7.43-7.36 (m, 1H), 7.10-7.02 (m, 1H), 7.01-6.92 (m, 2H), 4.58-4.53 (m, 2H), 4.43 (br, 1H), 4.37 (s, 2H), 4.29-4.27 (m 2H), 4.14-4.08 (m, 2H), 3.39-3.32 (m, 1H), 3.58-3.55 (m, 1H), 3.41 (s, 1H), 3.08-3.01 (m, 1H), 2.88 (d, *J =* 10.0 Hz, 1H), 2.78-2.75 (m, 1H), 2.38-2.30 (m, 2H), 1.80-1.72 (m, 2H). |
| Example 143 | Compound 143 | 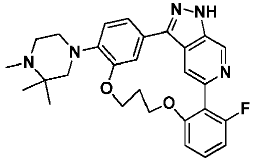 | ESI-MS: 488.3, [M+H]⁺. |
| | | | ¹H NMR (DMSO-*d*₆, 400 MHz) δ 13.81 (s, 1H), 9.09 (s, 1H), 8.75 (s, 1H), 7.84 (s, 1 H), 7.48-7.36 (m, 2H), 6.99-6.92 (m, 3H), 4.59-4.54 (m, 2H), 4.34 (s, 2H), 4.13-4.10 (m, 2H), 3.16 (br, 2H), 2.86 (br, 2H), 2.57 (br, 3H), 2.38-2.30 (m, 2H), 1.50-1.10 (m, 6H). |
| Example 144 | Compound 144 | 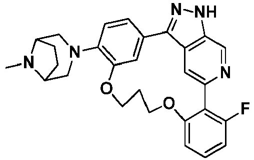 | ESI-MS: 486.3, [M+H]⁺. |
| | | | ¹H NMR (DMSO-*d*₆, 400 MHz) δ 13.59 (s, 1H), 9.09 (s, 1H), 8.75 (s, 1H), 7.84 (s, 1 H), 7.48-7.36 (m, 2H), 6.99-6.92 (m, 3H), 4.59-4.54 (m, 2H), 4.13-4.10 (m, 2H), 3.30-3.20 (m, 2H), 3.20-2.90 (m, 4H), 2.40 (br, 3H), 2.38-2.30 (m, 2H), 2.10-1.90 (m, 4H). |
| Example 145 | Compound 145 | 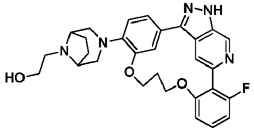 | ESI-MS: 516.3, [M+H]⁺. |
| | | | ¹H NMR (DMSO-*d*₆, 400 MHz) δ 13.59 (s, 1H), 9.08 (s, 1H), 8.74 (s, 1H), 7.82 (s, 1 H), 7.46-7.36 (m, 2H), 6.99-6.92 (m, 3H), 4.58-4.53 (m, 2H), 4.38 (br, 1H), 4.14-4.08 (m, 2H), 3.60-3.48 (m, 2H), 3.30-3.24 (m, 2H), 2.89 (br, 2H), 2.50-2.40 (m, 2H), 2.38-2.30 (m, 2H), 1.88 (br, 4H). |
| Example 146 | Compound 146 | 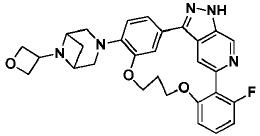 | ESI-MS: 514.3, [M+H]⁺. |
| | | | ¹H NMR (DMSO-*d*₆, 400 MHz) δ 13.53 (s, 1H), 9.08 (s, 1H), 8.72 (s, 1H), 7.78 (s, 1 H), 7.48-7.45 (m, 1H), 7.43-7.36 (m, 1H), 7.06 (d, *J* = 8.2 Hz, 1H), 7.01-6.93 (m, 2H), 4.62 (t, *J* = 6.0 Hz, 2H), 4.54-4.49 (m, 2H), 4.29-4.26 (m, 2H), 4.13-4.10 (m, 2H), 3.90-3.83 (m, 1H), 3.70-3.64 (m, 2H), 3.61 (d, *J* = 11.6 Hz, 2H), 3.51 (d, *J* = 11.6 Hz, 2H), 2.48-2.41 (m, 1H), 2.38-2.28 (m, 2H), 1.75-1.72 (m, 2H). |
| Example 147 | Compound 147 | 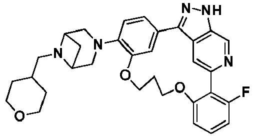 | ESI-MS: 556.3, [M+H]⁺. |
| | | | ¹H NMR (DMSO-*d*₆, 400 MHz) δ 13.57 (s, 1H), 9.08 (s, 1H), 8.73 (s, 1H), 7.78 (s, 1 H), 7.50-7.42 (m, 1H), 7.43-7.36 (m, 1H), 7.10-7.02 (m, 1H), 7.01-6.92 (m, 2H), 4.52-4.49 (m, 2H), 4.14-4.08 (m, 2H), 3.90-3.60 (m, 6H), 3.30-3.20 (m, 4H), 2.48-2.30 (m, 3H), 1.90-1.72 (m, 1H), 1.70-1.58 (m, 3H), 1.20-1.10 (m, 2H). |
| Example 148 | Compound 148 | 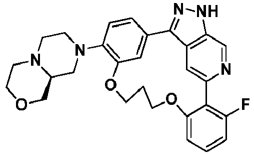 | ESI-MS: 502.3, [M+H]⁺. |
| | | | ¹H NMR (DMSO-*d*₆, 400 MHz) δ 13.61 (s, 1H), 9.09 (s, 1H), 8.75 (s, 1H), 7.84 (s, 1H), 7.50-7.46 (m, 1H), 7.43-7.36 (m, 1H), 7.02-6.93 (m, 3H), 4.58-4.53 (m, 2H), 4.12-4.10 (m, 2H), 3.79-3.75 (m, 1H), 3.71-3.67 (m, 1H), 3.58-3.47 (m, 2H), 3.28-3.20 (m, 1H), 3.16 (t, *J* = 10.2 Hz, 1H), 2.79-2.73 (m, 2H), 2.67-2.64 (m, 1H), 2.43-2.23 (m, 6H). |

### Example 9.

### Compound 9: the synthesis of 16-fluoro-5-(4-methylpiperazin-1-yl)-7,10-dioxa-19,22,23-triazapentacyclo[16.5.2.1^{2,6}.0^{12,17}.0^{21,24}]hexacosa-1(23),2(26),3,5,12,14,16,18,20,24-decaene

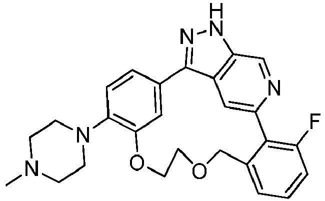

### Step 1: the synthesis of 2-((2-bromo-3-fluorobenzyl)oxy)ethanol

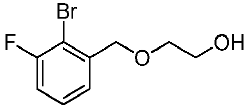

To a suspension of sodium hydride (82 mg, 2.05 mmol) in dry tetrahydrofuran (5 mL) was added ethylene glycol (1.758 g, 28.37 mmol) dropwise at 0°C under nitrogen. The mixture was then warmed to room temperature, and was added 2-bromo-1-(bromomethyl)-3-fluorobenzene (500 mg, 1.87 mmol). After stirring at room temperature for 2 hours, the mixture was warmed to 50°C and further stirred for 2 hours. The reaction mixture was then cooled to 0°C, quenched with water (50 mL), and extracted with ethyl acetate (50 mL × 2). The combined organic phase was washed with water (50 mL) and brine (50 mL), dried over anhydrous sodium sulfate, and filtered. The filtrate was concentrated to dryness to afford the title product (417 mg, colorless oil). Yield: 90%.

ESI-MS: 250.2, [M+H]⁺.

### Step 2: the synthesis of 2-(2-((2-bromo-3-fluorobenzyl)oxy)ethoxy)tetrahydro-2H-pyran

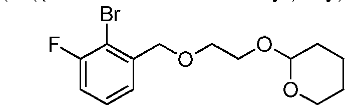

To a solution of 2-((2-bromo-3-fluorobenzyl)oxy)ethanol (417 mg, 1.67 mmol) in dichloromethane (5 mL) was added 3,4-dihydro-2H-pyran (169 mg, 2.01 mmol) and pyridinium p-toluenesulfonate (84 mg, 0.34 mmol), and stirred at room temperature for 14 hours. To the mixture was added sodium carbonate aqueous solution (40 mL), and then extracted with dichloromethane (40 mL). The organic phase was washed with water (40 mL), brine (40 mL), dried over anhydrous sodium sulfate, and then filtered. The filtrate was concentrated to dryness, and the residue was purified by column chromatography (petroleum ether/ethyl acetate = 10/1) to afford the title product (506 mg, colorless oil). Yield: 90.8%.

ESI-MS: 334.2, [M+H]⁺.

### Step 3: the synthesis of 2-(2-fluoro-6-{[2-(oxan-2-yloxy)ethoxy]methyl}phenyl)-4,4,5,5-tetramethyl-1,3,2-dioxaborolane

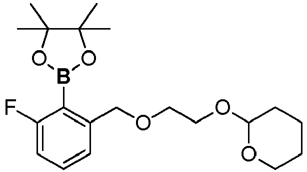

To a solution of 2-(2-((2-bromo-3-fluorobenzyl)oxy)ethoxy)tetrahydro-2H-pyran (386 mg, 1.16 mmol) in dry tetrahydrofuran (4 mL) cooled at -70°C in dry ice-ethanol bath under nitrogen was added n-butyl lithium solution in n-hexane (2.5M, 0.56 mL, 1.39 mmol) dropwise, while keeping the inner temperature below -60°C. After the addition, the mixture was stirred at -65°C for 1 hour, followed by the addition of 4,4,5,5-tetramethyl-2-(propan-2-yloxy)-1,3,2-dioxaborolane (259 mg, 1.39 mmol) dropwise. After stirring at -65°C for 1 hour, the mixture was warmed to 0°C, and further stirred for 1 hour. The reaction was then cooled to -10°C, quenched with ammonium chloride aqueous solution (40 mL), and extracted with ethyl acetate (40 mL × 2). The combined organic phase was washed with water (50 mL), brine (50 mL), dried over anhydrous sodium sulfate, and filtered. The filtrate was concentrated to afford the title compound (352 mg, pale yellow oil). Yield: 80%.

ESI-MS: 398.2, [M+NH₄]⁺.

### Step 4: the synthesis of tert-butyl 4-[2-(acetyloxy)-4-[5-(2-fluoro-6-{[2-(oxan-2-yloxy)ethoxy]methyl}phenyl)-1-{[2-(trimethylsilyl)ethoxy]methyl}-1H-pyrazolo[3,4-c]pyridin-3-yl]phenyl]piperazine-1-carboxylate

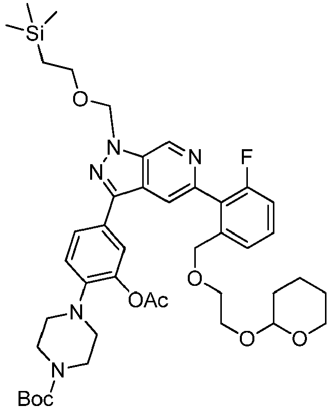

To a mixture of 2-(2-fluoro-6-{[2-(oxan-2-yloxy)ethoxy]methyl}phenyl)-4,4,5,5-tetramethyl-1,3,2-dioxaborolane (300 mg, 0.46 mmol), tert-butyl 4-[2-(acetyloxy)-4-(5-bromo-1- f [2-(trimethylsilyl)ethoxy]methyl}-1H-pyrazolo[3,4-c]pyridin-3-yl)phenyl]piperazine-1-carboxylate (352 mg, 0.93 mmol), sodium carbonate (148 mg, 1.39 mmol) and Xphos-Pd-G2 (37 mg, 0.05 mmol) under nitrogen was added 1,4-dioxane (5 mL) and water (1 mL), and the mixture was heated to 80°C and stirred for 4 hours. The mixture was then cooled to room temperature, diluted with water (60 mL), and extracted with ethyl acetate (50 mL × 2). The combined organic phase was washed with brine (50 mL), dried over anhydrous sodium sulfate, and filtered. The filtrate was concentrated to dryness, and the residue was purified by column chromatography (petroleum ether/ethyl acetate = 5/1 to 2/1) to afford the title product (322 mg, yellow solid). Yield: 84%.

ESI-MS: 820.4, [M+H]⁺.

### Step 5: the synthesis of tert-butyl 4-[4-(5-{2-fluoro-6-[(2-hydroxyethoxy)methyl]phenyl}-1-{[2-(trimethylsilyl)ethoxy]methyl}-1H-pyrazolo[3,4-c]pyridin-3-yl)-2-hydroxyphenyl]piperazine-1-carboxylate

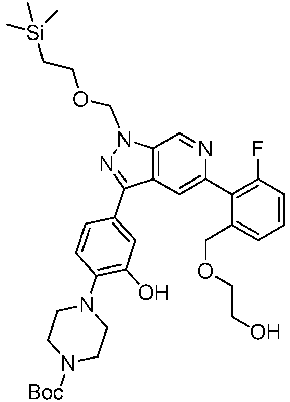

To a solution of *tert*-butyl 4-[2-(acetyloxy)-4-[5-(2-fluoro-6-{[2-(oxan-2-yloxy)ethoxy]methyl}phenyl)-1-{[2-(trimethylsilyl)ethoxy]methyl}-1*H*-pyrazolo[3,4-c]pyridin-3-yl]phenyl]piperazine-1-carboxylate (322 mg, 0.39 mmol) in methanol (5 mL) was added aqueous solution of lithium hydroxide (3M, 1.0 mL) at 0°C, and stirred for 1 hour. After completion of the reaction, the mixture was adjusted to pH = 7 with 5% citric acid solution, and then extracted with ethyl acetate (50 mL × 2). The organic phase washed with brine, dried over anhydrous sodium sulfate, and filtered. The filtrate was concentrated to dryness to afford the crude intermediate (286 mg). To a solution of the crude intermediate (286 mg, 0.37 mmol) in methanol (5 mL) was added p-toluenesulfonic acid hydrate (70 mg, 0.37 mmol) at room temperature, and stirred 40°C for 14 hours. The mixture was concentrated to dryness, and the residue was dissolved in dichloromethane (50 mL). The solution was washed with water (30 mL), brine (30 mL), dried over anhydrous sodium sulfate, and filtered. The filtrate was concentrated to dryness, and the residue was purified by column chromatography (dichloromethane/methanol = 15/1) to afford the title product (250 mg, pale yellow solid). Yield: 92%.

ESI-MS: 694.4, [M+H]⁺.

### Step 6: the synthesis of tert-butyl 4-(16-fluoro-22-{[2-(trimethylsilyl)ethoxy]methyl}-7,10-dioxa-19,22,23-triazapentacyclo[16.5.2.1^{2,6}.0^{12,17}.0^{21,24}]hexacosa-1(23),2(26),3,5,12,14,16,18,20,24-decaen-5-yl)piperazine-1-carboxylate

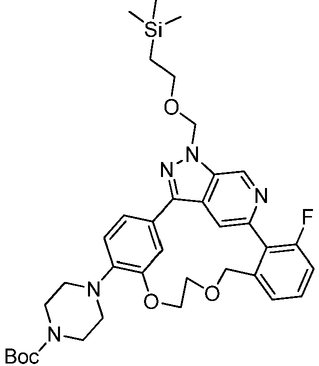

To a solution of TMAD (159 mg, 0.92 mmol) in dry tetrahydrofuran (20 mL) was added tributylphosphine (186 mg, 0.92 mmol) at room temperature under nitrogen. After stirring for 5 min, a solution of tert-butyl 4-[4-(5-{2-fluoro-6-[(2-hydroxyethoxy)methyl]phenyl}-1-{[2-(trimethylsilyl)ethoxy]methyl}-1H-pyrazolo[3,4-c]pyridin-3-yl)-2-hydroxyphenyl]piperazine-1-carboxylate (160 mg, 0.23 mmol) in tetrahydrofuran (5 mL) was added dropwise, and further stirred for 1 hour. The reaction was quenched with water (50 mL), and extracted with ethyl acetate (50 mL × 2). The combined organic phase was washed with brine (50 mL), dried over anhydrous sodium sulfate, and filtered. The filtrate was concentrated to dryness, and the residue was purified by column chromatography (petroleum ether/ethyl acetate = 5/1 to 1/1) to afford the title product (64 mg, white solid). Yield: 48%.

ESI-MS: 676.3, [M+H]⁺.

### Step 7: the synthesis of 16-fluoro-5-(piperazin-1-yl)-7,10-dioxa-19,22,23-triazapentacyclo[16.5.2.1^{2,6}.0^{12,17}.0^{21,24}]hexacosa-1(23),2(26),3,5,12,14,16,18,20,24-decaene

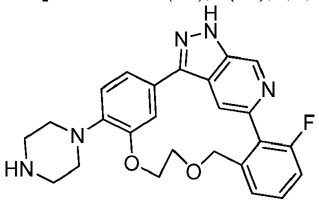

To a solution of tert-butyl 4-(16-fluoro-22-{[2-(trimethylsilyl)ethoxy]methyl}-7,10-dioxa-19,22,23-triazapentacyclo[16.5.2.1^{2,6}.0^{12,17}.0^{21,24}]hexacosa-1(23),2(26),3,5,12,14,16,18,20,24-decaen-5-yl)piperazine-1-carboxylate (50 mg, 0.07 mmol) in dichloromethane (5 mL) was added trifluoroacetic acid (1.0 mL) at 0°C, and stirred at room temperature for 14 hours. The reaction mixture was concentrated to dryness, and the residue was re-dissolved in dichloromethane (20 mL), and then treated with sodium carbonate aqueous solution (20 mL). The mixture was extracted with dichloromethane/isopropanol mixture (4/1, 20 mL × 3), and the combined organic phase was concentrated to dryness. To a solution of the residue in acetonitrile (5 mL) was added ammonia (0.5 mL), and stirred for 30 min. The mixture was then diluted with water (30 mL), and extracted with dichloromethane/isopropanol mixture (4/1, 20 mL × 3). The combined organic phase was washed with brine (20 mL), dried over anhydrous sodium sulfate and filtered. The filtrate was concentrated to dryness to afford the title product (33 mg, white solid). Yield: 100%.

ESI-MS: 446.2, [M+H]⁺.

### Step 8: the synthesis of 16-fluoro-5-(4-methylpiperazin-1-yl)-7,10-dioxa-19,22,23-triazapentacyclo[16.5.2.1^{2,6}.0^{12,17}.0^{21,24}]hexacosa-1(23),2(26),3,5,12,14,16,18,20,24-decaene

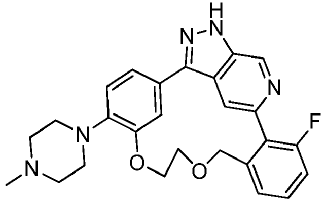

To a solution of 16-fluoro-5-(piperazin-1-yl)-7,10-dioxa-19,22,23-triazapentacyclo[16.5.2.1^{2,6}.0^{12,17}.0^{21,24}]hexacosa-1(23),2(26),3,5,12,14,16,18,20,24-decaene (33 mg, 0.07 mmol) in dichloromethane/methanol (1/1, 4 mL) was added 1*H*-benzotriazole-1-methanol (14 mg, 0.09 mmol), sodium acetate (12 mg, 0.14 mmol) and sodium triacetoxyborohydride (31 mg, 0.14 mmol), and stirred at room temperature for 12 hours. The reaction was quenched with sodium bicarbonate aqueous solution (30 mL), and was extracted with dichloromethane/isopropanol mixture (4/1, 20 mL × 3). The combined organic phase was washed with brine (30 mL), dried over anhydrous sodium sulfate and filtered. The filtrate was concentrated to dryness, and residue was purified by pre-TLC (dichloromethane/methanol = 7/1) to afford the title product (18 mg, white solid). Yield: 53%.

ESI-MS: 460.2, [M+H]⁺.

¹H NMR (DMSO-*d*₆, 400MHz) δ 13.76 (s, 1H), 9.15 (s, 1H), 9.00 (s, 1H), 7.96 (d, *J* = 1.5 Hz, 1H), 7.62 (dd, *J* = 8.2, 1.6 Hz, 1H), 7.49-7.44 (m, 2H), 7.38-7.33 (m, 1H), 7.03 (d, *J=* 8.4 Hz, 1H), 4.51 (br, 2H), 4.41 (s, 2H), 4.08 (s, 2H), 3.05 (s, 4H), 2.55-2.45 (m, 4H), 2.24 (m, 3H).

### Example 10.

### Compound 10: the synthesis of 17-fluoro-5-(piperazin-1-yl)-7,11-dioxa-20,23,24-triazapentacyclo[17.5.2.1^{2,6}.0^{13,18}.0^{22,25}]heptacosa-1(24),2(27),3,5,13,15,17,19,21,25-decaene

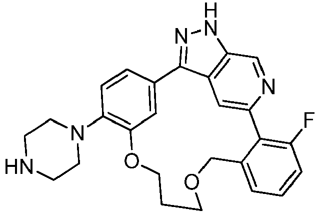

### Step 1: the synthesis of 2-((2-bromo-3-fluorobenzyl)oxy)propan-1-ol

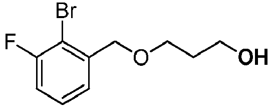

This compound was synthesized according to the procedure described in Example 9 (step 1) using 1,3-propanediol instead of ethylene glycol as the starting material. Yield: 100%. ESI-MS: 262.9, [M+H]⁺.

### Step 2: the synthesis of 2-{3-[(2-bromo-3-fluorophenyl)methoxy]propoxy}oxane

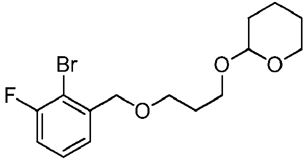

This compound was synthesized according to the procedure described in Example 9 (step 2) using 2-((2-bromo-3-fluorobenzyl)oxy)propan-1-ol instead of 2-((2-bromo-3-fluorobenzyl)oxy)ethanol as the starting material. Yield: 93.1%.

ESI-MS: 346.9, [M+H]⁺.

### Step 3: the synthesis of 2-(2-fluoro-6-{ [3-(oxan-2-yloxy)propoxy]methyl}phenyl)-4,4,5,5-tetramethyl-1,3,2-dioxaborolane

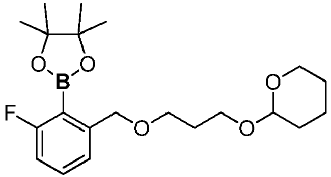

This compound was synthesized according to the procedure described in Example 9 (step 3) using 2-{3-[(2-bromo-3-fluorophenyl)methoxy]propoxy}oxane instead of 2-(2-((2-bromo-3-fluorobenzyl)oxy)ethoxy)tetrahydro-2H-pyran as the starting material. Yield: 93.1%.

ESI-MS: 412.2, [M+H]⁺.

### Step 4: the synthesis of tert-butyl 4-[2-(acetyloxy)-4-[5-(2-fluoro-6-{[3-(oxan-2-yloxy)propoxy]methyl}phenyl)-1-{[2-(trimethylsilyl)ethoxy]methyl}-1H-pyrazolo[3,4-c]pyridin-3-yl]phenyl]piperazine-1-carboxylate

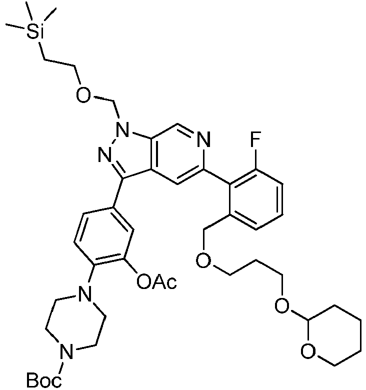

This compound was synthesized according to the procedure described in Example 9 (step 4) using 2-(2-fluoro-6- f [3-(oxan-2-yloxy)propoxy]methyl}phenyl)-4,4,5,5-tetramethyl-1,3,2-dioxaborolane instead of 2-(2-fluoro-6- f [2-(oxan-2-yloxy)ethoxy]methyl}phenyl)-4,4,5,5-tetramethyl-1,3,2-dioxaborolane as the starting material. Yield: 95%.

ESI-MS: 834.4, [M+H]⁺.

### Step 5: the synthesis of tert-butyl 4-[4-(5-{2-fluoro-6-[(3-hydroxypropoxy)methyl]phenyl}-1-{[2-(trimethylsilyl)ethoxy]methyl}-1H-pyrazolo[3,4-c]pyridin-3-yl)-2-hydroxyphenyl]piperazine-1-carboxylate

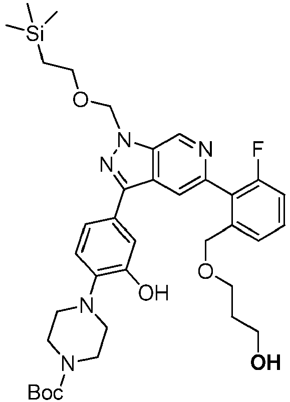

This compound was synthesized according to the procedure described in Example 9 (step 5) using tert-butyl 4-[2-(acetyloxy)-4-[5-(2-fluoro-6-{[3-(oxan-2-yloxy)propoxy]methyl}phenyl)-1-{[2-(trimethylsilyl)ethoxy]methyl}-1H-pyrazolo[3,4-c]pyridin-3-yl]phenyl]piperazine-1-carboxylate instead of tert-butyl 4-[2-(acetyloxy)-4-[5-(2-fluoro-6- f [2-(oxan-2-yloxy)ethoxy]methyl}phenyl)-1- f [2-(trimethylsilyl)ethoxy]methyl}-1H-pyrazolo[3,4-c]pyridin-3-yl]phenyl]piperazine-1-carboxylate as the starting material. Yield: 88%.

ESI-MS: 708.3, [M+H]⁺.

### Step 6: the synthesis of tert-butyl 4-(17-fluoro-23-{[2-(trimethylsilyl)ethoxy]methyl}-7,11-dioxa-20,23,24-triazapentacyclo[17.5.2.1^{2,6}.0^{13,18}.0^{22,25}]heptacosa-1(24),2(27),3,5,13,15,17,19,21,25-decaen-5-yl)piperazine-1-carboxlate

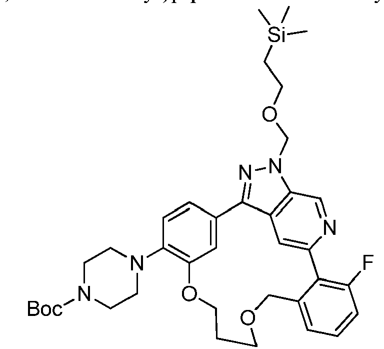

This compound was synthesized according to the procedure described in Example 9 (step 6) using tert-butyl 4-[4-(5-{2-fluoro-6-[(3-hydroxypropoxy)methyl]phenyl}-1-{[2-(trimethylsilyl)ethoxy]methyl}-1H-pyrazolo[3,4-c]pyridin-3-yl)-2-hydroxyphenyl]piperazine-1-carboxylate instead of tert-butyl 4-[4-(5-{2-fluoro-6-[(2-hydroxyethoxy)methyl]phenyl}-1-{[2-(trimethylsilyl)ethoxy]methyl}-1*H*-pyrazolo[3,4-c]pyridin-3-yl)-2-hydroxyphenyl]piperazine-1-carboxylate as the starting material. Yield: 77%.

ESI-MS: 690.3, [M+H]⁺.

### Step 7: the synthesis of 17-fluoro-5-(piperazin-1-yl)-7,11-dioxa-20,23,24-triazapentacyclo[17.5.2.1^{2,6}.0^{13,18}.0^{22,25}]heptacosa-1(24),2(27),3,5,13,15,17,19,21,25-decaene

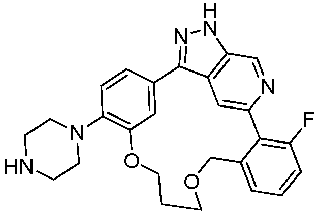

This compound was synthesized according to the procedure described in Example 9 (step 7) using tert-butyl 4-(17-fluoro-23-{[2-(trimethylsilyl)ethoxy]methyl}-7,11-dioxa-20,23,24-triazapentacyclo[17.5.2.1^{2,6}.0^{13,18}.0^{22,25}]heptacosa-1(24),2(27),3,5,13,15,17,19,21,25-decaen-5-yl)piperazine-1-carboxylate instead of tert-butyl 4-(16-fluoro-22-{[2-(trimethylsilyl)ethoxy]methyl}-7,10-dioxa-19,22,23-triazapentacyclo[16.5.2.1^{2,6}.0^{12,17}.0^{21,24}]hexacosa-1(23),2(26),3,5,12,14,16,18,20,24-decaen-5-yl)piperazine-1-carboxylate as the starting material. Yield: 100%.

ESI-MS: 460.3, [M+H]⁺.

### Example 11.

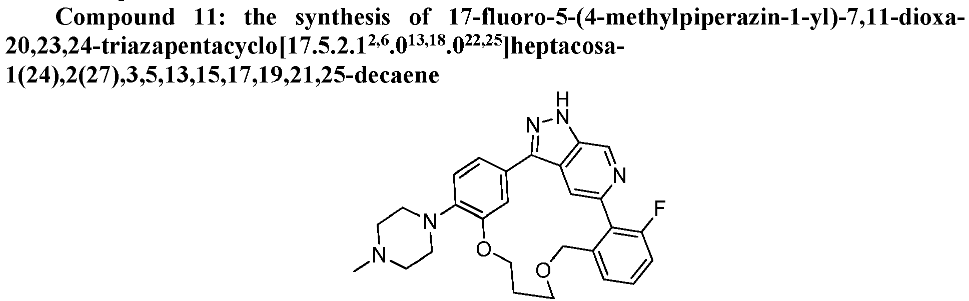

This compound was synthesized according to the procedure described in Example 9 (step 8) using 17-fluoro-5-(piperazin-1-yl)-7,11-dioxa-20,23,24-triazapentacyclo[17.5.2.1^{2,6}.0^{13,18}.0^{22,25}]heptacosa-1(24),2(27),3,5,13,15,17,19,21,25-decaene instead of 16-fluoro-5-(piperazin-1-yl)-7,10-dioxa-19,22,23-triazapentacyclo[16.5.2.1^{2,6}.0^{12,17}.0^{21,24}]hexacosa-1(23),2(26),3,5,12,14,16,18,20,24-decaene as the starting material. Yield: 54%.

ESI-MS: 474.2, [M+H]⁺.

¹H NMR (DMSO-*d*₆, 400 MHz) δ 13.76 (s, 1H), 9.14 (d, *J* = 0.8 Hz, 1H), 8.47 (s, 1H), 7.70 (d, *J=* 1.6 Hz, 1H), 7.54-7.50 (m, 1H), 7.50-7.45 (m, 1H), 7.43-7.38 (m, 1H), 7.36-7.29 (m, 1H), 7.03 (d, *J* = 8.0 Hz, 1H), 4.40 (t, *J* = 6.4 Hz, 2H), 4.33 (s, 2H), 3.76-3.66 (m, 2H), 3.05 (s, 4H), 2.54-2.44 (m, 4H), 2.24 (s, 3H), 2.12-2.02 (m, 2H).

### Example 12.

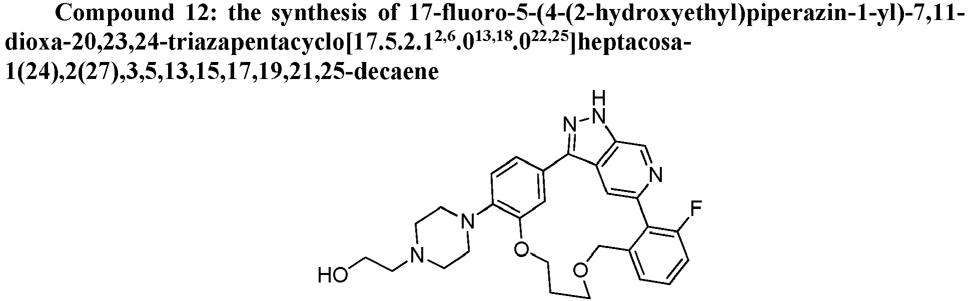

### Step 1: the synthesis of 5-(4-{2-[(tert-butyldimethylsilyl)oxy]ethyl}piperazin-1-yl)-17-fluoro-7,11-dioxa-20,23,24-triazapentacyclo[17.5.2.1^{2,6}.0^{13,18}.0^{22,25}]heptacosa-1(24),2(27),3,5,13,15,17,19,21,25-decaene

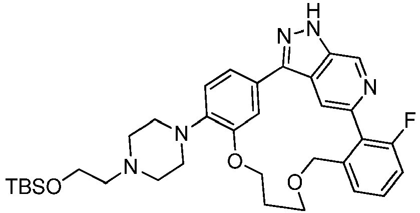

To a solution of 17-fluoro-5-(piperazin-1-yl)-7,11-dioxa-20,23,24-triazapentacyclo[17.5.2.1^{2,6}.0^{13,18}.0^{22,25}]heptacosa-1(24),2(27),3,5,13,15,17,19,21,25-decaene (20 mg, 0.04 mmol) in dichloromethane (1 mL) and dry methanol (1 mL) was added *2-[(tert*butyldimethylsilyl)oxy]acetaldehyde (16 mg, 0.09 mmol) at room temperature, and stirred for 1 hour. To the mixture was added sodium triacetoxyborohydride (28 mg, 0.13 mmol), and stirred for 12 hours. The reaction was quenched with sodium bicarbonate aqueous solution (10 mL), and was extracted with dichloromethane/isopropanol mixture (4/1, 20 mL × 2). The combined organic phase was washed with brine (20 mL), dried over anhydrous sodium sulfate and filtered. The filtrate was concentrated to dryness, and residue was purified by pre-TLC (dichloromethane/methanol = 10/1) to afford the title product (12 mg, white solid). Yield: 44%.

ESI-MS: 618.5, [M+H]⁺.

### Step 2: the synthesis of 17-fluoro-5-(4-(2-hydroxyethyl)piperazin-1-yl)-7,11-dioxa-20,23,24-triazapentacyclo[17.5.2.1^{2,6}.0^{13,18}.0^{22,25}]heptacosa-1(24),2(27),3,5,13,15,17,19,21,25-decaene

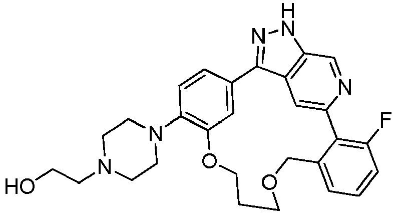

To a solution of 5-(4-{2-[(tert-butyldimethylsilyl)oxy]ethyl}piperazin-1-yl)-17-fluoro-7,11-dioxa-20,23,24-triazapentacyclo[17.5.2.1^{2,6}.0^{13,18}.0^{22,25}]heptacosa-1(24),2(27),3,5,13,15,17,19,21,25-decaene (12 mg, 0.02 mmol) in tetrahydrofuran (1 mL) was added 1M tetrabutylammonium fluoride solution in tetrahydrofuran (60 µL, 0.06 mmol), and stirred at room temperature for 14 hours. The reaction was quenched with water (10 mL), and was extracted with dichloromethane/isopropanol mixture (4/1, 10 mL × 3). The combined organic phase was washed with brine (10 mL), dried over anhydrous sodium sulfate and filtered. The filtrate was concentrated to dryness, and residue was purified by pre-TLC (dichloromethane/methanol = 7/1) to afford the title product (5 mg, white solid). Yield: 51%.

ESI-MS: 504.3, [M+H]⁺.

The following compounds in the Examples were synthesized according to procedures similar to those described in Example 9 and using appropriate intermediates.

| Example | Compound | Structure | Characterization data |
|---|---|---|---|
| Example 89 | Compound 89 | 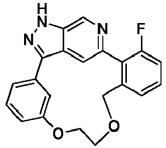 | ESI-MS: 362.1, [M+H]⁺. |
| | | | ¹H NMR (DMSO-*d*₆, 400 MHz) δ 13.86 (s, 1H), 9.19 (s, 1H), 9.00 (s, 1H), 8.04 (s, 1H), 7.68 (d, *J* = 7.6 Hz, 1H), 7.53-7.31 (m, 4H), 6.92 (dd, *J* = 8.1, 1.9 Hz, 1H), 4.49 (s, 2H), 4.43 (s ,2H), 4.08 (s, 2H). |
| Example 90 | Compound 90 | 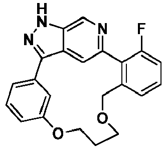 | ESI-MS: 376.2, [M+H]⁺. |
| | | | ¹H NMR (DMSO-*d*₆, 400 MHz) δ 13.86 (s, 1H), 9.17 (s, 1H), 8.53 (s, 1H), 7.80 (s, 1H), 7.58 (d, *J* = 7.6 Hz, 1H), 7.52-7.39 (m, 3H), 7.37 - 7.30 (m, 1H), 6.92 (dd, *J* = 8.0, 1.6 Hz, 1H), 4.38 (t, *J* = 6.8 Hz, 2H), 4.33 (s, 2H), 3.69 (m, 2H), 2.12 - 2.01 (m, 2H). |
| Example 91 | Compound 91 | 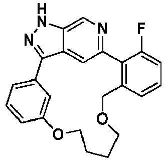 | ESI-MS: 390.2, [M+H]⁺. |
| | | | ¹H NMR (DMSO-*d*₆, 400 MHz) δ 13.86 (s, 1H), 9.17 (s, 1H), 8.53 (s, 1H), 7.80 (s, 1H), 7.58 (d, *J* = 7.6 Hz, 1H), 7.52-7.38 (m, 3H), 7.33 (t, *J* = 9.2 Hz, 1H), 6.92 (dd, *J =* 8.0, 1.6 Hz, 1H), 4.37 (t, *J =* 6.8 Hz, 2H), 4.33 (s, 2H), 3.69 (s, 2H), 2.51(s, 2H), 2.11-2.01 (m, 2H). |
| Example 92 | Compound 92 | 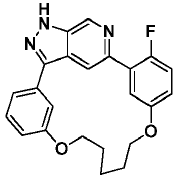 | ESI-MS: 390.1, [M+H]⁺. |
| | | | ¹H NMR (DMSO-*d*₆, 400 MHz) δ 13.85 (s, 1H), 9.18 (s, 1H), 8.12 (d, *J* = 19.7 Hz, 1H), 7.79 (s, 1H), 7.52 - 7.46 (m, 2H), 7.44 - 7.40 (m, 1H), 7.20 (dd, *J* = 10.2, 9.0 Hz, 1H), 7.01 - 6.93 (m, 2H), 4.38 - 4.24 (m, 2H), 4.19 (t, *J* = 7.3 Hz, 2H), 1.95 - 1.82 (m, 2H), 1.80 - 1.70 (m, 2H), 1.65 - 1.50 (m, 2H). |
| Example 105 | Compound 105 | 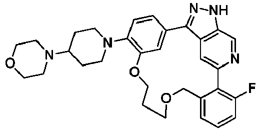 | ESI-MS: 544.3, [M+H]⁺. |
| | | | ¹H NMR (DMSO-*d*₆, 400 MHz) δ 13.72 (s, 1H), 9.14 (s,1H), 8.47 (s,1H), 7.70 (s,1H), 7.52-7.44 (m, 2H), 7.41 (d, *J* = 6.8 Hz, 1H), 7.32 (t, *J* = 9.0 Hz, 1H), 7.03 (d, *J* = 8.0 Hz, 1H), 4.39 (t, *J* = 6.6 Hz, 2H), 4.33 (s, 2H), 3.71 (s, 2H), 3.62-3.45 (m, 6H), 3.17 (d, *J* = 5.2 Hz, 2H), 2.59 (t, *J* = 11.2 Hz, 2H), 2.50-2.49 (m, 2H), 2.34-2.21 (m, 1H), 2.10-2.06 (m, 2H), 1.85 (d, *J* = 10.8 Hz, 2H), 1.63-1.48 (m, 2H). |
| Example 107 | Compound 107 | 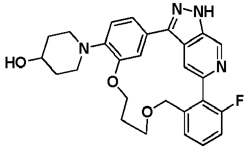 | ESI-MS: 475.2, [M+H]⁺. |
| | | | ¹H NMR (DMSO-*d*₆, 400 MHz) δ 13.71 (s, 1H), 9.13 (s, 1H), 8.47 (s, 1H), 7.69 (s, 1H), 7.52-7.44 (m, 2H), 7.41 (d, *J* = 6.8 Hz, 1H), 7.32 (t, *J* = 9.2 Hz, 1H), 7.04 (d, *J* = 8.0 Hz, 1H), 4.62 (d, *J* = 4.2 Hz, 1H), 4.39 (t, *J* = 6.6 Hz, 2H), 4.33 (s, 2H), 3.76-3.66 (m, 2H), 3.64-3.54 (m, 1H), 3.35-3.30 (m, 2H), 2.78-2.67 (m, 2H), 2.13-2.02 (m, 2H), 1.90-1.80 (m, 2H), 1.57-1.52 (m, 2H). |
| Example 109 | Compound 109 | 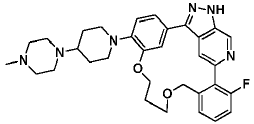 | ESI-MS: 557.3, [M+H]⁺. |
| | | | ¹H NMR (DMSO-*d*₆, 400 MHz) δ 13.75 (s, 1H), 9.13 (s, 1H), 8.47 (s, 1H), 7.69 (s, 1H), 7.53-7.44 (m, 2H), 7.41 (d, *J* = 7.1 Hz, 1H), 7.32 (t, *J* = 9.0 Hz, 1H), 7.03 (d, *J* = 8.4 Hz, 1H), 4.39 (t, *J* = 6.5 Hz, 2H), 4.33 (s, 2H), 3.71 (s, 2H), 3.51 (d, *J* = 11.1 Hz, 2H), 2.62-2.50 (m, 6 H), 2.41-2.25 (m, 5H), 2.17 (s, 3H), 2.07 (t, *J* = 4.8 Hz, 2H), 1.83 (d, *J* = 10.4 Hz, 2H), 1.64-1.49 (m, 2H). |
| Example 111 | Compound 111 | 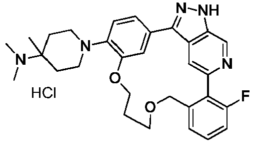 | ESI-MS: 516.2, [M+H-HCl]⁺. |
| | | | ¹H NMR (DMSO-*d*₆, 400 MHz) δ 13.78 (s, 1H), 10.22 (br, 1H), 9.14 (s, 1H), 8.47 (s, 1H), 7.72 (s, 1H), 7.55-7.45 (m, 2H), 7.42-7.39 (m, 1H), 7.36-7.30 (m, 1H), 7.08 (d, *J* = 8.2 Hz, 1H), 4.41 (t, *J* = 6.7 Hz, 1H), 4.34 (s, 2H), 3.75-3.66 (m, 2H), 3.54-3.40 (m, 2H), 2.78 (t, *J* = 11.4 Hz, 2H), 2.68 (s, 6H), 2.12-1.86 (m, 6H), 1.30 (s, 3H). |
| Example 112 | Compound 112 | 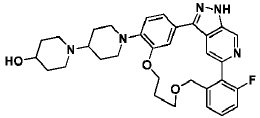 | ESI-MS: 558.3, [M+H]⁺. |
| | | | ¹H NMR (DMSO-*d*₆, 400 MHz) δ 13.72 (s, 1H), 9.13 (s, 1H), 8.47 (s, 1H), 7.69 (s, 1H), 7.54-7.38 (m, 3H), 7.32 (t, *J* = 9.0 Hz, 1H), 7.03 (d, *J* = 8.2 Hz, 1H), 4.51 (s, 1H), 4.39 (t, *J* = 6.5 Hz, 2H), 4.33 (s, 2H), 3.71 (m, 2H), 3.52 (d, *J =* 11.2 Hz, 2H), 3.45-3.38 (m, 1H), 2.81 (s, 2H), 2.58 (t, *J* = 11.0 Hz, 1H), 2.29-2.21 (m, 2H), 2.07 (m, 2H), 1.91 (s, 2H), 1.83-1.71 (m, 4H), 1.66-1.53 (m, 2H), 1.42-1.33 (m, 2H). |
| Example 115 | Compound 115 | 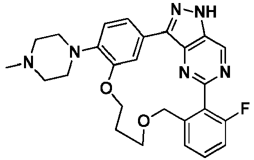 | ESI-MS: 475.3, [M+H]⁺. |
| | | | ¹H NMR (DMSO-*d*₆, 400 MHz) δ 13.98 (s, 1H), 9.46 (s, 1H), 8.67 (d, *J* = 1.6 Hz, 1H), 7.74-7.71 (m, 1H), 7.57-7.51 (m, 1H), 7.46 (d, *J* = 7.1 Hz, 1H), 7.35-7.30 (m, 1H), 7.01 (d, *J =* 8.2 Hz, 1H), 4.57 (s, 2H), 4.37 (t, *J* = 6.5 Hz, 2H), 3.71 (t, *J* = 5.3 Hz, 2H), 3.10-3.00 (m, 4H), 2.50-2.44 (m, 4H), 2.24 (s, 3H), 2.06-2.00 (m, 2H). |

### Example 17.

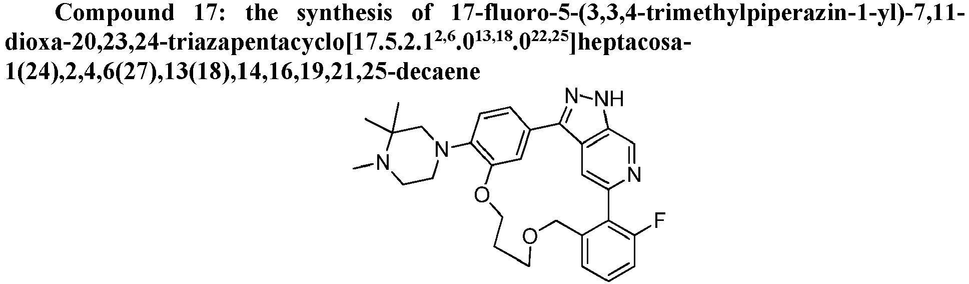

### Step 1: the synthesis of tert-butyl N-[4-(5-bromo-1-{[2-(trimethylsilyl)ethoxy]methyl}-1H-pyrazolo[3,4-c]pyridin-3-yl)-2-hydroxyphenyl]carbamate (Intermediate 17-1)

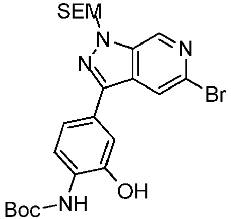

To a mixture of 5-bromo-3-iodo-1-{[2-(trimethylsilyl)ethoxy]methyl}-1*H*-pyrazolo[3,4-c]pyridine (454 mg, 1.00 mmol), *tert*-butyl 2-{[(*tert*-butoxy)carbonyl]amino}-5-(4,4,5,5-tetramethyl-1,3,2-dioxaborolan-2-yl)phenylcarbonate (435 mg, 1.00 mmol), Pd(dppf)Cl₂ (73 mg, 0.10 mmol) and potassium phosphate (637 mg, 3.0 mmol) was added 1,4-dioxane (10 mL) and water (2 mL), and stirred at 80°C for 14 hours under nitrogen. The reaction mixture was cooled to room temperature, diluted with water (60 mL), and extracted with ethyl acetate (50 mL × 2). The combined organic phase was washed with brine (50 mL), dried over anhydrous sodium sulfate, and filtered. The filtrate was concentrated to dryness, and the residue was purified by column chromatography (petroleum ether/ethyl acetate = 5/1 to 3/1) to afford the title product (227 mg, white solid). Yield: 42.4%.

ESI-MS: 635.2, [M+H]⁺.

### Step 2: the synthesis of tert-butyl N-{4-[5-(2-fluoro-6-{[3-(oxan-2-yloxy)propoxy]methyl}phenyl)-1-{[2-(trimethylsilyl)ethoxy]methyl}-1H-pyrazolo[3,4-c]pyridin-3-yl]-2-hydroxyphenyl}carbamate (Intermediate 17-2)

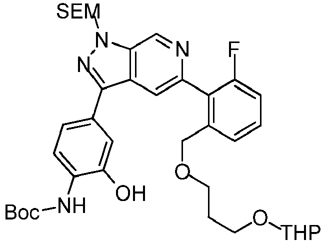

A mixture of 2-(2-fluoro-6-{[3-(oxan-2-yloxy)propoxy]methyl}phenyl)-4,4,5,5-tetramethyl-1,3,2-dioxaborolane (1.450 g, 2.62 mmol), **Intermediate 17-1** (2.062 g, 5.23 mmol), sodium carbonate (831 mg, 7.85 mmol) and Xphos-Pd-G2 (206 mg, 0.26 mmol) in 1,4-dioxane (30 mL) and water (6 mL) was heated to 80°C and stirred for 2 hours. The reaction mixture was cooled to room temperature, diluted with water (120 mL), and extracted with ethyl acetate (80 mL × 2). The combined organic phase was washed with brine (80 mL), dried over anhydrous sodium sulfate, and filtered. The filtrate was concentrated to dryness, and the residue was purified by column chromatography (petroleum ether/ethyl acetate = 10/1 to 2/1) to afford the title product (1.576 g, brown oil). Yield: 80.5%.

ESI-MS: 723.4, [M+H]⁺.

### Step 3: the synthesis of tert-butyl N-[4-(5-{2-fluoro-6-[(3-hydroxypropoxy)methyl]phenyl}-1-{[2-(trimethylsilyl)ethoxy]methyl}-1H-pyrazolo[3,4-c]pyridin-3-yl)-2-hydroxyphenyl]carbamate (Intermediate 17-3)

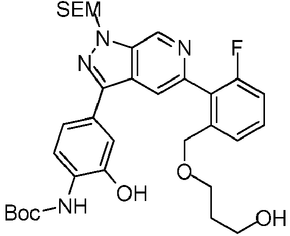

To a solution of **Intermediate 17-3** (1.576 g, 2.18 mmol) in methanol (25 mL) was added p-toluenesulfonic acid hydrate (435 mg, 2.29 mmol), heated to 40°C and stirred for 1.5 hours. The majority of methanol was removed by rotavap, and the residue was dissolved in dichloromethane (100 mL). The solution was washed with sodium bicarbonate aqueous solution (50 mL) and brine (50 mL), dried over anhydrous sodium sulfate, and filtered. The filtrate was concentrated to dryness, and the residue was purified by column chromatography (petroleum ether/ethyl acetate = 5/1 to 1/1) to afford the title product (1.055 g, white solid). Yield: 75.9%.

ESI-MS: 639.3, [M+H]⁺.

### Step 4: the synthesis of tert-butyl N-(17-fluoro-23-{[2-(trimethylsilyl)ethoxy]methyl}-7,11-dioxa-20,23,24-triazapentacyclo[17.5.2.1^{2,6}.0^{13,18}.0^{22,25}]heptacosa-1(24),2(27),3,5,13,15,17,19,21,25-decaen-5-yl)carbamate Intermediate 17-4)

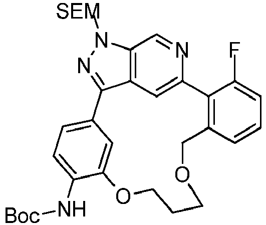

To a solution of TMAD (1.419 g, 8.26 mmol) in dry tetrahydrofuran (25 mL) was added tributylphosphine (1.668 g, 8.26 mmol) at room temperature under nitrogen. After stirring for 5 min, a solution of **Intermediate 17-3** (1.055 mg, 1.65 mmol) in tetrahydrofuran (25 mL) was added dropwise, and further stirred for 0.5 hour. The reaction was quenched with water (300 mL), and extracted with ethyl acetate (10 mL × 2). The combined organic phase was washed with brine (100 mL), dried over anhydrous sodium sulfate, and filtered. The filtrate was concentrated to dryness, and the residue was purified by column chromatography (petroleum ether/ethyl acetate = 10/1 to 1/1) to afford the title product (880 mg, white solid). Yield: 85.8%.

ESI-MS: 621.3, [M+H]⁺.

### Step 5: the synthesis of 17-fluoro-23-{[2-(trimethylsilyl)ethoxy]methyl}-7,11-dioxa-20,23,24-triazapentacyclo[17.5.2.1^{2,6}.0^{13,18}.0^{22,25}]heptacosa-1(24),2(27),3,5,13,15,17,19,21,25-decaen-5-amine (Intermediate 17-5)

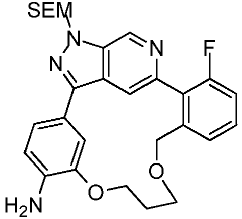

To a solution of **Intermediate 17-4** (823 mg, 1.33 mmol) in anhydrous methanol (15 mL) was added *p*-toluenesulfonic acid hydrate (630 mg, 3.31 mmol) at room temperature, heated to 60°C and stirred for 6 hours. The majority of methanol was removed by rotavap, and the residue was dissolved in dichloromethane (80 mL). The solution was washed with sodium bicarbonate aqueous solution (50 mL) and brine (50 mL), dried over anhydrous sodium sulfate, and filtered. The filtrate was concentrated to dryness, and the residue was purified by column chromatography (petroleum ether/ethyl acetate = 5/1 to 2/1) to afford the title product (640 mg, white solid). Yield: 93.6%.

ESI-MS: 521.3, [M+H]⁺.

### Step 6: the synthesis of 5-bromo-17-fluoro-23-{[2-(trimethylsilyl)ethoxy]methyl}-7,11-dioxa-20,23,24-triazapentacyclo[17.5.2.1^{2,6}.0^{13,18}.0^{22,25}]heptacosa-1(24),2(27),3,5,13,15,17,19,21,25-decaene (Intermediate 17-6)

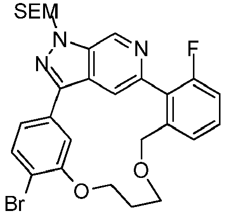

To a solution of **Intermediate 17-5** (568 mg, 1.09 mmol) in acetonitrile (15 mL) was added p-toluenesulfonic acid hydrate (415 mg, 2.18 mmol) and tert-butyl nitrite (146 mg, 1.42 mmol) under nitrogen, and stirred at room temperature for 20 min. To the mixture was then added copper(II) bromide (61 mg, 0.27 mmol) and tetrabutylammonium bromide (1.406 g, 4.36 mmol). The mixture was heated to 70°C and stirred for 2 hours under nitrogen. The mixture was then cooled to room temperature, quenched with water (100 mL), and extracted with ethyl acetate (60 mL × 2). The combined organic phase was washed with brine (60 mL), dried over anhydrous sodium sulfate, and filtered. The filtrate was concentrated to dryness, and the residue was purified by column chromatography (petroleum ether/ethyl acetate = 7/1 to 2/1) to afford the title product (425 mg, white solid). Yield: 66%.

ESI-MS: 584.3, [M+H]⁺.

### Step 7: the synthesis of tert-butyl 4-(17-fluoro-23-{[2-(trimethylsilyl)ethoxy]methyl}-7,11-dioxa-20,23,24-triazapentacyclo[17.5.2.1^{2,6}.0^{13,18}.0^{22,25}]heptacosa-1(24),2(27),3,5,13,15,17,19,21,25-decaen-5-yl)-2,2-dimethylpiperazine-1-carboxylate (Intermediate 17-7)

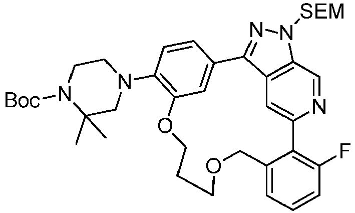

To a mixture of **Intermediate 17-6** (50 mg, 0.09 mmol), 1-tert-butoxycarbonyl-2,2-dimethylpiperazine (55 mg, 0.26 mmol), palladium acetate (2 mg, 0.009 mmol), BINAP (11 mg, 0.017 mmol) and cesium carbonate (84 mg, 0.26 mmol) was added toluene (2 mL), and heated to 100°C for 14 hours under nitrogen. The mixture was then cooled to room temperature, diluted with water (20 mL), and extracted with ethyl acetate (20 mL × 2). The combined organic phase was washed with water (20 mL) and brine (20 mL), dried over anhydrous sodium sulfate, and filtered. The filtrate was concentrated to dryness, and the residue was purified by column chromatography (dichloromethane/methanol = 10/1) to afford the title product (33 mg, colorless oil). Yield: 54%.

ESI-MS: 717.4, [M+H]⁺.

### Step 8: the synthesis of 5-(3,3-dimethylpiperazin-1-yl)-17-fluoro-7,11-dioxa-20,23,24-triazapentacyclo[17.5.2.1^{2,6}.0^{13,18}.0^{22,25}]heptacosa-1(24),2(27),3,5,13,15,17,19,21,25-decaene (Intermediate 17-8)

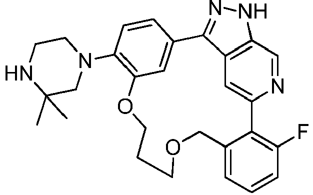

To a solution of **Intermediate 17-7** (33 mg, 0.05 mmol) in dichloromethane (3 mL) was added trifluoroacetic acid (0.5 mL), and stirred at room temperature for 14 hours. The reaction mixture was concentrated to dryness, and the residue was dissolved in acetonitrile (2 mL), and treated with ammonia (0.1 mL) for 30 min. The mixture was concentrated to dryness. The residue was dissolved in water (30 mL), and then extracted with dichloromethane/isopropanol mixture (4/1, 30 mL × 3). The combined organic phase was washed with brine (20 mL), dried over anhydrous sodium sulfate and filtered. The filtrate was concentrated to dryness to afford the title product (27 mg, yellow solid). Yield: 100%.

ESI-MS: 488.3, [M+H]⁺.

### Step 9: the synthesis of Compound 17

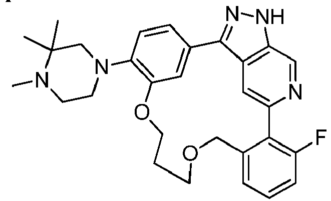

To a solution of **Intermediate 17-8** (27 mg, 0.05 mmol) in dichloromethane/methanol (1/1, 3 mL) was added 1*H*-benzotriazole-1-methanol (12 mg, 0.08 mmol), sodium acetate (14 mg, 0.17 mmol) and sodium triacetoxyborohydride (35 mg, 0.17 mmol), and stirred at room temperature for 5 hours. The reaction was quenched with sodium bicarbonate aqueous solution (20 mL), and was extracted with dichloromethane/isopropanol mixture (4/1, 20 mL × 2). The combined organic phase was washed with brine (30 mL), dried over anhydrous sodium sulfate and filtered. The filtrate was concentrated to dryness, and residue was purified by pre-TLC (dichloromethane/methanol = 7/1) to afford the title product (14 mg, white solid). Yield: 50%.

ESI-MS: 502.3, [M+H]⁺.

¹H NMR (DMSO-*d*₆, 400 MHz) δ 13.81 (s, 1H), 9.15 (s, 1H), 8.48 (s, 1H), 7.72 (s, 1H), 7.54 (m, 1H), 7.45-7.51 (m, 1H), 7.41 (m, 1H), 7.30-7.35 (m, 1H), 7.04 (d, *J* = 7.6 Hz, 1H), 4.39 (t, *J* = 6.4 Hz, 2H), 4.34 (s, 2H), 3.72 (d, *J* = 4.8 Hz, 2H), 3.15 (m, 4H), 2.86 (m, 2H), 2.6 (m, 3H), 2.07 (dd, *J* = 7.0, 3.1 Hz, 2H), 1.24 (m, 6H).

The following compounds in the Examples were synthesized according to procedures similar to those described in Example 17 and using appropriate intermediates.

| Example | Compound | Structure | Characterization data |
|---|---|---|---|
| Example 18 | Compound 18 | 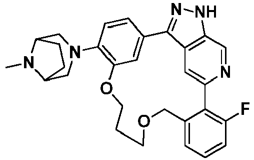 | ESI-MS: 474.2, [M+H]⁺. |
| | | | ¹H NMR (DMSO-*d*₆, 400 MHz) δ 13.79 (s, 1H), 9.13 (d, *J* = 0.8 Hz, 1H), 8.45 (s, 1H), 7.67 (d, *J* = 1.5 Hz, 1H), 7.51-7.44 (m, 2H), 7.42-7.39 (m, 1H), 7.35-7.29 (m, 1H), 6.93 (d, *J* = 8.3 Hz, 1H), 4.39-4.34 (m, 4H), 3.73 (t, *J* = 5.1 Hz, 2H), 3.25-3.20 (m, 2H), 3.11 (br, 2H), 2.83 (d, *J* = 10.1 Hz, 2H), 2.20 (s, 3H), 2.10-2.02 (m, 2H), 1.94-1.88 (m, 2H), 1.85-1.79 (m, 2H). |
| Example 100 | Compound 100 | 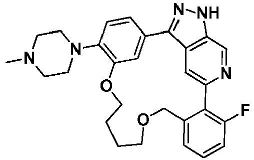 | ESI-MS: 488.3, [M+H]⁺. |
| | | | ¹H NMR (DMSO-*d*₆, 400 MHz) δ 13.83 (s, 1H), 9.15 (s, 1H), 8.15 (s, 1H), 7.50-7.43 (m, 3H), 7.39 (d, *J* = 6.8 Hz, 1H), 7.29 (t, *J* = 9.0 Hz, 1H), 7.05 (d, *J* = 8.0 Hz, 1H), 4.29 (s, 2H), 4.26-4.21 (m, 2H), 3.62 (t, *J* = 5.2 Hz, 2H), 3.07 (s, 4H), 2.62-2.51 (m,4H), 2.28 (s, 3H), 1.97-1.78 (m, 2H), 1.78-1.64 (m, 2H). |
| Example 101 | Compound 101 | 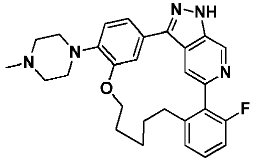 | ESI-MS: 472.2, [M+H]⁺. |
| | | | ¹H NMR (DMSO-*d*₆, 400 MHz) δ 13.75 (s, 1H), 9.15 (s, 1H), 8.12 (s, 1H), 7.56 (dd, *J* = 8.3, 1.7 Hz, 1H), 7.51 (d, *J* = 1.8 Hz, 1H), 7.46 - 7.33 (m, 1H), 7.27 (d, *J* = 7.4 Hz, 1H), 7.16 - 7.07 (m, 1H), 7.03 (d, *J* = 8.3 Hz, 1H), 4.33 (t, *J* = 6.2 Hz, 2H), 3.15 - 3.10 (m, 4H), 2.72 - 2.62 (m, 2H), 2.48 - 2.43 (m, 4H), 2.23 (s, 3H), 1.98 - 1.78 (m, 4H), 1.72 - 1.60 (m, 2H). |
| Example 103 | Compound 103 | 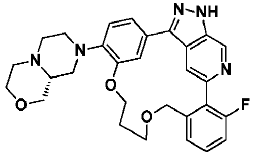 | ESI-MS: 516.2, [M+H]⁺. |
| | | | ¹H NMR (DMSO-*d*₆, 400 MHz) δ 13.72 (s, 1H), 9.13 (s, 1H), 8.47 (s, 1H), 7.71 (d, *J* = 1.5 Hz, 1H), 7.55-7.45 (m, 2H), 7.43-7.39 (m, 1H), 7.36-7.30 (m, 1H), 7.01 (d, *J* = 8.3 Hz, 1H), 4.40 (t, *J* = 13.2 Hz, 2H), 4.33 (s, 2H), 3.80-3.64 (m, 4H), 3.58-3.42 (m, 2H), 3.28-3.20 (m, 1H), 3.16 (t, *J* = 10.2 Hz, 1H), 2.82-2.71 (m, 2H), 2.67-2.64 (m, 1H), 2.41-2.22 (m, 4H), 2.12-2.02 (m, 2H). |
| Example 116 | Compound compound 116 | 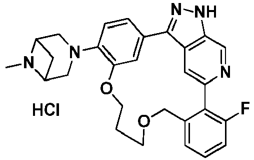 | ESI-MS: [M+H-HCl]⁺. |
| | | | 486.2, ¹H NMR (DMSO-*d*₆, 400 MHz) δ 13.79 (s, 1H), 9.93 (br, 1H), 9.16 (d, *J* = 0.8 Hz, 1H), 8.47 (s, 1H), 7.74 (d, *J* = 1.5 Hz, 1H), 7.59 (dd, *J* = 8.4, 1.8 Hz, 1H), 7.48 (dd, *J* = 8.0, 5.3 Hz, 1H), 7.44-7.41 (m, 1H), 7.36-7.30 (m, 1H), 7.17-7.10 (m, 1H), 4.41-4.24 (m, 6H), 4.16-3.76 (m, 6H), 3.40-3.20 (m, 3H), 2.90-2.82 (m, 1H), 2.20-2.02 (m, 3H). |
| Example 121 | Compound 121 | 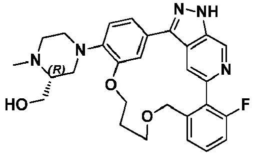 | ESI-MS: 504.2, [M+H]⁺. |
| | | | ¹H NMR (DMSO-*d*₆, 400 MHz) δ 13.76 (s, 1H), 9.14 (d, *J* = 0.8 Hz, 1H), 8.48 (s, 1H), 7.71 (d, *J* = 1.5 Hz, 1H), 7.55-7.45 (m, 2H), 7.43-7.39 (m, 1H), 7.36-7.30 (m, 1H), 7.02 (d, *J* = 8.3 Hz, 1H), 4.54 (br, 1H), 4.39 (t, *J* = 13.2 Hz, 2H), 4.34 (s, 2H), 3.76-3.62 (m, 3H), 3.53-3.46 (m, 1H), 3.40-3.30 (m, 4H), 2.84-2.70 (m, 2H), 2.56-2.50 (m, 1H), 2.42-2.19 (m, 5H), 2.12-2.02 (m, 2H). |
| Example 122 | Compound 122 | 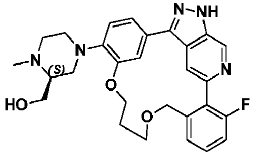 | ESI-MS: 504.2, [M+H]⁺. |
| | | | ¹H NMR (DMSO-*d*₆, 400 MHz) δ 13.76 (s, 1H), 9.14 (d, *J* = 0.8 Hz, 1H), 8.48 (s, 1H), 7.71 (d, *J* = 1.5 Hz, 1H), 7.55-7.45 (m, 2H), 7.43-7.39 (m, 1H), 7.36-7.30 (m, 1H), 7.02 (d, *J* = 8.3 Hz, 1H), 4.54 (br, 1H), 4.39 (t, *J* = 13.2 Hz, 2H), 4.34 (s, 2H), 3.76-3.62 (m, 3H), 3.53-3.46 (m, 1H), 3.40-3.30 (m, 4H), 2.82-2.70 (m, 2H), 2.56-2.50 (m, 1H), 2.42-2.20 (m, 5H), 2.12-2.02 (m, 2H). |
| Example 123 | Compound 123 | 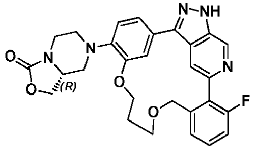 | ESI-MS: 516.2, [M+H]⁺. |
| | | | ¹H NMR (DMSO-*d*₆, 400 MHz) δ 13.78 (s, 1H), 9.15 (d, *J* = 0.8 Hz, 1H), 8.48 (s, 1H), 7.74 (d, *J* = 1.5 Hz, 1H), 7.55-7.45 (m, 2H), 7.43-7.39 (m, 1H), 7.36-7.30 (m, 1H), 7.07 (d, *J* = 8.3 Hz, 1H), 4.44-4.38 (m, 3H), 4.33 (s, 2H), 4.06-3.96 (m, 2H), 3.76-3.64 (m, 3H), 3.57-3.50 (m, 1H), 3.44-3.38 (m, 1H), 3.24-3.15 (m, 1H), 2.64-2.52 (m, 2H), 2.13-2.04 (m, 2H). |
| Example 124 | Compound 124 | 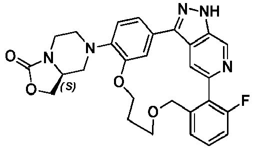 | ESI-MS: 516.2, [M+H]⁺. |
| | | | ¹H NMR (DMSO-*d*₆, 400 MHz) δ 13.78 (s, 1H), 9.15 (d, *J* = 0.8 Hz, 1H), 8.48 (s, 1H), 7.74 (d, *J* = 1.5 Hz, 1H), 7.55-7.45 (m, 2H), 7.43-7.39 (m, 1H), 7.36-7.30 (m, 1H), 7.07 (d, *J* = 8.3 Hz, 1H), 4.44-4.38 (m, 3H), 4.33 (s, 2H), 4.06-3.96 (m, 2H), 3.76-3.64 (m, 3H), 3.57-3.50 (m, 1H), 3.44-3.38 (m, 1H), 3.24-3.15 (m, 1H), 2.64-2.52 (m, 2H), 2.13-2.04 (m, 2H). |
| Example 125 | Compound 125 | 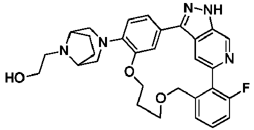 | ESI-MS: 530.3, [M+H]⁺. |
| | | | ¹H NMR (DMSO-*d*₆, 400 MHz) δ 13.79 (s, 1H), 9.13 (d, *J* = 0.8 Hz, 1H), 8.45 (s, 1H), 7.67 (d, *J* = 1.5 Hz, 1H), 7.51-7.44 (m, 2H), 7.42-7.39 (m, 1H), 7.35-7.29 (m, 1H), 6.93 (d, J = 8.3 Hz, 1H), 4.39-4.34 (m, 5H), 3.73 (t, *J* = 5.1 Hz, 2H), 3.60-3.48 (m, 2H), 3.30-3.24 (m, 2H), 2.89 (br, 2H), 2.38-2.30 (m, 2H), 2.10-2.02 (m, 2H), 1.88 (br, 4H). |
| Example 127 | Compound 127 | 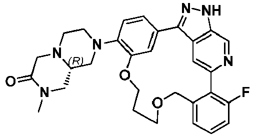 | ESI-MS: 543.2, [M+H]⁺. |
| | | | ¹H NMR (DMSO-*d*₆, 400 MHz) δ 13.76 (s, 1H), 9.15 (d, *J* = 0.8 Hz, 1H), 8.48 (s, 1H), 7.74 (d, *J* = 1.5 Hz, 1H), 7.55-7.45 (m, 2H), 7.43-7.39 (m, 1H), 7.36-7.30 (m, 1H), 7.05 (d, *J* = 8.3 Hz, 1H), 4.41 (t, *J* = 13.2 Hz, 2H), 4.33 (s, 2H), 3.75-3.68 (m, 2H), 3.52-3.40 (m, 2H), 3.36-3.28 (m, 2H), 3.24 (dd, *J* = 11.6, 4.1 Hz, 1H), 3.13 (t, *J* = 11.1 Hz, 1H), 2.92-2.86 (m, 1H), 2.85-2.72 (m, 5H), 2.70-2.60 (m, 1H), 2.50-2.44 (m, 1H), 2.36-2.26 (m, 1H), 2.12-2.02 (m, 2H). |
| Example 128 | Compound 128 | 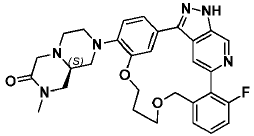 | ESI-MS: 543.2, [M+H]⁺. |
| | | | ¹H NMR (DMSO-*d*₆, 400 MHz) δ 13.76 (s, 1H), 9.15 (d, *J* = 0.8 Hz, 1H), 8.48 (s, 1H), 7.74 (d, *J* = 1.5 Hz, 1H), 7.55-7.45 (m, 2H), 7.43-7.39 (m, 1H), 7.36-7.30 (m, 1H), 7.05 (d, *J* = 8.3 Hz, 1H), 4.41 (t, *J* = 13.2 Hz, 2H), 4.33 (s, 2H), 3.75-3.68 (m, 2H), 3.52-3.40 (m, 2H), 3.36-3.28 (m, 2H), 3.24 (dd, *J* = 11.6, 4.1 Hz, 1H), 3.13 (t, *J* = 11.1 Hz, 1H), 2.92-2.86 (m, 1H), 2.85-2.72 (m, 5H), 2.70-2.60 (m, 1H), 2.50-2.44 (m, 1H), 2.36-2.26 (m, 1H), 2.12-2.02 (m, 2H). |
| Example 151 | Compound 151 | 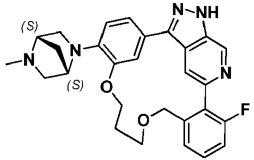 | ESI-MS: 486.2, [M+H]⁺. |
| | | | ¹H NMR (DMSO-*d*₆, 400 MHz) δ 13.67 (s, 1H), 9.13 (d, *J* = 0.8 Hz, 1H), 8.44 (s, 1H), 7.67-7.62 (m, 1H), 7.51-7.44 (m, 2H), 7.44-7.40 (m, 1H), 7.35-7.29 (m, 1H), 6.77 (d, J *J* = 8.3 Hz, 1H), 4.48-4.40 (m, 1H), 4.37 (s, 2H), 4.32-4.25 (m, 1H), 3.77 (t, *J* = 5.1 Hz, 2H), 3.54-3.51 (m, 1H), 3.10-2.70 (m, 3H), 2.38 (br, 3H), 2.09-2.00 (m, 2H), 1.98-1.76 (m, 2H). |
| Example 152 | Compound 152 | 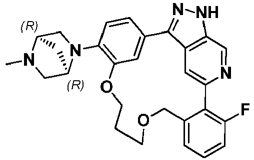 | ESI-MS: 486.2, [M+H]⁺. |
| | | | ¹H NMR (DMSO-*d*₆, 400 MHz) δ 13.64 (s, 1H), 9.12 (d, *J* = 0.8 Hz, 1H), 8.44 (s,1 H), 7.67-7.62 (m, 1H), 7.50-7.40 (m, 3H), 7.34-7.29 (m, 1H), 6.73 (d, *J* = 8.3 Hz, 1H), 4.42-4.34 (m, 3H), 4.32-4.25 (m, 1H), 3.77 (t, *J* = 5.1 Hz, 2H), 3.56-3.50 (m, 1H), 3.40-3.30 (m, 1H), 3.26-3.20 (m, 1H), 2.80-2.74 (m, 1H), 2.68 (d, *J* = 9.3 Hz, 1H), 2.26 (s, 3H), 2.08-1.98 (m, 2H), 1.82 (d, *J* = 9.1 Hz, 1H), 1.71 (d, *J* = 9.1 Hz, 1H). |
| Example 153 | Compound 153 | 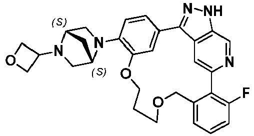 | ESI-MS: 528.2, [M+H]⁺. |
| | | | ¹H NMR (DMSO-*d*₆, 400 MHz) δ 13.62 (s, 1H), 9.12 (s, 1H), 8.44 (s, 1H), 7.67-7.62 (m, 1H), 7.51-7.45 (m, 2H), 7.44-7.41 (m, 1H), 7.34-7.29 (m, 1H), 6.74 (d, *J* = 8.3 Hz, 1H), 4.55-4.50 (m, 2H), 4.43 (br, 1H), 4.37 (s, 2H), 4.33 (t, *J* = 5.4 Hz, 1H), 4.30-4.24 (m 3H), 3.39-3.32 (m, 1H), 3.79-3.76 (m, 2H), 3.58-3.55 (m, 1H), 3.41 (s, 1H), 3.08-3.01 (m, 1H), 2.88 (d, *J* = 10.0 Hz, 1H), 2.78-2.75 (m, 1H), 2.08-1.98 (m, 2H), 1.80-1.72 (m, 2H). |
| Example 154 | Compound 154 | 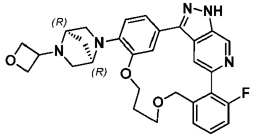 | ESI-MS: 528.2, [M+H]⁺. |
| | | | ¹H NMR (DMSO-*d*₆, 400 MHz) δ 13.62 (s, 1H), 9.12 (s, 1H), 8.44 (s, 1H), 7.67-7.62 (m, 1H), 7.51-7.45 (m, 2H), 7.44-7.41 (m, 1H), 7.34-7.29 (m, 1H), 6.74 (d, *J* = 8.3 Hz, 1H), 4.55-4.50 (m, 2H), 4.43 (br, 1H), 4.37 (s, 2H), 4.33 (t, *J* = 5.4 Hz, 1H), 4.30-4.24 (m 3H), 3.39-3.32 (m, 1H), 3.79-3.76 (m, 2H), 3.58-3.55 (m, 1H), 3.41 (s, 1H), 3.08-3.01 (m, 1H), 2.88 (d, J = 10.0 Hz, 1H), 2.78-2.75 (m, 1H), 2.08-1.98 (m, 2H), 1.80-1.72 (m, 2H). |

### Example 94.

### Compound 94: the synthesis of 17-fluoro-5-(4-methylpiperazin-1-yl)-12-oxa-8,20,23,24-tetraazapentacyclo[17.5.2.1^{2,6}.0^{13,18}.0^{22,25}]heptacosa-1(24),2(27),3,5,13(18),14,16,19,21,25-decaene

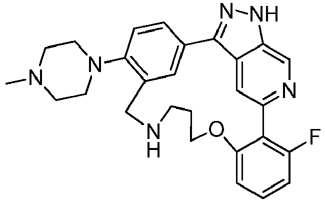

### Step 1: the synthesis of benzyl 4-[4-(5-bromo-1-{[2-(trimethylsilyl)ethoxy]methyl}-1H-pyrazolo[3,4-c]pyridin-3-yl)-2-({[(tert-butoxy)carbonyl](3-hydroxypropyl)amino}methyl)phenyl]piperazine-1-carboxylate (Intermediate 94-1)

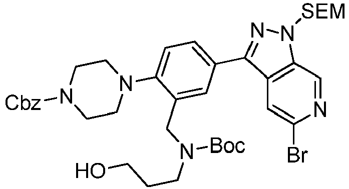

To a solution of benzyl 4-[2-({[(*tert*-butoxy)carbonyl](3-hydroxypropyl)amino}methyl)-4-(4,4,5,5-tetramethyl-1,3,2-dioxaborolan-2-yl)phenyl]piperazine-1-carboxylate (1.32 g, 2.17 mmol) in 1,4-dioxane/water (20/2 mL) was added 5-bromo-3-iodo-1-{[2-(trimethylsilyl)ethoxy]methyl}-1*H*-pyrazolo[3,4-c]pyridine (984 mg, 2.17 mmol), Pd(dppf)Cl2 (161 mg, 0.22 mmol) and potassium phosphate (1.38 g, 6.51 mmol), and then stirred at 80°C for 18 hours under nitrogen. The reaction mixture was cooled to room temperature, diluted with water (20 mL), and extracted with ethyl acetate (50 mL × 3). The combined organic phase was washed with brine (100 mL), dried over anhydrous sodium sulfate, and filtered. The filtrate was concentrated to dryness, and the residue was purified by column chromatography (petroleum ether/ethyl acetate = 1/1) to afford the title product (1.45 g, yellow oil). Yield: 82.6%.

ESI-MS: 809.3, [M+H]⁺.

### Step 2: the synthesis of benzyl 4-[2-({[(tert-butoxy)carbonyl](3-hydroxypropyl)amino}methyl)-4-[5-(2-fluoro-6-hydroxyphenyl)-1-{[2-(trimethylsilyl)ethoxy]methyl}-1H-pyrazolo[3,4-c]pyridin-3-yl]phenyl]piperazine-1-carboxylate (Intermediate 94-2)

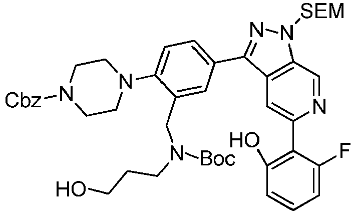

To a solution of **Intermediate 94-1** (1.45 g, 1.79 mmol) in 1,4-dioxane/water (20/2 mL) was added (2-fluoro-6-hydroxyphenyl)boronic acid (335 mg, 2.15 mmol), Xphos-Pd-G2 (704 mg, 0.89 mmol) and potassium phosphate (1.14 g, 5.37 mmol), and then stirred at 100°C for 3 hours under nitrogen. The mixture was then cooled to room temperature, diluted with water (30 mL), and extracted with ethyl acetate (100 mL × 2). The combined organic phase was washed with brine (100 mL), dried over anhydrous sodium sulfate, and filtered. The filtrate was concentrated to dryness, and the residue was purified by column chromatography (petroleum ether/ethyl acetate = 1/1) to afford the title product (1.42 g, yellow oil). Yield: 94.3%.

ESI-MS: 841.1, [M+H]⁺.

### Step 3: the synthesis of benzyl 4-(2-{[(3-bromopropyl)[(tert-butoxy)carbonyl]amino]methyl}-4-[5-(2-fluoro-6-hydroxyphenyl)-1-{[2-(trimethylsilyl)ethoxy]methyl}-1H-pyrazolo[3,4-c]pyridin-3-yl]phenyl)piperazine-1-carboxylate (Intermediate 94-3)

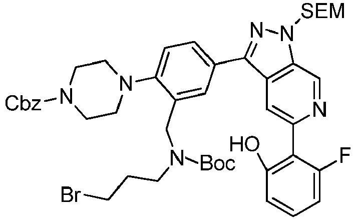

To a solution of **Intermediate 94-2** (1.20 g, 1.43 mmol) in dichloromethane (20 mL) was added triphenylphosphine (487 mg, 1.86 mmol) and *N*-bromosuccinimide (330 mg, 1.86 mmol), and stirred at room temperature for 18 hours under nitrogen. The reaction was quenched with water, and extracted with dichloromethane (50 mL × 2). The combined organic phase was washed with brine (100 mL), dried over anhydrous sodium sulfate, and filtered. The filtrate was concentrated to dryness, and the residue was purified by column chromatography (petroleum ether/ethyl acetate = 3/1) to afford the title product (1.13 g, yellow oil). Yield: 87.5%.

ESI-MS: 903.3, [M+H]⁺.

### Step 4: the synthesis of tert-butyl 5-{4-[(benzyloxy)carbonyl]piperazin-1-yl}-17-fluoro-23-{[2-(trimethylsilyl)ethoxy]methyl}-12-oxa-8,20,23,24-tetraazapentacyclo[17.5.2.1^{2,6}.0^{13,18}.0^{22,25}]heptacosa-1(24),2(27),3,5,13(18),14,16,19,21,25-decaene-8-carboxylate (Intermediate 94-4)

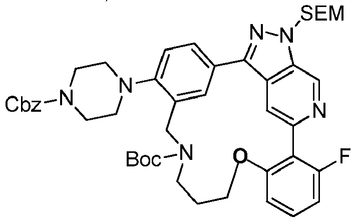

To a solution of **Intermediate 94-3** (1.10 g, 1.22 mmol) in DMF (80 mL) was added cesium carbonate (1.19 g, 3.66 mmol) and sodium iodide (183 mg, 1.22 mmol), and stirred at 100°C for 3 hours under nitrogen. The reaction was quenched with water, and extracted with ethyl acetate (100 mL × 3). The combined organic phase was washed with brine (100 mL), dried over anhydrous sodium sulfate, and filtered. The filtrate was concentrated to dryness, and the residue was purified by column chromatography (petroleum ether/ethyl acetate = 1/1) to afford the title product (796 mg, yellow oil). Yield: 79.6%.

ESI-MS: 823.4, [M+H]⁺.

### Step 5: the synthesis of tert-butyl 17-fluoro-5-(piperazin-1-yl)-23-{[2-(trimethylsilyl)ethoxy]methyl}-12-oxa-8,20,23,24-tetraazapentacyclo[17.5.2.1^{2,6}.0^{13,18}.0^{22,25}]heptacosa-1(24),2(27),3,5,13(18),14,16,19,21,25-decaene-8-carboxylate (Intermediate 94-5)

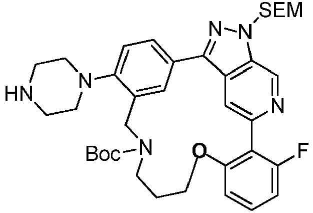

To a solution of **Intermediate 94-4** (796 mg, 1.22 mmol) in methanol (20 mL) was added Pd/C (80 mg), and stirred at room temperature under atmosphere of hydrogen for 18 hours. The mixture was filtered and concentrated to afford the crude product of title compound (712 mg, yellow oil).

ESI-MS: 689.4, [M+H]⁺.

### Step 6: the synthesis of tert-butyl 17-fluoro-5-(4-methylpiperazin-1-yl)-23-{[2-(trimethylsilyl)ethoxy]methyl}-12-oxa-8,20,23,24-tetraazapentacyclo[17.5.2.1^{2,6}.0^{13,18}.0^{22,25}]heptacosa-1(24),2(27),3,5,13(18),14,16,19,21,25-decaene-8-carboxylate (Intermediate 94-6)

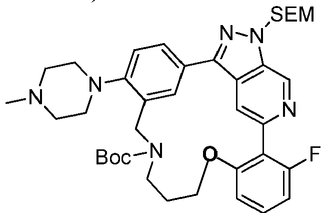

To a solution of **Intermediate 94-5** (712 mg, 1.03 mmol) in dichloromethane (20 mL) was added 1*H*-benzotriazole-1-methanol (230 mg, 1.55 mmol) and sodium triacetoxyborohydride (437 mg, 2.06 mmol) at 0°C, and stirred at room temperature for 3 hours. The reaction was quenched with water (50 mL), and was extracted with dichloromethane (50 mL × 3). The combined organic phase was washed with brine (100 mL), dried over anhydrous sodium sulfate and filtered. The filtrate was concentrated to dryness, and residue was purified by pre-TLC (dichloromethane/methanol = 10/1) to afford the title product (715 mg, yellow oil). Yield: 98.7%.

ESI-MS: 703.4, [M+H]⁺.

### Step 7: the synthesis of Compound 94

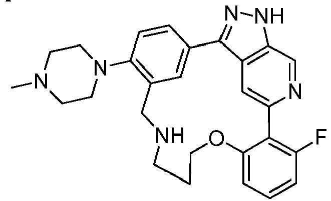

To a solution of **Intermediate 94-6** 49 mg, 0.07 mmol) in dichloromethane 5 mL) was added trifluoroacetic acid (1 mL), and stirred at room temperature for 18 hours. The reaction mixture was concentrated to dryness, and the residue was dissolved in acetonitrile (5 mL), and treated with ammonia (1 mL) for 30 min. The mixture was extracted with dichloromethane/isopropanol mixture (5/1, 10 mL × 3). The combined organic phase was washed with brine (20 mL), dried over anhydrous sodium sulfate and filtered. The filtrate was concentrated to dryness, and residue was purified by pre-TLC (dichloromethane/methanol = 5/1) to afford the title product (15.47 mg, white solid). Yield: 46.9%.

ESI-MS: 473.3, [M+H]⁺.

¹H NMR (DMSO-*d*₆, 400 MHz) δ 13.70 (s, 1H), 9.12 (d, *J* = 1.2 Hz, 1H), 8.16 (d, *J* = 1.3 Hz, 1H), 7.85 (d, *J* = 2.1 Hz, 1H), 7.62 (dd, *J* = 8.1, 2.0 Hz, 1H), 7.38 - 7.32 (m, 1H), 7.23 (d, *J* = 8.2 Hz, 1H), 6.97 (d, *J* = 8.5 Hz, 1H), 6.86 (dd, *J* = 10.2, 8.3 Hz, 1H), 4.15 (t, *J* = 4.9 Hz, 2H), 3.93 (s, 2H), 3.01 (t, *J* = 7.6 Hz, 2H), 2.90 (t, *J* = 4.7 Hz, 4H), 2.56 - 2.51 (m, 4H), 2.25 (s, 3H), 2.00 (s, 2H).

### Example 95.

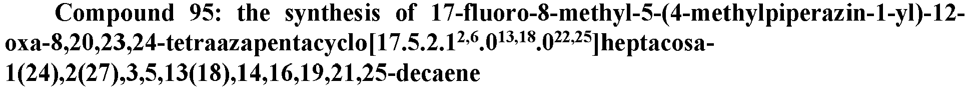

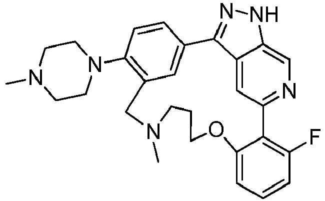

### Step 1: the synthesis of Compound 95

To a solution of **Compound 94** (51 mg, 0.11 mmol) in dichloromethane (8 mL) was added 1*H*-benzotriazole-1-methanol (33 mg, 0.22 mmol) and sodium triacetoxyborohydride (70 mg, 0.33 mmol) at 0°C, and stirred at room temperature for 3 hours. The reaction was quenched with saturated sodium bicarbonate aqueous solution (10 mL), and was extracted with dichloromethane (20 mL × 3). The combined organic phase was washed with brine (30 mL), dried over anhydrous sodium sulfate and filtered. The filtrate was concentrated to dryness, and residue was purified by pre-TLC (dichloromethane/methanol = 5/1) to afford the title product (19 mg, white solid). Yield: 36.5%.

ESI-MS: 487.3, [M+H]⁺.

¹H NMR (DMSO-*d*₆, 400 MHz) δ 13.66 (s, 1H), 9.12 (d, *J* = 1.1 Hz, 1H), 8.17 (d, *J* = 1.2 Hz, 1H), 7.72 (d, *J* = 2.2 Hz, 1H), 7.61 (dd, *J* = 8.2, 2.0 Hz, 1H), 7.38 - 7.32 (m, 1H), 7.25 (d, *J* = 8.2 Hz, 1H), 6.96 (d, *J* = 8.4 Hz, 1H), 6.88 (dd, *J =* 10.5, 8.3 Hz, 1H), 4.11 (t, *J* = 4.9 Hz, 2H), 3.67 (s, 2H), 2.98 - 2.84 (m, 6H), 2.60 - 2.52 (m, 4H), 2.39 (s, 3H), 2.26 (s, 3H), 2.04 (s, 2H).

The following compounds in the Examples were synthesized according to procedures similar to those described in Example 94 and using appropriate intermediates.

| Example | Compound | Structure | Characterization data |
|---|---|---|---|
| Example 93 | Compound 93 | 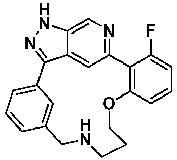 | ESI-MS: 573.3, [M+H]⁺. |
| | | | ¹H NMR (DMSO-*d*₆, 400 MHz) δ 13.73 (s, 1H), 9.15 (s, 1H), 8.16 (d, *J* = 1.0 Hz, 1H), 7.79 (s, 1H), 7.59 (d, *J* = 7.5 Hz, 1H), 7.47 (t, *J* = 7.6 Hz, 1H), 7.35 (dd, *J* = 14.9, 8.3 Hz, 1H), 7.25 (d, *J* = 7.6 Hz, 1H), 6.98 (d, J = 8.4 Hz, 1H), 6.93 - 6.81 (m, 1H), 4.27 - 4.09 (m, 2H), 3.98 (s, 2H), 2.99 (t, *J* = 7.6 Hz, 2H), 2.07 - 1.95 (m, 2H). |
| Example 96 | Compound 96 | 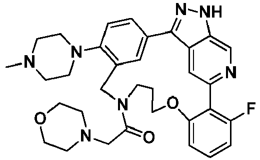 | ESI-MS: 600.3, [M+H]⁺. |
| | | | ¹H NMR (DMSO-*d*₆, 400 MHz) δ 13.74 (s, 1H), 9.13 (s, 1H), 8.30 (s, 1H), 7.87 - 7.71 (m, 2H), 7.42 - 7.35 (m, 2H), 7.10 - 7.00 (m, 2H), 6.95 (d, *J* = 10.1 Hz, 1H), 4.80 - 4.70 (m, 2H), 4.30 - 4.10 (m, 2H), 3.95 - 3.82 (m, 1H), 3.75 - 3.65 (m, 1H), 3.55 - 3.45 (m, 2H), 3.25 - 3.20 (m, 2H), 3.23 (s, 1H), 3.02 (s, 2H), 2.95 - 2.80 (m, 5H), 2.55 (s, 2H), 2.44 (s, 1H), 2.35 - 2.26 (m, 8H). |
| Example 97 | Compound 97 | 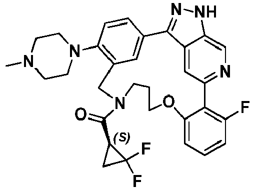 | ESI-MS: 577.3, [M+H]⁺. |
| | | | ¹H NMR (DMSO-*d*₆, 400 MHz) δ 13.76 (s, 1H), 9.14 (s, 1H), 8.29 (s, 1H), 8.06 - 7.81 (m, 1H), 7.86 - 7.58 (m, 1H), 7.44 - 7.35 (m, 2H), 7.25 - 6.83 (m, 2H), 4.99 - 4.66 (m, 2H), 4.40 - 4.10 (m, 2H), 4.08 - 3.81 (m, 1H), 3.80 - 3.60 (m, 1H), 3.00 - 2.77 (m, 5H), 2.50 - 2.40 (m, 5H), 2.25 (s, 3H), 2.12 - 1.98 (m, 1H), 1.97 - 1.82 (m, 1H), 1.82 - 1.70 (m, 1H). |
| Example 98 | Compound 98 | 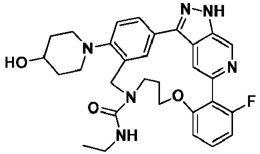 | ESI-MS: 544.3, [M+H]⁺. |
| | | | ¹H NMR (DMSO-*d*₆, 400 MHz) δ 13.75 (s, 1H), 9.14 (d, *J* = 1.1 Hz, 1H), 8.38 (d, *J* = 1.3 Hz, 1H), 7.83 (d, *J* = 7.4 Hz, 2H), 7.45 - 7.30 (m, 2H), 7.05 (d, *J* = 8.4 Hz, 1H), 6.93 (dd, *J* = 10.6, 8.2 Hz, 1H), 6.60 (t, *J* = 5.7 Hz, 1H), 4.50 (s, 2H), 4.20 (t, *J* = 5.1 Hz, 2H), 3.74 - 3.64 (m, 2H), 3.18 - 2.98 (m, 2H), 2.95 (t, *J=* 4.5 Hz, 4H), 2.64 - 2.52 (m, 4H), 2.27 (s, 3H), 2.20 - 2.10 (m, 2H), 1.01 (t, *J* = 7.1 Hz, 3H). |
| Example 99 | Compound 99 | 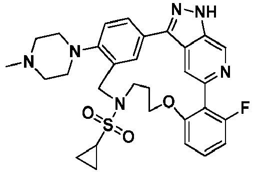 | ESI-MS: 577.3, [M+H]⁺. |
| | | | ¹H NMR (DMSO-*d*₆, 400 MHz) δ 13.74 (s, 1H), 9.14 (d, *J* = 1.2 Hz, 1H), 8.33 (s, 1H), 7.90 - 7.76 (m, 2H), 7.42 - 7.32 (m, 2H), 7.03 (d, *J* = 8.4 Hz, 1H), 6.93 (dd, *J* = 10.4, 8.3 Hz, 1H), 4.62 (s, 2H), 4.18 (t, *J* = 4.9 Hz, 2H), 3.72 (t, *J* = 8.1 Hz, 2H), 2.96 (s, 5H), 2.78 - 2.58 (m, 4H), 2.44 - 2.20 (m, 5H), 1.04 - 0.94 (m, 4H). |

### Example 119.

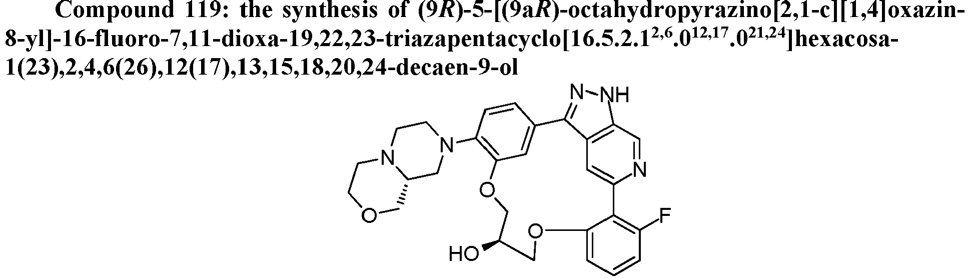

### Step 1: the synthesis of 3-{4-[(9aR)-octahydropyrazino[2,1-c][1,4]oxazin-8-yl]-3-{[(2R)-oxiran-2-yl]methoxy}phenyl}-5-bromo-1-{[2-(trimethylsilyl)ethoxy]methyl}-1H-pyrazolo[3,4-c]pyridine (Intermediate 119-1)

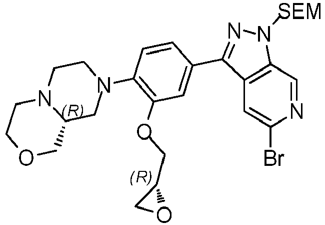

To a solution of 2-[(9a*R*)-octahydropyrazino[2,1-c][1,4]oxazin-8-yl]-5-(5-bromo-1-{[2-(trimethylsilyl)ethoxy]methyl}-1*H*-pyrazolo[3,4-c]pyridin-3-yl)phenol (200 mg, 0.36 mmol) in acetonitrile (10 mL) was added potassium carbonate (148 mg, 1.08 mmol), (2R)-glycidyl *p*-toluenesulfonate (164 mg, 0.72 mmol) and sodium iodide (52 mg, 0.36 mmol), and stirred at 75°C for 18 hours under nitrogen. The reaction was quenched with water (10 mL), and was extracted with ethyl acetate (20 mL × 3). The combined organic phase was washed with brine (50 mL), dried over anhydrous sodium sulfate and filtered. The filtrate was concentrated to dryness, and residue was purified column chromatography (dichloromethane/methanol = 20/1) to afford the title product (112 mg, yellow oil). Yield: 50%.

ESI-MS: 616.2, [M+H]⁺.

### Step 2: the synthesis of 2-(3-{4-[(9aR)-octahydropyrazino[2,1-c][1,4]oxazin-8-yl]-3-{[(2R)-oxiran-2-yl]methoxy}phenyl}-1-{[2-(trimethylsilyl)ethoxy]methyl}-1H-pyrazolo[3,4-c]pyridin-5-yl)-3-fluorophenol (Intermediate 119-2)

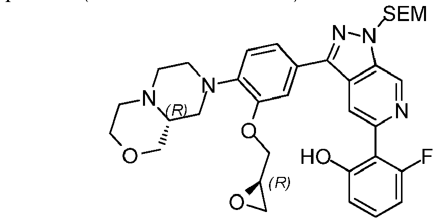

To a solution of **Intermediate 119-1** (112 mg, 0.18 mmol) in 1,4-dioxane/water (10/2 mL) was added (2-fluoro-6-hydroxyphenyl)boronic acid (34 mg, 0.22 mmol), Xphos-Pd-G2 (15 mg, 0.02 mmol) and potassium phosphate (115 mg, 0.54 mmol), and then stirred at 85°C for 3 hours under nitrogen. The mixture was then cooled to room temperature, diluted with water (30 mL), and extracted with ethyl acetate (20 mL × 3). The combined organic phase was washed with brine (50 mL), dried over anhydrous sodium sulfate, and filtered. The filtrate was concentrated to dryness, and the residue was purified by column chromatography (ethyl acetate/methanol = 50/1) to afford the title product (43 mg, yellow oil). Yield: 37%.

ESI-MS: 648.2, [M+H]⁺.

### Step 3: the synthesis of (9R)-5-[(9aR)-octahydropyrazino[2,1-c][1,4]oxazin-8-yl]-16-fluoro-22- f [2-(trimethylsilyl)ethoxy]methyl}-7,11-dioxa-19,22,23-triazapentacyclo[16.5.2.1^{2,6}.0^{12,17}.0^{21,24}]hexacosa-1(23),2(26),3,5,12,14,16,18,20,24-decaen-9-ol (Intermediate 119-3)

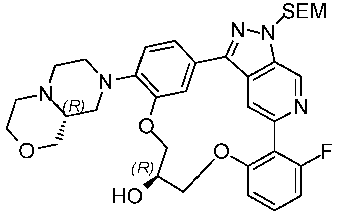

To a solution of **Intermediate 119-2** (43 mg, 0.07 mmol) in acetonitrile (5 mL) was added cesium carbonate (65 mg, 0.20 mmol), and stirred at 80°C for 3 hours under nitrogen. The mixture was then cooled to room temperature, diluted with water (10 mL), and extracted with ethyl acetate (20 mL × 3). The combined organic phase was washed with brine (80 mL), dried over anhydrous sodium sulfate, and filtered. The filtrate was concentrated to dryness, and the residue was purified by pre-TLC (ethyl acetate/methanol = 50/1) to afford the title product (17 mg, white solid). Yield: 39.5%.

ESI-MS: 648.2, [M+H]⁺.

### Step 4: the synthesis of Compound 119

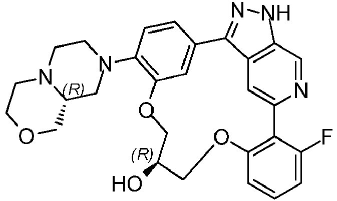

To a solution of **Intermediate 119-3** (17 mg, 0.03 mmol) in dichloromethane (5 mL) was added trifluoroacetic acid (1 mL) at 0°C, and then warmed to room temperature and stirred for 18 hours. The reaction mixture was concentrated to dryness, and the residue was dissolved in acetonitrile (5 mL), and treated with ammonia (1 mL) for 30 min. The mixture was extracted with dichloromethane/isopropanol mixture (5/1, 10 mL × 3). The combined organic phase was washed with brine (15 mL), dried over anhydrous sodium sulfate and filtered. The filtrate was concentrated to dryness, and residue was purified by pre-TLC (dichloromethane/methanol = 10/1) to afford the title product (5.34 mg, white solid). Yield: 39%.

ESI-MS: 518.2, [M+H]⁺.

¹H NMR (DMSO-*d*₆, 400 MHz) δ 13.62 (s, 1H), 9.09 (s, 1H), 8.68 (s, 1H), 7.74 (s, 1H), 7.59 - 7.24 (m, 2H), 7.24 - 6.83 (m, 3H), 5.78 - 5.47 (m, 1H), 4.74 - 4.70 (m, 1H), 4.35 - 4.26 (m, 2H), 4.04 (t, *J* = 9.4 Hz, 1H), 3.92 - 3.86 (m, 1H), 3.82 - 3.75 (m, 1H), 3.75 - 3.43 (m, 3H), 3.38 - 3.35 (m, 1H), 3.21 - 3.15 (m, 1H), 2.83 - 2.74 (m, 2H), 2.72 - 2.64 (m, 1H), 2.46 - 2.20 (m, 4H).

The following compounds in the Examples were synthesized according to procedures similar to those described in Example 119 and using appropriate intermediates.

| Example | Compound | Structure | Characterization data |
|---|---|---|---|
| Example 114 | Compound 114 | 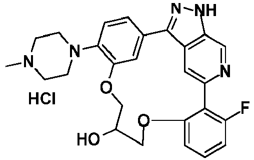 | ESI-MS: 476.2, [M+H-HCl]⁺. |
| | | | ¹H NMR (DMSO-*d*₆, 400 MHz) δ 13.67 (s, 1H), 10.30 (br, 1H), 9.10 (s, 1H), 8.68 (s, 1H), 7.89 (s, 1H), 7.50 (d, *J* = 7.6 Hz, 1H), 7.44-7.37 (m, 2H), 7.10 (d, *J* = 8.1 Hz, 1H), 7.05-6.96 (m, 2H), 5.63 (d, *J* = 4.6 Hz, 1H), 4.75 (d, *J* = 13.3 Hz, 1H), 4.40-4.22 (m, 2H), 4.05 (t, *J* = 9.4 Hz, 1H), 3.92-3.80 (m, 1H), 3.60-2.80 (br, 8H), 2.70 (br, 3H). |
| Example 120 | Compound 120 | 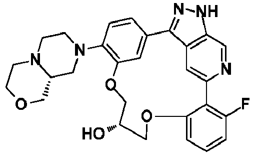 | ESI-MS: 518.2, [M+H]⁺. |
| | | | ¹H NMR (DMSO-*d*₆, 400 MHz) δ 13.62 (s, 1H), 9.09 (s, 1H), 8.68 (s, 1H), 7.74 (s, 1H), 7.59 - 7.24 (m, 2H), 7.24 - 6.83 (m, 3H), 5.78 - 5.47 (m, 1H), 4.74 - 4.70 (m, 1H), 4.35 - 4.26 (m, 2H), 4.04 (t, *J* = 9.4 Hz, 1H), 3.92 - 3.86 (m, 1H), 3.82 - 3.75 (m, 1H), 3.75 - 3.43 (m, 3H), 3.38 - 3.35 (m, 1H), 3.21 - 3.15 (m, 1H), 2.83 - 2.74 (m, 2H), 2.72 - 2.64 (m, 1H), 2.46 - 2.20 (m, 4H). |

### Example: Biological Activity Test and Results

### 1. HPK1 Kinase Activity Inhibition Test

The kinase activity of HPK1 is manifested as activity of autophosphorylation and phosphorylation of downstream substrates. In the process of autophosphorylation, additional substrates are not required, and ATP is consumed to generate ADP. The amount of the product is measured by ADP-Glo reagent and luminescence method to reflect kinase activity.

Tested compounds: compounds prepared in the Examples of this invention.

Preparation of compound stock solution: dissolve the test compound in 100% DMSO to make a 10 mM stock solution;

Preparation of 4×Kinase Reaction Buffer:

| Material | | Concentration of Stock Solution | | Volume | | Final Concentration | |
|---|---|---|---|---|---|---|---|
| | Tris | | 1 M (25X) | | 240µL | | 40 mM |
| | MgCl₂ | | 1 M (50X) | | 120µL | | 20 mM |
| | BSA | | 7.5%(75X) | | 80µL | | 0.1% |
| | DTT | | 1M(500X) | | 3µL | | 0.5 mM |
| | ddH₂O | | | | 5557µL | | |

Preparation of 2×HPK1 Kinase Solution:

| Material | Concentration of Stock Solution | Volume | 2×Final Concentration | Final Concentration |
|---|---|---|---|---|
| HPK1 | 4878 nM | 1µL | 10 nM | 5 nM |
| 1 × kinase reaction buffer solution | | 487µL | | |

Preparation of 4×ATP mixture:

| Material | ATP Km | Concentration of Stock Solution | Volume | 4×Final Concentration | Final Concentration |
|---|---|---|---|---|---|
| ATP | 1.669µM | 1 mM (125×) | 3µL | 8µM | 2µM |
| 4×kinase reaction buffer solution | | | 372µL | | |

### Procedures:

Dilute the stock solution of the test compound by 5 times with 100% DMSO, make a 4-fold equal dilution in a 96-well dilution plate, add 1 µL of the compound to 49 µL of kinase reaction buffer, and shake on a microplate shaker for 20 minutes. Transfer 2 µL of 2×HPK1 kinase solution to 384 reaction plate, add 1 µL of the test compound (prepared in step (1)) to the 384 reaction plate (Greiner, Cat# 784075), centrifuge for 1 minute (1000 rpm/min), incubate at 25°C for 10 minutes. Transfer 1 µL of the 4×ATP mixture to a 384 reaction plate, centrifuge for 1 minute (1000 rpm/min), and incubate at 25°C for 60 minutes. In the reaction system, the final concentration of DMSO is 0.5%. Transfer 4 µL of ADP-Glo to a 384 reaction plate, centrifuge for 1 minute (1000 rpm/min), and incubate at 25°C for 40 minutes. Transfer 8 µL detection solution to a 384 reaction plate, centrifuge for 1 minute (1000 rpm/min), and incubate at 25°C for 40 minutes. The fluorescence signal is read using a Biotek multi-function plate reader, and the IC50 (half inhibitory concentration) of the compound is obtained using a four-coefficient nonlinear fitting formula.

Compounds as shown in the Examples exhibit IC50 values in the following ranges: ++++ = IC50 ≤ 5 nM, +++ = 5 nM < IC50 ≤ 50 nM, ++ = 50 nM < IC50 ≤ 500 nM, + = IC50 > 500 nM.

**Table 1 Inhibitory effects of compounds on HPK1 kinase activity**

| Compound | IC50 | Compound | IC50 |
|---|---|---|---|
| 3 | ++++ | 116 | ++++ |
| 4 | +++ | 117 | ++++ |
| 5 | +++ | 119 | +++ |
| 6 | ++++ | 120 | ++++ |
| 7 | +++ | 121 | ++++ |
| 9 | ++++ | 122 | ++++ |
| 10 | ++++ | 123 | ++++ |
| 11 | ++++ | 124 | ++++ |
| 12 | ++++ | 127 | ++++ |
| 17 | ++++ | 128 | ++++ |
| 18 | ++++ | 130 | ++++ |
| 100 | +++ | 131 | ++++ |
| 101 | ++++ | 134 | ++++ |
| 102 | ++++ | 135 | ++++ |
| 103 | ++++ | 137 | ++++ |
| 104 | ++++ | 138 | ++++ |
| 105 | ++++ | 140 | ++++ |
| 106 | +++ | 141 | ++++ |
| 107 | ++++ | 142 | +++ |
| 108 | ++++ | 143 | ++++ |
| 109 | ++++ | 144 | ++++ |
| 110 | ++++ | 145 | ++++ |
| 111 | ++++ | 146 | ++++ |
| 112 | ++++ | 147 | ++++ |
| 113 | +++ | 151 | ++++ |
| 114 | ++++ | 152 | ++++ |
| 115 | +++ | 153 | ++++ |

The data in Table 1 show that the compounds of the Examples of the present invention have inhibitory effect on HPK1 kinase activity.

### 2. FLT3 Kinase Activity Inhibition Test

Tested compounds: compounds prepared in the Examples of this invention.

Prepare compound stock solution: dissolve the test compound in 100% DMSO to make a 10 mM stock solution. Dilute the stock solution of the test compound by 5 times with 100% DMSO, make a 4-fold equal dilution in a 96-well dilution plate, add 1 µL of the compound to 39 µL of kinase reaction buffer (1×kinase buffer, 5 mM MgCl2, 1 mM DTT), and shake on a microplate shaker for 20 minutes. 4×Positive control (4 µL of positive compound solution of the starting concentration, add 196 µL of kinase buffer) and 4×negative control (4 µL of 100% DMSO, add 196 µL of kinase buffer).

### Procedures

(1) Use 1×kinase reaction buffer to prepare 2.5×FLT3 kinase solution (160 pM), transfer 2 µL of the kinase solution to the 384 reaction plate, and add 1 µL of the test compound solution of gradient concentration to the 384 reaction plate (Greiner, Cat# 784075), centrifuge for 1 minute (1000 rpm), and incubate at 25°C for 10 minutes.
(2) Use 1×kinase reaction buffer to prepare 2.5×TK-substrate-biotin (2.5 µM) and 2.5× ATP (2.5 µM), mix well, and add 2 µL of the TK-substrate-biotin/ATP solution to the 384 reaction plate, centrifuge for 30 seconds (1000 rpm), and incubate at 25°C for 40 minutes.
(3) Use HTRF detection buffer to prepare 2×Sa-XL 665 (125 nM) and 1× TK-antibady-Cryptate. Add 5 µL of a mixture of Sa-XI, 665 and TK-antibody-Cryptate solutions to each well, centrifuge for 30 seconds (1000 rpm), and react at room temperature for 1 hour.
(4) The fluorescence signal is read using a Biotek multi-function plate reader, and the IC50 (half inhibitory concentration) of the compound is obtained using a four-coefficient nonlinear fitting formula.

Compounds as shown in Examples exhibit IC50 values in the following ranges: ++++ = IC50 ≤ 5 nM, +++ = 5 nM < IC50 ≤ 50 nM, ++ = 50 nM < IC50 ≤ 500 nM, + = IC50 > 500 nM.

**Table 2 Inhibitory effects of compounds on FLT3 kinase activity**

| Compound | IC50 | Compound | IC50 |
|---|---|---|---|
| 3 | +++ | 102 | ++++ |
| 6 | ++++ | 103 | ++++ |
| 9 | ++++ | 119 | ++++ |
| 10 | ++++ | 122 | ++++ |
| 11 | ++++ | 144 | ++++ |
| 12 | ++++ | 151 | ++++ |

The data in Table 2 show that the compounds of the Examples of the present invention have inhibitory effect on FLT3 kinase activity.

### 3. KDR Kinase Activity Inhibition Test

Tested compounds: compounds prepared in the Examples of this invention.

Prepare compound stock solution: dissolve the test compound in 100% DMSO to make a 10 mM stock solution. Dilute the stock solution of the test compound by 5 times with 100% DMSO, make a 4-fold equal dilution in a 96-well dilution plate, add 1 µL of the compound to 39 µL of kinase reaction buffer (1×kinase buffer, 5 mM MgCl2, 1 mM DTT), and shake on a microplate shaker for 20 minutes. 4×Positive control (4 µL of positive compound solution of the starting concentration, add 196 µL of kinase buffer) and 4×negative control (4 µL of 100% DMSO, add 196 µL of kinase buffer).

### Procedures

(1) Use 1×kinase reaction buffer to prepare 2.5×KDR kinase solution (250 pM), transfer 2 µL of the kinase solution to the 384 reaction plate, and add 1 µL of the test compound solution of gradient concentration to the 384 reaction plate (Greiner, Cat# 784075), centrifuge for 1 minute (1000 rpm), and incubate at 25°C for 10 minutes.
(2) Use 1×kinase reaction buffer to prepare 2.5×TK-substrate-biotin (2.5 µM) and 2.5× ATP (2.5 µM), mix well, and add 2 µL of the TK-substrate-biotin/ATP solution to the 384 reaction plate, centrifuge for 30 seconds (1000 rpm), and incubate at 25°C for 40 minutes.
(3) Use HTRF detection buffer to prepare 2×Sa-XL 665 (125 nM) and 1× TK-antibady-Cryptate. Add 5 µL of a mixture of Sa-XI, 665 and TK-antibody-Cryptate solutions to each well, centrifuge for 30 seconds (1000 rpm), and react at room temperature for 1 hour.
(4) The fluorescence signal is read using a Biotek multi-function plate reader, and the IC50 (half inhibitory concentration) of the compound is obtained using a four-coefficient nonlinear fitting formula.

Compounds as shown in the Examples exhibit IC50 values in the following ranges: ++++ = IC50 ≤ 5 nM, +++ = 5 nM < IC50 ≤ 50 nM, ++ = 50 nM < IC50 ≤ 500 nM, + = IC50 > 500 nM.

**Table 2 Inhibitory effects of compounds on KDR kinase activity**

| Compound | IC50 | Compound | IC50 |
|---|---|---|---|
| 3 | +++ | 102 | ++++ |
| 6 | ++++ | 103 | ++++ |
| 9 | ++++ | 119 | ++++ |
| 10 | +++ | 122 | ++++ |
| 11 | ++++ | 144 | ++++ |
| 12 | +++ | 151 | ++++ |

### 4. Detection of IL-2 production by Jurkat cells using ELISA

Tested compounds: compounds prepared in the Examples of this invention.

### Procedures:

Human Jurkat-E6-1 cells were incubated with various concentrations of tested compounds for 30 minutes in a humidified incubator at 37°C and 5% CO2. Cells were transferred to cell culture plates pre-coated with anti-human CD3 antibody, then soluble anti-human CD28 antibody was added, and cells were stimulated for 24 hours at 37°C and 5% CO₂ in a humidified incubator. The cell culture medium was collected after centrifugation, and was then transferred to a 96-well transparent microtiter plate (Thermo) pre-coated with anti-human IL-2 antibody, incubated at room temperature for 2 hours with gently shaking, washed with washing buffer for 4 times, and then followed the ELISA MAX Deluxe Set Human IL-2 (BioLegend) kit procedures, used a microplate reader (Molecular Device, i3X) to read the OD value at 450nm. The best standard curve was selected by the microplate reader application software, and the corresponding concentration was calculated according to the OD value of the standard. Results are expressed as a percentage (%) of the amount of IL-2 secreted by compound-treated vs DMSO-treated cells.

**Table 4. Effect of compounds on secretion of IL-2 from human Jurkat cells.**

| Compound | Percentage of the amount of IL-2 secreted by compound treated vs DMSO-treated cells | Concentration of the Tested Compound (µM) |
|---|---|---|
| 3 | 823% | 1.0 |
| 4 | 801% | 3.0 |
| 5 | 717% | 3.0 |
| 6 | 542% | 1.0 |
| 7 | 404% | 3.0 |
| 9 | 568% | 1.0 |
| 10 | 298% | 1.0 |
| 17 | 439% | 0.33 |
| 18 | 486% | 0.33 |
| 100 | 235% | 1.0 |
| 101 | 257% | 0.33 |
| 102 | 702% | 1.0 |
| 103 | 580% | 0.33 |
| 104 | 273% | 0.33 |
| 105 | 313% | 0.33 |
| 106 | 504% | 1.0 |
| 107 | 345% | 1.0 |
| 108 | 213% | 0.33 |
| 109 | 264% | 1.0 |
| 110 | 222% | 0.33 |
| 111 | 388% | 0.33 |
| 112 | 245% | 1.0 |
| 113 | 265% | 0.33 |
| 114 | 239% | 0.33 |
| 115 | 263% | 1.0 |
| 116 | 450% | 0.33 |
| 117 | 525% | 1.0 |
| 119 | 591% | 0.33 |
| 120 | 494% | 1.0 |
| 121 | 467% | 0.33 |
| 122 | 345% | 0.33 |
| 123 | 440% | 1.0 |
| 124 | 630% | 1.0 |
| 127 | 475% | 0.33 |
| 128 | 452% | 0.33 |
| 144 | 517% | 1.0 |
| 145 | 512% | 1.0 |
| 146 | 476% | 1.0 |
| 147 | 429% | 0.33 |
| 151 | 416% | 1.0 |
| 152 | 432% | 0.33 |
| 153 | 369% | 0.33 |

The data in Table 4 shows that the example compounds in the invention have significant enhancement on the secretion of cytokine IL-2, as compared to DMSO controls.

### 5. Detection of MV-4-11 cell activity

The CellTiter-Glo (CTG) luminescence method was used to detect the effect of compounds on the activity of MV-4-11 cells.

Tested compounds: compound prepared in the Examples of the present invention.

### Procedures:

Add 100 µL containing 20,000 MV-4-11 (human myeloid monocytic leukemia cells, from the Cell Bank of the Chinese Academy of Sciences, Cat. No. SCSP-5031) cell suspension to 96-well flat-bottom clear-bottomed cell plates (Corning, Cat# 3603), and then add 100 µL of cell culture medium containing a series of concentrations of the compound to be tested, with a total volume of 200 µL per well. The final gradient concentrations of the compounds were 500nM, 125nM, 31.25nM, 7.813nM, 1.953nM, 0.488nM, 0.122nM, 0.031nM, and 0.008nM, and the final DMSO content was 0.5%. Set cells with the cell culture medium containing 0.5% DMSO but without compounds as the 100% control; set the culture medium wells without cells as 0% background control. Incubate the cell plate in a humidified incubator at 37°C and 5% CO2 for 5 days. After 5 days, take out the cell plate, centrifuge (1000 rpm) for 5 minutes, remove and discard 100 µL supernatant, add 100µL CTG reagent (Promega, Cat#G7572) to each well, shake at room temperature for 10 minutes to promote cell lysis, and incubate at room temperature for 30 minutes to stabilize the luminescence signal. Use a microplate reader (Molecular Device, i3X) to read the Luminescent fluorescence value, use GraphPad Prism 5.0 software to analyze the original data, use nonlinear S-curve regression to fit the data to obtain the dose-effect curve (cell survival rate %), and calculate the EC50 value.

**Table 5. Inhibitory effects of compounds on MV-4-11 cell activity**

| Compound | EC50 (nM) |
|---|---|
| 3 | 32.0 |
| 5 | 50.0 |
| 6 | 3.8 |
| 9 | 7.0 |
| 11 | 6.1 |

Unless otherwise defined, the terms used in this application are the meanings commonly understood by those skilled in the art.

The embodiments described in this application are only for exemplary purposes and are not intended to limit the protection scope of this application. Those skilled in the art can make various other substitutions, changes and improvements within the scope of this application. Therefore, this application is not limited to the above-described embodiments, but only by the claims.

## Claims

1. A macrocyclic azaindazole compound of Formula (I), 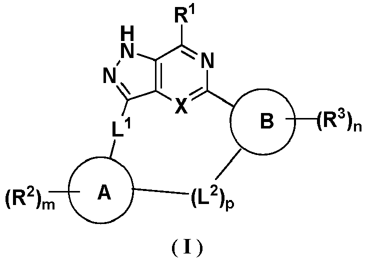
or an isomer, pharmaceutically acceptable salt, polymorph, isotope labeled compound, active metabolite or prodrug thereof,
wherein, the structure and atomic number of the azaindazole ring are as shown in Formula (Ia), 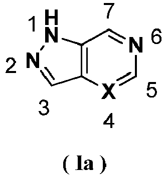
in the structure of Formula (I), X is selected from the group consisting of CR^{x} and N, wherein R^{x} is independently selected from the group consisting of hydrogen and halogen;
R¹ is selected from the group consisting of:
1) hydrogen, halogen, cyano, -C(=O)OR^{a1}, -C(=O)NR^{a1}R^{b1}, -OR^{a1}, -NR^{a1}R^{b1}, -NR^{e1}C(=O)R^{a1}, -NR^{e1}C(=O)NR^{a1}R^{b1}, -SR^{a1}, -S(=O)R^{a1} and -S(=O)₂R^{a1};
2) C₁₋₆ alkyl, C₂₋₄ alkenyl, C₂₋₄ alkynyl, C₃₋₈ cycloalkyl, and 3- to 8-membered aliphatic heterocyclyl, optionally substituted with 0, 1, 2, 3 or 4 of R¹¹;
wherein, R^{a1}, R^{b1} and R^{e1} are each independently selected from the group consisting of:
1) hydrogen;
2) C₁₋₆ alkyl, C₃₋₆ monocyclic cycloalkyl, and 3- to 6-membered monocyclic aliphatic heterocyclyl, optionally substituted with 0, 1, 2, 3 or 4 of R¹¹;
or, R^{a1} and R^{b1} attached to the same nitrogen atom, taken together with the nitrogen atom to which they are attached, form a 4- to 6-membered aliphatic heterocyclyl, optionally substituted with 0, 1, 2, 3 or 4 of R¹¹;
wherein, R¹¹ is selected from the group consisting of oxo, halogen, hydroxyl, amino, cyano, C₁₋₃ alkyl, C₁₋₃ alkoxy, C₁₋₃ alkylamino, C₃₋₆ cycloalkyl and C₃₋₆ cycloalkoxy;
L¹ is selected from the group consisting of:
1) single bond;
2) 6- to 10-membered aryl, and 5- to 10-memebred heteroaryl; said aryl or heteroaryl group is connected to the azaindazole ring at the 3-position and to the ring A through a single bond, respectively; in addition to being connected to the azaindazole ring at the 3-position and to the ring A, said aryl or heteroaryl group is optionally substituted with 0, 1, 2, 3, 4, 5 or 6 of R4;
ring A is selected from the group consisting of monocyclic or bicyclic 3- to 12-membered carbocyclyl group and 3- to 12-membered heterocyclyl group, said carbocyclyl or heterocyclyl group is connected to L¹ and L² through two different atoms respectively; and in addition to being connected to L¹ and L², said carbocyclyl or heterocyclyl group is optionally substituted with m of R²;
ring B is selected from the group consisting of monocyclic or bicyclic 6- to 12-membered carbocyclyl group and 5- to 12-membered heterocyclyl group, said carbocyclyl or heterocyclyl group is connected to L² and to the azaindazole ring at the 5-position through two different atoms respectively; wherein,
1) when ring B is selected from the group consisting of monocyclic carbocyclyl and monocyclic heterocyclyl group, the shortest distance between the two atoms on ring B, through which ring B is connected to L² and to the azaindazole ring at the 5-position, is 1, 2 or 3 chemical bonds;
2) when ring B is selected from the group consisting of bicyclic carbocyclyl and bicyclic heterocyclyl group, the shortest distance between the two atoms on ring B, through which ring B is connected to L² and to the azaindazole ring at the 5-position, is 1, 2, 3, 4 or 5 chemical bonds;
and, in addition to being connected to L² and to the azaindazole ring at the 5-position, ring B is optionally substituted with n of R³;
R² is each independently selected from the group consisting of:
1) oxo, halogen, cyano, -C(=O)R^{a2}, -C(=O)OR^{a2}, -C(=O)NR^{a2}R^{b2}, -C(=NR^{d2})NR^{a2}R^{b2}, -OR^{a2}, -OC(=O)R^{a2}, -OC(=O)OR^{c2}, -OC(=O)NR^{a2}R^{b2}, -SR^{a2}, -S(=O)R^{c2}, -S(=O)₂R^{c2}, sulfonic acid group, -S(=O)NR^{a2}R^{b2}, -S(=O)₂NR^{a2}R^{b2}, -S(=O)(=NR^{d2})R^{c2}, -NR^{a2}R^{b2}, -NR^{a2}C(=O)R^{b2}, -NR^{a2}C(=O)OR^{c2}, -NR^{e2}C(=O)NR^{a2}R^{b2}, -NR^{e2}C(=NR^{d2})NR^{a2}R^{b2}, -NR^{a2}S(=O)₂R^{c2}, -NR^{e2}S(=O)₂NR^{a2}R^{b2}, nitro, -PR^{c2}R^{f2}, -P(=O)R^{c2}R^{f2} and phosphonic acid group;
2) C₁₋₆ alkyl, C₂₋₆ alkenyl, C₂₋₆ alkynyl, phenyl, 5- to 10-membered heteroaryl, C₃₋₁₂ cycloalkyl and 3- to 12-membered aliphatic heterocyclyl, optionally substituted with 0, 1, 2, 3, 4, 5 or 6 of R²²;
3) divalent bridged C₁₋₅ alkylene, 3- to 6-membered oxaalkylene, and 2- to 6-membered azaalkylene connected to ring A through two different ring-forming atoms thereof; said alkylene, oxaalkylene and azaalkylene are optionally substituted with 0, 1, 2, 3, 4, 5 or 6 of R²²;
or, two R² groups attached to one or two ring-forming atom(s) of the ring A, taken together with the one or two ring-forming atom(s) to which they are attached, form a C₅₋₁₂ aliphatic carbocyclyl, or 5- to 12-membered aliphatic heterocyclyl, optionally substituted with 0, 1, 2, 3, 4 or 5 of R²²;
wherein, R^{a2}, R^{b2} and R^{e2} are each independently selected from the group consisting of:
1) hydrogen;
2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4, 5 or 6 of R²²;
or, R^{a2} and R^{b2} attached to the same nitrogen atom, taken together with the nitrogen atom to which they are attached, form a 3- to 12-membered aliphatic heterocyclyl, optionally substituted with 0, 1, 2, 3, 4 or 5 of R²²;
R^{c2} and R^{f2} are each independently selected from the group consisting of C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4 or 5 of R²²; or, R^{c2} and R^{f2} attached to the same phosphine atom, taken together with the phosphine atom to which they are attached, form a 3- to 12-membered aliphatic heterocyclyl, optionally substituted with 0, 1, 2, 3, 4 or 5 of R²²;
R^{d2} is selected from the group consisting of:
1) hydrogen, cyano, nitro and -S(=O)₂R^{G};
2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4 or 5 of R²²;
R²² is selected from the group consisting of:
1) oxo, halogen, cyano, -C(=O)R^{a22}, -C(=O)OR^{a22}, -C(=O)NR^{a22}R^{b22}, -C(=NR^{d22})NR^{a22}R^{b22}, -OR^{a22}, -OC(=O)R^{a22}, -OC(=O)OR^{c22}, -OC(=O)NR^{a22}R^{b22}, -SR^{a22}, -S(=O)R^{c22}, -S(=O)₂R^{c22}, sulfonic acid group, -S(=O)NR^{a22}R^{b22}, -S(=O)₂NR^{a22}R^{b22}, -S(=O)(=NR^{d22})R^{c22}, -NR^{a22}R^{b22}, -NR^{a22}C(=O)R^{b22}, -NR^{a22}C(=O)OR^{c22}, -NR^{e22}C(=O)NR^{a22}R^{b22}, -NR^{e22}C(=NR^{d22})NR^{a22}R^{b22}, -NR^{a22}S(=O)₂R^{c22}, -NR^{e22}S(=O)₂NR^{a22}R^{b22}, nitro, -PR^{c22}R^{f22}, -P(=O)R^{c22}R^{f22}, phosphonic acid group, and =N-R^{d22};
2) C₁₋₆ alkyl, C₁₋₆ alkylene, C₂₋₆ alkenyl, C₂₋₆ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R²³;
R^{a22}, R^{b22} and R^{e22} are each independently selected from the group consisting of:
1) hydrogen;
2) C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R²³;
or, R^{a22} and R^{b22} attached to the same nitrogen atom, taken together with the nitrogen atom to which they are attached, form a 3- to 10-membered aliphatic heterocyclyl, optionally substituted with 0, 1, 2, 3, 4 or 5 of R²³;
R^{c22} and R^{f22} are each independently selected from the group consisting of C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R²³; or, R^{c22} and R^{f22} attached to the same phosphine atom, taken together with the phosphine atom to which they are attached, form a 3- to 10-membered aliphatic heterocyclyl, optionally substituted with 0, 1, 2, 3, 4 or 5 of R²³;
R^{d22} is selected from the group consisting of:
1) hydrogen, cyano, nitro and -S(=O)₂R^{G};
2) C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R²³;
R²³ is selected from the group consisting of:
1) oxo, halogen, cyano, -C(=O)R^{a23}, -C(=O)OR^{a23}, -C(=O)NR^{a23}R^{b23}, -C(=NR^{d23})NR^{a23}R^{b23}, -OR^{a23}, -OC(=O)R^{a23}, -OC(=O)OR^{c23}, -OC(=O)NR^{a23}R^{b23}, -SR^{a23}, -S(=O)R^{c23}, -S(=O)₂R^{c23}, sulfonic acid group, -S(=O)NR^{a23}R^{b23}, -S(=O)₂NR^{a23}R^{b23}, -S(=O)(=NR^{d23})R^{c23}, -NR^{a23}R^{b23}, -NR^{a23}C(=O)R^{b23}, -NR^{a23}C(=O)OR^{c23}, -NR^{e23}C(=O)NR^{a23}R^{b23}, -NR^{e23}C(=NR^{d23})NR^{a23}R^{b23}, -NR^{a23}S(=O)₂R^{c23}, -NR^{e23}S(=O)₂NR^{a23}R^{b23}, nitro, -PR^{c23}R^{f23}, -P(=O)R^{c23}R^{f23}, phosphonic acid group, and =N-R^{d23};
2) C₁₋₄ alkyl, C₁₋₄ alkylene, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
R^{a23}, R^{b23} and R^{e23} are each independently selected from the group consisting of:
1) hydrogen;
2) C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
or, R^{a23} and R^{b23} attached to the same nitrogen atom, taken together with the nitrogen atom to which they are attached, form a 3- to 8-membered aliphatic heterocyclyl, optionally substituted with 0, 1, 2, 3, or 4 of R^{G};
R^{c23} and R^{f23} are each independently selected from the group consisting of C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3, or 4 of R^{G};
or, R^{c23} and R^{f23} attached to the same phosphine atom, taken together with the phosphine atom to which they are attached, form a 3- to 8-membered aliphatic heterocyclyl, optionally substituted with 0, 1, 2, 3 or 4 of R^{G};
R^{d23} is selected from the group consisting of:
1) hydrogen, cyano, nitro and -S(=O)₂R^{G};
2) C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
R³ is independently selected from the group consisting of:
1) oxo, halogen, cyano, -C(=O)R^{a3}, -C(=O)OR^{a3}, -C(=O)NR^{a3}R^{b3}, -C(=NR^{d3})NR^{a3}R^{b3}, -OR^{a3}, -OC(=O)R^{a3}, -OC(=O)OR^{c3}, -OC(=O)NR^{a3}R^{b3}, -SR^{a3}, -S(=O)R^{c3}, -S(=O)₂R^{c3}, sulfonic acid group, -S(=O)NR^{a3}R^{b3}, -S(=O)₂NR^{a3}R^{b3}, -S(=O)(=NR^{d3})R^{c3}, -NR^{a3}R^{b3}, -NR^{a3}C(=O)R^{b3}, -NR^{a3}C(=O)OR^{c3}, -NR^{e3}C(=O)NR^{a3}R^{b3}, -NR^{e3}C(=NR^{d3})NR^{a3}R^{b3}, -NR^{a3}S(=O)₂R^{c3}, -NR^{e3}S(=O)₂NR^{a3}R^{b3}, nitro, -PR^{c3}R^{f3}, -P(=O)R^{c3}R^{f3} and phosphonic acid group;
2) C₁₋₆ alkyl, C₂₋₆ alkenyl, C₂₋₆ alkynyl, 6- to 10-membered aryl, 5- to 10-membered heteroaryl, C₃₋₁₂ cycloalkyl and 3- to 12-membered aliphatic heterocyclyl, optionally substituted with 0, 1, 2, 3, 4, 5 or 6 of R³²,
3) divalent bridged C₁₋₆ alkylene connected to ring B through two different ring-forming atoms thereof;
or, two R³ groups attached to one or two ring-forming atom(s) of the ring B, taken together with the one or two ring-forming atom(s) to which they are attached, form a C₅₋₁₂ aliphatic carbocyclyl, or 5- to 12-membered aliphatic heterocyclyl, optionally substituted with 0, 1, 2, 3, 4 or 5 of R³²;
wherein, R^{a3}, R^{b3} and R^{e3} are each independently selected from the group consisting of:
1) hydrogen;
2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4, 5 or 6 of R³²,
or, R^{a3} and R^{b3} attached to the same nitrogen atom, taken together with the nitrogen atom to which they are attached, form a 3- to 12-membered aliphatic heterocyclyl, optionally substituted with 0, 1, 2, 3, 4 or 5 of R³²,
R^{c3} and R^{f3} are each independently selected from the group consisting of C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4 or 5 of R³²,
or, R^{c3} and R^{f3} attached to the same phosphine atom, taken together with the phosphine atom to which they are attached, form a 3- to 12-membered aliphatic heterocyclyl, optionally substituted with 0, 1, 2, 3, 4 or 5 of R³²,
R^{d3} is selected from the group consisting of:
1) hydrogen, cyano, nitro and -S(=O)₂R^{G};
2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4 or 5 of R³²,
R³² is selected from the group consisting of:
1) oxo, halogen, cyano, -C(=O)R^{a32}, -C(=O)OR^{a32}, -C(=O)NR^{a32}R^{b32}, -C(=NR^{d32})NR^{a32}R^{b32}, -OR^{a32}, -OC(=O)R^{a32}, -OC(=O)OR^{c32}, -OC(=O)NR^{a32}R^{b32}, -SR^{a32}, -S(=O)R^{c32}, -S(=O)₂R^{c32}, sulfonic acid group, -S(=O)NR^{a32}R^{b32}, -S(=O)₂NR^{a32}R^{b32}, -S(=O)(=NR^{d32})R^{c32}, -NR^{a32}R^{b32}, -NR^{a32}C(=O)R^{b32}, -NR^{a32}C(=O)OR^{c32}, -NR^{e32}C(=O)NR^{a32}R^{b32}, -NR^{e32}C(=NR^{d32})NR^{a32}R^{b32}, -NR^{a32}S(=O)₂R^{c32}, -NR^{e32}S(-O)₂NR^{a32}R^{b32}, nitro, -PR^{c32}R^{f32}, -P(=O)R^{c32}R^{f32}, phosphonic acid group, and =N-R^{d32};
2) C₁₋₆ alkyl, C₁₋₆ alkylene, C₂₋₆ alkenyl, C₂₋₆ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R³³;
R^{a32}, R^{b32} and R^{e32} are each independently selected from the group consisting of:
1) hydrogen;
2) C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R³³;
or, R^{a32} and R^{b32} attached to the same nitrogen atom, taken together with the nitrogen atom to which they are attached, form a 3- to 10-membered aliphatic heterocyclyl, optionally substituted with 0, 1, 2, 3, 4 or 5 of R³³;
R^{c32} and R^{f32} are each independently selected from the group consisting of C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R³³;
or, R^{c32} and R^{f32} attached to the same phosphine atom, taken together with the phosphine atom to which they are attached, form a 3- to 10-membered aliphatic heterocyclyl, optionally substituted with 0, 1, 2, 3, 4 or 5 of R³³;
R^{d32} is selected from the group consisting of:
1) hydrogen, cyano, nitro and -S(=O)₂R^{G};
2) C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R³³;
R³³ is selected from the group consisting of:
1) oxo, halogen, cyano, -C(=O)R^{a33}, -C(=O)OR^{a33}, -C(=O)NR^{a33}R^{b33}, -C(=NR^{d33})NR^{a33}R^{b33}, -OR^{a33}, -OC(=O)R^{a33}, -OC(=O)OR^{c33}, -OC(=O)NR^{a33}R^{b33}, -SR^{a33}, -S(=O)R^{c33}, -S(=O)₂R^{c33}, sulfonic acid group, -S(=O)NR^{a33}R^{b33}, -S(=O)₂NR^{a33}R^{b33}, -S(=O)(=NR^{d33})R^{c33}, -NR^{a33}R^{b33}, -NR^{a33}C(=O)R^{b33}, -NR^{a33}C(=O)OR^{c33}, -NR^{e33}C(=O)NR^{a33}R^{b33}, -NR^{e33}C(=NR^{d33})NR^{a33}R^{b33}, -NR^{a33}S(=O)₂R^{c33}, -NR^{e33}S(=O)₂NR^{a33}R^{b33}, nitro, -PR^{c33}R^{f33}, -P(=O)R^{c33}R^{f33}, phosphonic acid group, and =N-R^{d33};
2) C₁₋₄ alkyl, C₁₋₄ alkylene, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
R^{a33}, R^{b33} and R^{e33} are each independently selected from the group consisting of:
1) hydrogen;
2) C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
or, R^{a33} and R^{b33} attached to the same nitrogen atom, taken together with the nitrogen atom to which they are attached, form a 3- to 8-membered aliphatic heterocyclyl, optionally substituted with 0, 1, 2, 3, or 4 of R^{G};
R^{c33} and R^{f33} are each independently selected from the group consisting of C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3, or 4 of R^{G};
or, R^{c33} and R^{f33} attached to the same phosphine atom, taken together with the phosphine atom to which they are attached, form a 3- to 8-membered aliphatic heterocyclyl, optionally substituted with 0, 1, 2, 3 or 4 of R^{G};
R^{d33} is selected from the group consisting of:
1) hydrogen, cyano, nitro and -S(=O)₂R^{G};
2) C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
R⁴ is independently selected from the group consisting of:
1) oxo, halogen, cyano, -C(=O)R^{a4}, -C(=O)OR^{a4}, -C(=O)NR^{a4}R^{b4}, -C(=NR^{d4})NR^{a4}R^{b4}, -OR^{a4}, -OC(=O)R^{a4}, -OC(=O)OR^{c4}, -OC(=O)NR^{a4}R^{b4}, -SR^{a4}, -S(=O)R^{c4}, -S(=O)₂R^{c4}, -S(=O)NR^{a4}R^{b4}, -S(=O)₂NR^{a4}R^{b4}, -S(=O)(=NR^{d4})R^{c4}, -NR^{a4}R^{b4}, -NR^{a4}C(=O)R^{b4}, -NR^{a4}C(=O)OR^{c4}, -NR^{e4}C(=O)NR^{a4}R^{b4}, -NR^{e4}C(=NR^{d4})NR^{a4}R^{b4}, -NR^{a4}S(=O)₂R^{c4} and -NR^{e4}S(=O)₂NR^{a4}R^{b4};
2) C₁₋₆ alkyl, C₂₋₆ alkenyl, C₂₋₆ alkynyl, phenyl, 5- to 10-membered heteroaryl, C₃₋₁₂ cycloalkyl and 3- to 12-membered aliphatic heterocyclyl, optionally substituted with 0, 1, 2, 3, 4, 5 or 6 of R⁴²;
or, two R⁴ groups attached to two ring-forming atoms of the ring L¹, taken together with the two ring-forming atoms to which they are attached, form a C₅₋₁₂ aliphatic carbocyclyl, or 5-to 12-membered aliphatic heterocyclyl, optionally substituted with 0, 1, 2, 3, 4, 5 or 6 of R⁴²,
R^{a4}, R^{b4} and R^{e4} are each independently selected from the group consisting of:
1) hydrogen;
2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4, 5 or 6 of R⁴²;
or, R^{a4} and R^{b4} attached to the same nitrogen atom, taken together with the nitrogen atom to which they are attached, form a 3- to 12-membered aliphatic heterocyclyl, optionally substituted with 0, 1, 2, 3, 4 or 5 of R⁴²;
R^{c4} and R^{f4} are each independently selected from the group consisting of C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4 or 5 of R⁴²;
R^{d4} is selected from the group consisting of:
1) hydrogen, cyano, nitro and -S(=O)₂R^{G};
2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3, 4 or 5 of R⁴²;
R⁴² is selected from the group consisting of:
1) oxo, halogen, cyano, -C(=O)R^{a42}, -C(=O)OR^{a42}, -C(=O)NR^{a42}R^{b42}, -C(=NR^{d42})NR^{a42}R^{b42}, -OR^{a42}, -OC(=O)R^{a42}, -OC(=O)OR^{c42}, -OC(=O)NR^{a42}R^{b42}, -SR^{a42}, -S(=O)R^{c42}, -S(=O)₂R^{c42}, -S(=O)NR^{a42}R^{b42}, -S(=O)₂NR^{a42}R^{b42}, -S(=O)(=NR^{d42})R^{c42}, -NR^{a42}R^{b42}, -NR^{a42}C(=O)R^{b42}, -NR^{a42}C(=O)OR^{c42}, -NR^{e42}C(=O)NR^{a42}R^{b42}, -NR^{e42}C(=NR^{d42})NR^{a42}R^{b42}, -NR^{a42}S(=O)₂R^{c42}, -NR^{e42}S(=O)₂NR^{a42}R^{b42} and =N-R^{d42};
2) C₁₋₆ alkyl, C₁₋₆ alkylene, C₂₋₆ alkenyl, C₂₋₆ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R⁴³;
R^{a42}, R^{b42} and R^{e42} are each independently selected from the group consisting of:
1) hydrogen;
2) C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R⁴³;
or, R^{a42} and R^{b42} attached to the same nitrogen atom, taken together with the nitrogen atom to which they are attached, form a 3- to 10-membered aliphatic heterocyclyl, optionally substituted with 0, 1, 2, 3, 4 or 5 of R⁴³;
R^{c42} and R^{f42} are each independently selected from the group consisting of C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R⁴³;
R^{d42} is selected from the group consisting of:
1) hydrogen, cyano, nitro and -S(=O)₂R^{G};
2) C₁₋₄ alkyl, C₂₋₄ alkenyl, C₂₋₄ alkynyl, phenyl, 5- to 6-membered heteroaryl, C₃₋₁₀ cycloalkyl and 3- to 10-membered aliphatic heterocyclyl, optionally substituted with 0, 1, 2, 3, 4 or 5 of R⁴³;
R⁴³ is selected from the group consisting of:
1) oxo, halogen, cyano, -C(=O)R^{a43}, -C(=O)OR^{a43}, -C(=O)NR^{a43}R^{b43}, -C(=NR^{d43})NR^{a43}R^{b43}, -OR^{a43}, -OC(=O)R^{a43}, -OC(=O)OR^{c43}, -OC(=O)NR^{a43}R^{b43}, -SR^{a43}, -S(=O)R^{c43}, -S(=O)₂R^{c43}, -S(=O)NR^{a43}R^{b43}, -S(=O)₂NR^{a43}R^{b43}, -S(=O)(=NR^{d43})R^{c43}, -NR^{a43}R^{b43}, -NR^{a43}C(=O)R^{b43}, -NR^{a43}C(=O)OR^{c43}, -NR^{e43}C(=O)NR^{a43}R^{b43}, -NR^{e43}C(=NR^{d43})NR^{a43}R^{b43}, -NR^{a43}S(=O)₂R^{c4}₃, -NR^{e43}S(=O)₂NR^{a43}R^{b43} and =N-R^{d43};
2) C₁₋₄ alkyl, C₁₋₄ alkylene, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
R^{a43}, R^{b43} and R^{e43} are each independently selected from the group consisting of:
1) hydrogen;
2) C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
or, R^{a43} and R^{b43} attached to the same nitrogen atom, taken together with the nitrogen atom to which they are attached, form a 3- to 8-membered aliphatic heterocyclyl, optionally substituted with 0, 1, 2, 3, or 4 of R^{G};
R^{c43} and R^{f43} are each independently selected from the group consisting of C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3, or 4 of R^{G};
R^{d43} is selected from the group consisting of:
1) hydrogen, cyano, nitro and -S(=O)₂R^{G};
2) C₁₋₄ alkyl, C₂₋₄ alkenyl, C₂₋₄ alkynyl, phenyl, 5- to 6-membered heteroaryl, C₃₋₈ cycloalkyl and 3- to 8-membered aliphatic heterocyclyl, optionally substituted with 0, 1, 2, 3 or 4 of R^{G};
L² is each independently selected from the group consisting of -CH₂-, -NH-, -O-, and -S-; wherein, each L² is independently substituted with 0, 1, or 2 of R⁵; two adjacent L² may be connected through single bond, double bond, or triple bond, provided that the chemical valence allows; p of L² connected sequentially form a linker connecting ring A and ring B; wherein the two L² at both ends are attached to ring A and ring B through single bond, respectively;
R⁵ is independently selected from the group consisting of:
1) oxo, halogen, cyano, -C(=O)R^{a5}, -C(=O)OR^{a5}, -C(=O)NR^{a5}R^{b5}, -C(=NR^{d5})NR^{a5}R^{b5}, -OR^{a5}, -OC(=O)R^{a5}, -OC(=O)OR^{c5}, -OC(=O)NR^{a5}R^{b5}, -OP(=O)(R^{G1})₂, -SR^{a5}, -S(=O)R^{c5}, -S(=O)₂R^{c5}, sulfonic acid group, -S(=O)NR^{a5}R^{b5}, -S(=O)₂NR^{a5}R^{b5}, -S(=O)(=NR^{d5})R^{c5}, -NR^{a5}R^{b5}, -NR^{a5}C(=O)R^{b5}, -NR^{a5}C(=O)OR^{c5}, -NR^{e5}C(=O)NR^{a5}R^{b5}, -NR^{e5}C(=NR^{d5})NR^{a5}R^{b5}, -NR^{a5}S(=O)₂R^{c5}, -NR^{e5}S(=O)₂NR^{a5}R^{b5}, -NR^{G2}P(=O)(R^{G1})₂, -P(=O)R^{c5}R^{f5}, -P(=O)(R^{G1})₂ and =N-R^{d5};
2) C₁₋₆ alkyl, C₂₋₆ alkenyl, C₂₋₆ alkynyl, phenyl, 5- to 6-membered heteroaryl, C₃₋₆ cycloalkyl and 3- to 6-membered aliphatic heterocyclyl, optionally substituted with 0, 1, 2, 3 or 4 of R⁵²;
3) two R⁵ groups attached to the same L² or attached to two adjacent L², taken together with the one or two L² atom(s) to which they are attached, form a C₃₋₆ aliphatic carbocyclyl, or 3- to 6-membered aliphatic heterocyclyl, optionally substituted with 0, 1, 2, 3, or 4 of R⁵²;
4) two R⁵ groups attached to two nonadjacent L², taken together with the two L² atoms to which they are attached and all other L² atom(s) between said two L² atoms, form a C₃₋₆ aliphatic carbocyclyl, or 3- to 6-membered aliphatic heterocyclyl, optionally substituted with 0, 1, 2, 3, or 4 of R⁵²,
R^{a5}, R^{b5} and R^{e5} are independently selected from the group consisting of:
1) hydrogen;
2) C₁₋₄ alkyl, C₂₋₄ alkenyl, C₂₋₄ alkynyl, phenyl, 5- to 6-membered heteroaryl, C₃₋₁₂ cycloalkyl and 3- to 12-membered aliphatic heterocyclyl, optionally substituted with 0, 1, 2, 3 or 4 of R⁵²;
or, R^{a5} and R^{b5} attached to the same nitrogen atom, taken together with the nitrogen atom to which they are attached, form a 3- to 6-membered aliphatic heterocyclyl, optionally substituted with 0, 1, 2, 3 or 4 of R⁵²;
R^{c5} and R^{f5} are each independently selected from the group consisting of C₁₋₄ alkyl, C₂₋₄ alkenyl, C₂₋₄ alkynyl, phenyl, 5- to 6-membered heteroaryl, C₃₋₆ cycloalkyl and 3- to 6-membered aliphatic heterocyclyl, optionally substituted with 0, 1, 2, 3 or 4 of R⁵²,
or, R^{c5} and R^{f5} attached to the same phosphine atom, taken together with the phosphine atom to which they are attached, form a 3- to 6-membered aliphatic heterocyclyl, optionally substituted with 0, 1, 2, 3 or 4 of R⁵²;
R^{d5} is selected from the group consisting of:
1) hydrogen, hydroxyl, C₁₋₄ alkoxy, cyano, nitro and -S(=O)₂R^{G};
2) C₁₋₄ alkyl, C₂₋₄ alkenyl, C₂₋₄ alkynyl, phenyl, 5- to 6-membered heteroaryl, C₃₋₆ cycloalkyl and 3- to 6-membered aliphatic heterocyclyl, optionally substituted with 0, 1, 2, 3 or 4 of R⁵²;
R⁵² is selected from the group consisting of:
1) oxo, halogen and cyano;
2) -C(=O)R^{G1}, -C(=O)R^{G2}, -C(=NR^{G3})N(R^{G2})₂, -OC(=O)R^{G2}, -OP(=O)(R^{G1})₂, -S(=O)R^{G2}, -S(=O)₂R^{G2}, sulfonic acid group, -S(=O)N(R^{G2})₂, -S(=O)₂N(R^{G2})₂, -S(=O)(=NR^{G3})R^{G2}, -N(R^{G2})C(=O)R^{G1}, -N(R^{G2})C(=O)R^{G2}, -N(R^{G2})C(=NR^{G3})N(R^{G2})₂, -N(R^{G2})S(=O)₂R^{G2}, -N(R^{G2})S(=O)₂N(R^{G2})₂, -N(R^{G2})P(=O)(R ^{G1})_{2,} -P(=O)(R^{G2})₂, -P(=O)(R^{G1})₂, =N-R^{G3} and R^{G1};
3) R^{G2};
R^{G} is selected from the group consisting of:
1) halogen, oxo, cyano, carboxyl, hydroxyl, C₁₋₄ alkoxy, amino, C₁₋₄ alkylamino, C₁₋₄ alkyl sulfinyl, C₁₋₄ alkyl sulfonyl, and C₁₋₄ alkylaminosulfonyl;
2) C₁₋₄ alkyl, C₁₋₄ alkylene, C₂₋₄ alkenyl, C₂₋₄ alkynyl, C₃₋₆ cycloalkyl and 3- to 8-membered aliphatic heterocyclyl, optionally substituted with 0, 1, 2, 3 or 4 substituents independently selected from the group consisting of: oxo, halogen, hydroxyl, hydroxymethyl, carboxyl, cyano, C₁₋₃ alkoxy, amino, and C₁₋₃ alkylamino;
R^{G1} is selected from the group consisting of -OR^{G2} and -N(R^{G2})₂;
R^{G2} is selected from the group consisting of:
1) hydrogen;
2) C₁₋₄ alkyl, C₁₋₄ alkylene, C₃₋₆ cycloalkyl, 3- to 6-membered aliphatic heterocyclyl and phenyl, optionally substituted with 0, 1, 2 or 3 substituents independently selected from the group consisting of: oxo, halogen, hydroxyl, hydroxymethyl, carboxyl, cyano, C₁₋₃ alkoxy, amino, and C₁₋₃ alkylamino;
3) two R^{G2} attached to the same nitrogen atom, taken together with the nitrogen atom to which they are attached, form a 3- to 6-membered aliphatic heterocyclyl, optionally substituted with 0, 1, 2 or 3 substituents independently selected from the group consisting of: oxo, halogen, hydroxyl, hydroxymethyl, carboxyl, cyano, C₁₋₃ alkoxy, amino, and C₁₋₃ alkylamino;
R^{G3} is selected from the group consisting of: hydrogen, cyano, nitro, -OR^{G2}, -S(=O)₂R^{G2} and R^{G2};
p is selected from the group consisting of 1, 2, 3, 4, 5, 6, 7, 8, 9 and 10;
m is selected from the group consisting of 0, 1, 2, 3, 4, 5 and 6;
n is selected from the group consisting of 0, 1, 2, 3, 4, 5 and 6.

2. The macrocyclic azaindazole compound according to claim 1, wherein,
X is CH; or X is CF; or X is N;
R¹ is hydrogen.

3. The macrocyclic azaindazole compound according to claim 1, wherein,
the compound is represented by Formula (II), 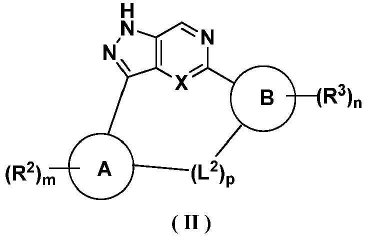 wherein,
ring A is selected from the group consisting of monocyclic or bicyclic 3- to 12-membered carbocyclyl group and 3- to 12-membered heterocyclyl group, and is connected to the azaindazole ring at the 3-position and to L² through two different atoms respectively; in addition to being connected to the azaindazole ring at the 3-position and to L², ring A is optionally substituted with m of R²;
X, ring B, R², R³, L², p, m, and n are as defined in claim 1.

4. The macrocyclic azaindazole compound according to claim 3, wherein,
X is selected from the group consisting of CH and N;
ring A is selected from the group consisting of monocyclic or bicyclic C₆₋₁₂ carbocyclyl group and 5- to 12-membered heterocyclyl group, and is connected to the azaindazole ring at the 3-position and to L² through two different ring-forming atoms respectively, and the single ring directly connected to the azaindazole ring at the 3-position is aromatic ring; wherein,
1) when ring A is selected from the group consisting of phenyl and monocyclic heteroaryl, the shortest distance between the two ring-forming atoms on ring A attached to the azaindazole ring at the 3-position and to L² respectively, is 1, 2 or 3 chemical bonds;
2) when ring A is selected from the group consisting of bicyclic carbocyclyl and bicyclic heterocyclyl, the shortest distance between the two ring-forming atoms on ring A attached to the azaindazole ring at the 3-position and to L² respectively, is 1, 2, 3, 4 or 5 chemical bonds;
ring B is selected from the group consisting of phenyl, naphthyl, 5- to 6-membered monocyclic heteroaryl, and 9- to 10-membered bicyclic heteroaryl, and is connected to L² and to the azaindazole ring at the 5-position through two different ring-forming atoms thereof, respectively:
1) when ring B is selected from phenyl and 5- to 6-membered monocyclic heteroaryl, the shortest distance between said two ring-forming atoms is 1, 2 or 3 chemical bonds;
2) when ring B is selected from naphthyl and 9- to 10-membered bicyclic heteroaryl, the shortest distance between said two ring-forming atoms is 1, 2, 3, 4 or 5 chemical bond;
R² is each independently selected from the group consisting of:
1) halogen, cyano, hydroxyl, C₁₋₄ alkoxy, amino, C₁₋₄ alkylamino, and C₁₋₃ alkyl sulfonyl;
2) C₁₋₄ alkyl, C₂₋₄ alkenyl, C₂₋₄ alkynyl, phenyl, 5- to 6-membered heteroaryl, C₃₋₆ cycloalkyl and 3- to 8-membered aliphatic heterocyclyl, optionally substituted with 0, 1, 2, 3 or 4 of R²²;
3) C₁₋₅ alkylene, 3- to 6-membered oxaalkylene, and 2- to 6-membered azaalkylene, optionally substituted with 0, 1, 2, 3 or 4 of R²².
R²² is each independently selected from the group consisting of:
1) oxo, halogen, cyano, hydroxyl, C₁₋₄ alkoxy, amino, C₁₋₄ alkylamino, C₁₋₃ alkyl sulfonyl, and carboxyl;
2) C₁₋₄ alkyl, C₂₋₄ alkenyl, C₂₋₄ alkynyl, C₃₋₆ cycloalkyl and 3- to 8-membered aliphatic heterocyclyl, optionally substituted with 0, 1, 2, 3 or 4 substituents independently selected from the group consisting of: oxo, halogen, hydroxyl, hydroxy-C₁₋₃ alkyl, C₁₋₃ alkoxy-C₁₋₃ alkyl, cyano, C₁₋₃ alkoxy, amino, C₁₋₃ alkylamino, amino-C₁₋₃ alkyl, and C₁₋₃ alkylamino-C₁₋₃ alkyl;
3) C₁₋₅ alkylene, 3- to 6-membered oxaalkylene, and 2- to 6-membered azaalkylene, optionally substituted with 0, 1, 2, 3 or 4 substituents independently selected from the group consisting of: oxo, halogen, hydroxyl, hydroxy-C₁₋₃ alkyl, C₁₋₃ alkoxy-C₁₋₃ alkyl, cyano, C₁₋₃ alkoxy, amino, C₁₋₃ alkylamino, amino-C₁₋₃ alkyl, and C₁₋₃ alkylamino-C₁₋₃ alkyl;
R³ is independently selected from the group consisting of:
1) halogen, cyano, -C(=O)NR^{a3}R^{b3} and -OR^{a3};
2) C₁₋₃ alkyl, C₂₋₆ alkynyl, phenyl, 5- to 6-membered heteroaryl, C₃₋₆ cycloalkyl and 3- to 6-membered aliphatic heterocyclyl, optionally substituted with 0, 1, 2, 3 or 4 of R³²;
wherein, R^{a3} and R^{b3} are each independently selected from the group consisting of:
1) hydrogen;
2) C₁₋₄ alkyl, C₃₋₆ cycloalkyl and 4- to 6-membered aliphatic heterocyclyl, optionally substituted with 0, 1, 2, 3 or 4 of R³²;
or, R^{a3} and R^{b3} attached to the same nitrogen atom, taken together with the nitrogen atom to which they are attached, form a 4- to 6-membered aliphatic heterocyclyl, optionally substituted with 0, 1, 2, 3 or 4 of R³²;
R³² is each independently selected from the group consisting of:
1) oxo, halogen, cyano, hydroxyl, C₁₋₄ alkoxy, amino, and C₁₋₄ alkylamino;
2) C₁₋₄ alkyl, C₃₋₆ cycloalkyl and 3- to 6-membered aliphatic heterocyclyl, optionally substituted with 0, 1 or 2 substituents independently selected from the group consisting of: oxo, halogen, hydroxyl, hydroxymethyl, cyano, C₁₋₃ alkoxy, amino, and C₁₋₃ alkylamino;
L² is each independently selected from the group consisting of -CH₂-, -NH-, -O-, and -S-; wherein, each L² is independently substituted with 0, 1, or 2 of R⁵; two adjacent L² may be connected through single bond; p of L² connected sequentially form a linker connecting ring A and ring B;
when L² is selected from -CH₂-, the R⁵ thereof is each independently selected from the group consisting of:
1) oxo, halogen, cyano, -C(=O)R^{a5}, carboxyl, -C(=O)NR^{a5}R^{b5}, -OR^{a5}, -NR^{a5}R^{b5}, -NR^{a5}C(=O)R^{b5}, NR^{e5}C(=O)NR^{a5}R^{b5}, -NR^{a5}S(=O)₂R^{c5}, -NR^{e5}S(=O)₂NR^{a5}R^{b5}, and -NR^{G2}P(=O)(R^{G1})₂;
2) C₁₋₄ alkyl, C₃₋₆ cycloalkyl and 3- to 6-membered aliphatic heterocyclyl, optionally substituted with 0, 1, 2, 3 or 4 of R⁵²;
3) two R⁵ groups attached to the same L², taken together with the L² atom to which they are attached, form a cyclic group selected from the group consisting of: 1,1-cyclopropyl, 1,1-cyclobutyl, 1,1-cyclopentyl, 1,1-cyclohexyl, 3,3-oxetanyl, 3,3-azetidinyl, 3,3-tetrahydrofuryl, 3,3-tetrahydropyrrolyl, 3,3-tetrahydropyranyl, 4,4-tetrahydropyranyl, 3,3-piperidinyl and 4,4-piperidyl, optionally substituted with 0, 1, 2, 3, or 4 of R⁵²,
4) two R⁵ groups attached to two adjacent L², taken together with the two L² atoms to which they are attached, form a cyclic group selected from the group consisting of: 1,2-cyclopropyl, 1,2-cyclobutyl, 1,2-cyclopentyl, 1,2-cyclohexyl, 3,4-tetrahydrofuryl, 3,4-tetrahydropyrrolyl, 3,4-tetrahydropyranyl and 3,4-piperidinyl, optionally substituted with 0, 1, 2, 3, or 4 of R⁵²,
5) two R⁵ groups attached to two nonadjacent L², taken together with the two L² atoms to which they are attached and all other L² atom(s) between said two L² atoms, form a cyclic group selected from the group consisting of: 1,3-cyclobutyl, 1,3-cyclopentyl, 1,3-cyclohexyl, 1,4-cyclohexyl, 2,5-tetrahydrofuryl, 2,5-tetrahydropyrrolyl, 2,6-tetrahydropyranyl, 3,5-tetrahydropyranyl, 2,6-piperidinyl, 3,5-piperidinyl, 2,6-morpholinyl, 2,5-morpholinyl, 3,5-morpholinyl, 2,6-piperazinyl and 2,5-piperazinyl, optionally substituted with 0, 1, 2, 3, or 4 of R⁵²;
when L² is selected from -NH-, the R⁵ thereof is each independently selected from the group consisting of:
1) -C(=O)R^{a5}, -C(=O)NR^{a5}R^{b5}, -C(=NR^{d5})NR^{a5}R^{b5}, -S(=O)₂R^{c5}, -S(=O)₂NR^{a5}R^{b5}, -S(=O)(=NR^{d5})R^{c5} and -P(=O)(R^{G1})₂;
2) C₁₋₄ alkyl, C₃₋₆ cycloalkyl and 3- to 6-membered aliphatic heterocyclyl, optionally substituted with 0, 1, 2, 3 or 4 of R⁵²;
3) two R⁵ groups attached to two adjacent L², taken together with the two L² atoms to which they are attached, form a cyclic group selected from the group consisting of: 1,2-aziridinyl, 1,2-azetidinyl, 1,2-tetrahydropyrrolyl, 1,2-piperidinyl, 3,4-morpholinyl and 1,2-piperanyl, optionally substituted with 0, 1, 2, 3, or 4 of R⁵²,
4) two R⁵ groups attached to two nonadjacent L², taken together with the two L² atoms to which they are attached and all other L² atom(s) between said two L² atoms, form a cyclic group selected from the group consisting of: 1,3-azetidinyl, 1,3-tetrahydropyrrolyl, 1,3-piperidinyl, 1,4-piperidinyl and 1,4-piperazinyl, optionally substituted with 0, 1, 2, 3, or 4 of R⁵²;
when L² is selected from -S-, the R⁵ thereof is each independently selected from oxo;
wherein, R^{a5}, R^{b5} and R^{e5} are independently selected from the group consisting of:
1) hydrogen;
2) C₁₋₃ alkyl, C₃₋₆ cycloalkyl, 3-oxetanyl, 3-azetidinyl, tetrahydrofuryl, tetrahydropyrrolyl, tetrahydropyranyl and piperidinyl, optionally substituted with 0, 1, 2, 3 or 4 of R⁵²;
or, R^{a5} and R^{b5} attached to the same nitrogen atom, taken together with the nitrogen atom to which they are attached, form a cyclic group selected from the group consisting of: 1-azetidinyl, 1-tetrahydropyrrolyl, 1-piperidinyl, 1-piperazinyl and morpholinyl, optionally substituted with 0, 1, 2, 3 or 4 of R⁵²;
R^{c5} is each independently selected from the group consisting of C₁₋₃ alkyl, C₃₋₆ cycloalkyl, 3-oxetanyl, 3-azetidinyl, tetrahydrofuryl, tetrahydropyrrolyl, tetrahydropyranyl and piperidinyl, optionally substituted with 0, 1, 2, 3 or 4 of R⁵²,
R^{d5} is selected from the group consisting of:
1) hydrogen, cyano, nitro and -S(=O)₂-(C₁₋₃ alkyl);
2) C₁₋₃ alkyl and cyclopropyl;
R⁵² is selected from the group consisting of: oxo, fluorine, cyano, hydroxyl, C₁₋₃ alkoxy, cyclopropoxy, C₁₋₃ alkyl, cyclopropyl, difluoromethyl, trifluoromethyl, hydroxymethyl, 1-hydroxyethyl, 2-hydroxyethyl, 2-hydroxy-2-propyl, amino and C₁₋₃ alkylamino;
R^{G1} is selected from the group consisting of -OR^{G2} and -N(R^{G2})₂;
R^{G2} is selected from the group consisting of:
1) hydrogen;
2) C₁₋₄ alkyl, C₁₋₄ alkylene, C₃₋₆ cycloalkyl, 3- to 6-membered aliphatic heterocyclyl and phenyl, optionally substituted with 0, 1, 2 or 3 substituents independently selected from the group consisting of: oxo, halogen, hydroxyl, hydroxymethyl, carboxyl, cyano, C₁₋₃ alkoxy, amino, and C₁₋₃ alkylamino;
3) two R^{G2} attached to the same nitrogen atom, taken together with the nitrogen atom to which they are attached, form a 3- to 6-membered aliphatic heterocyclyl, optionally substituted with 0, 1, 2 or 3 substituents independently selected from the group consisting of: oxo, halogen, hydroxyl, hydroxymethyl, carboxyl, cyano, C₁₋₃ alkoxy, amino, and C₁₋₃ alkylamino;
R^{G3} is selected from the group consisting of: hydrogen, cyano, nitro, -OR^{G2}, -S(=O)₂R^{G2} and R^{G2},
m is selected from the group consisting of 0, 1, 2, 3, 4, 5 and 6;
n is selected from the group consisting of 0, 1, 2, 3, 4, 5 and 6;
p is selected from the group consisting of 2, 3, 4, 5, 6, 7, 8 and 9.

5. The macrocyclic azaindazole compound according to claim 4, wherein, the compound is represented by Formula (IIa), 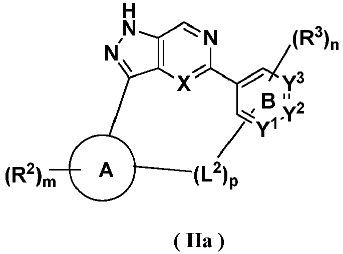
X is selected from the group consisting of CH and N;
ring A is selected from the group consisting of phenyl and 5- to 6-membered heteroaryl, and is connected to the azaindazole ring at the 3-position and to L² through two different ring-forming atoms respectively; in addition to being connected to the azaindazole ring at the 3-position and to L², ring A is optionally substituted with m of R²;
ring B is connected to L² and to the azaindazole ring at the 5-position through two different ring-forming atoms respectively; wherein, the shortest distance between said two ring-forming atoms is 1, 2 or 3 chemical bonds; wherein, Y¹, Y² and Y³ are each independently selected from the group consisting of CR³ and N, provided that no more than two of Y¹, Y² and Y³ is selected from N;
m is selected from the group consisting of 0, 1, 2, 3 and 4;
n is selected from the group consisting of 0, 1, 2 and 3;
R², R³, L² and p are as defined in claim 4.

6. The macrocyclic azaindazole compound according to claim 4, wherein, the compound is represented by Formula (IIb), 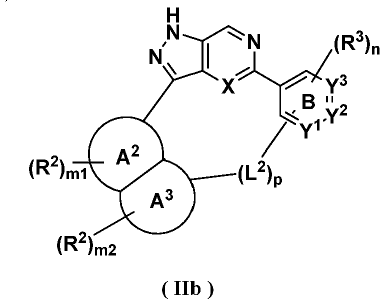 wherein,
X is selected from the group consisting of CH and N;
ring A² and ring A³ form a fused ring system, wherein: ring A² is selected from the group consisting of phenyl and 5- to 6-membered heteroaryl, and is connected to the azaindazole ring at the 3-position through a ring-forming atom thereof; ring A³ is selected from the group consisting of C₅, C₆, and C₇ monocyclic carbocyclyl and 5-, 6- and 7-membered monocyclic heterocyclyl, and is connected to L² through a ring-forming atom thereof; and in addition to being connected to the azaindazole ring at the 3-position, ring A² is optionally substituted with m1 of R²; and in addition to being connected to L², ring A³ is unsubstituted or optionally substituted with m2 of R²;
ring B is connected to L² and to the azaindazole ring at the 5-position through two different ring-forming atoms respectively; wherein, the shortest distance between said two ring-forming atoms is 1, 2 or 3 chemical bonds; wherein, Y¹, Y² and Y³ are each independently selected from the group consisting of CR³ and N, provided that no more than two of Y¹, Y² and Y³ is selected from N;
m1 and m2 is selected from integers between 0 and 4; the sum of m1 and m2 is m, and m is selected from the group consisting of 0, 1, 2, 3 and 4;
n is selected from the group consisting of 0, 1, 2 and 3;
R², R³, L² and p are as defined in claim 4.

7. The macrocyclic azaindazole compound according to claim 4, wherein, the compound is represented by Formula (IIc), 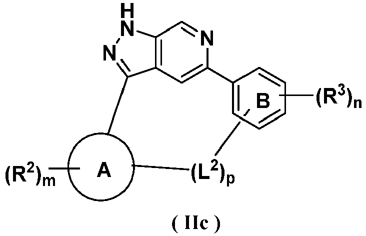 wherein, ring A, R², R³, L², p, m, and n are as defined in claim 4.

8. The macrocyclic azaindazole compound according to any one of claims 3-5 and claim 7, wherein, ring A is selected from one of the following structures: 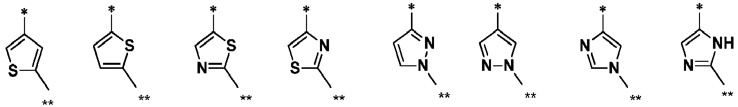 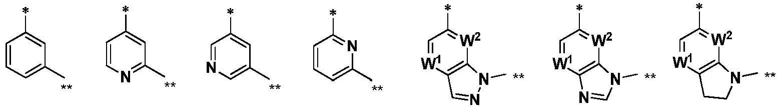 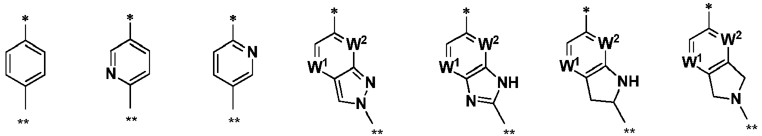 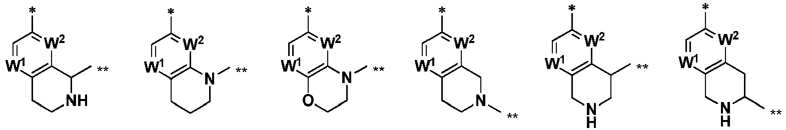 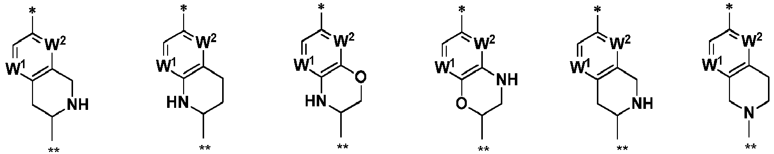 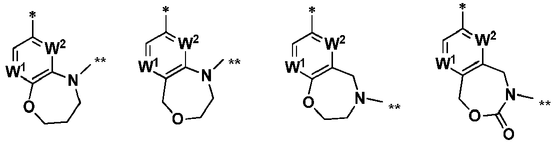 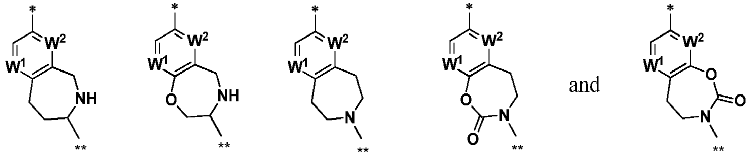
wherein, the "*" at the end of the chemical bond in each structure means that the structure is connected to the azaindazole ring at the 3-position through the bond, the "**" at the end of the chemical bond in each structure means that the structure is connected to L² through the bond;
W¹ and W² are each independently selected from the group consisting of CH and N;
the ring-forming carbon atom(s) and/or ring-forming nitrogen atom(s) in these formulas is optionally substituted with 0, 1, 2, 3 or 4 of R².

9. The macrocyclic azaindazole compound according to any one of claims 3-5 and claim 7, wherein, ring A is selected from one of the following structures: 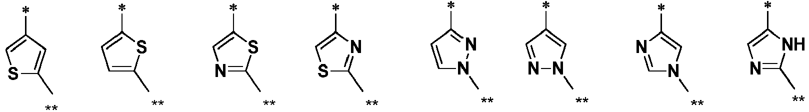 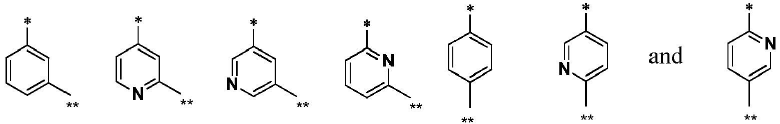
wherein, the "*" at the end of the chemical bond in each structure means that the structure is connected to the azaindazole ring at the 3-position through the bond, the "**" at the end of the chemical bond in each structure means that the structure is connected to L² through the bond;
the ring-forming carbon atom(s) in these formulas is optionally substituted with 0, 1 or 2 of R².

10. The macrocyclic azaindazole compound according to claim 6, wherein, the fused ring system formed of ring A² and ring A³ is selected from one of the following structures: 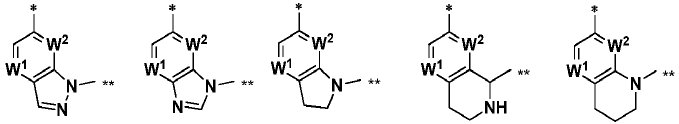 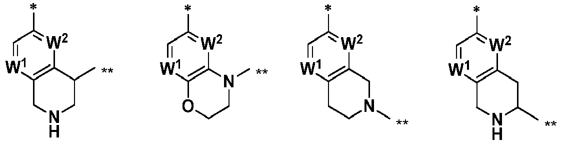 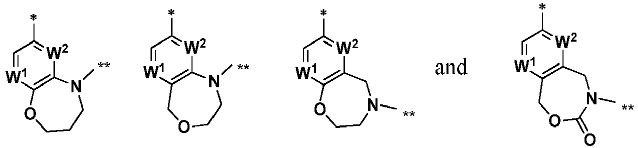
wherein, the "*" at the end of the chemical bond in each structure means that the structure is connected to the azaindazole ring at the 3-position through the bond, the "**" at the end of the chemical bond in each structure means that the structure is connected to L² through the bond;
W¹ and W² are each independently selected from the group consisting of CH and N;
the ring-forming carbon atom(s) and/or ring-forming nitrogen atom(s) in these formula is optionally substituted with 0, 1, 2, 3 or 4 of R².

11. The macrocyclic azaindazole compound according to any one of claims 1-10, wherein, ring B is selected from the group consisting of phenyl and 6-membered heteroaryl.

12. The macrocyclic azaindazole compound according to any one of claims 1-10, wherein,
R² is each independently selected from the group consisting of:
1) oxo, fluorine, chlorine, cyano, hydroxyl, methoxy, ethoxy, isopropoxy, cyclopropoxy, methyl, ethyl, 1-propyl, isopropyl, cyclopropyl, difluoromethyl, trifluoromethyl, hydroxymethyl, 1-hydroxyethyl, 2-hydroxyethyl, 2-hydroxy-2-propyl, 2-amino-2-propyl, N-methyl-2-amino-2-propyl, N,N-dimethyl-2-amino-2-propyl, and carboxyl;
2) one of the following aliphatic heterocyclyl, wherein the "*" at the end of the chemical bond in each structure means that the structure is connected to the ring A through the bond: 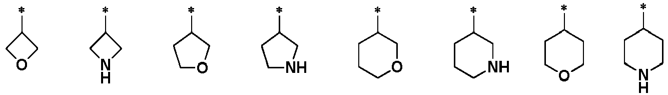 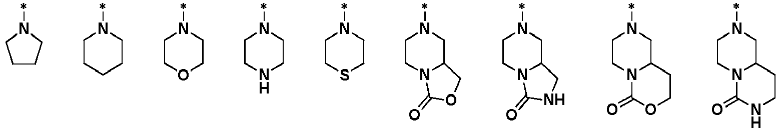 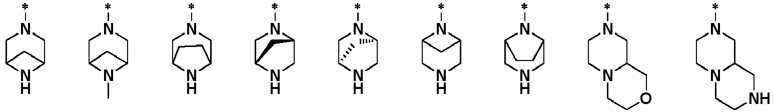 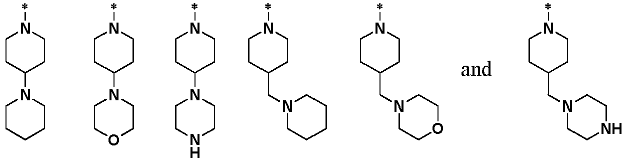 wherein, said aliphatic heterocyclyl is optionally substituted with 0, 1, 2 or 3 of substituent R²²¹ independently selected from the group consisting of: oxo, fluorine, chlorine, cyano, hydroxyl, C₁-C₆ alkoxy, C₁-C₆ alkylamino, C₁-C₆ alkyl, C₁-C₆ fluoroalkyl, C₁-C₆ hydroxyalkyl, C₁-C₆ aminoalkyl, C₃-C₆ cycloalkyl, C₃-C₆ oxacycloalkyl, -C(=O)-(C₁-C₆ alkyl), and -S(=O)₂-(C₁-C₆ alkyl);
3) ethylene, propylene, butylene, pentylene, oxapropylene, oxabutylene, oxapentylene, azapropylene, azabutylene and azapentylene, optionally substituted with 0, 1 or 2 substituents selected from the group consisting of fluorine, hydroxyl and methyl;
4) phenyl, pyridyl, pyrimidinyl, imidazolyl, pyrazolyl and thiazolyl, optionally substituted with 0, 1, 2 or 3 substituents independently selected from the group consisting of: oxo, fluorine, chlorine, cyano, hydroxyl, C₁-C₆ alkoxy, C₁-C₆ alkyl, C₁-C₆ fluoroalkyl, C₁-C₆ hydroxyalkyl, C₁-C₆ aminoalkyl, C₃-C₆ cycloalkyl, and C₃-C₆ oxacycloalkyl;
m is selected from the group consisting of 0, 1 and 2.

13. The macrocyclic azaindazole compound according to claim 1, wherein, the compound is represented by Formula (IIIa), 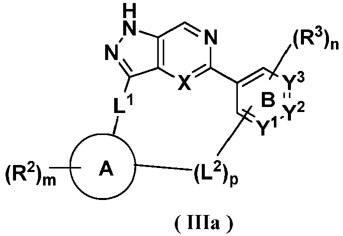 wherein,
X is selected from the group consisting of CH and N;
L¹ is selected from the group consisting of: phenyl, 5- to 6-memebred heteroaryl, and 9- to 10-memebred bicyclic heteroaryl; said aryl or heteroaryl group is connected to the azaindazole ring at the 3-position and to the ring A through two nonadjacent ring-forming atoms respectively; in addition to being connected to the azaindazole ring and to the ring A, said aryl and heteroaryl group is optionally substituted with 0, 1, 2, 3, 4, 5 or 6 of R⁴;
ring A is selected from the group consisting of monocyclic or bicyclic C₅, C₆, C₇, C₈, and C₉ aliphatic carbocyclyl group and 5-, 6-, 7-, 8-, and 9-membered aliphatic heterocyclyl group, and is connected to L¹ and L² through two different atoms thereof, respectively; said monocyclic or bicyclic structure does not contain aromatic ring;
ring B is connected to L² and to the azaindazole ring at the 5-position through two different ring-forming atoms respectively; wherein, the shortest distance between the two ring-forming atoms thereof, is 1, 2 or 3 chemical bonds; wherein, Y¹, Y² and Y³ are each independently selected from the group consisting of CR³ and N, provided that no more than two of Y¹, Y² and Y³ is selected from N;
R² is each independently selected from the group consisting of:
1) oxo, halogen, cyano, hydroxyl, C₁₋₄ alkoxy, amino, C₁₋₄ alkylamino, and C₁₋₃ alkyl sulfonyl;
2) C₁₋₄ alkyl, C₁₋₅ alkylene, 3- to 6-membered oxaalkylene, 2- to 6-membered azaalkylene, C₂₋₄ alkenyl, C₂₋₄ alkynyl, C₃₋₆ cycloalkyl and 3- to 8-membered aliphatic heterocyclyl, optionally substituted with 0, 1, 2, 3 or 4 substituents independently selected from the group consisting of: oxo, halogen, hydroxyl, hydroxy-C₁₋₃ alkyl, C₁₋₃ alkoxy-C₁₋₃ alkyl, cyano, C₁₋₃ alkoxy, amino, C₁₋₃ alkylamino, amino-C₁₋₃ alkyl, and C₁₋₃ alkylamino-C₁₋₃ alkyl;
R⁴ is each independently selected from the group consisting of:
1) halogen, cyano, hydroxyl, C₁₋₄ alkoxy, amino, C₁₋₄ alkylamino, and C₁₋₃ alkyl sulfonyl;
2) C₁₋₄ alkyl, C₂₋₄ alkenyl, C₂₋₄ alkynyl, C₃₋₆ cycloalkyl and 3- to 8-membered aliphatic heterocyclyl, optionally substituted with 0, 1, 2, 3 or 4 substituents independently selected from the group consisting of: oxo, halogen, hydroxyl, hydroxy-C₁₋₃ alkyl, C₁₋₃ alkoxy-C₁₋₃ alkyl, cyano, C₁₋₃ alkoxy, amino, C₁₋₃ alkylamino, amino-C₁₋₃ alkyl, and C₁₋₃ alkylamino-C₁₋₃ alkyl;
3) phenyl and 5- to 6-membered heteroaryl, optionally substituted with 0, 1, 2 or 3 of R⁴²,
wherein, R⁴² is each independently selected from the group consisting of:
1) oxo, halogen, cyano, hydroxyl, C₁₋₃ alkoxy, amino, C₁₋₃ alkylamino, and C₁₋₃ alkyl sulfonyl;
2) C₁₋₃ alkyl, C₂₋₄ alkynyl, C₃₋₆ cycloalkyl and 3- to 6-membered aliphatic heterocyclyl, optionally substituted with 0, 1, 2, or 3 substituents independently selected from the group consisting of: oxo, halogen, hydroxyl, hydroxy-C₁₋₃ alkyl, C₁₋₃ alkoxy-C₁₋₃ alkyl, cyano, C₁₋₃ alkoxy, amino, C₁₋₃ alkylamino, amino-C₁₋₃ alkyl, and C₁₋₃ alkylamino-C₁₋₃ alkyl;
L² is each independently selected from the group consisting of -CH₂-, -NH-, -O-, and -S-; wherein, each L² is independently substituted with 0, 1, or 2 of R⁵; two adjacent L² may be connected through single bond; p of L² connected sequentially form a linker connecting ring A and ring B;
when L² is selected from -CH₂-, the R⁵ thereof is each independently selected from the group consisting of:
1) oxo, halogen, cyano, -C(=O)R^{a5}, carboxyl, -C(=O)NR^{a5}R^{b5}, -OR^{a5}, -NR^{a5}R^{b5}, -NR^{a5}C(=O)R^{b5}, -NR^{e5}C(=O)NR^{a5}R^{b5}, -NR^{a5}S(=O)₂R^{c5}, -NR^{e5}S(=O)₂NR^{a5}R^{b5}, and -NR^{G2}P(=O)(R^{G1})₂;
2) C₁₋₄ alkyl, C₃₋₆ cycloalkyl and 3- to 6-membered aliphatic heterocyclyl, optionally substituted with 0, 1, 2, 3 or 4 of R⁵²;
3) two R⁵ groups attached to the same L², taken together with the L² atom to which they are attached, form a cyclic group selected from the group consisting of: 1,1-cyclopropyl, 1,1-cyclobutyl, 1,1-cyclopentyl, 1,1-cyclohexyl, 3,3-oxetanyl, 3,3-azetidinyl, 3,3-tetrahydrofuryl, 3,3-tetrahydropyrrolyl, 3,3-tetrahydropyranyl, 4,4-tetrahydropyranyl, 3,3-piperidinyl and 4,4-piperidyl, optionally substituted with 0, 1, 2, 3, or 4 of R⁵²,
4) two R⁵ groups attached to two adjacent L², taken together with the two L² atoms to which they are attached, form a cyclic group selected from the group consisting of: 1,2-cyclopropyl, 1,2-cyclobutyl, 1,2-cyclopentyl, 1,2-cyclohexyl, 3,4-tetrahydrofuryl, 3,4-tetrahydropyrrolyl, 3,4-tetrahydropyranyl and 3,4-piperidinyl, optionally substituted with 0, 1, 2, 3, or 4 of R⁵²,
5) two R⁵ groups attached to two nonadjacent L², taken together with the two L² atoms to which they are attached and all other L² atom(s) between said two L² atoms, form a cyclic group selected from the group consisting of: 1,3-cyclobutyl, 1,3-cyclopentyl, 1,3-cyclohexyl, 1,4-cyclohexyl, 2,5-tetrahydrofuryl, 2,5-tetrahydropyrrolyl, 2,6-tetrahydropyranyl, 3,5-tetrahydropyranyl, 2,6-piperidinyl, 3,5-piperidinyl, 2,6-morpholinyl, 2,5-morpholinyl, 3,5-morpholinyl, 2,6-piperazinyl and 2,5-piperazinyl, optionally substituted with 0, 1, 2, 3, or 4 of R⁵²;
when L² is selected from -NH-, the R⁵ thereof is each independently selected from the group consisting of:
1) -C(=O)R^{a5}, -C(=O)NR^{a5}R^{b5}, -C(=NR^{d5})NR^{a5}R^{b5}, -S(=O)₂R^{c5}, -S(=O)₂NR^{a5}R^{b5}, -S(=O)(=NR^{d5})R^{c5} and -P(=O)(R^{G1})₂;
2) C₁₋₄ alkyl, C₃₋₆ cycloalkyl and 3- to 6-membered aliphatic heterocyclyl, optionally substituted with 0, 1, 2, 3 or 4 of R⁵²;
3) two R⁵ groups attached to two adjacent L², taken together with the two L² atoms to which they are attached, form a cyclic group selected from the group consisting of: 1,2-aziridinyl, 1,2-azetidinyl, 1,2-tetrahydropyrrolyl, 1,2-piperidinyl, 3,4-morpholinyl and 1,2-piperanyl, optionally substituted with 0, 1, 2, 3, or 4 of R⁵²,
4) two R⁵ groups attached to two nonadjacent L², taken together with the two L² atoms to which they are attached and all other L² atom(s) between said two L² atoms, form a cyclic group selected from the group consisting of: 1,3-azetidinyl, 1,3-tetrahydropyrrolyl, 1,3-piperidinyl, 1,4-piperidinyl and 1,4-piperazinyl, optionally substituted with 0, 1, 2, 3, or 4 of R⁵²;
when L² is selected from -S-, the R⁵ thereof is each independently selected from oxo;
wherein, R^{a5}, R^{b5} and R^{e5} are independently selected from the group consisting of:
1) hydrogen;
2) C₁₋₃ alkyl, C₃₋₆ cycloalkyl, 3-oxetanyl, 3-azetidinyl, tetrahydrofuryl, tetrahydropyrrolyl, tetrahydropyranyl and piperidinyl, optionally substituted with 0, 1, 2, 3 or 4 of R⁵²;
or, R^{a5} and R^{b5} attached to the same nitrogen atom, taken together with the nitrogen atom to which they are attached, form a cyclic group selected from the group consisting of: 1-azetidinyl, 1-tetrahydropyrrolyl, 1-piperidinyl, 1-piperazinyl and morpholinyl, optionally substituted with 0, 1, 2, 3 or 4 of R⁵²;
R^{c5} is each independently selected from the group consisting of C₁₋₃ alkyl, C₃₋₆ cycloalkyl, 3-oxetanyl, 3-azetidinyl, tetrahydrofuryl, tetrahydropyrrolyl, tetrahydropyranyl and piperidinyl, optionally substituted with 0, 1, 2, 3 or 4 of R⁵²,
R^{d5} is selected from the group consisting of:
1) hydrogen, cyano, nitro and -S(=O)₂-(C₁₋₃ alkyl);
2) C₁₋₃ alkyl and cyclopropyl;
R⁵² is selected from the group consisting of: oxo, fluorine, cyano, hydroxyl, C₁₋₃ alkoxy, cyclopropoxy, C₁₋₃ alkyl, cyclopropyl, difluoromethyl, trifluoromethyl, hydroxymethyl, 1-hydroxyethyl, 2-hydroxyethyl, 2-hydroxy-2-propyl, amino and C₁₋₃ alkylamino;
R^{G1} is selected from the group consisting of -OR^{G2} and -N(R^{G2})₂;
R^{G2} is selected from the group consisting of:
1) hydrogen;
2) C₁₋₄ alkyl, C₁₋₄ alkylene, C₃₋₆ cycloalkyl, 3- to 6-membered aliphatic heterocyclyl and phenyl, optionally substituted with 0, 1, 2 or 3 substituents independently selected from the group consisting of: oxo, halogen, hydroxyl, hydroxymethyl, carboxyl, cyano, C₁₋₃ alkoxy, amino, and C₁₋₃ alkylamino;
3) two R^{G2} attached to the same nitrogen atom, taken together with the nitrogen atom to which they are attached, form a 3- to 6-membered aliphatic heterocyclyl, optionally substituted with 0, 1, 2 or 3 substituents independently selected from the group consisting of: oxo, halogen, hydroxyl, hydroxymethyl, carboxyl, cyano, C₁₋₃ alkoxy, amino, and C₁₋₃ alkylamino;
R^{G3} is selected from the group consisting of: hydrogen, cyano, nitro, -OR^{G2}, -S(=O)₂R^{G2} and R^{G2},
m is selected from the group consisting of 0, 1, 2, 3, 4, 5 and 6;
p is selected from the group consisting of 2, 3, 4, 5, 6, 7, 8 and 9;
n is selected from the group consisting of 0, 1, 2 and 3;
R³ is as defined in claim 1.

14. The macrocyclic azaindazole compound according to any one of claims 1 and 13, wherein,
L¹ is selected from the group consisting of:
1) phenyl, thienyl and pyridyl;
2) 5- and 6-membered monocyclic heteroaryl containing two ring-forming heteroatoms, wherein one of the ring-forming heteroatom is selected from nitrogen, and the other ring-forming heteroatom is selected from the group consisting of nitrogen, oxygen and sulfur; wherein, L¹ is connected to the azaindazole ring at the 3-position and to the ring A through two nonadjacent ring-forming atoms respectively; in addition to being connected to the azaindazole ring and to the ring A, L¹ is optionally substituted with 0, 1 or 2 of R⁴;
3) quinolyl, isoquinolyl, indolyl, indazolyl, benzimidazolyl, benzothiazolyl and benzisothiazolyl; wherein, L¹ is connected to the azaindazole ring at the 3-position and to the ring A through two nonadjacent ring-forming atoms respectively, said two ring-forming atoms belong to the benzene ring; in addition to being connected to the azaindazole ring and to the ring A, L¹ is optionally substituted with 0, 1, 2 or 3 of R⁴.

15. The macrocyclic azaindazole compound according to claim 14, wherein,
L¹ is selected from one of the following structures; wherein, the "*" at the end of the chemical bond in each structure means that the structure is connected to the azaindazole ring at the 3-position through the bond, the "**" at the end of the chemical bond in each structure means that the structure is connected to ring A through the bond: 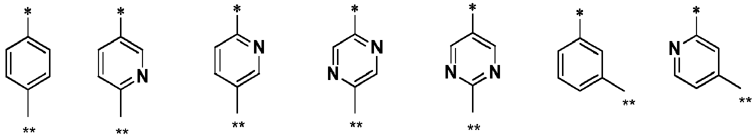 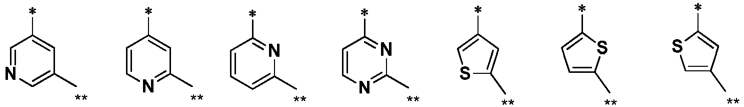 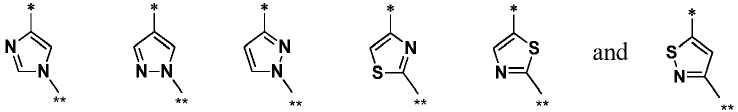
and, in addition to being connected to the azaindazole ring and to the ring A, L¹ is optionally substituted with 0, 1 or 2 of R⁴.

16. The macrocyclic azaindazole compound according to any one of claims 14-15, wherein,
ring A is selected from one of the following structures; the "*" at the end of the chemical bond in each structure means that the structure is connected to L¹ through the bond, the "**" at the end of the chemical bond in each structure means that the structure is connected to L² through the bond: 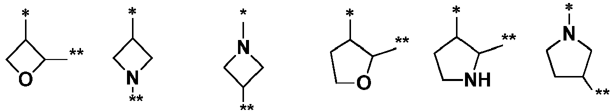 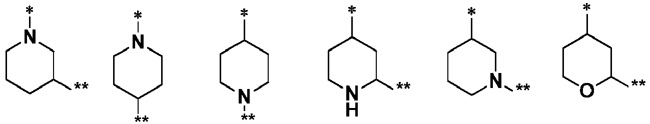 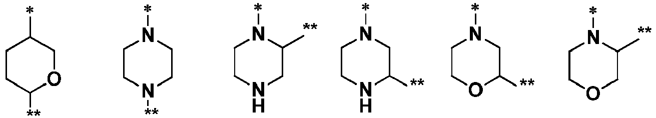 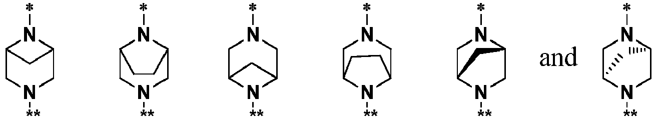
and, in addition to being connected to L¹ and L², ring A is optionally substituted with 0, 1 or 2 of R².

17. The macrocyclic azaindazole compound according to any of claim 1 and claims 13-16, wherein,
R² is independently selected from the group consisting of:
1) oxo, fluorine, chlorine, cyano, hydroxyl, methoxy, ethoxy, isopropoxy, cyclopropoxy, methyl, ethyl, 1-propyl, isopropyl, cyclopropyl, difluoromethyl, trifluoromethyl, hydroxymethyl, 1-hydroxyethyl, 2-hydroxyethyl, 2-hydroxy-2-propyl, 2-amino-2-propyl, *N*-methyl-2-amino-2-propyl, *N*,N-dimethyl-2-amino-2-propyl, and carboxyl;
2) cyclopropyl, cyclobutyl, cyclopentyl, cyclohexyl, oxetanyl, tetrahydrofuryl, tetrahydropyranyl, azetidinyl, tetrahydropyrrolyl, piperidinyl, piperazinyl, and morpholinyl, optionally substituted with 0, 1, 2 or 3 of substituent R²²¹ independently selected from the group consisting of: oxo, fluorine, chlorine, cyano, hydroxyl, C₁-C₆ alkoxy, C₁-C₆ alkylamino, C₁-C₆ alkyl, C₁-C₆ fluoroalkyl, C₁-C₆ hydroxyalkyl, C₁-C₆ aminoalkyl, C₃-C₆ cycloalkyl, and C₃-C₆ oxacycloalkyl;
3) ethylene, propylene, butylene, pentylene, oxapropylene, oxabutylene, oxapentylene, azapropylene, azabutylene or azapentylene, optionally substituted with 0, 1 or 2 of substituents selected from the group consisting of fluorine, hydroxy and methyl;
m is selected from the group consisting of 0, 1 and 2.

18. The macrocyclic azaindazole compound according to claim 17, wherein,
R²²¹ is each independently selected from the group consisting of: oxo, fluorine, chlorine, cyano, hydroxyl, methoxy, ethoxy, isopropoxy, cyclopropoxy, dimethylamino, methyl, ethyl, 1-propyl, isopropyl, cyclopropyl, difluoromethyl, trifluoromethyl, hydroxymethyl, 1-hydroxyethyl, 2-hydroxyethyl, 2-hydroxy-2-propyl, 2-amino-2-propyl, *N*-methyl-2-amino-2-propyl, *N,N*-dimethyl-2-amino-2-propyl, oxetanyl and tetrahydropyranyl.

19. The macrocyclic azaindazole compound according to any one of claims 1-18, wherein,
R³ is each independently selected from the group consisting of: fluorine, chlorine, cyano, C₁₋₃ alkoxy, difluoromethoxy, trifluoromethoxy, cyclopropoxy, C₁₋₃ alkyl, difluoromethyl, trifluoromethyl and cyclopropyl, and at least one of R³ is located in the *ortho*-position of the ring-forming atom of ring B connected to the azaindazole ring at the 5-position; said R³ located in the *ortho*-position of the ring-forming atom of ring B connected to the azaindazole ring at the 5-position is selected from the group consisting of fluorine, chlorine, methoxy, methyl and ethyl;
n is selected from the group consisting of 1, 2 and 3.

20. The macrocyclic azaindazole compound according to claim 19, wherein,
R³ is independently selected from the group consisting of: fluorine, chlorine, cyano, methoxy, methyl and ethyl, and at least one of R³ is located in the *ortho*-position of the ring-forming atom of ring B connected to the azaindazole ring at the 5-position; said R³ located in the *ortho*-position of the ring-forming atom of ring B connected to the azaindazole ring at the 5-position is selected from the group consisting of fluorine, chlorine, methoxy, methyl and ethyl;
n is selected from the group consisting of 1 and 2.

21. The macrocyclic azaindazole compound according to claim 20, wherein,
R³ is independently selected from the group consisting of: fluorine, chlorine, cyano and methoxy, and at least one of R³ is located in the *ortho*-position of the ring-forming atom of ring B connected to the azaindazole ring at the 5-position; said R³ located in the *ortho-*position of the ring-forming atom of ring B connected to the azaindazole ring at the 5-position is selected from the group consisting of fluorine, chlorine and methoxy; preferably, said R³ is selected from fluorine;
n is selected from the group consisting of 1 and 2; preferably, n is selected from 1.

22. The macrocyclic azaindazole compound according to claim 21, wherein,
R³ is independently selected from the group consisting of: fluorine and methoxy; and at least one of R³ is located in the *ortho-*position of the ring-forming atom of ring B connected to the azaindazole ring at the 5-position;
n is selected from the group consisting of 1 and 2; preferably, n is selected from 1.

23. The macrocyclic azaindazole compound according to any one of claims 1-22, wherein,
ring B is selected from the group consisting of phenyl and 5- to 6-membered monocyclic heteroaryl, and is connected to L² and to the azaindazole ring at the 5-position through two different ring-forming atoms respectively; wherein the shortest distance between said two ring-forming atoms is 1, 2 or 3 chemical bonds.

24. The macrocyclic azaindazole compound according to any one of claims 1-22, wherein,
ring B is selected from the group consisting of phenyl, and ring B is connected to L² and to the azaindazole ring at the 5-position through two different ring-forming atoms respectively; wherein said two ring-forming atoms are in *ortho-, meta-* or *para*-position relationship.

25. The macrocyclic azaindazole compound according to claim 4, wherein, the compound is represented by Formula (IIIb), 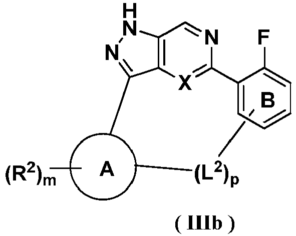 wherein,
X is selected from the group consisting of N and CH;
ring A is selected from the group consisting of phenyl and pyridyl, and is connected to the azaindazole ring at the 3-position and to L² through two different ring-forming atoms respectively; wherein the shortest distance between said two ring-forming atoms is 1 or 2 chemical bonds; preferably, the shortest distance between said two ring-forming atoms is 2 chemical bonds;
ring B is selected from the group consisting of phenyl, and is connected to L² and to the azaindazole ring at the 5-position through two different ring-forming atoms respectively; wherein said two ring-forming atoms are in *ortho-* or *meta*-position relationship; preferably, said two ring-forming atoms are in *ortho*-position relationship;
R² is each independently selected from the group consisting of:
1) fluorine, chlorine, cyano, hydroxyl, methoxy, ethoxy, isopropoxy, cyclopropoxy, methyl, ethyl, 1-propyl, isopropyl, cyclopropyl, difluoromethyl, trifluoromethyl, hydroxymethyl, 1-hydroxyethyl, 2-hydroxyethyl, 2-hydroxy-2-propyl, 2-amino-2-propyl, *N*-methyl-2-amino-2-propyl, and *N,N-*dimethyl-2-amino-2-propyl;
2) one of the following aliphatic heterocyclyl, wherein the "*" at the end of the chemical bond in each structure means that the structure is connected to the ring A through the bond: 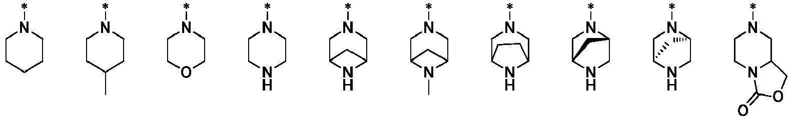 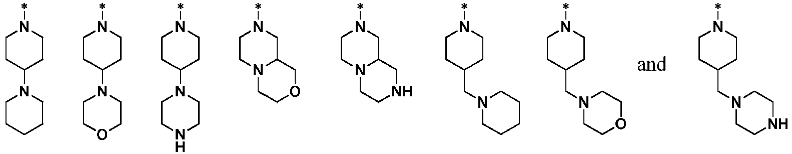
wherein, said aliphatic heterocyclyl is optionally substituted with 0, 1, 2 or 3 of substituent R²²¹ independently selected from the group consisting of:
oxo, fluorine, chlorine, cyano, hydroxyl, methoxy, ethoxy, isopropoxy, cyclopropoxy, dimethylamino, methyl, ethyl, 1-propyl, isopropyl, cyclopropyl, difluoromethyl, trifluoromethyl, hydroxymethyl, 1-hydroxyethyl, 2-hydroxyethyl, 2-hydroxy-2-propyl, 2-amino-2-propyl, *N*-methyl-2-amino-2-propyl, *N,N*-dimethyl-2-amino-2-propyl, oxetanyl and tetrahydropyranyl;
L², p, and m are as defined in claim 4.

26. The macrocyclic azaindazole compound according to any one of claims 1-25, wherein,
L² is each independently selected from the group consisting of -CH₂-, -NH-, -O-, and -S-; wherein, each L² is independently substituted with 0, 1, or 2 of R⁵; two adjacent L² may be connected through single bond; p of L² connected sequentially form a linker connecting ring A and ring B;
R⁵ is independently selected from the group consisting of:
1) oxo, halogen, cyano, hydroxyl, C₁₋₃ alkoxy, amino, C₁₋₃ alkylamino, and C₁₋₃ alkyl;
2) when L² is selected from carbon, two R⁵ groups attached to the same L², taken together with said L² atom to which they are attached, form C₃, C₄ and C₅ cycloalkylene; wherein said cycloalkylene is optionally substituted with 0, 1, 2, 3, or 4 of R⁵²,
3) when two adjacent L² are selected from the group consisting of carbon and nitrogen, two R⁵ groups attached to said two L², taken together with said two L² atoms to which they are attached, form C₄, C₅ and C₆ cycloalkylene or C₄, C₅ and C₆ heterocyclylene; wherein said cycloalkylene and heterocyclylene is optionally substituted with 0, 1, 2, 3, or 4 of R⁵²,
4) when two nonadjacent L² are selected from the group consisting of carbon and nitrogen, two R⁵ groups attached to said two L², taken together with said two L² atoms to which they are attached, form C₄, C₅ and C₆ cycloalkylene or C₄, C₅ and C₆ heterocyclylene; wherein said cycloalkylene and heterocyclylene is optionally substituted with 0, 1, 2, 3, or 4 of R⁵²,
wherein,
R⁵² is selected from the group consisting of: halogen, cyano, hydroxyl, C₁₋₃ alkoxy and C₁₋₃ alkyl;
p is selected from the group consisting of 2, 3, 4, 5, 6, 7, 8 and 9.

27. The macrocyclic azaindazole compound according to any one of claims 1-26, wherein, X is CH.

28. The macrocyclic azaindazole compound according to claim 1, wherein, the compound is selected from:
| Compound No. | Structure | Name |
|---|---|---|
| Compound 1 | 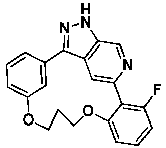 | 16-fluoro-7,11-dioxa-19,22,23-triazapentacyclo[16.5.2.1^{2,6}.0^{12,17}.0^{21,24}]hexacosa -1(23),2(26),3,5,12,14,16,18,20,24-decaene |
| Compound 2 | 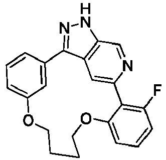 | 17-fluoro-7,12-dioxa-20,23,24-triazapentacyclo[17.5.2.1^{2,6}.0^{13,18}.0^{22,25}]heptacos a-1(24),2(27),3,5,13,15,17,19,21,25-decaene |
| Compound 3 | 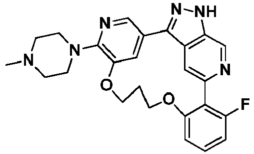 | 16-fluoro-5-(4-methylpiperazin-1 -yl)-7,11-dioxa-4,19,22,23-tetraazapentacyclo[16.5.2.1^{2,6}.0^{12,17}.0^{21,24}]hexac osa-1(23),2(26),3,5, 12, 14, 16, 18,20,24-decaene |
| Compound 4 | 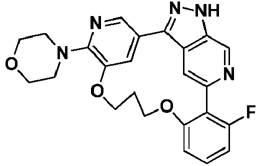 | 16-fluoro-5-(morpholin-4-yl)-7,11-dioxa-4,19,22,23-tetraazapentacyclo[16.5.2.1^{2,6}.0^{12,17}.0^{21,24}]hexac osa-1(23),2(26),3,5, 12, 14, 16, 18,20,24-decaene |
| Compound 5 | 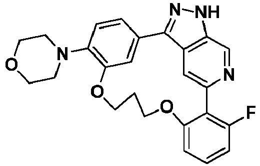 | 16-fluoro-5-(morpholin-4-yl)-7,11-dioxa-19,22,23-triazapentacyclo[16.5.2.1^{2,6}.0^{12,17}.0^{21,24}]hexacosa -1(23),2(26),3,5,12,14,16,18,20,24-decaene |
| Compound 6 | 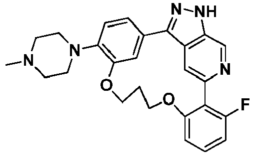 | 16-fluoro-5-(4-methylpiperazin-1 -yl)-7,11-dioxa-19,22,23-triazapentacyclo[16.5.2.1^{2,6}.0^{12,17}.0^{21,24}]hexacosa -1(23),2(26),3,5,12,14,16,18,20,24-decaene |
| Compound 7 | 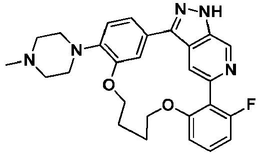 | 17-fluoro-5-(4-methylpiperazin-1-yl)-7,12-dioxa-20,23,24-triazapentacyclo[17.5.2.1^{2,6}.0^{13,18}.0^{22,25}]heptacos a-1(24),2(27),3,5,13,15,17,19,21,25-decaene |
| Compound 8 | 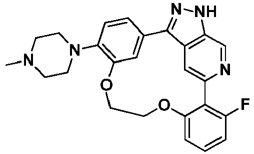 | 15-fluoro-5-(4-methylpiperazin-1-yl)-7,10-dioxa-18,21,22-triazapentacyclo[15.5.2.1^{2,6}.0^{11,16}.0^{20,23}]pentacos a-1(22),2(25),3,5,11,13,15,17,19,23-decaene |
| Compound 9 | 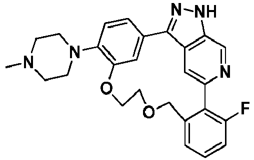 | 16-fluoro-5-(4-methylpiperazin-1-yl)-7,10-dioxa-19,22,23-triazapentacyclo[16.5.2.1^{2,6}.0^{12,17}.0^{21,24}]hexacosa -1(23),2(26),3,5,12,14,16,18,20,24-decaene |
| Compound 10 | 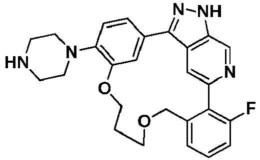 | 17-fluoro-5-(piperazin-1-yl)-7,11-dioxa-20,23,24-triazapentacyclo[17.5.2.1^{2,6}.0^{13,18}.0^{22,25}]heptacos a-1(24),2(27),3,5,13,15,17,19,21,25-decaene |
| Compound 11 | 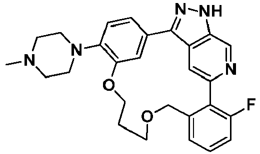 | 17-fluoro-5-(4-methylpiperazin-1-yl)-7,11-dioxa-20,23,24-triazapentacyclo[17.5.2.1^{2,6}.0^{13,18}.0^{22,25}]heptacos a-1(24),2(27),3,5,13,15,17,19,21,25-decaene |
| Compound 12 | 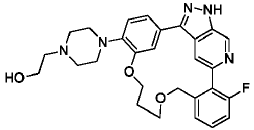 | 17-fluoro-5-(4-(2-hydroxyethyl)piperazin-1-yl)-7,11-dioxa-20,23,24-triazapentacyclo[17.5.2.1^{2,6}.0^{13,18}.0^{22,25}]heptacos a-1(24),2(27),3,5,13,15,17,19,21,25-decaene |
| Compound 13 | 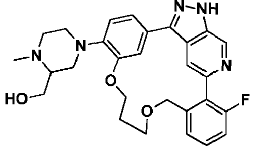 | 17-fluoro-5-(3-hydroxymethyl-4-methylpiperazin-1-yl)-7,11-dioxa-20,23,24-triazapentacyclo[17.5.2.1^{2,6}.0^{13,18}.0^{22,25}]heptacos a-1(24),2(27),3,5,13,15,17,19,21,25-decaene |
| Compound 14 | 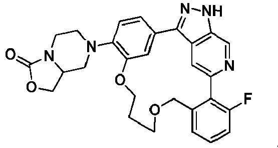 | 7-{17-fluoro-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-5-yl}-hexahydro-1*H*-[1,3]oxazolo[3,4-a]pyrazin-3-one |
| Compound 17 | 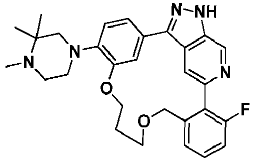 | 17-fluoro-5-(3,3,4-trimethylpiperazin-1-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 18 | 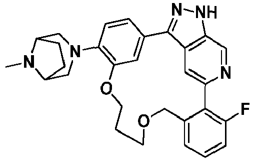 | 17-fluoro-5- f 8-methyl-3,8-diazabicyclo[3.2. 1]octan-3-yl 1-7,1 1-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 19 | 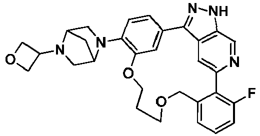 | 17-fluoro-5-[5-(oxetan-3-yl)-2,5-diazabicyclo[2.2.1]heptan-2-yl]-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 20 | 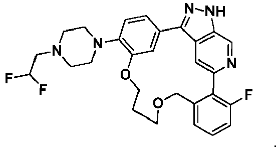 | 17-fluoro-5-(4-(2,2-difluoroethyl)-piperazin-1-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 21 | 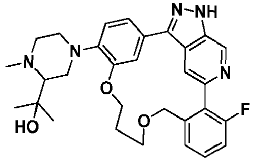 | 17-fluoro-5-(3-(2-hydroxy-2-methylethyl)-4-methylpiperazin-1-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 22 | 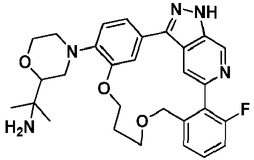 | 17-fluoro-5-(2-(2-amino-2-methylethyl)-morpholin-4-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 23 | 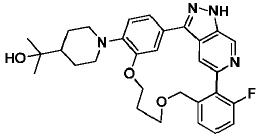 | 17-fluoro-5-(4-(2-hydroxy-2-methylethyl)-piperidinyl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 24 | 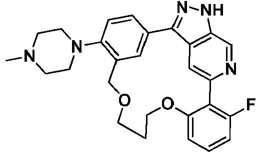 | 17-fluoro-5-(4-methylpiperazin-1-yl)-8,12-dioxa-20,23,24-triazapentacyclo[17.5.2.1^{2,6}.0^{13,18}.0^{22,25}]heptacos a-(24),2,4,6(27),13(18),14,16,19,21,25-decaene |
| Compound 25 | 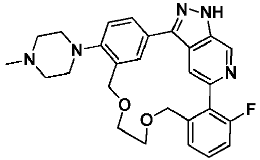 | 17-fluoro-5-(4-methylpiperazin-1-yl)-8,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 26 | 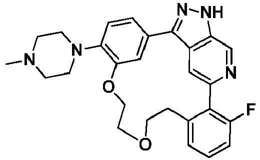 | 17-fluoro-5-(4-methylpiperazin-1-yl)-7,10-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 27 | 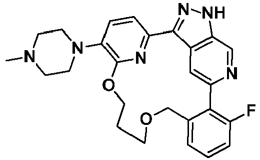 | 17-fluoro-5-(4-methylpiperazin-1-yl)-7,11-dioxa-20,23,24,27-tetraazapentacyclo[17.5.2.1^{2,6}.0^{13,18}.0^{22,25}]heptac osa- 1 (24),2,4,6(27),13 (18),14,16,19,21,2 5 - decaene |
| Compound 28 | 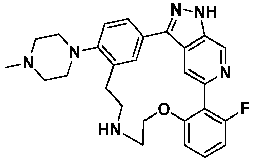 | 17-fluoro-5 -(4-methylpiperazin-1 -yl)-12-oxa-9,20,23,24-tetraazapentacyclo[17.5.2.1^{2,6}.0^{l3,18}.0^{22,25}]heptac osa-1(24),2,4,6(27), 13(18), 14, 16, 19,21,25-decaene |
| Compound 29 | 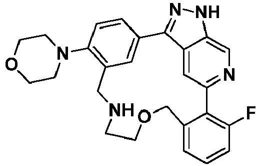 | 17-fluoro-5-(morpholin-4-yl)-11-oxa-8,20,23,24-tetraazapentacyclo[17.5.2.1^{2,6}.0^{13,18}.0^{22,25}]heptac osa-1(24),2,4,6(27), 13(18), 14, 16, 19,21,25-decaene |
| Compound 30 | 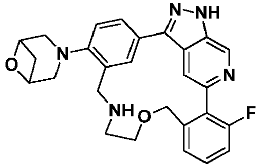 | 17-fluoro-5-{6-oxa-3-azabicyclo[3.1.1]heptan-3-yl}-11-oxa-8,20,23,24-tetraazapentacyclo[17.5.2.1^{2,6}.0^{13,18}.0^{22,25}]heptac osa-1(24),2,4,6(27), 13(18), 14, 16, 19,21,25-decaene |
| Compound 31 | 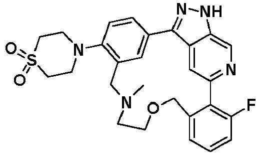 | 17-fluoro-8-methyl-5-(1,1-dioxothiomorpholin-4-yl)-11-oxa-8,20,23,24-tetraazapentacyclo[17.5.2.1^{2,6}.0^{13,18}.0^{22,25}]heptac osa-1 (24),2,4,6(27), 13(18), 14,16,19,21,25-decaene |
| Compound 32 | 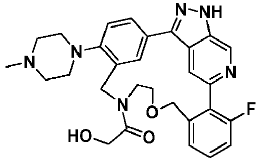 | 1-[17-fluoro-5-(4-methylpiperazin-1-yl)-11-oxa-8,20,23,24-tetraazapentacyclo[17.5.2.1^{2,6}.0^{13,18}.0^{22,25}]heptac osa-1 (24),2,4,6(27), 13(18), 14,16,19,21,25-decaen-8-yl]-2-hydroxyethan-1-one |
| Compound 33 | 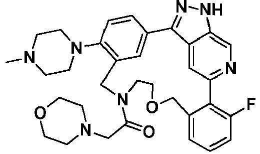 | 1-[17-fluoro-5-(4-methylpiperazin-1-yl)-11-oxa-8,20,23,24-tetraazapentacyclo[17.5.2.1^{2,6}.0^{13,18}.0^{22,25}]heptac osa-1 (24),2,4,6(27), 13(18), 14,16,19,21,25-decaen-8-yl]-2-(morpholin-4-yl)ethan-1-one |
| Compound 34 | 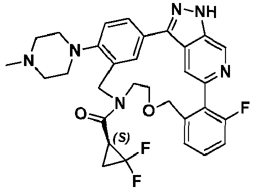 | 8-[(1S)-2,2-difluorocyclopropanecarbonyl]-17-fluoro-5-(4-methylpiperazin-1 -yl)-11 -oxa-8,20,23,24-tetraazapentacyclo[17.5.2.1^{2,6}.0^{13,18}.0^{22,25}]heptac osa-1 (24),2,4,6(27), 13(18), 14,16,19,21,25-decaene |
| Compound 35 | 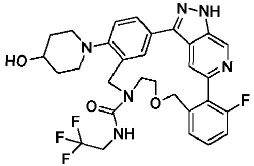 | 17-fluoro-5-(4-hydroxypiperidin-1-yl)-N (2,2,2-trifluoroethyl)-11-oxa-8,20,23,24-tetraazapentacyclo[17.5.2.1^{2,6}.0^{13,18}.0^{22,25}]heptac osa-1 (24),2,4,6(27), 13(18), 14,16,19,21,25-decaene-8-carboxamide |
| Compound 36 | 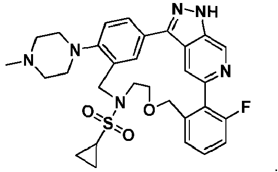 | 8-(cyclopropanesulfonyl)-17-fluoro-5-(4-methylpiperazin-1 -yl)-11 -oxa-8,20,23,24-tetraazapentacyclo[17.5.2.1^{2,6}.0^{13,18}.0^{22,25}]heptac osa-1 (24),2,4,6(27), 13(18), 14,16,19,21,25-decaene |
| Compound 37 | 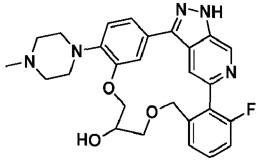 | 17-fluoro-9-hydroxy-5-(4-methylpiperazin-1-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 38 | 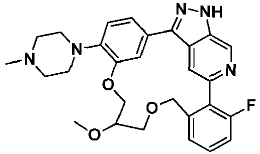 | 17-fluoro-9-methoxy-5-(4-methylpiperazin-1-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 39 | 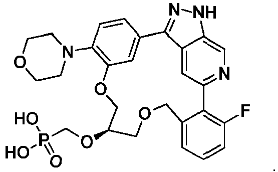 | ({[(9*R*)-17-fluoro-5-(morpholin-4-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-9-yl]oxy}methyl)phosphonic acid |
| Compound 40 | 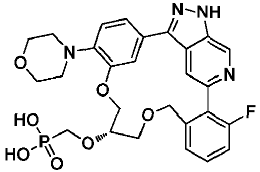 | ({[(9*S*)-17-fluoro-5-(morpholin-4-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-9-yl]oxy}methyl)phosphonic acid |
| Compound 41 | 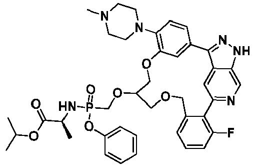 | Isopropyl (2*S*)-2-{[({[17-fluoro-5-(4-methylpiperazin-1-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-9-yl]oxy}methyl)(phenoxy)phosphoryl]amino}propanoate |
| Compound 42 | 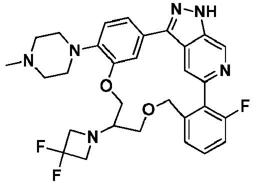 | 9-(3,3-difluoroazetidin-1-yl)-17-fluoro-5-(4-methylpiperazin-1-yl)-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 43 | 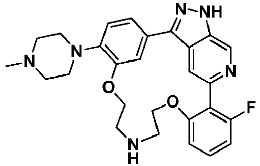 | 18-fluoro-5-(4-methylpiperazin-1-yl)-7,13-dioxa-10,21,24,25-tetraazapentacyclo[18.5.2.1^{2,6}.0^{14,19}.0^{23,26}]octaco sa-1(25),2,4,6(28), 14(19), 15, 17,20,22,26-decaene |
| Compound 44 | 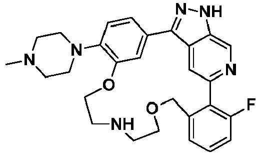 | 19-fluoro-5-(4-methylpiperazin-1-yl)-7,13-dioxa-10,22,25,26-tetraazapentacyclo[19.5.2.1^{2,6}.0^{15,20}.0^{24,27}]nonac osa-1 (26),2,4,6(29), 15(20), 16,18,21,23,27-decaene |
| Compound 45 | 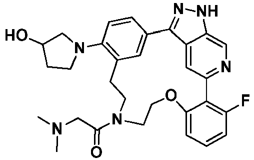 | 2-(dimethylamino)-1-[17-fluoro-5-(3-hydroxypyrrolidin-1-yl)-12-oxa-9,20,23,24-tetraazapentacyclo[17.5.2.1^{2,6}.0^{13,18}.0^{22,25}]heptac osa-1 (24),2,4,6(27), 13(18), 14,16,19,21,25-decaen-9-yl]ethan-1-one |
| Compound 46 | 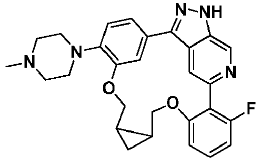 | 18-fluoro-5-(4-methylpiperazin-1-yl)-7,13-dioxa-21,24,25-triazahexacyclo[18.5.2.1^{2,6}.0^{9,11}.0^{14,19}.0^{23,26}]octa cosa-1(25),2,4,6(28), 14(19), 15, 17,20,22,26-decaene |
| Compound 47 | 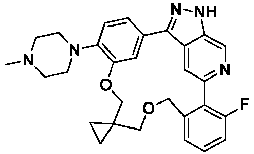 | 17'-fluoro-5'-(4-methylpiperazin-1-yl)-7',11'-dioxa-20',23',24'-triazaspiro[cyclopropane-1,9'-pentacyclo[17.5.2.1^{2,6}.0^{13,18}.0^{22,25}]heptacosane]-1'(24'),2',4',6'(27'),13'(18'),14',16',19',21',25'-decaene |
| Compound 48 | 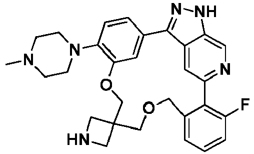 | 17'-fluoro-5'-(4-methylpiperazin-1-yl)-7',11'-dioxa-20',23',24'-triazaspiro[azetidine-3,9'-pentacyclo[17.5.2.1^{2,6}.0^{13,18}.0^{22,25}]heptacosane]-1'(24'),2',4',6'(27'),13'(18'),14',16',19',21',25'-decaene |
| Compound 49 | 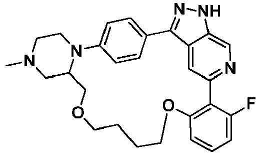 | 23-fluoro-9-methyl-13,18-dioxa-6,9,26,29,30-pentaazahexacyclo[23.5.2.2^{2,5}.0^{6,11}.0^{19,24}.0^{28,31}]te tratriaconta-1 (3 0),2,4,19(24),20,22,25,27,31,33-decaene |
| Compound 50 | 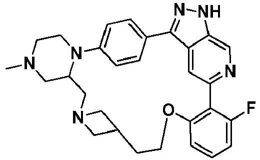 | 23-fluoro-9-methyl-18-oxa-6,9,13,26,29,30-hexaazaheptacyclo[23.5.2.2^{2,5}.1^{13,15}.0^{6,11}.0^{19,24}.0^{2 8,31}]pentatriaconta-1 (30),2,4,19(24),20,22,25,27,31,34-decaene |
| Compound 51 | 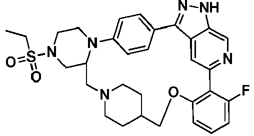 | 9-(ethanesulfonyl)-23-fluoro-18-oxa-6,9,13,26,29,30-hexaazaheptacyclo[23.5.2.2^{2,5}.2^{11,16}.0^{6,11}.0^{19,24}.0^{2 8,31}]hexatriaconta-1(30),2,4,19(24),20,22,25,27,31,35-decaene |
| Compound 52 | 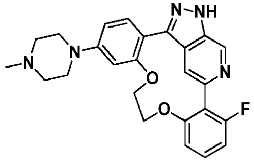 | 16-fluoro-5-(4-methylpiperazin-1-yl)-8,11-dioxa-19,22,23-triazapentacyclo[16.5.2.0^{2,7}.0^{12,17}.0^{21,24}]pentacos a-1(23),2,4,6,12(17),13,15,18,20,24-decaene |
| Compound 53 | 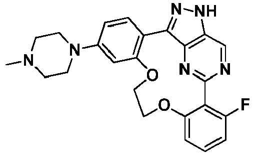 | 16-fluoro-5-(4-methylpiperazin-1-yl)-8,11-dioxa-19,22,23,25-tetraazapentacyclo[16.5.2.0^{2,7}.0¹2^{,17}.0^{21,24}]pentac osa-1(23),2,4,6, 12(17), 13, 15, 18,20,24-decaene |
| Compound 55 | 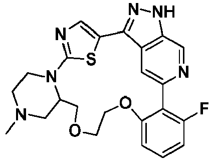 | 21-fluoro-9-methyl-13,16-dioxa-31-thia-4,6,9,24,27,28-hexaazahexacyclo[21.5.2.1^{2,5}.0^{6,11}.0^{17,22}.0^{26,29}]he ntriaconta-1(28),2,4, 17(22), 18,20,23,25,29-nonaene |
| Compound 56 | 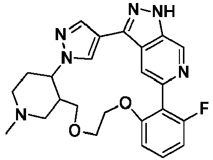 | 21-fluoro-9-methyl-13,16-dioxa-4,5,9,24,27,28-hexaazahexacyclo[21.5.2.1^{2,5}.0^{6,11}.0^{17,22}.0^{26,29}]he ntriaconta-1(28),2(31),3,17(22),18,20,23,25,29-nonaene |
| Compound 57 | 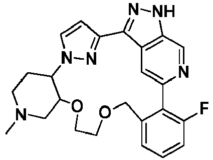 | 21-fluoro-9-methyl-12,15-dioxa-5,9,24,27,28,31-hexaazahexacyclo[21.5.2.1^{2,5}.0^{6,11}.0^{17,22}.0^{26,29}]he ntriaconta-1(28),2(31),3,17(22),18,20,23,25,29-nonaene |
| Compound 58 | 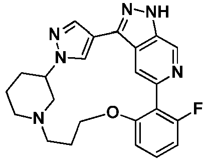 | 19-fluoro-14-oxa-4,5,10,22,25,26-hexaazahexacyclo[19.5.2.1^{2,5}.1^{6,10}.0^{15,20}.0^{24,27}]tri aconta-1(26),2(30),3,15(20),16,18,21,23,27-nonaene |
| Compound 59 | 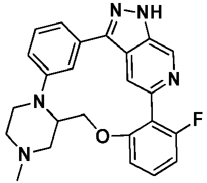 | 19-fluoro-10-methyl-14-oxa-7,10,22,25,26-pentaazahexacyclo[19.5.2.1^{2,6}.0^{7,12}.0^{15,20}.0^{24,27}]n onacosa-1(26),2,4,6(29), 15(20), 16,18,21,23,27-decaene |
| Compound 60 | 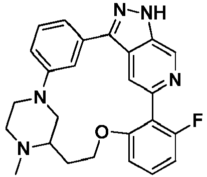 | 19-fluoro-10-methyl-14-oxa-7,10,22,25,26-pentaazahexacyclo[19.5.2.1^{2,6}.1^{7,11}.0^{15,20}.0^{24,27}]tr iaconta-1(26),2,4,6(30), 15(20), 16, 18,21,23,27-decaene |
| Compound 61 | 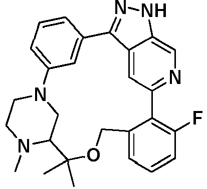 | 19-fluoro-10,12,12-trimethyl-13-oxa-7,10,22,25,26-pentaazahexacyclo[19.5.2.1^{2,6}.1^{7,11}.0^{15,20}.0^{24,27}]tr iaconta-1(26),2,4,6(30), 15(20), 16, 18,21,23,27-decaene |
| Compound 62 | 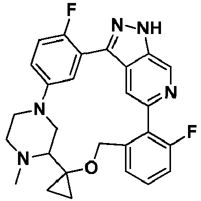 | 3',19'-difluoro-10'-methyl-13'-oxa-7',10',22',25',26'-pentaazaspiro[cyclopropane-1,12'-hexacyclo[19.5.2.1^{2,6}.1^{7,11}.0^{15,20}.0^{24,27}]triacontan e]-1'(26'),2',4',6'(30'),15'(20'),16',18',21',23',27'-decaene |
| Compound 63 | 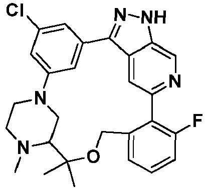 | 4-chloro-19-fluoro-10,12,12-trimethyl-13-oxa-7,10,22,25,26-pentaazahexacyclo[19.5.2.1^{2,6}.1^{7,11}.0^{15,20}.0^{24,27}]tr iaconta-1(26),2,4,6(30), 15(20), 16, 18,21,23,27-decaene |
| Compound 64 | 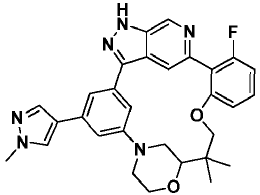 | 19-fluoro-12,12-dimethyl-4-(1-methyl-1H-pyrazol-4-yl)-10,14-dioxa-7,22,25,26-tetraazahexacyclo[19.5.2.1^{2,6}.1^{7,11}.0^{15,20}.0^{24,27}]tri aconta-1(26),2,4,6(30),15(20),16,18,21,23,27-decaene |
| Compound 65 | 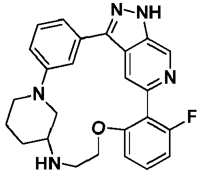 | 20-fluoro-15-oxa-7,12,23,26,27-pentaazahexacyclo[20.5.2.1^{2,6}.1^{7,11}.0^{16,21}.0^{25,28}]h entriaconta-1 (27),2,4,6(31), 16(21), 17,19,22,24,28-decaene |
| Compound 66 | 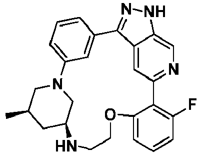 | (9*R*,11*S*)-20-fluoro-9-methyl-15-oxa-7,12,23,26,27-pentaazahexacyclo[20.5.2.1^{2,6}.1^{7,11}.0^{16,21}.0^{25,28}]h entriaconta-1 (27),2,4,6(31), 16(21), 17,19,22,24,28-decaene |
| Compound 67 | 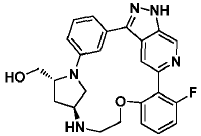 | [(8*R*,10*S*)-19-fluoro-14-oxa-7,11,22,25,26-pentaazahexacyclo[19.5.2.1^{2,6}.1^{7,10}.0^{15,20}.0^{24,27}]tr iaconta-1(26),2,4,6(30), 15(20), 16, 18,21,23,27-decaen-8-yl]methanol |
| Compound 68 | 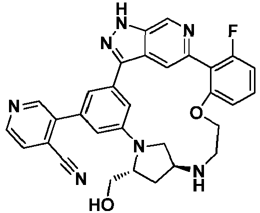 | 3-[(8*R*,10*S*)-19-fluoro-8-(hydroxymethyl)-14-oxa-7,11,22,25,26-pentaazahexacyclo[19.5.2.1^{2,6}.1^{7,10}.0^{15,20}.0^{24,27}]tr iaconta-1(26),2,4,6(30), 15(20), 16, 18,21,23,27-decaen-4-yl]pyridine-4-carbonitrile |
| Compound 69 | 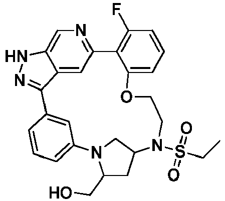 | [11-(ethanesulfonyl)-19-fluoro-14-oxa-7,11,22,25,26-pentaazahexacyclo[19.5.2.1^{2,6}.1^{7,10}.0^{15,20}.0^{24,27}]tr iaconta-1(26),2,4,6(30),15(20),16,18,21,23,27-decaen-8-yl]methanol |
| Compound 70 | 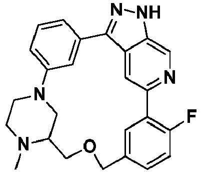 | 18-fluoro-10-methyl-13-oxa-7,10,21,24,25-pentaazahexacyclo[18.5.2.1^{2,6}.1^{7,11}.1^{15,19}.0^{23,26}]tr iaconta-1(25),2,4,6(30),15,17,19(28),20,22,26-decaene |
| Compound 71 | 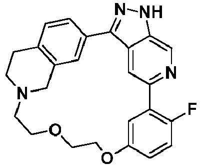 | 10-fluoro-14,17-dioxa-3,4,20,30-tetraazahexacyclo[18.5.3.2^{5,8}.1^{9,13}.0^{2,6}.0^{23,27}]hent riaconta-1(25),2,5(31),6,8(30),9(29),10,12,23,26-decaene |
| Compound 72 | 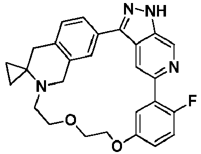 | 10'-fluoro-14',17'-dioxa-3',4',20',30'-tetraazaspiro[cyclopropane-1,21'-hexacyclo[18.5.3.2^{5,8}.1^{9,13}.0^{2,6}.0^{23,27}]hentriaconta ne]-1'(25'),2',5'(31'),6',8'(30'),9'(29'),10',12',23',26'-decaene |
| Compound 73 | 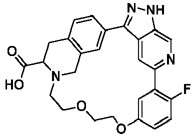 | 10-fluoro-14,17-dioxa-3,4,20,30-tetraazahexacyclo[18.5.3.2^{5,8}.1^{9,13}.0^{2,6}.0^{23,27}]hent riaconta-1(25),2,5(31),6,8(30),9(29),10,12,23,26-decaene-21-carboxylic acid |
| Compound 74 | 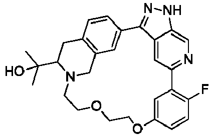 | 10-fluoro-14,17-dioxa-21-(2-hydroxyprop-2-yl)-3,4,20,30-tetraazahexacyclo[18.5.3.2^{5,8}.1^{9,13}.0^{2,6}.0^{23,27}]hent riaconta-1(25),2,5(31),6,8(30),9(29),10,12,23,26-decaene |
| Compound 75 | 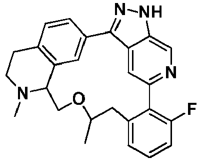 | 10-fluoro-16,20-dimethyl-17-oxa-3,4,20,28-tetraazahexacyclo[17.6.2.2^{5,8}.0^{2,6}.0^{9,14}.0^{23,27}]non acosa-1(25),2,5(29),6,8(28),9(14), 1 0,12,23,26-decaene |
| Compound 76 | 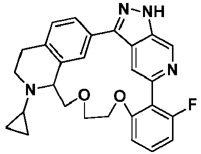 | 21-cyclopropyl-10-fluoro-15,18-dioxa-3,4,21,29-tetraazahexacyclo[18.6.2.2^{5,8}.0^{2,6}.0^{9,14}.0^{24,28}]triac onta-1(26),2,5(30),6,8(29),9(14),10,12,24,27-decaene |
| Compound 77 | 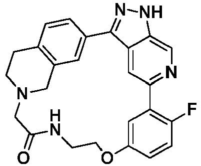 | 10-fluoro-14-oxa-3,4,17,20,30-pentaazahexacyclo[18.5.3.2^{5,8}.1^{9,13}.0^{2,6}.0^{23,27}]hen triaconta-1(25),2,5(31),6,8(30),9(29),10,12,23,26-decaen-18-one |
| Compound 78 | 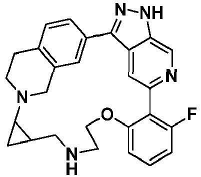 | 14-fluoro-9-oxa-1,6,20,21,33-pentaazaheptacyclo[21.5.3.2^{16,19}.0^{2,4}.0^{10,15}.0^{18,22}. 0^{26,30}]tritriaconta-10(15),11,13,16(33),17,19(32),21,23,25,30-decaene |
| Compound 79 | 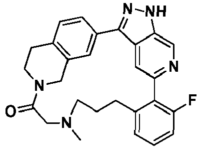 | 10-fluoro-18-methyl-3,4,18,21,30-pentaazahexacyclo[19.5.3.2^{5,8}.0^{2,6}.0^{9,14}.0^{24,28}]hen triaconta-1(26),2,5(31),6,8(30),9(14),10,12,24,27-decaen-20-one |
| Compound 80 | 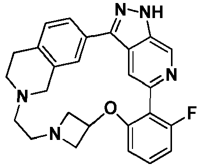 | 10-fluoro-15-oxa-3,4,18,21,31-pentaazaheptacyclo[19.5.3.2^{5,8}.1^{16,18}.0^{2,6}.0^{9,14}.0^{24 ,28}]dotriaconta-1(26),2,5(32),6,8(31),9(14),10,12,24,27-decaene |
| Compound 81 | 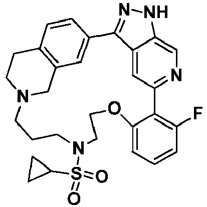 | 18-(cyclopropanesulfonyl)-10-fluoro-15-oxa-3,4,18,22,31-pentaazahexacyclo[20.5.3.2^{5,8}.0^{2,6}.0^{9,14}.0^{25,29}]dot riaconta-1(27),2,5(32),6,8(31),9(14),10,12,25,28-decaene |
| Compound 82 | 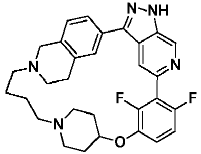 | 22,32-difluoro-18-oxa-9,14,25,28,29-pentaazaheptacyclo[22.5.2.2^{14,17}.1^{2,6}.1^{5,9}.1^{19,23}.0^{2 7,30}]hexatriaconta-1(29),2,4,6(36),19,21,23(32),24,26,30-decaene |
| Compound 83 | 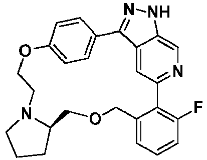 | (13*R*)-21-fluoro-6,15-dioxa-9,24,27,28-tetraazahexacyclo[21.5.2.2^{2,5}.0^{9,11}.0^{17,22}.0^{26,29}]do triaconta-1(28),2,4,17(22),18,20,23,25,29,31-decaene |
| Compound 84 | 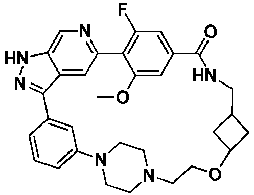 | 37-fluoro-3-methoxy-12-oxa-7,15,18,25,26,29-hexaazaheptacyclo[22.5.2.2^{2,5}.2^{15,18}.1^{9,1}.1^{19,23}.0^{2 7,31}]heptatriaconta-1(29),2,4,19(32),20,22,24,27,30,36-decaen-6-one |
| Compound 85 | 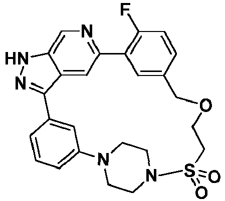 | 19-fluoro-14-oxa-11lambda6-thia-7,10,22,25,26-pentaazahexacyclo[19.5.2.2^{7,10}.1^{2,6}.1^{16,20}.0^{24,27}]d otriaconta-1(26),2,4,6(32),6,18,20(29),21,23,27-decaene-11,11-dione |
| Compound 86 | 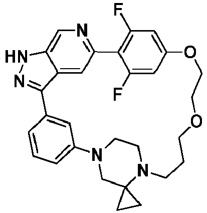 | 3',34'-difluoro-6',9'-dioxa-13',16',23',24',27'-pentaazaspiro[cyclopropane-1,14'-hexacyclo[20.5.2.2^{2,5}.2^{13,16}.1^{17,21}.0^{25,29}]tetratriac ontane]-1'(27'),2',4',17'(30'),18',20',22',25',28',33'-decaene |
| Compound 87 | 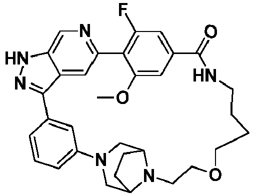 | 38-fluoro-3-methoxy-12-oxa-7,15,21,28,29,32-hexaazaheptacyclo[25.5.2.2^{2,5}.1^{16,21}.1^{22,26}.0^{15,19}.0 ^{30,34}]octatriaconta-1(32),2,4,22(35),23,25,27,30,33,37-decaen-6-one |
| Compound 88 | 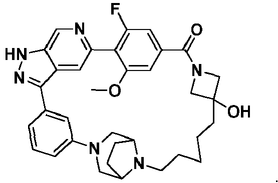 | 39-fluoro-9-hydroxy-3-methoxy-7,15,21,28,29,32-hexaazaoctacyclo[25.5.2.2^{2,5}.1^{7,9}.1^{16,21}.1^{22,26}.0^{15,1 9}.0^{30,34}]nonatriaconta-1(32),2,4,22(35),23,25,27,30,33,38-decaen-6-one |
| Compound 89 | 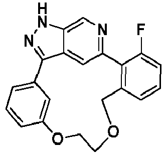 | 16-fluoro-7,10-dioxa-19,22,23-triazapentacyclo[16.5.2.1^{2,6}.0^{12,17}.0^{21,24}]hexacosa -1(23),2(26),3,5,12(17),13,15,18,20,24-decaene |
| Compound 90 | 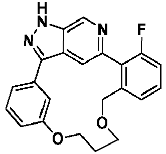 | 17-fluoro-7,11-dioxa-20,23,24-triazapentacyclo[17.5.2.1^{2,6}.0^{13,18}.0^{22,25}]heptacos a-1(24),2(27),3,5,13(18),14,16,19,21,25-decaene |
| Compound 91 | 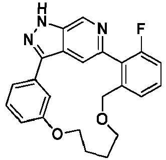 | 18-fluoro-7,12-dioxa-21,24,25-triazapentacyclo[18.5.2.1^{2,6}.0^{14,11}.0^{21,26}]octacosa -1(25),2(28),3,5,14(19),15,17,20,22,26-decaene |
| Compound 92 | 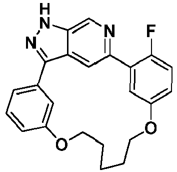 | 17-fluoro-7,13-dioxa-20,23,24-triazapentacyclo[17.5.2.1^{2,6}.1^{14,18}.0^{22,25}]octacosa -1(24),2(28),3,5,14,16,18(27),19,21,25-decaene |
| Compound 93 | 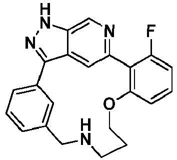 | 17-fluoro-12-oxa-8,20,23,24-tetraazapentacyclo[17.5.2.1^{2,6}.0^{l3,18}.0^{22,25}]heptac osa-1(24),2(27),3,5,13(18),14,16,19,21,25-decaene |
| Compound 94 | 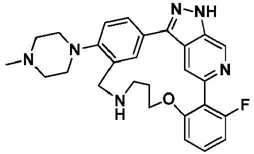 | 17-fluoro-5 -(4-methylpiperazin-1 -yl)-12-oxa-8,20,23,24-tetraazapentacyclo[17.5.2.1^{2,6}.0^{l3,18}.0^{22,25}]heptac osa-1(24),2(27),3,5,13(18),14,16,19,21,25-decaene |
| Compound 95 | 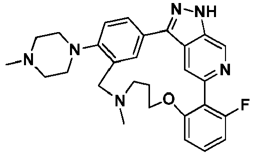 | 17-fluoro-8-methyl-5-(4-methylpiperazin-1-yl)-12-oxa-8,20,23,24-tetraazapentacyclo[17.5.2.1^{2,6}.0^{l3,18}.0^{22,25}]heptac osa-1(24),2(27),3,5,13(18),14,16,19,21,25-decaene |
| Compound 96 | 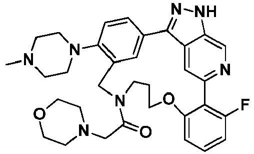 | 1-[17-fluoro-5-(4-methylpiperazin-1-yl)-12-oxa-8,20,23,24-tetraazapentacyclo[17.5.2.1^{2,6}.0^{13,18}.0^{22,25}]heptac osa-1(24),2(27),3,5,13(18),14,16,19,21,25-decaen-8-yl]-2-(morpholin-4-yl)ethan-1-one |
| Compound 97 | 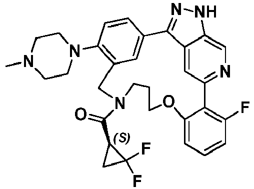 | 8-[(1*S*)-2,2-difluorocyclopropanecarbonyl]-17-fluoro-5-(4-methylpiperazin-1-yl)-12-oxa-8,20,23,24-tetraazapentacyclo[17.5.2.1^{2,6}.0^{13,18}.0^{22,25}]heptac osa-1(24),2(27),3,5,13(18),14,16,19,21,25-decaene |
| Compound 98 | 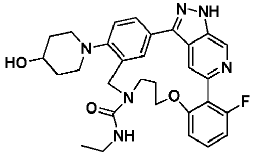 | *N*-ethyl-17-fluoro-5-(4-hydroxypiperidin-1 -yl)-12-oxa-8,20,23,24-tetraazapentacyclo[17.5.2.1^{2,6}.0^{13,18}.0^{22,25}]heptac osa-1(24),2(27),3,5,13(18),14,16,19,21,25-decaene-8-carboxamide |
| Compound 99 | 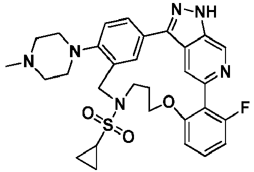 | 8-(cyclopropanesulfonyl)-17-fluoro-5-(4-methylpiperazin-1-yl)-12-oxa-8,20,23,24-tetraazapentacyclo[17.5.2.1^{2,6}.0^{l3,18}.0^{22,25}]heptac osa-1(24),2(27),3,5,13(18),14,16,19,21,25-decaene |
| Compound 100 | 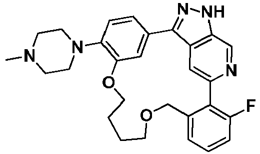 | 18-fluoro-5-(4-methylpiperazin-1-yl)-7,12-dioxa-21,24,25-triazapentacyclo[18.5.2.1^{2,6}.0^{14,19}.0^{21,26}]octacosa -1(25),2(28),3,5,14(19),15,17,20,22,26-decaene |
| Compound 101 | 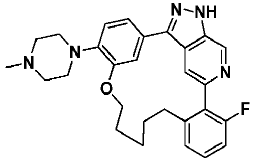 | 17-fluoro-5-(4-methylpiperazin-1-yl)-7-oxa-20,23,24-triazapentacyclo[17.5.2.1^{2,6}.0^{13,18}.0^{22,25}]heptacos a-1(24),2(27),3,5,13(18),14,16,19,21,25-decaene |
| Compound 102 | 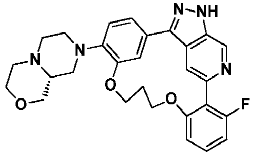 | 5-[(9aR)-octahydropyrazino[2,1-c][1,4]oxazin-8-yl]-16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 103 | 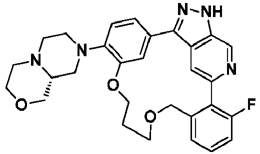 | 5-[(9aR)-octahydropyrazino[2,1-c][1,4]oxazin-8-yl]-17-fluoro-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 104 | 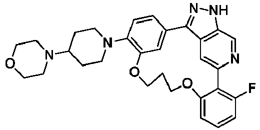 | 16-fluoro-5-[4-(morpholin-4-yl)piperidin-1-yl]-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 105 | 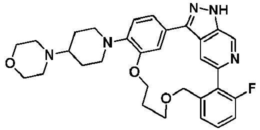 | 17-fluoro-5-[4-(morpholin-4-yl)piperidin-1-yl]-7,11-dioxa-20,23,24-triazapentacyclo[17.5.2.1^{2,6}.0^{11,18}.0^{22,25}]heptacos a-1(24),2,4,6(27),13(18),14,16,19,21,25-decaene |
| Compound 106 | 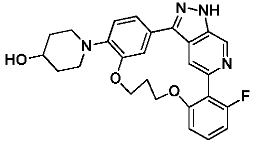 | 16-fluoro-5-(4-hydroxypiperidin-1-yl)-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 107 | 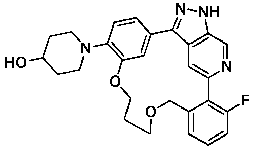 | 17-fluoro-5-(4-hydroxypiperidin-1-yl)-7,11-dioxa-20,23,24-triazapentacyclo[17.5.2.1^{2,6}.0^{11,18}.0^{22,25}]heptacos a-1(24),2,4,6(27),13(18),14,16,19,21,25-decaene |
| Compound 108 | 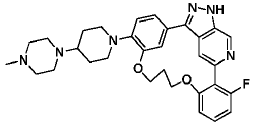 | 16-fluoro-5-[4-(4-methylpiperazin-1-yl)piperidin-1-yl]-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 109 | 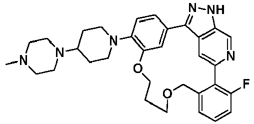 | 17-fluoro-5-[4-(4-methylpiperazin-1-yl)piperidin-1 -yl]-7,11 -dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 110 | 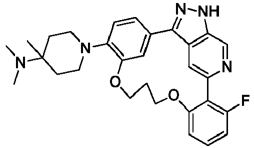 | 1-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-*N,N*,4-trimethylpiperidin-4-amine |
| Compound 111 | 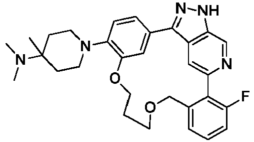 | 1-{17-fluoro-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-5-yl}-*N,N*,4-trimethylpiperidin-4-amine |
| Compound 112 | 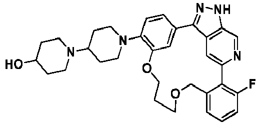 | 1'-{17-fluoro-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-5-yl}-[1,4'-bipiperidin]-4-ol |
| Compound 113 | 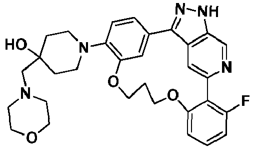 | 1-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl 1-4-[(morpholin-4-yl)methyl]piperidin-4-ol |
| Compound 114 | 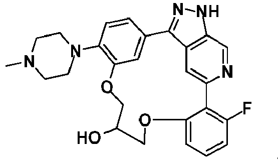 | 16-fluoro-5-(4-methylpiperazin-1-yl)-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-9-ol |
| Compound 115 | 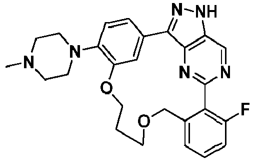 | 17-fluoro-5-(4-methylpiperazin-1 -yl)-7,11-dioxa-20,23,24,26-tetraazapentacyclo[17.5.2.1^{2,6}.0^{l3,18}.0^{22,25}]heptac osa-1(24),2,4,6(27),13(18),14,16,19,21,25-decaene |
| Compound 116 | 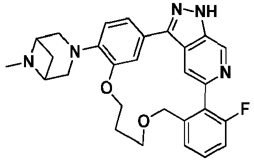 | 17-fluoro-5-{6-methyl-3,6-diazabicyclo[3.1.1]heptan-3-yl}-7,11-dioxa-20,23,24-triazapentacyclo[17.5.2.1^{2,6}.0^{11,18}.0^{22,25}]heptacos a-1(24),2,4,6(27),13(18),14,16,19,21,25-decaene |
| Compound 117 | 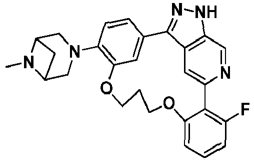 | 16-fluoro-5-{6-methyl-3,6-diazabicyclo[3.1.1]heptan-3-yl}-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 118 | 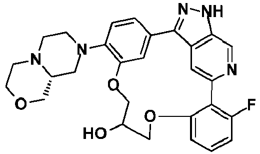 | 5-[(9aR)-octahydropyrazino[2,1-c][1,4]oxazin-8-yl]-16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-9-ol |
| Compound 119 | 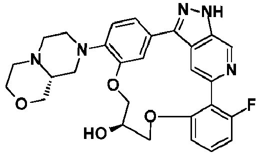 | (9*R*)-5-[(9a*R*)-octahydropyrazino[2,1-c][1,4]oxazin-8-yl]-16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-9-ol |
| Compound 120 | 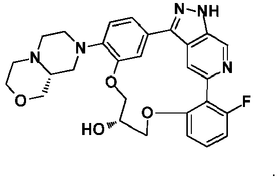 | (9*S*)-5-[(9a*R*)-octahydropyrazino[2,1-c][1,4]oxazin-8-yl]-16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-9-ol |
| Compound 121 | 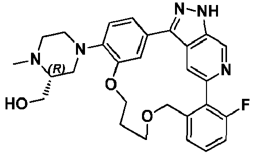 | [(2*R*)-4-{17-fluoro-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-5-yl}-1-methylpiperazin-2-yl]methanol |
| Compound 122 | 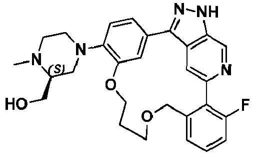 | [(2*S*)-4-{17-fluoro-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-5-yl} -1 -methylpiperazin-2-yl]methanol |
| Compound 123 | 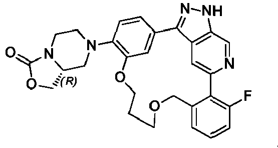 | (8a*R*)-7-{17-fluoro-7,11-dioxa-20,23,24-triazapentacyclo[17.5.2.1^{2,6}.0^{11,18}.0^{22,25}]heptacos a-1(24),2,4,6(27),13(18),14,16,19,21,25-decaen-5-yl}-hexahydro-1*H*-[1,3]oxazolo[3,4-a]pyrazin-3-one |
| Compound 124 | 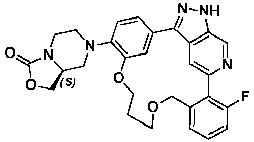 | (8a*S*)-7-{17-fluoro-7,11-dioxa-20,23,24-triazapentacyclo[17.5.2.1^{2,6}.0^{11,18}.0^{22,25}]heptacos a-1(24),2,4,6(27),13(18),14,16,19,21,25-decaen-5-yl}-hexahydro-1*H*-[1,3]oxazolo[3,4-a]pyrazin-3-one |
| Compound 125 | 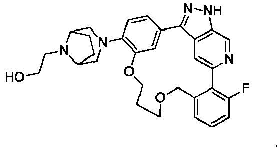 | 2-(3-{17-fluoro-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-5-yl}-3,8-diazabicyclo[3.2.1]octan-8-yl)ethan-1-ol |
| Compound 126 | 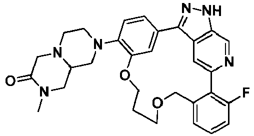 | 8-{17-fluoro-7,11-dioxa-20,23,24-triazapentacyclo[17.5.2.1^{2,6}.0^{13,18}.0^{22,25}]heptacos a-1(24),2,4,6(27),13(18),14,16,19,21,25-decaen-5-yl}-2-methyl-octahydro-1*H*-[1,4]diazino[1,2-a]pyrazin-3-one |
| Compound 127 | 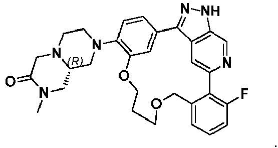 | (9a*R*)-8-{17-fluoro-7,11-dioxa-20,23,24-triazapentacyclo[17.5.2.1^{2,6}.0^{11,18}.0^{22,25}]heptacos a-1(24),2,4,6(27), 13(18), 14, 16, 19,21,25-decaen-5-yl}-2-methyl-octahydro-1*H*-[1,4]diazino[1,2-a]pyrazin-3-one |
| Compound 128 | 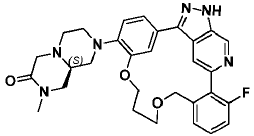 | (9a*S*)-8-{17-fluoro-7,11-dioxa-20,23,24-triazapentacyclo[17.5.2.1^{2,6}.0^{11,18}.0^{22,25}]heptacos a-1(24),2,4,6(27),13(18),14,16,19,21,25-decaen-5-yl}-2-methyl-octahydro-1*H*-[1,4]diazino[1,2-a]pyrazin-3-one |
| Compound 129 | 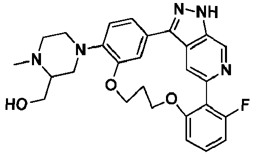 | [4-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-1-methylpiperazin-2-yl]methanol |
| Compound 130 | 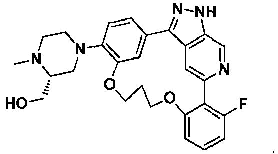 | [(2*R*)-4-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-1-methylpiperazin-2-yl]methanol |
| Compound 131 | 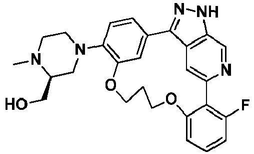 | [(2*S*)-4-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-1-methylpiperazin-2-yl]methanol |
| Compound 132 | 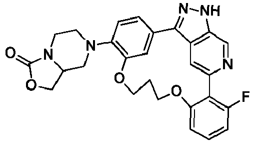 | 7-{16-fluoro-7,11 -di oxa-19,22,23 - triazapentacyclo[16.5.2.1^{2,6}.0^{12,17}.0^{21,24}]hexacosa -1(23),2,4,6(26),12(17),13,15,18,20,24-decaen-5-yl}-hexahydro-1*H*-[1,3]oxazolo[3,4-a]pyrazin-3-one |
| Compound 134 | 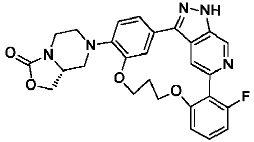 | (8a*R*)-7-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-hexahydro-1*H*-[1,3]oxazolo[3,4-a]pyrazin-3-one |
| Compound 135 | 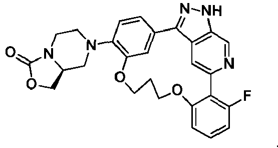 | (8a*S*)-7-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-hexahydro-1*H*-[1,3]oxazolo[3,4-a]pyrazin-3-one |
| Compound 136 | 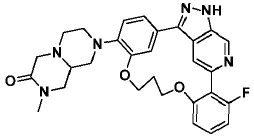 | 8-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-2-methyl-octahydro-1*H*-[1,4]diazino[1,2-a]pyrazin-3-one |
| Compound 137 | 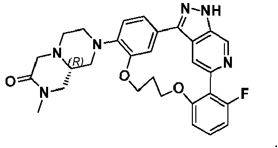 | (9a*R*)-8-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-2-methyl-octahydro-1*H*-[1,4]diazino[1,2-a]pyrazin-3-one |
| Compound 138 | 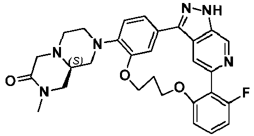 | (9a5)-8-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-2-methyl-octahydro-1*H*-[1,4]diazino[1,2-a]pyrazin-3-one |
| Compound 139 | 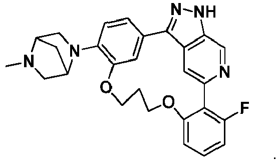 | 16-fluoro-5-[5-methyl-2,5-diazabicyclo[2.2.1]heptan-2-yl]-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 140 | 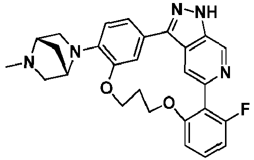 | 16-fluoro-5-[(1*S*,4*S*)-5-methyl-2,5-diazabicyclo[2.2.1]heptan-2-yl]-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 141 | 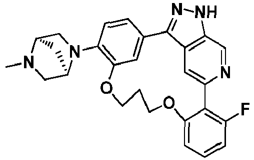 | 16-fluoro-5-[(1*R*,4*R*)-5-methyl-2,5-diazabicyclo[2.2.1]heptan-2-yl]-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 142 | 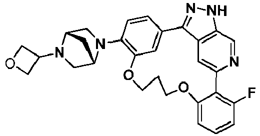 | 16-fluoro-5-[(1*S*,4*S*)-5-(oxetan-3-yl)-2,5-diazabicyclo[2.2.1]heptan-2-yl]-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 143 | 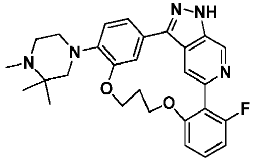 | 16-fluoro-5-(3,3,4-trimethylpiperazin-1-yl)-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 144 | 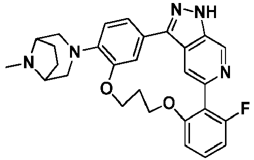 | 16-fluoro-5-{8-methyl-3,8-diazabicyclo[3.2.1]octan-3-yl}-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 145 | 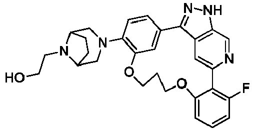 | 2-(3-{16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaen-5-yl}-3,8-diazabicyclo[3.2.1]octan-8-yl)ethan-1-ol |
| Compound 146 | 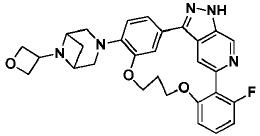 | 16-fluoro-5-[6-(oxetan-3-yl)-3,6-diazabicyclo[3.1.1]heptan-3-yl]-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 147 | 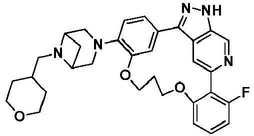 | 16-fluoro-5-{6-[(oxan-4-yl)methyl]-3,6-diazabicyclo[3.1.1]heptan-3-yl}-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 148 | 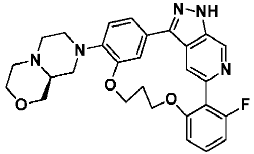 | 5-[(9a*S*)-octahydropyrazino[2,1-c][1,4]oxazin-8-yl]-16-fluoro-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 149 | 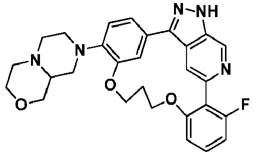 | 16-fluoro-5-{octahydropyrazino[2,1-c][1,4]oxazin-8-yl}-7,11-dioxa-19,22,23-triazapentacyclo[16.5.2.1^{2,6}.0^{12,17}.0^{21,24}]hexacosa -1(23),2,4,6(26),12(17),13,15,18,20,24-decaene |
| Compound 150 | 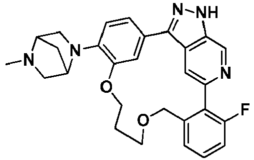 | 17-fluoro-5-[5-methyl-2,5-diazabicyclo[2.2.1]heptan-2-yl]-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 151 | 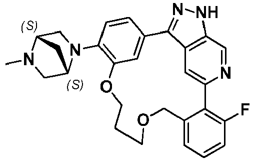 | 17-fluoro-5-[(1*S*,4*S*)-5-methyl-2,5-diazabicyclo[2.2.1]heptan-2-yl]-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 152 | 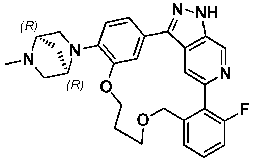 | 17-fluoro-5-[(1*R*,4*R*)-5-methyl-2,5-diazabicyclo[2.2.1]heptan-2-yl]-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 153 | 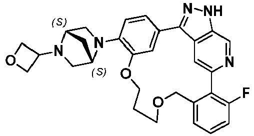 | 17-fluoro-5-[(1*S*,4*S*)-5-(oxetan-3-yl)-2,5-diazabicyclo[2.2.1]heptan-2-yl]-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |
| Compound 154 | 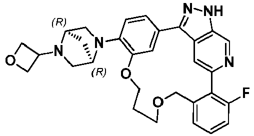 | 17-fluoro-5-[(1*R*,4*R*)-5-(oxetan-3-yl)-2,5-diazabicyclo[2.2.1]heptan-2-yl]-7,11-dioxa-20,23,24-triazapentacyclo[17.5.2.1^{2,6}.0^{13,18}.0^{22,25}]heptacos a-1(24),2,4,6(27),13(18),14,16,19,21,25-decaene |

29. A pharmaceutical composition comprising the compound according to any one of claims 1-28, or a pharmaceutically acceptable salt, hydrate, solvate, active metabolite, polymorph, isotope labeled compound, isomer or prodrug thereof, and a pharmaceutically acceptable carrier.

30. Use of the compound according to any one of claims 1-28, or a pharmaceutically acceptable salt, hydrate, solvate, active metabolite, polymorph, isotope labeled compound, isomer or prodrug thereof, or the pharmaceutical composition according to claim 29 in the preparation of a medicament for preventing, ameliorating or treating of a disease mediated by HPK1 or other protein kinases; wherein said diseases comprise tumors, myelodysplastic syndromes and diseases caused by viruses.

31. The use according to claim 30, wherein the tumor comprises at least one tumor selected from the group consisting of: chronic or acute leukemia, primary central nervous system lymphoma, lymphoma, multiple myeloma, lung cancer, hepatocellular carcinoma, cholangiocarcinoma, gallbladder cancer, gastric cancer, colorectal cancer, intestinal leiomyosarcoma, breast cancer, triple-negative breast cancer, ovarian cancer, cervical cancer, endometrial cancer, fallopian tube cancer, vaginal cancer, vulvar cancer, malignant teratoma, pancreatic cancer, pancreatic ductal adenocarcinoma, nasopharyngeal cancer, oral cancer, laryngeal cancer, esophageal squamous cell carcinoma, esophageal adenocarcinoma, thyroid cancer, kidney cancer, bladder cancer, malignant brain tumor, rhabdomyosarcoma, osteosarcoma, chondrosarcoma, osteofibrosarcoma, Ewing sarcoma, myxoma, malignant thymoma, malignant peripheral nerve sheath tumor, prostate cancer, testicular cancer, penile cancer, urethral cancer, and skin malignant tumors (comprising squamous cell carcinoma, basal cell carcinoma, malignant melanoma, and the like).

32. The use according to claim 30, wherein the virus comprises at least one virus selected from the group consisting of: hepatitis virus, human immunodeficiency virus, human papillomavirus, herpes simplex virus, measles virus, norovirus, Boca virus, Coxsackie virus, Ebola virus, enterovirus, lymphocytic meningitis virus, influenza virus, SARS virus and COVID-19 virus.

33. A method for preventing, ameliorating or treating diseases mediated by HPK1 or other protein kinases, comprising administering to a patient in need a therapeutically effective amount of a compound according to any one of claims 1 to 28, or a pharmaceutically acceptable hydrate, solvate, active metabolite, polymorph, isotope labeled compound, isomer or prodrug thereof, or the pharmaceutical composition according to claim 29, wherein said diseases comprise tumors, myelodysplastic syndrome symptoms and diseases caused by viruses.
